# EUROPEAN PATENT SPECIFICATION

(11) **EP 1 960 535 B1**
(45) Date of publication and mention of the grant of the patent: **08.05.2024**
(21) Application number: 06838389.2
(22) Date of filing: 24.11.2006
(51) Int. Cl.: G01N 33/50, C12Q 1/50

(54) **COMPOUNDS AND METHODS OF IDENTIFYING, SYNTHESIZING, OPTIMIZING AND PROFILING PROTEIN MODULATORS**
VERBINDUNGEN UND VERFAHREN ZUR IDENTIFIZIERUNG, SYNTHESE, OPTIMIERUNG UND PROFILERSTELLUNG VON PROTEINMODULATOREN
COMPOSES ET PROCEDES D IDENTIFICATION, DE SYNTHESE, D OPTIMISATION ET DE CREATION DE PROFILS DE MODULATEURS DE PROTEINES

(30) Priority: 23.11.2005 US 739477 P; 23.11.2005 US 739476 P; 02.12.2005 US 741767 P; 16.12.2005 US 751030 P; 13.03.2006 US 783106 P; 23.03.2006 US 785904 P; 23.03.2006 US 785817 P; 04.04.2006 US 789379 P; 29.08.2006 WO PCT/US2006/033890
(43) Date of publication of application: 27.08.2008
(73) Proprietor: Housey, Gerard M., Southfield, MI 48034 (US)
(72) Inventor: HOUSEY, Gerard, M., Southfield, MI 48034 (US)
(74) Representative: Witthoff Jaekel Steinecke Patentanwälte PartG mbB
(86) International application number: PCT/US2006/045394
(87) International publication number: WO 2007/062213

(56) References cited:
- WO-A1-2005/115992
- WO-A2-2007/037898
- HURON ET AL.: 'A Novel Pyridopyrimidine Inhibitor of Abl Kinase is a Picomolar Inhibitor of Bcr-abl-driven K562 Cells and Is Effective against STI571-resistant Bcr-abl Mutants' CLINICAL CANCER RESEARCH vol. 9, April 2003, pages 1267 - 1273, XP003023718
- VON BUBNOFF ET AL.: 'Inhibition of Wild-Type and Mutant Bcr-Abl by Pyrido-Pyrimidine Type Small Molecule Kinase Inhibitors' CANCER RESEARCH vol. 63, 01 October 2003, pages 6395 - 6404, XP003023719
- WOLFF NICHOLAS C ET AL: "PD166326, a novel tyrosine kinase inhibitor, has greater antileukemic activity than imatinib mesylate in a murine model of chronic myeloid leukemia", BLOOD, vol. 105, no. 10, May 2005 (2005-05), pages 3995-4003, ISSN: 0006-4971(print)
- T. O'hare ET AL: "Inhibition of wild-type and mutant Bcr-Abl by AP23464, a potent ATP-based oncogenic protein kinase inhibitor: implications for CML", BLOOD, vol. 104, no. 8, 15 October 2004 (2004-10-15), pages 2532-2539, XP55452820, US ISSN: 0006-4971, DOI: 10.1182/blood-2004-05-1851
- L C Crossman ET AL: "A single nucleotide polymorphism in the coding region of ABL and its effects on sensitivity to imatinib", LEUKEMIA., vol. 19, no. 11, 8 September 2005 (2005-09-08), pages 1859-1862, XP055452823, US ISSN: 0887-6924, DOI: 10.1038/sj.leu.2403935
- CARTER TODD A ET AL: "Inhibition of drug-resistant mutants of ABL, KIT, and EGF receptor kinases", PROCEEDINGS OF THE NATIONAL ACADEMY OF SCIENCES, NATIONAL ACADEMY OF SCIENCES, US, vol. 102, no. 31, 2 August 2005 (2005-08-02), pages 11011-11016, XP002440841, ISSN: 0027-8424, DOI: 10.1073/PNAS.0504952102
- Todd A. Carter: "Inhibition of drug-resistant mutants of ABL, KIT, and EGF receptor kinases | PNAS", PNAS (Article + Suppl.), 2 August 2005 (2005-08-02), XP055658460, Retrieved from the Internet: URL:https://www.pnas.org/content/102/31/11 011/tab-figures-data#F5 [retrieved on 2020-01-15]
- Todd A. Carter: "Inhibition of drug-resistant mutants of ABL, KIT, and EGF receptor kinases", PNAS (Supplementary Information), 2 August 2005 (2005-08-02), XP055658459,
- PEDEMONTE NICOLETTA ET AL: "SMALL-MOLECULE CORRECTORS OF DEFECTIVE DELTAF508-CFTR CELLULAR PROCESSING IDENTIFIED BY HIGH-THROUGHPUT SCREENING", JOURNAL OF CLINICAL INVESTIGATION, B M J GROUP, GB, vol. 115, no. 9, 1 September 2005 (2005-09-01), pages 2564-2571, XP009083656, ISSN: 0021-9738, DOI: 10.1172/JCI24898
- Paul La Rosee ET AL: "Activity of the Bcr-Abl kinase inhibitor PD180970 against clinically relevant Bcr-Abl isoforms that cause resistance to imatinib mesylate (Gleevec, STI571).", Cancer Research, vol. 62, no. 24, 1 December 2002 (2002-12-01), pages 7149-7153, XP055049991, US ISSN: 0008-5472

## Description

This application claims priority of PCT International Application PCT/US06/33890, filed August 29, 2006, U.S. Ser. No. 60/739,477, filed November 23, 2005, U.S. Ser. No. 60/739,476, filed November 23, 2005, U.S. Ser. No. 60/741,767, filed December 2, 2005, U.S. Ser. No. 60/751,030, filed December 16, 2005, U.S. Ser. No. 60/783,106, filed March 13, 2006, U.S. Ser. No. 60/785,904, filed March 23, 2006, U.S. Ser. No. 60/785,817, filed March 23, 2006, and U.S. Ser. No. 60/789,379, filed April 4, 2006.

### FIELD OF THE INVENTION

This invention relates to *in vitro* cell-based screening methods of identifying, synthesizing, optimizing and profiling compounds that are inhibitors or activators of proteins, both naturally occurring endogenous proteins as well as certain variant forms of endogenous proteins, and in particular to the embodiments as characterized in the claims. The method accelerates the identification and development of compounds as potential therapeutically effective drugs by simplifying the pharmaceutical discovery and creation process through improvements in hit identification, lead optimization, biological profiling, and rapid elimination of toxic compounds. Implementation results in overall cost reductions in the drug discovery process resulting from the corresponding increases in efficiency.

### BACKGROUND OF THE INVENTION

Important components of modern new drug discovery/creation methods that are directed towards a selected protein target present in a human cell include:
1. identification of "hit" compounds which inhibit or activate the selected target protein. (A hit is defined for these purposes as a compound that scores positively in a given assay and may posess some of the effects and pharmacological properties that the investigator desires. In modern pharmaceutical research, however, hits are virtually never final clinical candidates without substantial further modification);
2. selection of a lead compound upon which to base further studies and refinements of the initial hit compound;
3. optimization of a lead compound (whose chemical structure is either related to or identical to the original hit compound) by making a series of chemical modifications designed primarily to improve the inhibitory or activating properties of the lead compound with respect to the target protein, but which may also improve bioavailability, plasma half-life, or reduce toxicity;
4. profiling the spectrum of biological activity of a given lead compound (including an optimized lead) in order to determine its relative specificity and selectivity for the chosen target protein as compared to other non-target proteins, some of which may be closely related to the target protein itself (such as other members of a protein family);
5. preclinical *in-vitro* and *in-vivo* animal studies designed to evaluate dosing ranges, carcinogenicity, absorption, distribution, metabolism, excretion, pharmakokinetics, oral bioavailability (if desired), pharmacodynamics, toxicity, and related parameters;
6. clinical trials in healthy volunteers and in patients afflicted with the disease for which the potential therapeutic treatment is thought to be beneficial.

This invention is characterized in the claims and directed toward a novel approach which substantially improves steps 1-4 as given above. The method can also be used to create and optimize compounds that are substantially more effective and less toxic than typical experimental drugs that have been identified, optimized or profiled using standard, less sophisticated approaches that are currently in use.

The methodology described herein has been developed as part of an intensive effort to develop advanced new pharmaceutical technologies that convert the "drug discovery" process into one more accurately described as a "drug creation" process by inventing predictable, reliable methodologies that provide the skilled investigator with the necessary tools to create new drugs that target specific proteins of importance in human disease while reducing the time and immense costs associated with the drug discovery/development process.

The progressive development of drug resistance in a patient is the hallmark of chronic treatment with many classes of drugs, especially in the therapeutic areas of cancer and infectious diseases. Molecular mechanisms have been identified which mediate certain types of drug resistance phenomena, whereas in other cases the mechanisms of acquired as well as *de novo* resistance remain unknown today.

One mechanism of induced (acquired) drug resistance originally thought to be relevant in the area of cancer therapy involves increased expression of a protein known as P-glycoprotein (P-gp). P-gp is located in the cell membrane and functions as a drug efflux pump. The protein is capable of pumping toxic chemical agents, including many classical anti-cancer drugs, out of the cell. Consequently, upregulation of P-glycoprotein usually results in resistance to multiple drugs. Upregulation of P-glycoprotein in tumor cells may represent a defense mechanism which has evolved in mammalian cells to prevent damage from toxic chemical agents. Other related drug resistance proteins have now been identified with similar functions to P-gp, including multidrug-resistance-associated protein family members such as MRP1 and ABCG2. In any event, with the advent of the development of compounds that are specific for a given target protein, and less toxic, the importance of P-glycoprotein and related ATP-binding cassette (ABC) transporter proteins in clinically significant drug resistance has lessened.

Another possible molecular mechanism of acquired drug resistance is that alternative signal pathways are responsible for continued survival and metabolism of cells, even though the original drug is still effective against its target. Furthermore, alterations in intracellular metabolism of the drug can lead to loss of therapeutic efficacy as well. In addition, changes in gene expression as well as gene amplification events can occur, resulting in increased or decreased expression of a given target protein and frequently requiring increasing dosages of the drug to maintain the same effects. (Adcock and Lane, 2003)

Mutation induced drug resistance is a frequently occurring event in the infectious disease area. For example, several drugs have been developed that inhibit either the viral reverse transcriptase or the viral protease encoded in the human immunodeficiency (HIV) viral genome. It is well established in the literature that repeated treatment of HIV-infected AIDS patients using, for example, a reverse transcriptase inhibitor eventually gives rise to mutant forms of the virus that have reduced sensitivity to the drug. Mutations that have arisen in the gene encoding reverse transcriptase render the mutant form of the enzyme less affected by the drug.

The appearance of drug resistance during the course of HIV treatment is not surprising considering the rate at which errors are introduced into the HIV genome. The HIV reverse transcriptase enzyme is known to be particularly error prone, with a forward mutation rate of about 3.4 × 10⁻⁵ mutations per base pair per replication cycle (Mansky et al., J. Virol. 69:5087-94 (1995)). However, analogous mutation rates for endogenous genes encoded in mammalian cells are more than an order of magnitude lower.

New evidence shows that drug resistance can also arise from a mutational event involving the gene encoding the drug target (Gorre et al., Science, 2001; WO 02102976 A2). In this case, exposure of the patient to a specific therapeutic substance such as a given cancer drug that targets a specific ***protein-of-interest*** (POI, or "target" protein) may be followed by the outgrowth of a group of cells harboring a mutation occurring in the gene encoding the protein that is the target of the therapeutic substance. Whether the outgrowth of this population of cells results from a small percentage of pre-existing cells in the patient which already harbor a mutation which gives rise to a drug-resistant POI, or whether such mutations arise *de novo* during or following exposure of the animal or human being to a therapeutic agent capable of activating or inhibiting said POI, is presently unknown. In either case, such mutation events may result in a mutated protein (defined below as a ***theramutein*)** which is less affected, or perhaps completely unaffected, by said therapeutic substance.

Chronic myelogenous leukemia (CML) is characterized by excess proliferation of myeloid progenitors that retain the capacity for differentiation during the stable or chronic phase of the disease. Multiple lines of evidence have established deregulation of the Abl tyrosine kinase as the causative oncogene in certain forms of CML. The deregulation is commonly associated with a chromosomal translocation known as the Philadelphia chromosome (Ph), which results in expression of a fusion protein comprised of the *BCR* gene product fused to the Abelson tyrosine kinase, thus forming p210^{Bcr-Abl} which has tyrosine kinase activity. A related fusion protein, termed p190^{Bcr-Abl}, that arises from a different breakpoint in the *BCR* gene, has been shown to occur in patients with Philadelphia chromosome positive (Ph+) Acute Lymphoblastic Leukemia (ALL) (Melo, 1994; Ravandi et al., 1999). Transformation appears to result from activation of multiple signal pathways including those involving RAS, MYC, and JUN. Imatinib mesylate ("STI-571" or "Gleevec^{®}") is a 2-phenylamino pyrimidine that targets the ATP binding site of the kinase domain of Abl (Druker et al, NEJM 2001, p. 1038). Subsequently it has also been found by other methods to be an inhibitor of platelet-derived growth factor (PDGF) β receptor, and the Kit tyrosine kinase, the latter of which is involved in the development of gastrointestinal stromal tumors (see below).

Until recently, it had not been observed that during the course of treatment with a specific inhibitor of a given endogenous cellular protein that a mutation in its corresponding endogenous gene could lead to the expression of protein variants whose cellular functioning was resistant to the inhibitor. Work by Charles Sawyers and colleagues (Gorre et al., Science 293:876-80 (2001); WO 02/102976 A2) demonstrated for the first time that treatment of a patient with a drug capable of inhibiting the p210^{Bcr-Abl} tyrosine kinase (i.e., STI-571) could be followed by the emergence of a clinically significant population of cells within said patient harboring a mutation in the gene encoding the p210^{Bcr-Abl} cancer causing target protein which contains the Abelson tyrosine kinase domain. Various such mutations gave rise to mutant forms of p210^{Bcr-Abl} which were less responsive to Gleevec treatment than was the original cancer causing version. Notably, the mutations that emerged conferred upon the mutant protein a relative resistance to the effects of the protein kinase inhibitor drug, while maintaining a certain degree of the original substrate specificity of the mutant protein kinase. Crossman et al. (2005) reported on a single nucleotide polymorphism in the coding region of Abl and its effects on sensitivity to imatinib. Prior to the work of Gorre et al., it was generally believed by those skilled in the art that the types of resistance that would be observed in patients exposed to a compound which inhibited the Abelson protein kinase, such as STI-571, would have resulted from one or more of the other mechanisms of drug resistance listed above, or by some other as yet unknown mechanism, but that in any event said resistance would involve a target (protein or otherwise) which was distinct from the drug's target POI.

Accordingly, the ability to treat clinically relevant resistant mutant forms of proteins that are otherwise the targets of an existing therapy would be extremely useful. Such mutated proteins (theramuteins as defined below) are beginning to be recognized and understood to be important targets in recurring cancers, and will become important in other diseases as well. There exists a need for therapeutic agents that are active against such drug resistant variant forms of cellular proteins that may arise before, during or following normally effective drug therapies. WO 03/031608 A2 and WO 2004/063195 A1 teach methods including contacting a test compound with at least one kinase and determining whether the kinase activity of the protein is decreased. Comparing the inhibitory activity among several different kinases or forms of kinases would allow one to identify a compound which is selective. Furthermore, compounds may be identified based on co-crystallization studies and computer modeling. A key purpose of this invention is to provide a generalizable methodology that the skilled investigator may utilize to identify hits from high throughput screening (HTS) systems, create and optimize lead compounds, and profile the spectrum of biological activity of such compounds, all without reliance upon older methods such as cell free radioligand binding assays and the like. An additional key purpose of this invention is to provide compounds that may serve as potential therapeutic agents useful in overcoming mutation-induced drug resistance in endogenously occurring proteins.

### BRIEF SUMMARY OF THE INVENTION

The present invention relates to the embodiments characterized in the claims. The method described herein involves the generation of an *in vitro* cellular response-based drug discovery and creation system that utilizes modulations of a defined, pre-determined characteristic of a cell termed a *phenoresponse* as a tool to measure the ability of a given compound (chemical agent, modulator) to activate or inhibit a selected target protein. Through the iterative application of this process, the methodology described herein may be utilized to identify protein modulators (as herein defined), perform lead optimization on such modulators, and biologically profile the target protein specificity and selectivity of such modulators.

The invention described herein may be utilized with any target protein and any eukaryotic cell type, provided however that an essential element of the invention which is termed the ***phenoresponse*** is first identified and utilized according to the teachings herein. One embodiment of the method provides the skillled investigator with the ability to identify inhibitors or activators of a selected target protein. Another embodiment allows the skilled investigator to do rapid lead optimization studies in order to arrive at a potential clinical candidate compound. Still another embodiment provides the skilled investigator with the ability to design compounds possessing a desired degree of specificity for a given target protein as well as selectivity for that protein relative to distinct yet closely related family members of the target protein that may exist with certain targets.

Improvement of the therapeutic efficacy of a compound, including an already approved medication, is an important recurring problem in pharmaceutical research. A commonly utilized approach is to start with known chemical structure and make additional chemical modifications to the structure for the purpose of improving its potency, specificity (for the target protein), or other parameter relevant to its therapeutic efficacy in the patient. In some cases the starting structure may be a known drug. In other instances it may simply be an initial screening hit identified either using a cell-free or primary cell-based screening assay. In still other instances, the compound may be an initial chemical structure defined in its minimal terms based upon a screening hit or other model structure, and frequently termed a "scaffold". For the purposes of this invention, a scaffold is defined as a chemical structure with one or more side chains or ring substituents that have been removed relative to a representative compound that otherwise shares the same scaffold. By way of example, the third compound in Table 4 may be thought of as a scaffold.

An important contribution of the present invention is the use of the phenoresponse, taken together with determination of the cellular specificity of a first compound relative to a second compound in order to determine whether the first compound exhibits an improved cellular specificity relative to the second compound. This approach, reported for the first time in the invention described herein, represents a fundamental advance over the prior art. The prior art relies upon cell-free assay systems utilizing purified or recombinantly produced proteins for assaying the activity of a compound, and compares the effect of a given compound on a target protein with its effects on other proteins generally related (closely or distantly) to the target protein. Numerous examples of this type of prior art approach are found in the literature, including Hanke et. al., 1996, Warmuth et. al., US 2003/0162222 A1, Knight and Shokat, 2005, and references therein. Such older types of cell-free approaches are markedly less effective or completely ineffective as compared to the present invention in identifying and optimizing the cellular specificity and therapeutic efficacy of a given scaffold. The substantial improvement of the present invention results from at least three key elements.

First, the concept of the phenoresponse, when utilized together with the measurement of the cellular specificity of a given compound (as measured by determination of its CSG as defined in the claims), provides a system which allows the identification of compounds that may interact with the target protein in an improved, more functionally effective manner.

Second, the present invention provides a method of identifying compounds that are *also* capable of interacting with other cellular components distinct from the target protein (which include but are not limited to upstream or downstream components of a signal transduction pathway involving the target protein such as monomeric or multi-subunit proteins, protein complexes, protein/nucleic acid complexes, and the like), that are functional in the specific signal tranduction pathways or peripheral to the signal transduction pathways in which the target protein functions within the cell, to promote the disease state of interest such as a selected form of human cancer. Due to the complexity of the signal transduction cascades present in the cells of higher ordered organisms such as humans, the current state of the art is incapable of complete knowledge regarding all of the mechanism in which a given target protein functions within the cell.

Third, the present invention eliminates compounds that cross react with other non-target proteins that do NOT participate in the signal transduction pathways that underlie the disease state in which the target protein functions. This ability of the present invention to eliminate such compounds (which will have untoward side effects in the patient) arises from the direct comparative measurement of the cellular specificity of the compound using the phenoresponse, which inherently eliminates effects upon the control cell. If the effect of a given test compound results in a reduced cellular specificity as compared to the reference compound, the compound can be eliminated immediately. Whether the test compound is less effective against the target protein, or cross-reacts with other non-target proteins that do not participate in the signal tranduction pathways of the target protein that modulate the phenoresponse linked to the target protein, or is simply cytotoxic, is irrelevant and only of academic interest. The essential point is that the test compound will be a less effective therapeutic and can be eliminated from further consideration. This saves the skilled investigator time and effort in evaluating variant chemical structures. It is important for the reader to recognize that compounds that may be very potent and highly effective against the target in cell-free assay systems may nevertheless show relatively low CSG determinations and may therefore be rapidly eliminated, saving time and precious resources.

The aforementioned key advantages of the present invention are nowhere to be found in the prior art, and provide the essential improvements of the present invention over the prior art. These advantages are applicable to all potential therapeutic target proteins, but are especially important in the case of the intractable, highly drug resistant target proteins known as theramuteins (WO 2005/115992).

As a result of the use of this invention, the problem of improving and optimizing a given compound relative to other less effective compounds is greatly simplified and enhanced. The skilled investigator simply begins with a first compound, whether it be an approved drug, a screening hit, or a basic scaffold which is known to inhibit or activate the protein of interest, and uses this first compound as a starting point for reference purposes. Additional compounds that are analogs, homologs, isomers, and the like, of the first compound (also referred to herein as the "starting compound" or "reference compound") are then synthesized using basic methods of medicinal chemistry synthesis which are now standard in the art. Some of these chemical synthesis methods have already been referred to in other sections herein, and the reader may also refer to Burbaum et al., 1995 and Goodnow et al., 2003 as general references for such procedures. Once the additional compounds are synthesized, the skilled investigator then proceeds to use the methods of the invention rather than the prior art method of constantly referring to the results obtained with cell-free assays by testing the new compounds on both the target protein and an array of other non-target proteins in an attempt to minimize the cross-reactivity of the compound with other proteins. Instead, through the use of this invention, the skilled investigator may guide the improvement of the chemical structure of the starting compound through direct reference to the results obtained from determinations of the CSG of each compound to be tested using the phenoresponse-based cellular assay system of the present invention. Most importantly, continuous reliance upon the results of cell-free, purified protein assays, including "kinase panels" as referenced above in Hanke et al. (1996) and Knight and Shokat (2005) is eliminated in its entirety, and yet the compounds that result from the implementation of the present method are superior to those obtained by the older methods, as shown by the activities of the compounds identified herein that are effective against the highly drug-resistant theramutein p210 Bcr-Abl T3151, as shown in Table 4. Nothing limits the skilled investigator to independently test any resulting compounds in a cell-free system for independent verification if so desired, but this is in no way required in order to practice the invention.

Prior to this invention, it has not been demonstrated that a cellular response-based drug discovery system is capable of identifying and rank ordering inhibitors or activators of a selected target protein without prior reference to a cell-free, purified protein ligand binding assay or enzyme assay (when the target protein is an enzyme) in order to establish that the compounds under investigation are actually binding to the target protein.

These results demonstrate, for the first time, the use of a cellular response-based assay system as a primary tool to identify inhibitors or activators of a given target protein from compounds that score positively in a high-throughput screen (HTS). These results also demonstrate that once a hit or lead compound capable of activating or inhibiting a given target protein is identified (by any method, including the embodiments disclosed herein or via classical cell-free HTS methods), said compound may also be *chemically optimized* (i.e. lead optimization may be performed on said compound) entirely using the phenoresponse-based cellular assay system without subsequent dependence upon a cell-free purified protein assay system to independently verify/confirm that the inhibitory or activating ability of each subsequent compound synthesized during the lead optimization process. This embodiment alone saves the skilled investigator a substantial amount of time, effort and significant laboratory resources that would normally be spent on generating and independently confirming inhibitory or activating properties using classical cell-free purified protein assays, radioligand binding assays, and the like.

The method is demonstrated herein using a specific mutated form of a cancer-causing protein involved in the development and progression of chronic myelogenous leukemia (CML). This protein, termed the Abelson protein kinase, in its cancer causing form is a known target for certain tyrosine kinase inhibitors such as imatinib mesylate. However, as discussed in detail below, this target protein can arise in a patient in a mutated form that becomes resistant to the inhibitory effects of imatinib. Such forms of the Abelson kinase are termed theramuteins. In an embodiment of the invention, suitable lead compounds capable of inhibiting or activating a given theramutein are identified. In another embodiment of this invention, a lead compound is optimized. The method is effective for the identification of hits, for lead optimization of such hits (regardless of how such hits were initially identified), and for biological profiling of compounds directed towards non-theramutein endogenous target proteins. The general utility of the method is demonstrated using a theramutein consisting of a mutated form of the Abelson kinase harboring a T315I mutation that confers a high degree of drug resistance.

This invention further relates to agents that are inhibitors or activators of variant forms of proteins. The invention also relates to agents that are inhibitors or activators of certain variant forms of endogenous proteins. Of particular interest are inhibitors and activators of endogenous protein variants, encoded by genes which have mutated, which variants often arise or are at least first identified as having arisen following exposure to a chemical agent which is known to be an inhibitor or activator of the corresponding unmutated endogenous protein. Such protein variants (mutant proteins) are herein termed "theramuteins," and may occur either spontaneously in an organism (and be pre-existing mutations in some cases), or said mutants may arise as a result of selective pressure which results when the organism is treated with a given chemical agent capable of inhibiting the non-mutated form of said theramutein (herein termed a "prototheramutein"). It will be understood that in some cases a prototheramutein may be a "wild type" form of a POI (*e.g.,* a protein that gives rise to a disease due to disregulation). In other cases, the prototheramutein will be a disease causing variant of a "wild type" protein, having already mutated and thereby contributing to the development of the diseased state as a result of said prior mutation. One example of the latter type of prototheramutein is the P210^{BCR-ABL} oncoprotein, and a mutant form of this protein harboring a threonine (T) to isoleucine (I) mutation at position 315 is termed P210^{BCR-ABL-T315I} and is one example of a theramutein. As used herein, the designation "P210 ^{BCR-ABL} " is synonymous with the term "p210^{Bcr-Abl} ", the "wild-type Bcr-Abl protein", and the like.

Theramuteins are a rare class of endogenous proteins that harbor mutations that render said proteins resistant to drugs that are known to inhibit or activate in a therapeutically effective manner their non-mutated counterparts. The endogenous genes encoding a few such proteins are presently known to exhibit such mutations under certain circumstances. In one embodiment, this invention is directed toward compositions that inhibit certain drug-resistant mutants (theramuteins) of the Abelson tyrosine kinase protein, originally termed P210-Bcr-Abl in the literature that is involved in the development of chronic myelogenous leukemia.

The present method is particularly directed toward the identification of *specific* inhibitors or *specific* activators of proteins. Use of the term "specific" in the context of the terms "inhibitor" or "activator" (see definitions below) means that said inhibitor or activator binds to the protein and inhibits or activates the cellular functioning of the protein without also binding to and activating or inhibiting a wide variety of other proteins or non-protein targets in the cell. The skilled investigator is well aware that there is a certain degree of variability in the medical literature with respect to the concept of a *specific inhibitor* or a *specific activator,* and of the related concept of target protein "specificity" when discussing the actions of inhibitors or activators of a protein. Accordingly, for the purposes of this invention, a substance is a specific inhibitor or a specific activator of a given protein if said substance is capable of inhibiting or activating said protein at a given concentration such that a corresponding phenoresponse is modulated in the appropriate manner, without having an appreciable effect at the same given concentration upon the phenoresponse (if any) of a corresponding control cell that essentially does not express either the protein.

In certain embodiments, a substance may be a modulator of two closely relted proteins such as a prototheramutein and one of its corresponding theramuteins. In other embodiments, in addition to being a modulator of the prototheramutein and theramutein, a substance may also modulate the activities of proteins that have similar functions. As discussed above, in addition to inhibiting the p210^{Bcr Abl} tyrosine kinase, imatinib mesylate is also capable of inhibiting the c-kit oncogene product (also a tyrosine kinase) which is overexpressed in certain gastrointestinal stromal tumors, as well as the PDGF β receptor (also a tyrosine kinase), which is expressed in certain chronic myelomonocytic leukemias (CMML). Such a compound is sometimes termed a "moderately specific" inhibitor.

The method of the present invention can be used to identify substances that will activate or inhibit a theramutein, to the same extent, and preferably to an even greater extent than a known drug substance is capable of inhibiting the corresponding "wild type" form of that protein. (The skilled artisan is well aware, however, that said "wild type" forms of such proteins may have already mutated in the course of giving rise to the corresponding disease in which said protein participates.)

The present application discloses inhibitors of the P210^{BCR-ABL-T315I} theramutein having the formula I 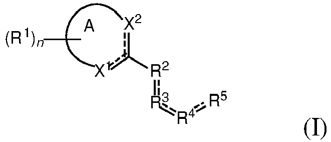 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹,-(CH₂)*ₚ*N(R¹¹)(CH₂)*_{q}*C(O)R¹¹, -(CH₂)*ₚ*N(R¹²)(R¹³), -(CH₂)*ₚ*N(R¹¹)(CH₂)*_{q}*R¹¹, -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
R² is selected from -CR²*ₐ*-, -NR²²*_{b}*-, and -(C=R²³)-;
   each R²¹ is independently selected from H, halo, -NH₂, -N(H)(C₁₋₃ alkyl), -N(C₁₋₃ alkyl)₂,
   -O-(C₁₋₃ alkyl), OH and C₁₋₃ alkyl;
   each R²² is independently selected from H and C₁₋₃ alkyl;
   R²³ is selected from O, S, N-R°, and N-OR⁰;
R³ is selected from -CR³¹*_{c}*- , -NR³²*_{d}*-, -SO₂-, and -(C=R³³)-;
   each R³¹ group is selected from H, halo, -NH₂, -N(H)(R⁰), -N(R⁰)₂, -O-R⁰, OH and C₁₋₃ alkyl;
   each R³² group is selected from H, alkyl, cycloalkyl, alkenyl, alkynyl, aralkyl, CO₂R⁰, C(O)R⁰, aryl, and a heterocyclic ring;
   R³³ is selected from O, S, N-R³⁴, and N-OR⁰;
   R³⁴ is selected from H, NO₂, CN, alkyl, cycloalkyl, alkenyl, alkynyl, aralkyl, aryl and a heterocyclic ring;
R⁴ is selected from -CR⁴¹*ₑ*-, -NR⁴²*_{f}*-, -(C=R⁴³)-, -SO₂-, and -O-;
   each R⁴¹ is selected from H, alkyl, cycloalkyl, alkenyl, alkynyl, CO₂R⁰, C(O)R⁰, aralkyl, aryl, and a heterocyclic ring;
   each R⁴² group is selected from H, alkyl, cycloalkyl, alkenyl, alkynyl, aralkyl, CO₂R⁰, C(O)R⁰, aryl, and a heterocyclic ring;
   each R⁴³ is selected from O, S, N-R°, and N-OR⁰;
with the provisos that when R² is -NR²²*_{b}*- and R⁴ is -NR⁴²*_{f}*-, then R³ is not -NR³²*_{d}*-; that both R³ and R⁴ are not simultaneously selected from -(C=R³³)- and -(C=R⁴³)-, respectively; and that R³ and R⁴ are not simultaneously selected from -SO₂-;
R⁵ is selected from -Y-R⁶ and -Z-R⁷;
   Y is selected from a chemical bond, O, NR⁰,
   R⁶ is selected from alkyl, cycloalkyl, alkenyl, alkynyl, aralkyl, aryl, and a heterocyclic ring;
   Z is a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
   R⁷ is H or is selected from aryl and a heterocyclic ring;
each R⁰ is independently selected from H, alkyl, cycloalkyl, aralkyl, aryl and a heterocyclic ring;
*a* is 1 or 2;
*b* is 0 or 1;
c is 1 or 2;
*d* is 0 or 1;
*e* is 1 or 2; and
*f* is 0 or 1.

The invention provides for a fundamentally new way of treating cancer and other diseases where treatment with an existing drug compound, by whatever mechanism, is followed by identifiable (clinically significant) theramutein-mediated drug resistance, by providing alternative drugs that can be administered as theramuteins arise and are identified as such (Wakai et al., 2004, reports an example wherein a theramutein may arise during the course of an on-going treatment regimen), or preemptively before the outgrowth of clinically significant populations of theramutein expressing cells. Further, where a drug treatment for a particular disease is less effective in a subset of individuals that express a certain theramutein of a protein that the drug targets, the invention enables the tailoring of treatments for those subjects by providing alternative drug substances that will be effective against said theramutein.

The application discloses a method of determining whether a chemical agent is at least as effective a modulator of a theramutein in a cell as a known substance is a modulator of a corresponding prototheramutein. One embodiment of the method involves contacting a control cell that expresses the prototheramutein and is capable of exhibiting a responsive phenotypic characteristic (linked to the functioning of the prototheramutein in the cell) with the known modulator of the prototheramutein, contacting a test cell that expresses the theramutein and is also capable of exhibiting the responsive phenotypic characteristic (linked to the functioning of the theramutein in the cell) with the chemical agent, and comparing the response of the treated test cell with the response of the treated control cell; to determine that the chemical agent is at least as effective a modulator of the theramutein as the known substance is a modulator of the prototheramutein. In certain other embodiments, one type of control cell may not express the prototheramutein at all. In other embodiments, the control cell may express about the same amount of the prototheramutein as the test cell expresses of the theramutein. In still other embodiments, the control cell may be capable of exhibiting the responsive phenotypic characteristic to about the same extent as the test cell under certain conditions. In the embodiments of the present inventon, the test cell expresses a given protein, whereas the control cell expresses little or essentially none of the protein.

Proteins of the invention that are of particular interest are those involved in regulatory function, such as enzymes, protein kinases, tyrosine kinases, receptor tyrosine kinases, serine threonine protein kinases, dual specificity protein kinases, proteases, matrix metalloproteinases, phosphatases, cell cycle control proteins, docking proteins such as the IRS family members, cell-surface receptors, G-proteins, ion channels, DNA- and RNA-binding proteins, polymerases, and the like. No limitation is intended on the type of theramutein or other protein that may be used in the invention. At the present time, three theramuteins are known: BCR-ABL, c-Kit, and EGFR.

Any responsive phenotypic characteristic that can be linked to the presence of the protein (including, *e.g.,* a theramutein or prototheramutein) in the cell can be employed for use in the method, including, for example, growth or culture properties, the phosphorylation state (or other modification) of a substrate of the theramutein, and any type of transient characteristic of the cell, as will be defined and discussed in detail.

### DESCRIPTION OF THE FIGURES

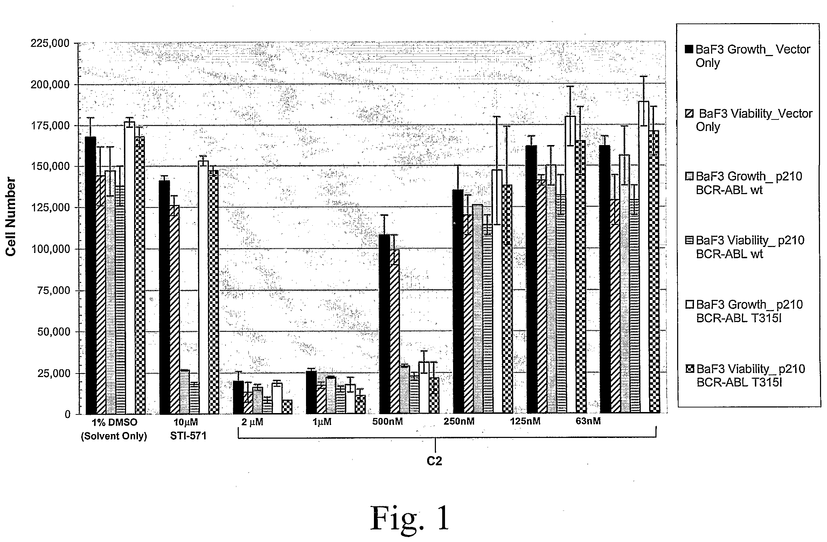

Figure 1 shows the effect on growth and viability of different concentrations of Compound 2 (C2) for non-transformed vector control BalF3 cells (which are IL-3 dependent) as well as Ba/F3 cells expressing the "wild type" p210^{Bcr-Abl} (designated p210^{Bcr-Abl-wt}), and BalF3 cells expressing the p210^{Bcr-Abl-T315I} drug resistant mutant. Cell counts and viability were determined on an automated cell counter as discussed in detail in the specification. Cell counts are shown by the solid color bars; cell viability is shown by the hashed bars. Note that STI-571 potently inhibits growth of the P210 cell line (grey bar) whereas it is unable to inhibit the growth of the T315I cell line (white bar) even at 10 µM concentration. 500 nM C2 shows the largest specificity gap within this dose-response series. Compare STI-571 at 10 µM to C2 at 500 nM on the T3151 cell line (white bars). Abbreviations: DMSO: dimethylsulfoxide (solvent used for drug dissolution).
Figure 2 shows the effect on growth and viability of different concentrations of Compound 6 (C6) for non-transformed vector control BalF3 cells as well as Ba/F3 cells expressing the p210^{Bcr-Abl-T315I} drug resistant mutant. All other details are as per Fig. 1.

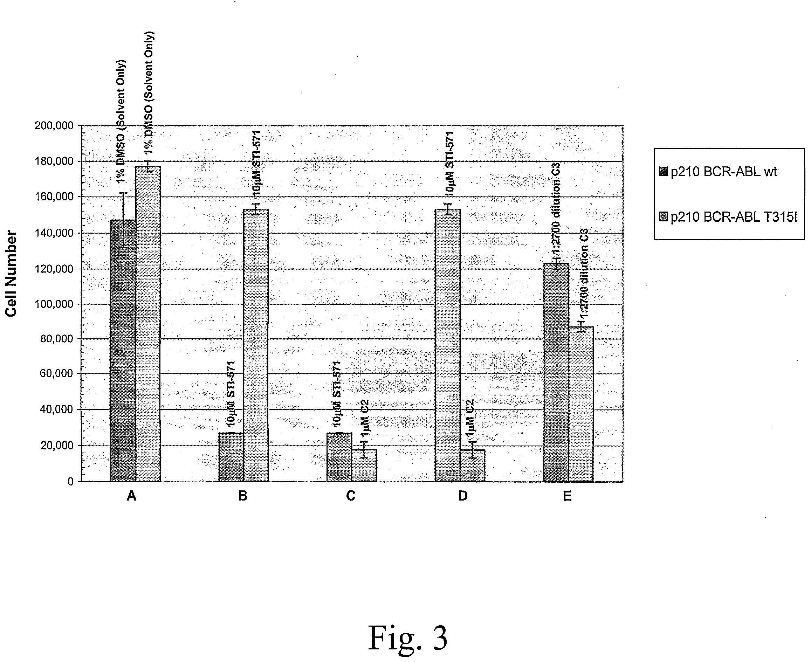

Figure 3 shows various determinations of the specificity gap by comparing the effects of various compounds identified in the screen in terms of their effects on the prototheramutein- and theramutein-expressing cell lines. Compound 3 (C3) shows the best example of the ability of the method to identify a compound that exerts an even greater effect on the theramutein than on its corresponding prototheramutein. (Panel E). Panel A: control DMSO treatments; B: negative heterologous specificity gap; C: slightly positive heterologous specificity gap; D: large positive homologous specificity gap; E: positive heterologous specificity gap. See text for explanations.

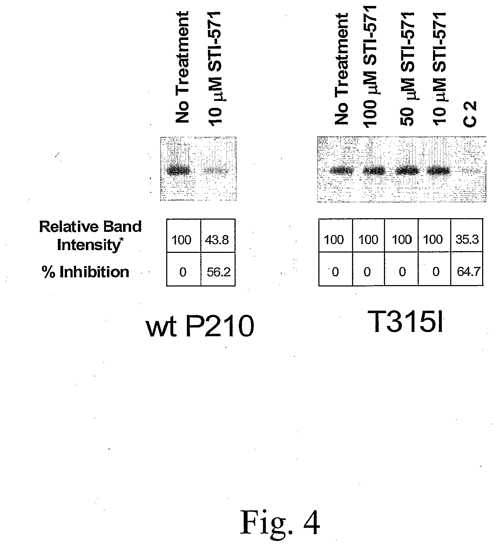

Figure 4 shows an autoradiograph of recombinant P210 Bcr-Abl wild type and T315I mutant kinase domains assayed for autophosphorylation activity. 200 ng of protein were preincubated with test substances for 10 minutes under standard autophosphoryation reaction conditions and then radiolabelled ATP was added and the reactions proceeded for 30 minutes at 30^{O}C, after which the samples were separated by SDS-PAGE. The gels were silver-stained, dried down under vacuum and exposed to X-ray film. Note that whereas 10 µM STI 571 is effective against wild type P210 Bcr-Abl, it is virtually ineffective against the T315I kinase domain, even at concentrations up to 100 µM. "P210 cell line" refers to cells expressing p210 ^{BCR-ABL-wt}. "T315I cell line" refers to cells expressing p210 ^{BCR-ABL-}T3151

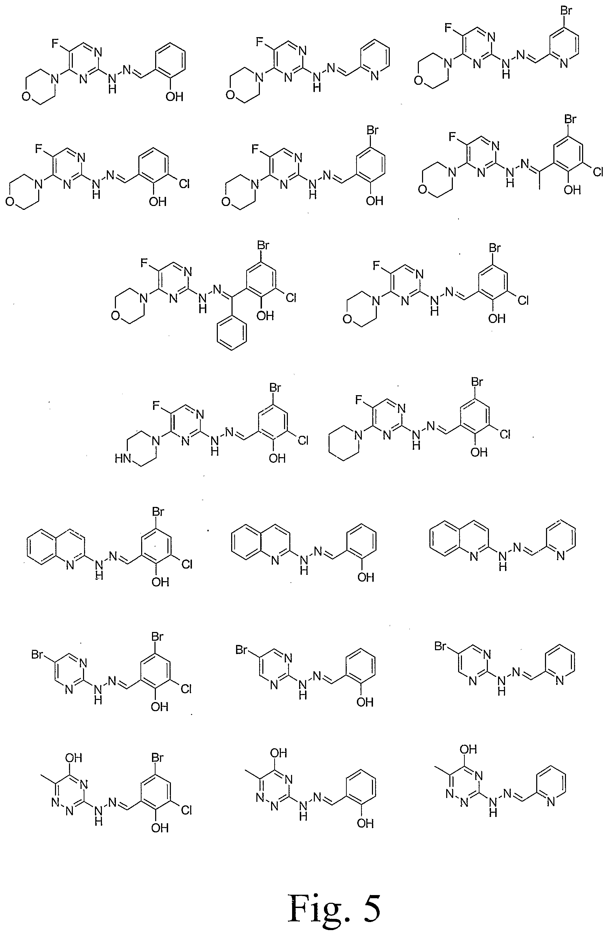

Figure 5 shows the chemical structures of representative compounds of the present application.

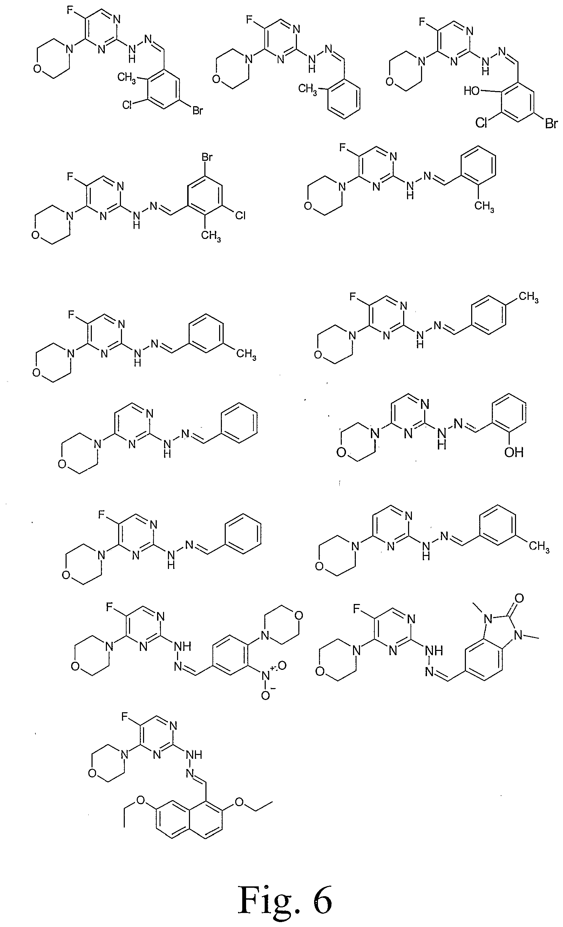

Figure 6 shows the chemical structures of representative compounds of the present applicattion.

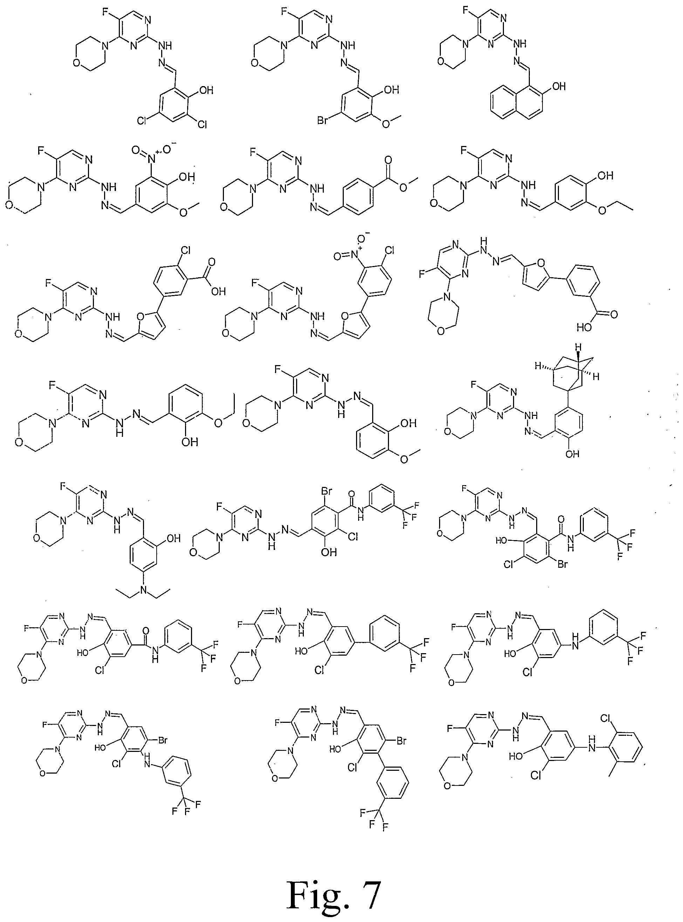

Figure 7 shows the chemical structures of representative compounds of the present application.

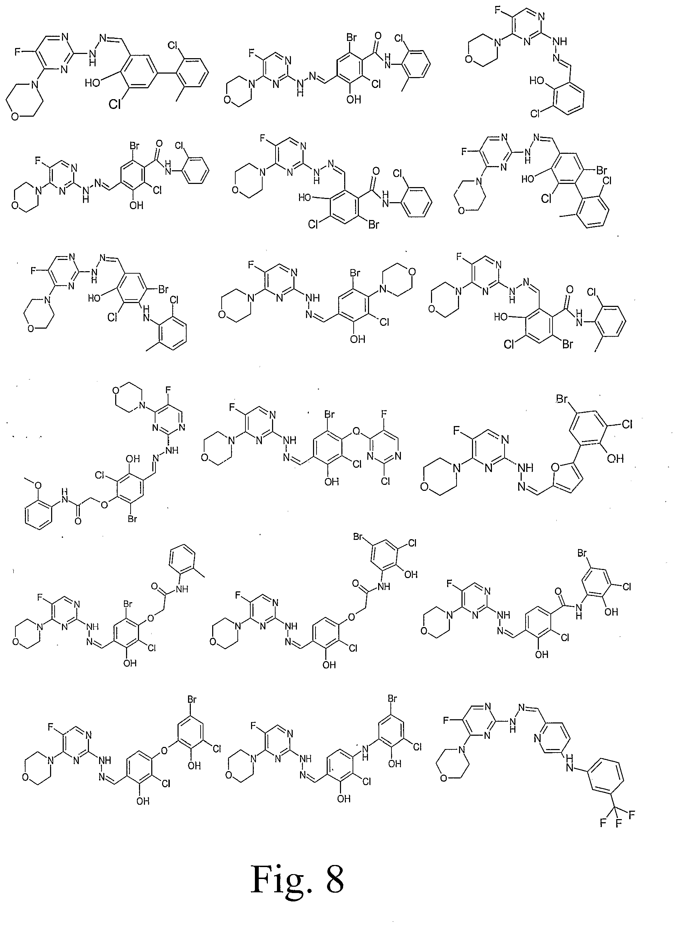

Figure 8 shows the chemical structures of representative compounds of the present application.

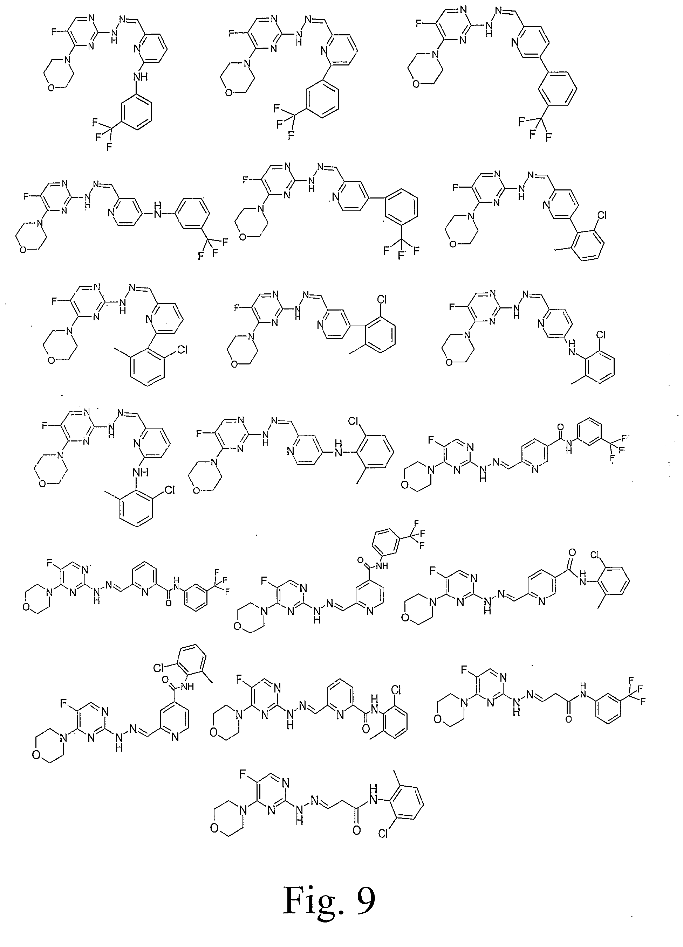

Figure 9 shows the chemical structures of representative compounds of the present application.

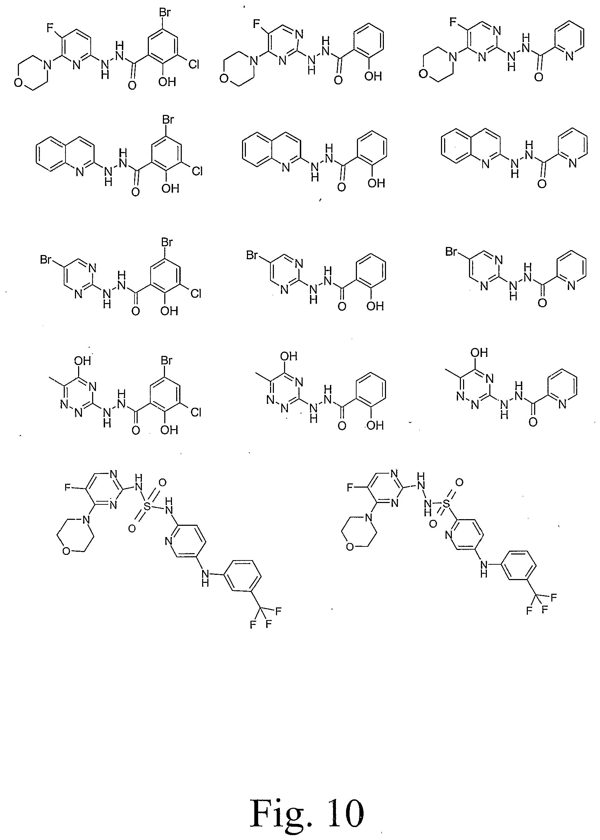

Figure 10 shows the chemical structures of representative compounds of the present application.

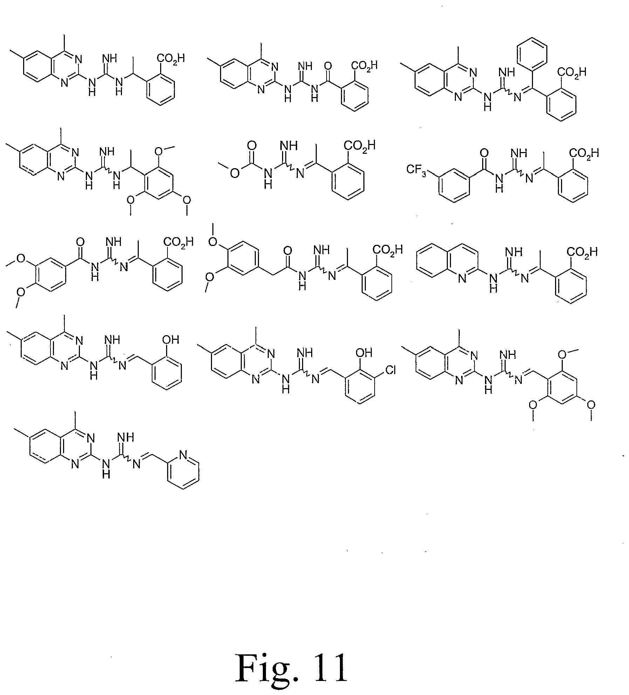

Figure 11 shows the chemical structures of representative compounds of the present application.

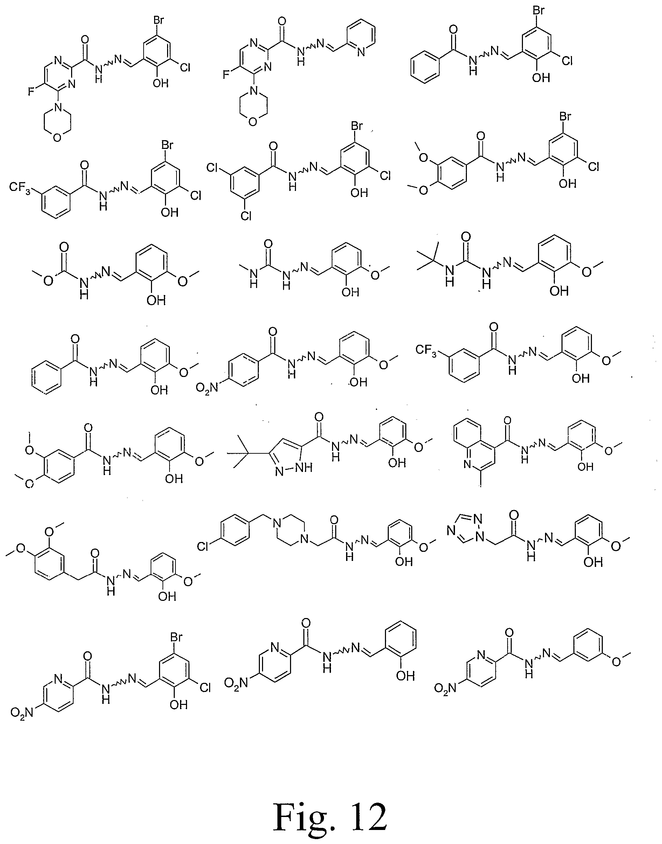

Figure 12 shows the chemical structures of representative compounds of the present application.

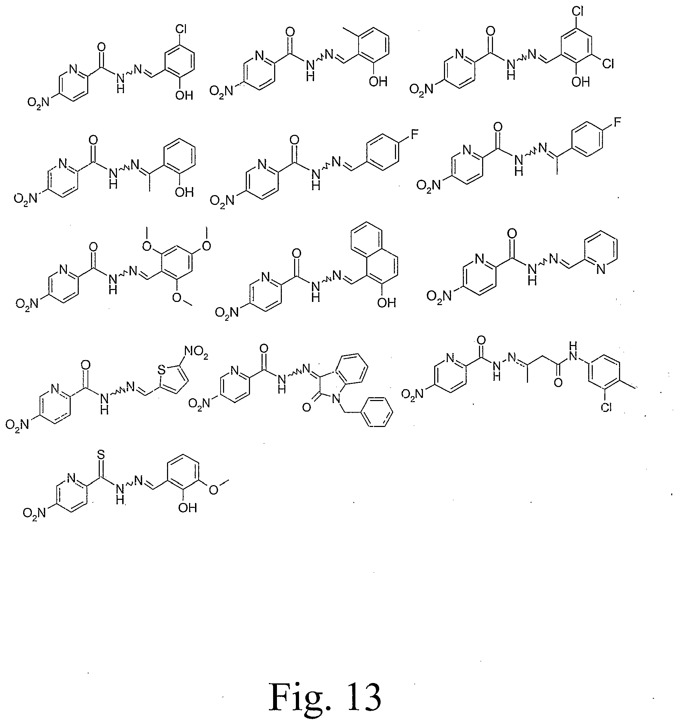

Figure 13 shows the chemical structures of representative compounds of the present application.
Figure 14 shows the inhibitory effect on growth rate of a hypothetical compound having a cellular specificity gap of 1 with respect to a test cell and a control cell.
Figure 15 shows the inhibitory effect on growth rate of a hypothetical compound having a cellular specificity gap of 40 with respect to a test cell and a control cell.
Figure 16 shows the growth inhibitory effect of imatinib mesylate at concentrations significantly below the apparent IC₅₀ for cellular toxicity.
Figure 17 shows the effect on growth of different concentrations of C2 and various C2 analogues for BalF3 cells expressing the p210^{Bcr-Abl-T315I} drug resistant mutant.
Figure 18 shows the results of a standard cell free protein kinase autophosphorylation assay for T315I mutant kinase domains in the presence of C2 and various C2 analogues at a concentration of 20 µM.

### DETAILED DESCRIPTION OF THE INVENTION

The term "halo" or "halogen" as used herein includes fluorine, chlorine, bromine and iodine.

The term "alkyl" as used herein contemplates substituted and unsubstituted, straight and branched chain alkyl radicals having from 1 to 6 carbon atoms. Preferred alkyl groups include methyl, ethyl, propyl, isopropyl, butyl, isobutyl, tert-butyl, and the like. Additionally, the alkyl group may be optionally substituted with one or more substituents selected from halo, CN, CO₂R, C(O)R, C(O)NR₂, NR₂, cyclic-amino, NO₂, and OR.

The term "cycloalkyl" as used herein contemplates substituted and unsubstituted cyclic alkyl radicals. Preferred cycloalkyl groups are those with a single ring containing 3 to 7 carbon atoms and include cyclopropyl, cyclopentyl, cyclohexyl, and the like. Other cycloalkyl groups may be selected from C₇ to C₁₀ bicyclic systems or from C₉ to C₁₄ tricyclic systems. Additionally, the cycloalkyl group may be optionally substituted with one or more substituents selected from halo, CN, CO₂R, C(O)R, C(O)NR₂, NR₂, cyclic-amino, NO₂, and OR.

The term "alkenyl" as used herein contemplates substituted and unsubstituted, straight and branched chain alkene radicals. Preferred alkenyl groups are those containing two to six carbon atoms. Additionally, the alkenyl group may be optionally substituted with one or more substituents selected from halo, CN, CO₂R, C(O)R, C(O)NR₂, NR₂, cyclic-amino, NO₂, and OR.

The term "alkynyl" as used herein contemplates substituted and unsubstituted, straight and branched chain alkyne radicals. Preferred alkynyl groups are those containing two to six carbon atoms. Additionally, the alkynyl group may be optionally substituted with one or more substituents selected from halo, CN, CO₂R, C(O)R, C(O)NR₂, NR₂, cyclic-amino, NO₂, and OR.

The term "aralkyl" as used herein contemplates an alkyl group which has as a substituent an aromatic group, which aromatic group may be substituted and unsubstituted. The aralkyl group may be optionally substituted on the aryl with one or more substituents selected from halo, CN, CF₃, NR₂, cyclic-amino, NO₂, OR, CF₃, -(CH₂)*ₓ*R, -(CH₂)ₓC(O)(CH₂)*_{y}*R, -(CH₂)*ₓ*C(O)N(R')(R"), -(CH₂)*ₓ*C(O)O(CH₂)*_{y}*R, -(CH₂)*ₓ*N(R')(R"), -N(R)SO₂R, -O(CH₂)ₓC(O)N(R')(R"), -SO₂N(R')(R"), -(CH₂)*ₓ*N(R)-(CH₂)*_{y}*-R, -(CH₂)*ₓ*N(R)-C(O)-(CH₂)*_{y}*-R, -(CH₂)*ₓ*N(R)-C(O)-O-(CH₂)*_{y}*R, -(CH₂)*ₓ*-C(O)-N(R)-(CH₂)*_{y}*-R, -(CH₂)*ₓ*C(O)N(R)-(CH₂)*_{y}*-R, -O-(CH₂)*ₓ*-C(O)-N(R)-(CH₂)*_{y}*-R, substituted and unsubstituted alkyl, substituted and unsubstituted cycloalkyl, substituted and unsubstituted aralkyl, substituted and unsubstituted alkenyl, substituted and unsubstituted alkynyl, substituted and unsubstituted aryl, and a substituted and unsubstituted heterocyclic ring, wherein the substituted alkyl, substituted cycloalkyl, substituted aralkyl, substituted alkenyl, substituted alkynyl, substituted aryl, and substituted heterocyclic ring may be substituted with one of more halo, CN, CF₃, CO₂R, C(O)R, C(O)NR₂, NR₂, cyclic-amino, NO₂, and OR.

The term "heterocyclic group" or "heterocyclic ring" as used herein contemplates aromatic and non-aromatic cyclic radicals having at least one heteroatom as a ring member. Preferred heterocyclic groups are those containing 5 or 6 ring atoms which includes at least one hetero atom, and includes cyclic amines such as morpholino, piperidino, pyrrolidino, and the like, and cyclic ethers, such as tetrahydrofuran, tetrahydropyran, and the like. Aromatic heterocyclic groups, also termed "heteroaryl" groups contemplates single-ring hetero-aromatic groups that may include from one to three heteroatoms, for example, pyrrole, furan, thiophene, imidazole, oxazole, thiazole, triazole, pyrazole, pyridine, pyrazine, pyridazine, pyrimidine, and the like. The term heteroaryl also includes polycyclic hetero-aromatic systems having two or more rings in which two atoms are common to two adjoining rings (the rings are "fused") wherein at least one of the rings is a heteroaryl, e.g., the other rings can be cycloalkyls, cycloalkenyls, aryl, heterocycles and/or heteroaryls. Examples of polycyclic heteroaromatic systems include quinoline, isoquinoline, tetrahydroisoquinoline, quinoxaline, quinaxoline, benzimidazole, benzofuran, purine, imidazopyridine, benzotriazole, and the like. Additionally, the heterocyclic groups may be optionally substituted with halo, CN, CF₃, NR₂, cyclic-amino, NO₂, OR, CF₃, -(CH₂)*ₓ*C(O)(CH₂)*_{y}*R, -(CH₂)*ₓ*C(O)N(R')(R"), -(CH₂)*ₓ*C(O)O(CH₂)*_{y}*R, -(CH₂)*ₓ*N(R')(R"), -N(R)SO₂R, -O(CH₂)*ₓ*C(O)N(R')(R"), -SO₂N(R')(R"), -(CH₂)*ₓ*N(R)-(CH₂)*_{y}*-R, -(CH₂)*ₓ*N(R)-C(O)-(CH₂)*_{y}*R, -(CH₂)*ₓ*N(R)-C(O)-O-(CH₂)*_{y}*-R, -(CH₂)*ₓ*-C(O)-N(R)-(CH₂)*_{y}*-R, -(CH₂)*ₓ*C(O)N(R)-(CH₂)*_{y}*-R, -O-(CH₂)*ₓ*-C(O)-N(R)-(CH₂)*_{y}*-R, substituted and unsubstituted alkyl, substituted and unsubstituted cycloalkyl, substituted and unsubstituted aralkyl, substituted and unsubstituted alkenyl, substituted and unsubstituted alkynyl, substituted and unsubstituted aryl, and a substituted and unsubstituted heterocyclic ring, wherein the substituted alkyl, substituted cycloalkyl, substituted aralkyl, substituted alkenyl, substituted alkynyl, substituted aryl, and substituted heterocyclic ring may be substituted with one of more halo, CN, CF₃, CO₂R, C(O)R, C(O)NR₂, NR₂, cyclic-amino, NO₂, and OR.

The term "cyclic-amino" as used herein contemplates aromatic and non-aromatic cyclic radicals having at least one nitrogen as a ring member. Preferred cyclic amino groups are those containing 5 or 6 ring atoms, which includes at least one nitrogen, and includes morpholino, piperidino, pyrrolidino, piperazino, imidazole, oxazole, thiazole, triazole, pyrazole, pyridine, pyrazine, pyridazine, pyrimidine and the like. Additionally, the cyclic-amino may be optionally substituted with halo, CN, CF₃, NR₂, NO₂, OR, CF₃, substituted and unsubstituted alkyl, substituted and unsubstituted cycloalkyl, substituted and unsubstituted aralkyl, substituted and unsubstituted alkenyl, substituted and unsubstituted alkynyl, substituted and unsubstituted aryl, and a substituted and unsubstituted heterocyclic ring, wherein the substituted alkyl, substituted cycloalkyl, substituted aralkyl, substituted alkenyl, substituted alkynyl, substituted aryl, and substituted heterocyclic ring may be substituted with one or more of halo, CN, CF₃, CO₂R, C(O)R, C(O)NR₂, NR₂, NO₂, and OR.

The term "aryl" or "aromatic group" as used herein contemplates single-ring aromatic groups (for example, phenyl, pyridyl, pyrazole, etc.) and polycyclic ring systems (naphthyl, quinoline, etc.). The polycyclic rings may have two or more rings in which two atoms are common to two adjoining rings (the rings are "fused") wherein at least one of the rings is aromatic, e.g., the other rings can be cycloalkyls, cycloalkenyls, aryl, heterocycles and/or heteroaryls. Additionally, the aryl groups may be optionally substituted with one or more substituents selected from halo, CN, CF₃, NR₂, cyclic-amino, NO₂, OR, CF₃, -(CH₂)*ₓ*C(O)(CH₂)*_{y}*R, -(CH₂)*ₓ*C(O)N(R')(R"), -(CH₂)*ₓ*C(O)O(CH₂)*_{y}*R, -(CH₂)*ₓ*N(R')(R"), -N(R)SO₂R, -O(CH₂)*ₓ*C(O)N(R')(R"), -SO₂N(R')(R"), -(CH₂)*ₓ*N(R)-(CH₂)*_{y}*-R, -(CH₂)*ₓ*N(R)-C(O)-(CH₂)*_{y}*-R, -(CH₂)*ₓ*N(R)-C(O)-O-(CH₂)*_{y}-*R, -(CH₂)*ₓ*-C(O)-N(R)-(CH₂)*_{y}*-R, -CH₂)ₓC(O)N(R)-(CH₂)_{y}-R, -O-(CH₂)*ₓ*-C(O)-N(R)-(CH₂)*_{y}*-R, substituted and unsubstituted alkyl, substituted and unsubstituted cycloalkyl, substituted and unsubstituted aralkyl, substituted and unsubstituted alkenyl, substituted and unsubstituted alkynyl, substituted and unsubstituted aryl, and a substituted and unsubstituted heterocyclic ring, wherein the substituted alkyl, substituted cycloalkyl, substituted aralkyl, substituted alkenyl, substituted alkynyl, substituted aryl, and substituted heterocyclic ring may be substituted with one of more halo, CN, CF₃, CO₂R, C(O)R, C(O)NR₂, NR₂, cyclic-amino, NO₂, and OR.

The term "heteroatom", particularly as a ring heteroatom, refers to N, O, and S.

Each R is independently selected from H, substituted and unsubstituted alkyl, substituted and unsubstituted cycloalkyl, substituted and unsubstituted aralkyl, substituted and unsubstituted aryl and a substituted and unsubstituted heterocyclic ring, wherein the substituted alkyl, substituted cycloalkyl, substituted aralkyl, substituted aryl and substituted heterocyclic ring may be substituted with one or more halo, CN, CF₃, OH, CO₂H, NO₂, C₁₋₆alkyl, -O-(C₁₋₆alkyl), -NH₂, -NH(C₁₋₆alkyl) and -N(C₁₋₆alkyl)₂. Each R' and R" are independently selected from H, or substituted and unsubstituted alkyl, substituted and unsubstituted cycloalkyl, substituted and unsubstituted aralkyl, substituted and unsubstituted aryl and a substituted and unsubstituted heterocyclic ring, wherein the substituted alkyl, substituted cycloalkyl, substituted aralkyl, substituted aryl and substituted heterocyclic ring may be substituted with one or more halo, CN, CF₃, OH, CO₂H, NO₂, C₁₋₆alkyl, -O-(C₁₋₆alkyl), -NH₂, -NH(C₁₋₆alkyl) and -N(C₁₋₆alkyl)₂; or R' and R" may be taken together with the nitrogen to which they are attached form a 5- to 7- membered ring which may optionally contain up to three further heteroatoms, which heteroatoms may be substituted by C₁₋₆alkyl. Each *x* and each *y* are independently selected from 0 to 4.

The present application discloses inhibitors of the P210^{BCR-ABL-T315I} theramutein having the formula I 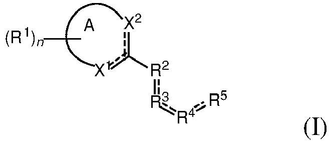 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹,-(CH₂)*ₚ*N(R¹¹)(CH₂)*_{q}*C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
R² is selected from -CR²¹*ₐ*-, -NR²²*_{b}*-, and -(C=R²³)-;
   each R²¹ is independently selected from H, halo, -NH₂, -N(H)(C₁₋₃ alkyl), -N(C₁₋₃ alkyl)₂, -O-(C₁₋₃ alkyl), OH and C₁₋₃ alkyl;
   each R²² is independently selected from H and C₁₋₃ alkyl;
   R²³ is selected from O, S, N-R°, and N-OR⁰;
R³ is selected from -CR³¹*_{c}*-, -NR³²*_{d}*-, -SO₂-, and -(C=R³³)-;
   each R³¹ group is selected from H, halo, -NH₂, -N(H)(R⁰), -N(R⁰)₂, -O-R⁰, OH and C₁₋₃ alkyl;
   each R³² group is selected from H, alkyl, cycloalkyl, alkenyl, alkynyl, aralkyl, CO₂R⁰, C(O)R⁰, aryl, and a heterocyclic ring;
   R³³ is selected from O, S, N-R³⁴, and N-OR⁰;
   R³⁴ is selected from H, NO₂, CN, alkyl, cycloalkyl, alkenyl, alkynyl, aralkyl, aryl and a heterocyclic ring;
R⁴ is selected from -CR⁴¹*ₑ*-, -NR⁴²*_{f}*-, -(C=R⁴³)- , -SO₂-, and -O-;
   each R⁴¹ is selected from H, alkyl, cycloalkyl, alkenyl, alkynyl, CO₂R⁰, C(O)R⁰, aralkyl, aryl, and a heterocyclic ring;
   each R⁴² group is selected from H, alkyl, cycloalkyl, alkenyl, alkynyl, aralkyl, CO₂R⁰, C(O)R⁰, aryl, and a heterocyclic ring;
   each R⁴³ is selected from O, S, N-R°, and N-OR⁰;
with the provisos that when R² is -NR²²*_{b}*- and R⁴ is -NR⁴²*_{f}*-, then R³ is not -NR³²*_{d}*-; that both R³ and R⁴ are not simultaneously selected from -(C=R³³)- and -(C=R⁴³)-, respectively; and
that R³ and R⁴ are not simultaneously selected from -SO₂-;
R⁵ is selected from -Y-R⁶ and -Z-R⁷;
   Y is selected from a chemical bond, O, NR⁰,
   R⁶ is selected from alkyl, cycloalkyl, alkenyl, alkynyl, aralkyl, aryl, and a heterocyclic ring;
   Z is a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
   R⁷ is H or is selected from aryl and a heterocyclic ring;
each R⁰ is independently selected from H, alkyl, cycloalkyl, aralkyl, aryl and a heterocyclic ring;
*a* is 1 or 2;
*b* is 0 or 1;
*c* is 1 or 2;
*d* is 0 or 1;
*e* is 1 or 2; and
*f* is 0 or 1.

An important component and conceptual teaching described herein is that neither the R² nor the R³ positions of the compounds are members of any aromatic or non-aromatic ring structure. Compounds having the R² and/or the R³ positions as members of any aromatic or non-aromatic ring structure do not effectively inhibit the T3151 theramutein, whereas the compounds that lack such a ring component at these positions, in addition to having other preferred chemical groups, are potent inhibitors of the T315I theramutein.

In preferred embodiments, ring A is an aromatic ring.

In preferred embodiments, X¹ or X² is N. In another preferred embodiment, both X¹ and X² are N. In particularly preferred embodiments Ring A is a pyridine ring or a pyrimidine ring. In still further preferred embodiments, Ring A is selected from the structures provided below:

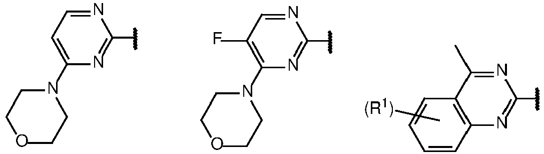

In preferred embodiments, R⁵ is a group having the formula 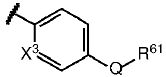 wherein:
X³ is N or CH;
R⁶¹ is selected from aryl and a heterocyclic ring;
Q is selected from a chemical bond or a group having the formula -O-, -(CH₂)*ᵢ*-, -(CH₂)*ᵢ*C(O)(CH₂)*ⱼ*-, -(CH₂)*ᵢ*-N(R⁶²)-(CH₂)*ⱼ*-, -(CH₂)*ᵢ*C(O)-N(R⁶²)-(CH₂)*ⱼ*-, -(CH₂)*ᵢ*C(O)O(CH₂)*ⱼ*-, -(CH₂)*ᵢ*N(R⁶²)C(O)-(CH₂)*ⱼ*-, -(CH₂)*ᵢ*OC(O)N(R⁶²)-(CH₂)*ⱼ*-, and -O-(CH₂)*ᵢ*-C(O)N(R⁶²)-(CH₂)*ⱼ*-;
R⁶² is selected from H, alkyl, aryl, and a heterocyclic ring;
each R⁰ is independently selected from H, alkyl, cycloalkyl, aralkyl, aryl and a heterocyclic ring;
*h* is 0 to 4;
*i* is 0 to 4; and
*j* is 0 to 4.

In further preferred embodiments, R⁵ is a group having the formula 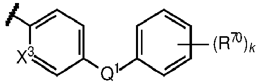 wherein:
X³ is N or CH;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
each R⁷⁰ is selected from halo, alkyl, CN, N(R⁷¹)₂, cyclic-amino, NO₂, OR⁷¹, and CF₃,
each R⁷¹ is selected from H, alkyl, aryl, aralkyl and a heterocyclic ring; and
*k* is 0 to 4.

In further preferred embodiments, R⁵ is a group having the formula 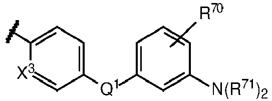 wherein
X³ is N or CH;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
R⁷⁰ is selected from halo, alkyl, CN, N(R⁷¹)₂, cyclic-amino, NO₂, OR⁷¹, and CF₃; and
each R⁷¹ is selected from H, alkyl, aryl, aralkyl and a heterocyclic ring.
In particularly preferred embodiments one or more of the following selections is made: Q¹ is -NH-; X³ is N; each R⁷¹ is independently selected from H, methyl, and ethyl, and preferably each R⁷¹ is methyl; and/or R⁷⁰ is selected from OH, OCH₃, halo, and CF₃.

In a preferred embodiment, if R² or R⁴ is selected to be -NR²²*_{b}*- or -NR⁴²-, respectively, then R³¹ is not selected from halo, -NH₂, -N(H)(R⁰), -N(R⁰)₂, -O-R⁰, or OH.

The present application further discloses inhibitors of the P210^{BCR-ABL-T315I} theramutein having the formula Iₐ 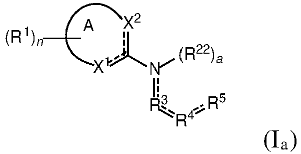 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)(CH₂)_{q}C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
each R²² is independently selected from H and C₁₋₃ alkyl;
R³ is selected from -CR³¹*_{c}*- , -NR³²*_{d}*-, -SO₂-, and -(C=R³³)-;
   each R³¹ group is selected from H, halo, -NH₂, -N(H)(R⁰), -N(R⁰)₂, -O-R⁰, OH and C₁₋₃ alkyl;
   each R³² group is selected from H, alkyl, cycloalkyl, alkenyl, alkynyl, aralkyl, CO₂R⁰, C(O)R⁰, aryl, and a heterocyclic ring;
   R³³ is selected from O, S, N-R³⁴, and N-OR⁰;
   R³⁴ is selected from H, NO₂, CN, alkyl, cycloalkyl, alkenyl, alkynyl, aralkyl, aryl and a heterocyclic ring;
R⁴ is selected from -CR⁴¹*ₑ*-, -NR⁴²*_{f}*-, -(C=R⁴³)-, -SO₂-, and -O-;
   each R⁴¹ is selected from H, alkyl, cycloalkyl, alkenyl, alkynyl, CO₂R⁰, C(O)R⁰, aralkyl, aryl, and a heterocyclic ring;
   each R⁴² group is selected from H, alkyl, cycloalkyl, alkenyl, alkynyl, aralkyl, CO₂R⁰, C(O)R⁰, aryl, and a heterocyclic ring;
   each R⁴³ is selected from O, S, N-R°, and N-OR⁰;
with the provisos that when R⁴ is -NR⁴²*_{f}*- then R³ is not -NR³²*_{d}*-; and that both R³ and R⁴ are not simultaneously selected from -(C=R³³)- and -(C=R⁴³)-, respectively; and that R³ and R⁴ are not simultaneously selected from -SO₂-;
R⁵ is selected from -Y-R⁶ and -Z-R⁷;
   Y is selected from a chemical bond, O, N-R⁰,
   R⁶ is selected from alkyl, cycloalkyl, alkenyl, alkynyl, aralkyl, aryl, and a heterocyclic ring;
   Z is a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
   R⁷ is H or is selected from aryl and a heterocyclic ring;
each R⁰ is independently selected from H, alkyl, cycloalkyl, aralkyl, aryl and a heterocyclic ring;
*a* is 1 or 2;
*b* is 0 or 1;
*c* is 1 or 2;
*d* is 0 or 1;
*e* is 1 or 2; and
*f* is 0 or 1.

The present application further provides inhibitors of the P210^{BCR-ABL-T315I} theramutein having the formula I_{b} 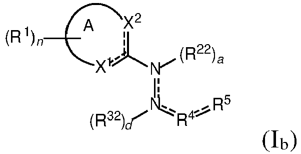 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)(CH₂)_{q}C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
each R²² is independently selected from H and C₁₋₃ alkyl;
each R³² group is selected from H, alkyl, cycloalkyl, alkenyl, alkynyl, aralkyl, CO₂R⁰, C(O)R⁰, aryl, and a heterocyclic ring;
R⁴ is selected from -CR⁴¹*ₑ*-, -(C=R⁴³)-, -SO₂-, and -O-;
   each R⁴¹ is selected from H, alkyl, cycloalkyl, alkenyl, alkynyl, CO₂R⁰, C(O)R⁰, aralkyl, aryl, and a heterocyclic ring;
   each R⁴³ is selected from O, S, N-R⁰, and N-OR⁰;
R⁵ is selected from -Y-R⁶ and -Z-R⁷;
   Y is selected from a chemical bond, O, N-R⁰,
   R⁶ is selected from alkyl, cycloalkyl, alkenyl, alkynyl, aralkyl, aryl, and a heterocyclic ring;
   Z is a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
   R⁷ is H or is selected from aryl and a heterocyclic ring;
each R⁰ is independently selected from H, alkyl, cycloalkyl, aralkyl, aryl and a heterocyclic ring;
*a* is 1 or 2;
*b* is 0 or 1;
*c* is 1 or 2;
*d* is 0 or 1;
*e* is 1 or 2; and
*f* is 0 or 1.

The present application further discloses inhibitors of the P210^{BCR-ABL-T315I} theramutein having the formula I_{c} 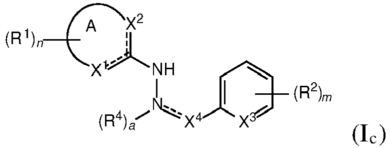 wherein
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)(CH₂)_{q}C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
X³ is N, CH or C-R²;
each R² is independently selected from the group consisting of alkyl, cycloalkyl, alkenyl, alkynyl, aralkyl, CN, CF₃, NO₂, OR²¹, -(CH₂)*ᵣ*C(O)(CH₂)*ₛ*R²¹, -(CH₂)*ᵣ*C(O)N(R²²)(R²³), -(CH₂)*ᵣ*C(O)O(CH₂)*ₛ*R²¹, -(CH₂)*ᵣ*N(R²¹)C(O)R²¹, -(CH₂)*ᵣ*N(R²²)(R²³), -N(R²¹)SO₂R²¹, -OC(O)N(R²²)(R²³), -SO₂N(R²²)(R²³), halo, aryl, and a heterocyclic ring, and additionally or alternatively, two R² groups on adjacent ring atoms form a 5- or 6-membered fused ring which contains from 0 to 3 heteroatoms;
   R²¹ is selected from H, alkyl, cycloalkyl, alkenyl, alkynyl, aralkyl, aryl, and a heterocyclic ring;
   R²² and R²³ are independently selected from H, alkyl, cycloalkyl, alkenyl, alkynyl, aralkyl, aryl, and a heterocyclic ring; or R²² and R²³ may be taken together with the nitrogen to which they are attached form a 5- to 7- membered ring which may optionally contain a further heteroatom, wherein the 5- to 7- membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *r* is 0 to 4;
   *s* is 0 to 4;
*m* is 0 to 4;
R⁴ is selected from H, alkyl, cycloalkyl, alkenyl, alkynyl, aralkyl, CO₂R⁰, C(O)R⁰, aryl, and a heterocyclic ring;
*a* is 0 or 1;
X⁴ is selected from 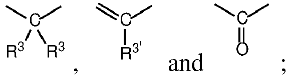
each R³ is independently selected from the group consisting of H, -N(R⁰)₂, alkyl, cycloalkyl, alkenyl, alkynyl, CO₂R⁰, C(O)R⁰, aralkyl, aryl, and a heterocyclic ring,
R³' is selected from H, -N(R⁰)₂, alkyl, cycloalkyl, aralkyl, aryl and a heterocyclic ring, and
each R⁰ is independently selected from H, alkyl, cycloalkyl, aralkyl, aryl and a heterocyclic ring.

In preferred embodiments, R², R³ and R⁴ of formula I are selected to give the following chemical groups:

-N(R²²)-N=C(R⁴¹)-

-N(R²²)-N(R³²)-C(=O)-

-N(R²²)-N(R³²)-C(R⁴¹)(R⁴¹)-

-N(R²²)-C(R³¹)(R³¹)-C(R⁴¹)(R⁴¹)-

-N(R²²)-C(R³¹)(R³¹)-C(=O)-

-N=N-C(R⁴¹)(R⁴¹)-

-C(R²¹)=C=C(R⁴¹)-

-C(R²¹)=C(R³¹)-C(=O)-

-C(R²¹)=C(R³¹)-C(R⁴¹)(R⁴¹)-

-C(R²¹)(R²¹)-C(R³¹)=C(R⁴¹)-

-C(R²¹)(R²¹)-C(R³¹)(R³¹)-C(=O)-

-C(R²¹)(R²¹)-C(R³¹)(R³¹)-C(R⁴¹)(R⁴¹)-

-C(R²¹)(R²¹)-N(R³²)-C(=O)-

-C(R²¹)(R²¹)-N(R³²)-C(R⁴¹)(R⁴¹)-

-N(R²²)-C(=O)-C(R⁴¹)(R⁴¹)-

-N(R²²)-C(=O)-N(R⁴¹)-

-N(R²²)-C(=O)-O-

-C(R²¹)(R²¹)-C(=O)-C(R⁴¹)(R⁴¹)-

-C(R²¹)(R²¹)-C(=O)-N(R⁴²)-

-N(R²²)-C(=NR³⁴)-N(R⁴²)-

-C(=O)-N(R¹²)-N(R⁴²).

Particularly preferred chemical groups for R², R³ and R⁴ include:

-N(R²²)-N=C(R⁴¹)-

-N(R²²)-N(R³²)-C(=O)-

-N(R²²)-C(R³¹)(R³¹)-C(R⁴¹)(R⁴¹)-

-N(R²²)-C(R³¹)(R³¹)-C(=O)-

-C(R²¹)(R²¹)-C(=O)-C(R⁴¹)(R⁴¹)-

-C(R²¹)(R²¹)-C(=O)-N(R⁴²)-

-N(R²²)-C(=NR³⁴)-N(R⁴²)-

-C(=O)-N(R³²)-N(R⁴²).

In further preferred embodiment, R⁶ or R⁷ is an aryl group, which may be optionally substituted. Particularly preferred aryl groups include substituted or unsubstituted phenyl and pyridyl. In additional or alternative embodiments, it is preferred that the substituents R²¹ and R²² are independently selected from groups which have small steric bulk and are preferably selected from H and CH₃, and more preferably are H.

The present application further discloses inhibitors of the P210^{BCR-ABL-T315I} theramutein having the formula II 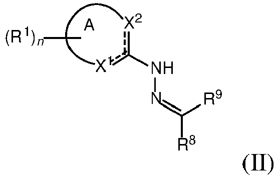 wherein
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)(CH₂)*_{q}*C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
R⁸ is selected from the group consisting of is selected from H, alkyl, cycloalkyl, alkenyl, alkynyl, CO₂R⁰, C(O)R⁰, aralkyl, aryl, and a heterocyclic ring;
R⁹ is selected from -Y-R⁶ and -Z-R⁷;
   Y is selected from a chemical bond, O, N-R⁰,
   R⁶ is selected from alkyl, cycloalkyl, alkenyl, alkynyl, aralkyl, aryl, and a heterocyclic ring;
   Z is a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
   R⁷ is H or is selected from aryl and a heterocyclic ring; and
each R⁰ is independently selected from H, alkyl, cycloalkyl, aralkyl, aryl and a heterocyclic ring.

The present application further discloses inhibitors of the P210^{BCR-ABL-T315I} theramutein having the formula IIₐ 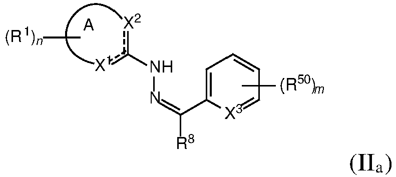 wherein
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
R⁸ is selected from the group consisting of is selected from H, alkyl, cycloalkyl, alkenyl, alkynyl, CO₂R⁰, C(O)R⁰, aralkyl, aryl, and a heterocyclic ring;
X³ is N, CH or C-R⁵⁰;
each R⁵⁰ is independently selected from the group consisting of alkyl, cycloalkyl, alkenyl, alkynyl, aralkyl, CN, CF₃, NO₂, OR⁵¹, -(CH₂)*ᵣ*C(O)(CH₂)*ₛ*R⁵¹, -(CH₂)*ᵣ*C(O)N(R⁵²)(R⁵³), -(CH₂)*ᵣ*C(O)O(CH₂)*ₛ*R⁵¹, -(CH₂)*ᵣ*N(R⁵¹)C(O)R⁵¹, -(CH₂)*ᵣ*N(R⁵²)(R⁵³), -N(R⁵¹)SO₂R⁵¹, -OC(O)N(R⁵²)(R⁵³), -SO₂N(R⁵²)(R⁵³), halo, aryl, and a heterocyclic ring, and additionally or alternatively, two R⁵⁰ groups on adjacent ring atoms form a 5- or 6-membered fused ring which contains from 0 to 3 heteroatoms;
   R⁵¹ is selected from H, alkyl, cycloalkyl, alkenyl, alkynyl, aralkyl, aryl, and a heterocyclic ring;
   R⁵² and R⁵³ are independently selected from H, alkyl, cycloalkyl, alkenyl, alkynyl, aralkyl, aryl, and a heterocyclic ring; or R⁵² and R⁵³ may be taken together with the nitrogen to which they are attached form a 5- to 7- membered ring which may optionally contain a further heteroatom, wherein the 5- to 7- membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *r* is 0 to 4;
   *s* is 0 to 4;
*m* is 0 to 4; and
each R⁰ is independently selected from H, alkyl, cycloalkyl, aralkyl, aryl and a heterocyclic ring.

The present application further discloses inhibitors of the P210^{BCR-ABL-T315I} theramutein having the formula II_{b} 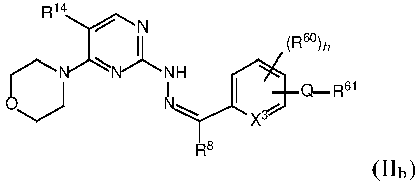 wherein:
R¹⁴ is selected from H and F;
R⁸ is selected from the group consisting of is selected from H, alkyl, cycloalkyl, alkenyl, alkynyl, CO₂R⁰, C(O)R⁰, aralkyl, aryl, and a heterocyclic ring;
X³ is N, CH or C-R⁶⁰;
each R⁶⁰ is independently selected from the group consisting of alkyl, cycloalkyl, alkenyl, alkynyl, aralkyl, CN, CF₃, NO₂, OR⁰, halo, aryl, and a heterocyclic ring;
R⁶¹ is selected from aryl and a heterocyclic ring;
Q is selected from a chemical bond or a group having the formula -O-, -(CH₂)*ᵢ*-, -(CH₂)*ᵢ*C(O)(CH₂)*ⱼ*-, -(CH₂)*ᵢ*-N(R⁶²)-(CH₂)*ⱼ*-, -(CH₂)*ᵢ*C(O)-N(R⁶²)-(CH₂)*ⱼ*-, -(CH₂)*ᵢ*C(O)O(CH₂)*ⱼ*-, -(CH₂)*ᵢ*N(R⁶²)C(O)-(CH₂)*ⱼ*-, -(CH₂)*ᵢ*OC(O)N(R⁶²)-(CH₂)*ⱼ*-, and - O-(CH₂)*ᵢ*-C(O)N(R⁶²)-(CH₂)*ⱼ*-;
R⁶² is selected from H, alkyl, aryl, and a heterocyclic ring;
each R⁰ is independently selected from H, alkyl, cycloalkyl, aralkyl, aryl and a heterocyclic ring;
*h* is 0 to 4;
*i* is 0 to 4; and
*j* is 0 to 4.

In preferred embodiments of compounds of the formula II_{b}, R⁶⁰ is selected from halo, CF₃, and OH. In other preferred embodiments, R⁸ is selected from H and CH₃.

In preferred embodiments of compounds of the formula II_{b}, X³ is N. In further preferred embodiments, Q is selected to be -(CH₂)*ᵢ*-N(R⁶²)-(CH₂)*ⱼ*-, and particularly in preferred embodiments, Q is -N(R⁶²)-.

The present application further discloses inhibitors of the P210^{BCR-ABL-T315I} theramutein having the formula II_{c} 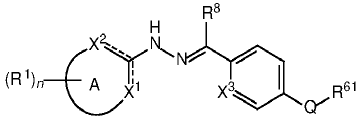 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   q is 0 to 4;
R⁸ is selected from H and methyl;
X³ is N or CH;
R⁶¹ is selected from aryl and a heterocyclic ring;
Q is selected from a chemical bond or a group having the formula -O-, -(CH₂)*ᵢ*-, -(CH₂)*ᵢ*C(O)(CH₂)*ⱼ*-, -(CH₂)*ᵢ*-N(R⁶²)-(CH₂)*ⱼ*-, -(CH₂)*ᵢ*C(O)-N(R⁶²)-(CH₂)*ⱼ*-, -(CH₂)*ᵢ*C(O)O(CH₂)*ⱼ*-, -(CH₂)*ᵢ*N(R⁶²)C(O)-(CH₂)*ⱼ*-, -(CH₂)*ᵢ*OC(O)N(R⁶²)-(CH₂)*ⱼ*-, and -O-(CH₂)*ᵢ*-C(O)N(R⁶²)-(CH₂)*ⱼ*-;
R⁶² is selected from H, alkyl, aryl, and a heterocyclic ring;
each R⁰ is independently selected from H, alkyl, cycloalkyl, aralkyl, aryl and a heterocyclic ring;
*h* is 0 to 4;
*i* is 0 to 4; and
*j* is 0 to 4.

The present application further discloses inhibitors of the P210^{BCR-ABL-T315I} theramutein having the formula II_{d} 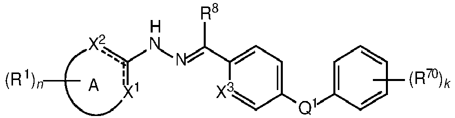 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
each R⁰ is independently selected from H, alkyl, cycloalkyl, aralkyl, aryl and a heterocyclic ring;
R⁸ is selected from H and CH₃;
X³ is N or CH;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
each R⁷⁰ is selected from halo, alkyl, CN, N(R⁷¹)₂, cyclic-amino, NO₂, OR⁷¹, and CF₃,
each R⁷¹ is selected from H, alkyl, aryl, aralkyl and a heterocyclic ring; and
*k* is 0 to 4.

The present application further discloses inhibitors of the P210^{BCR-ABL-T315I} theramutein having the formula IIₑ 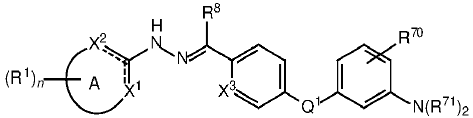 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
each R⁰ is independently selected from H, alkyl, cycloalkyl, aralkyl, aryl and a heterocyclic ring;
R⁸ is selected from H and CH₃;
X³ is N or CH;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
each R⁷⁰ is selected from halo, alkyl, CN, N(R⁷¹)₂, cyclic-amino, NO₂, OR⁷¹, and CF₃; and
each R⁷¹ is selected from H and alkyl.

The present application further discloses inhibitors of the P210^{BCR-ABL-T315I} theramutein having the formula II_{f} 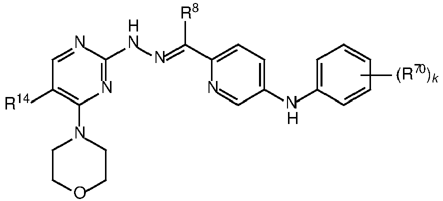 wherein
R¹⁴ is selected from H and F;
R⁸ is selected from H and CH₃;
each R⁷⁰ is selected from halo, alkyl, CN, N(R⁷¹)₂, cyclic-amino, NO₂, OR⁷¹, and CF₃,
each R⁷¹ is selected from H, alkyl, aryl, aralkyl and a heterocyclic ring; and
*k* is 0 to 4.

Exemplary compounds of the formula II, IIₐ, II_{b}, II_{c}, II_{d}, IIₑ or II_{f} includes the following structures:

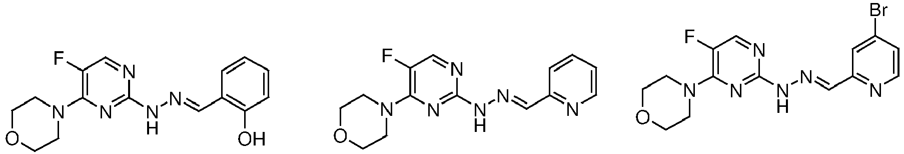

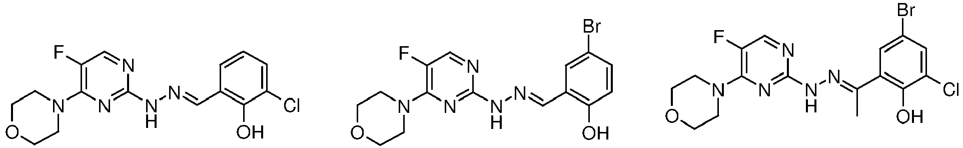

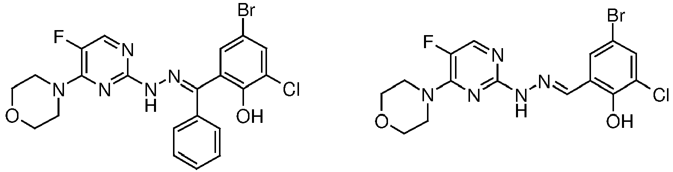

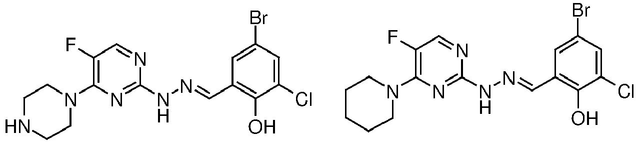

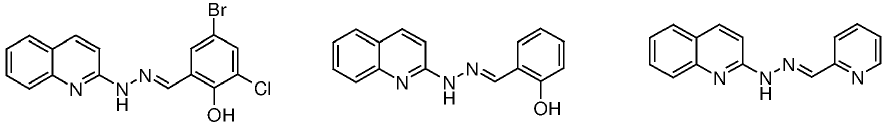

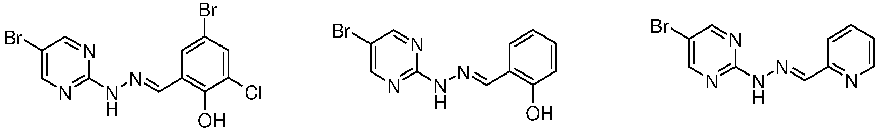

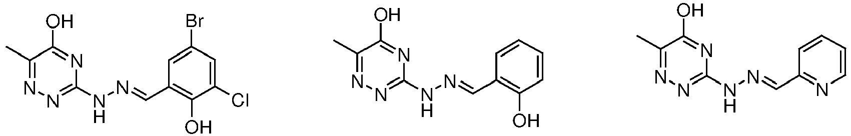

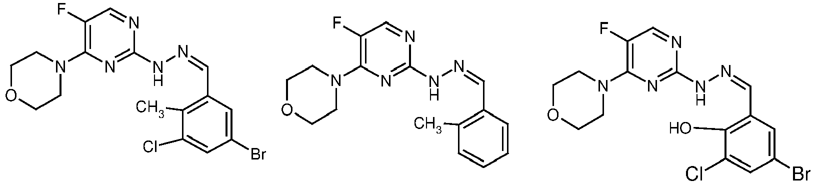

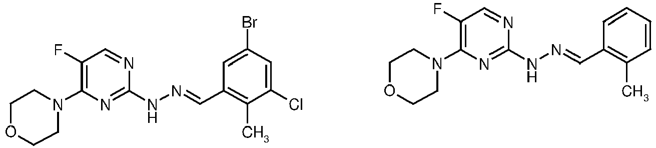

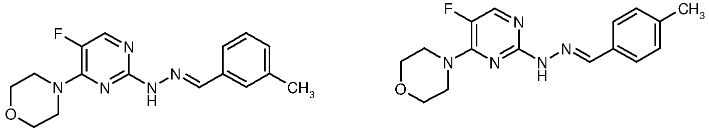

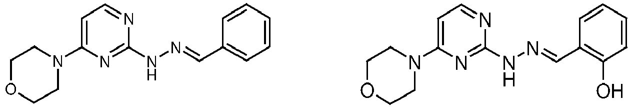

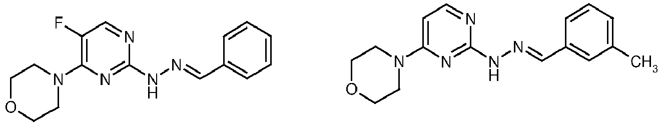

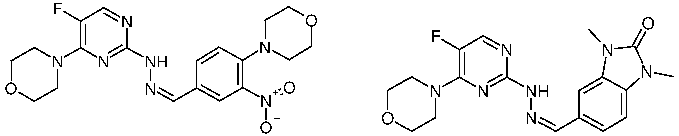

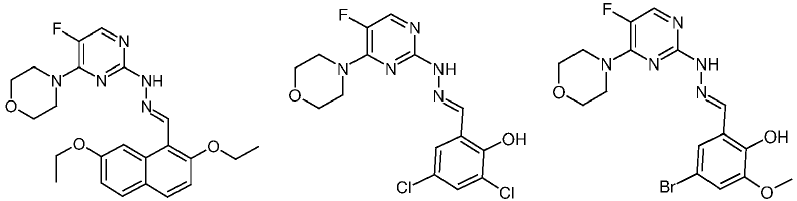

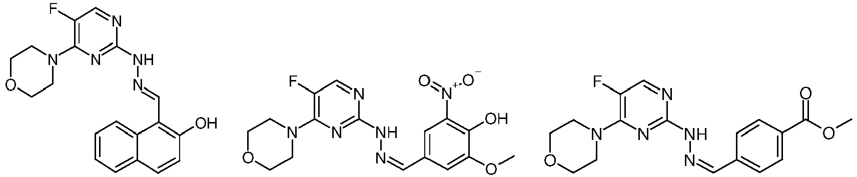

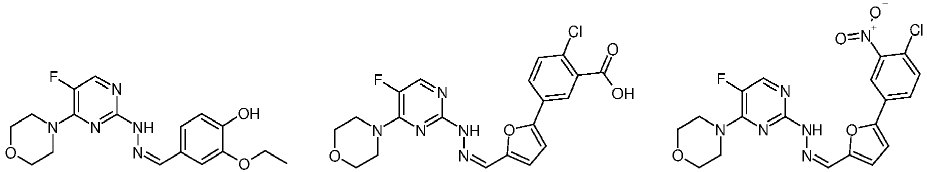

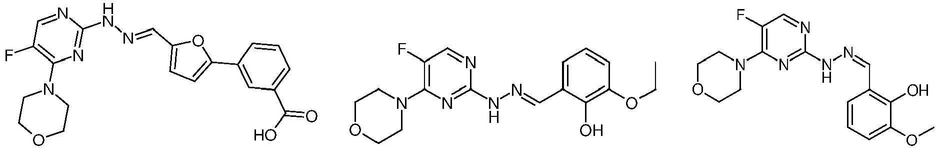

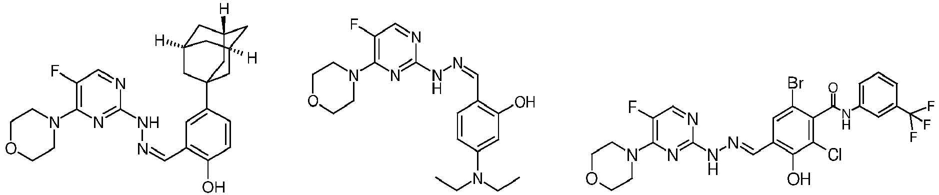

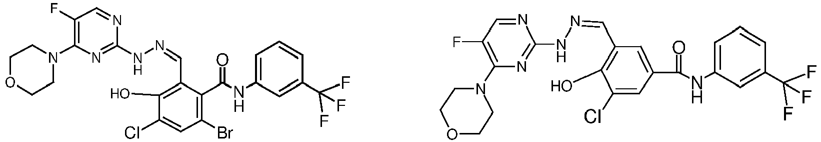

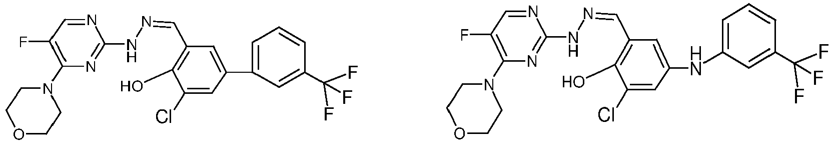

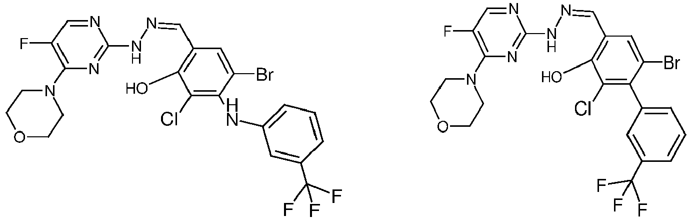

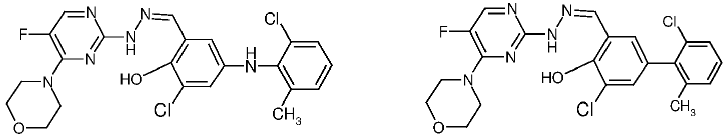

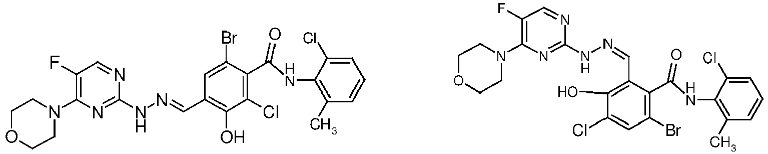

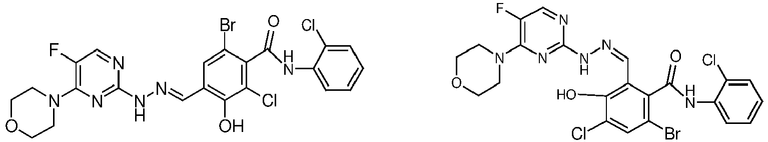

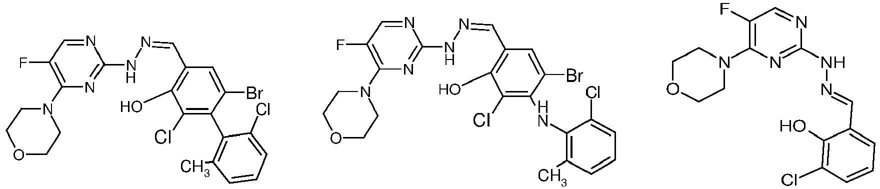

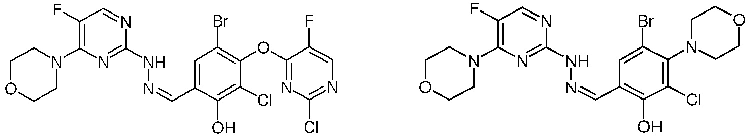

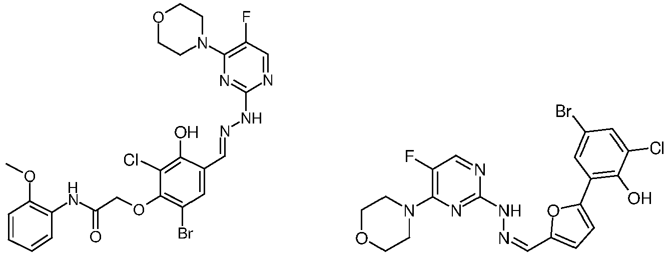

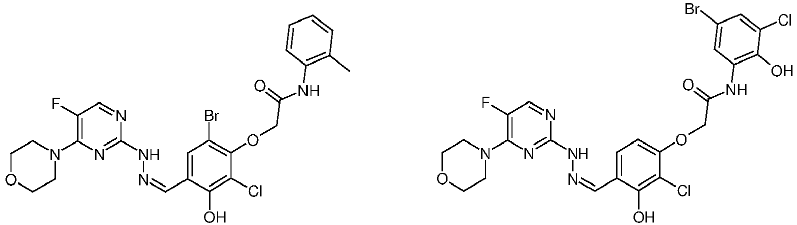

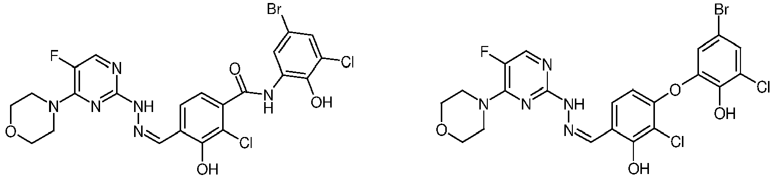

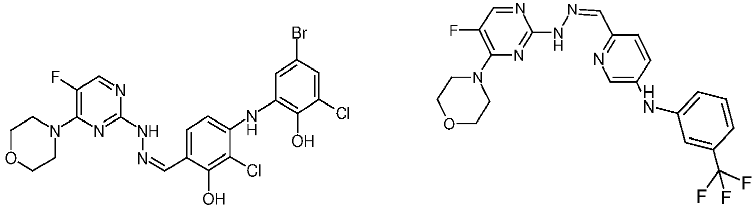

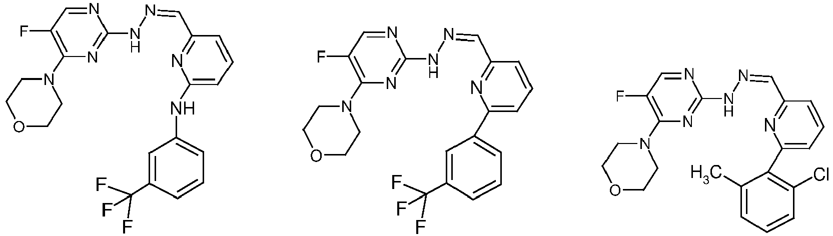

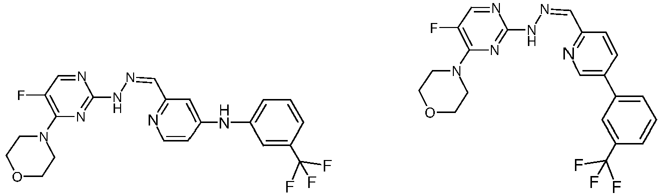

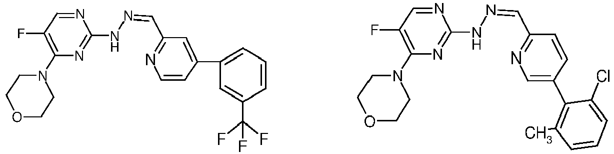

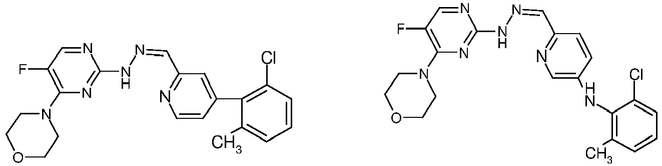

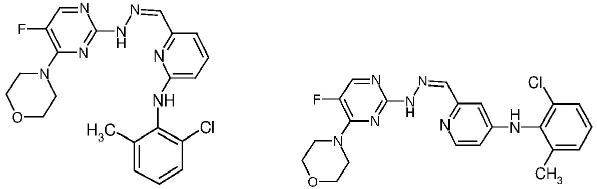

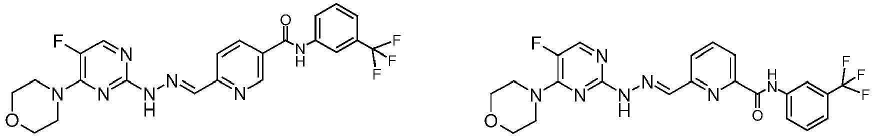

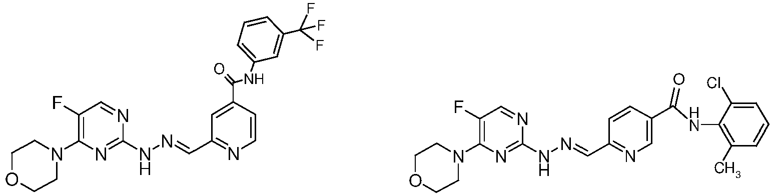

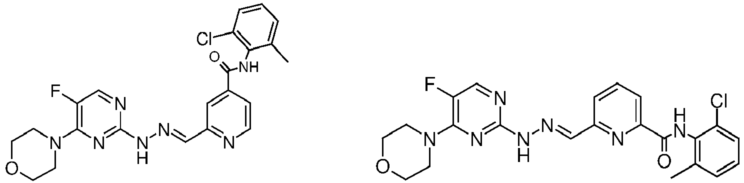

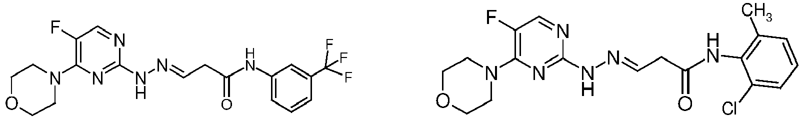

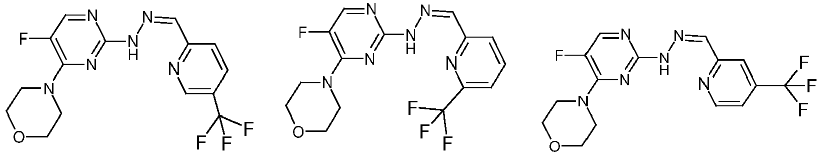

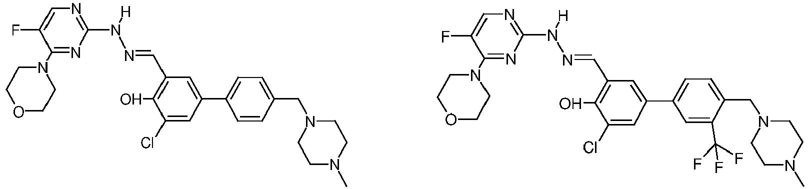

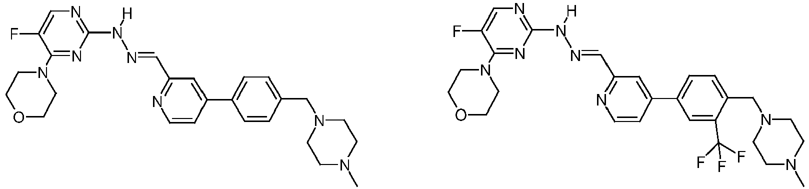

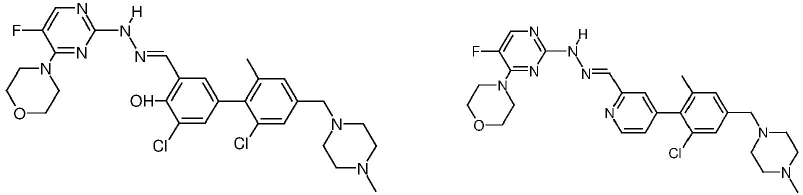

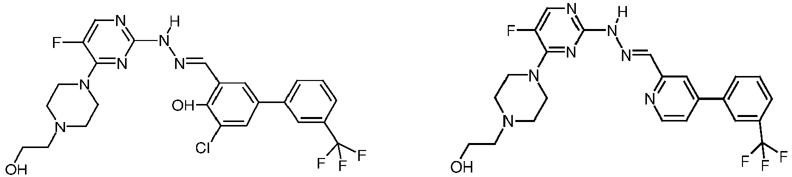

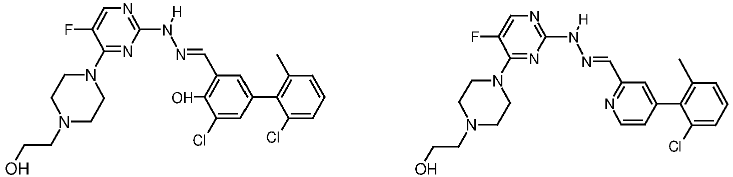

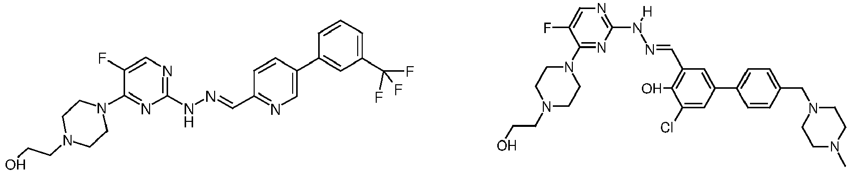

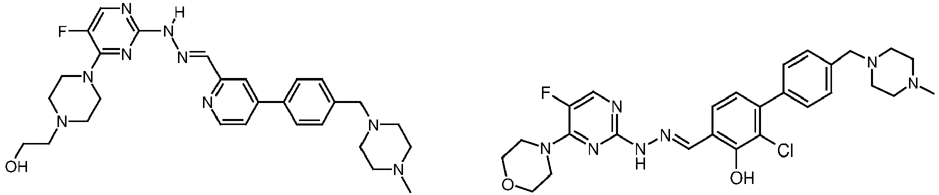

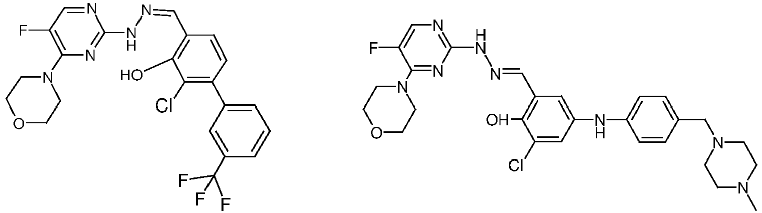

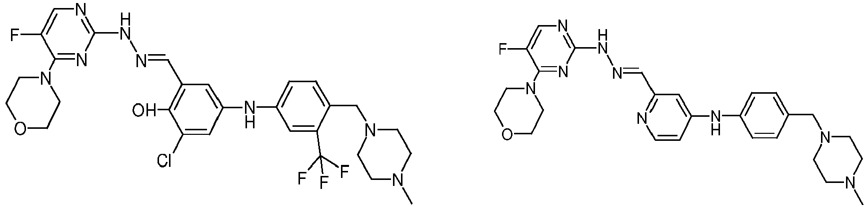

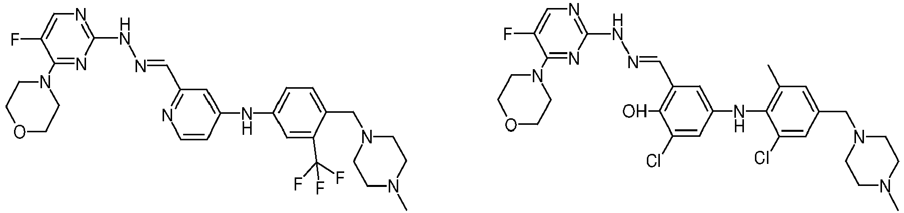

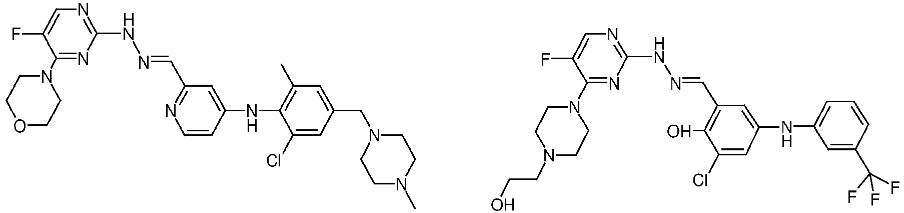

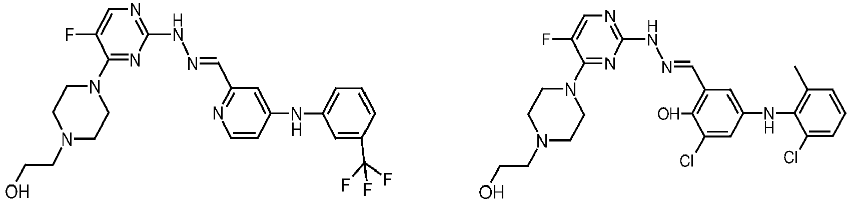

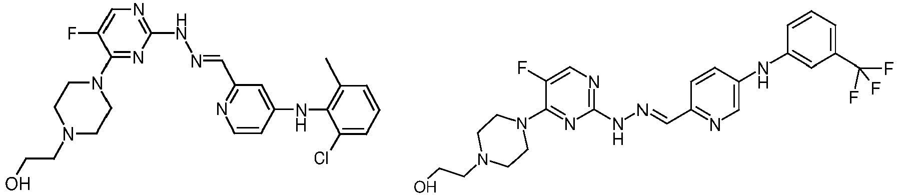

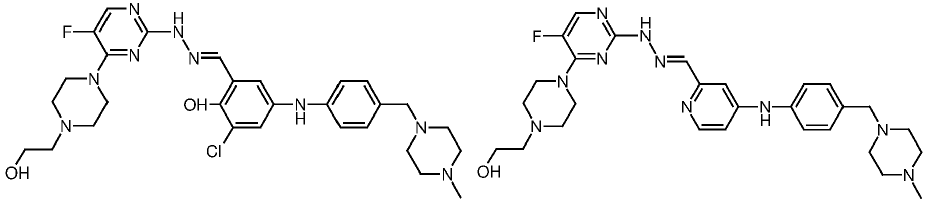

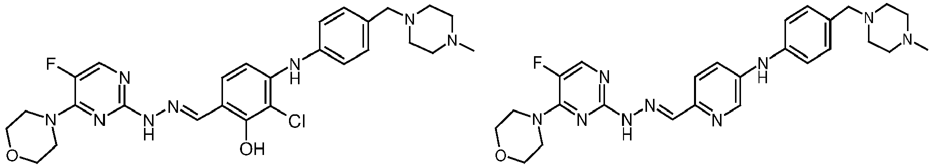

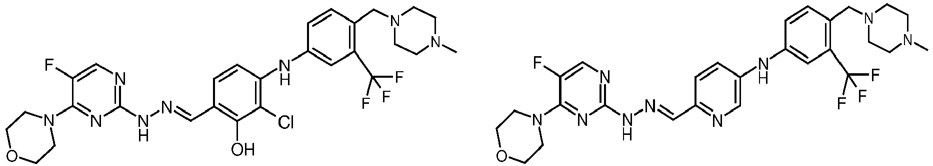

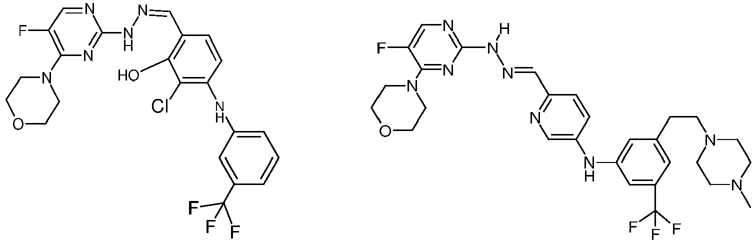

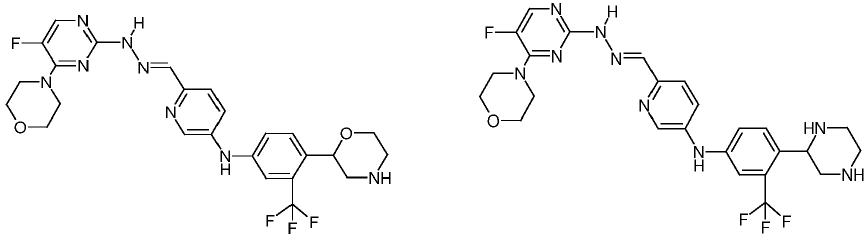

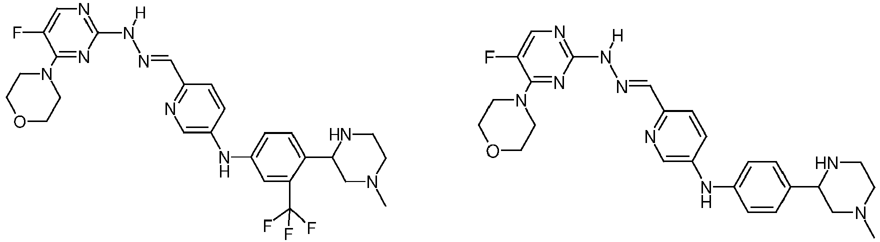

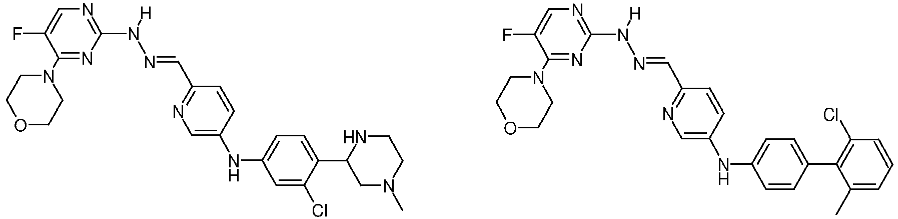

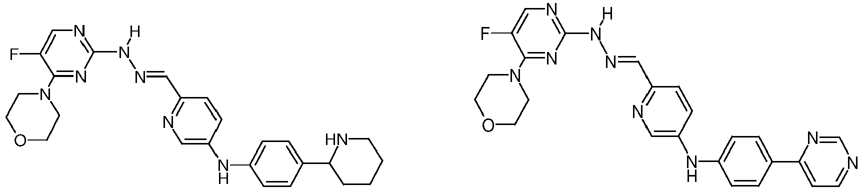

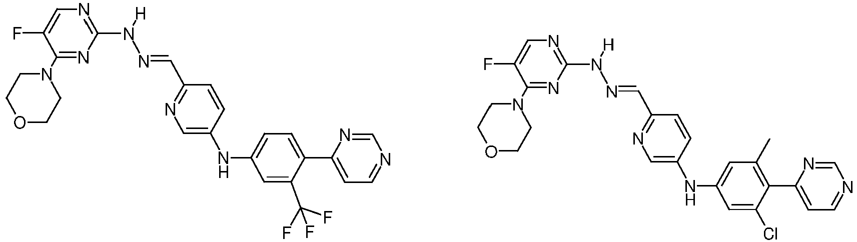

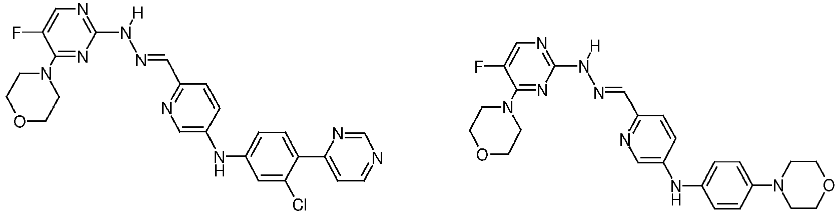

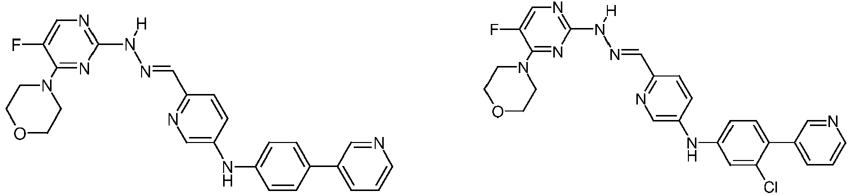

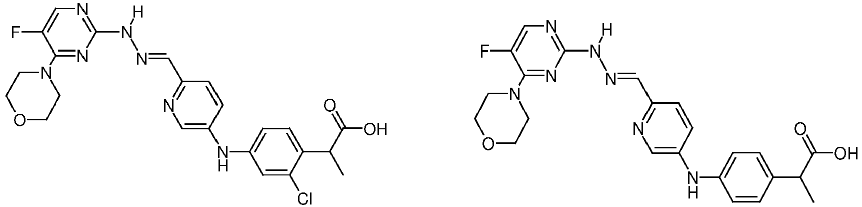

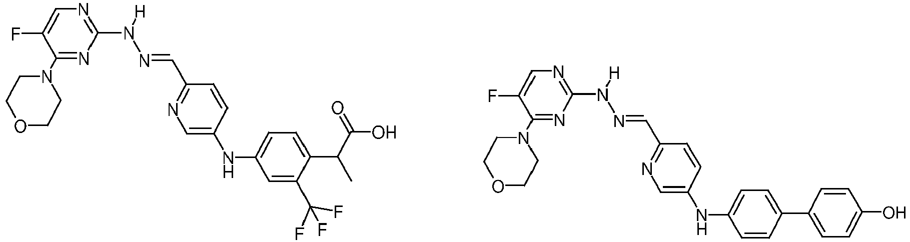

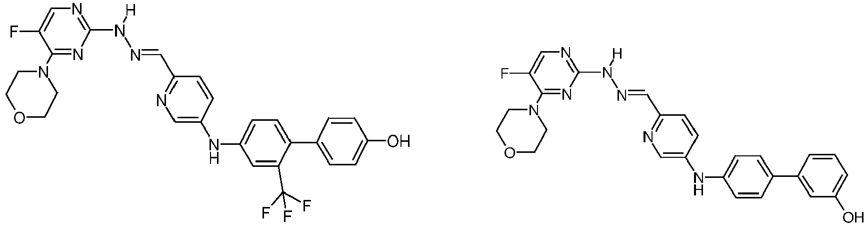

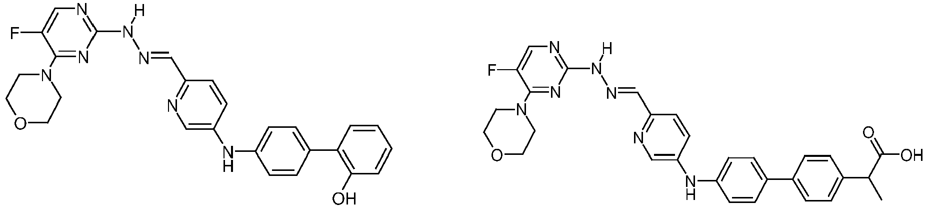

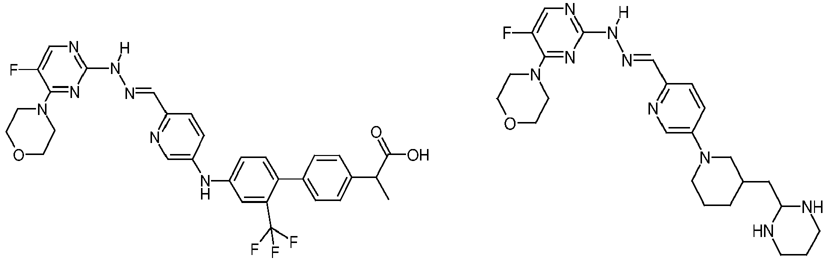

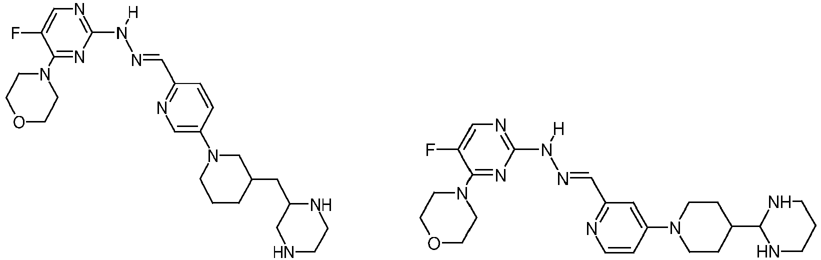

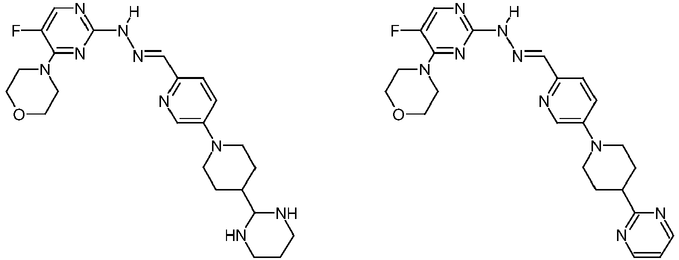

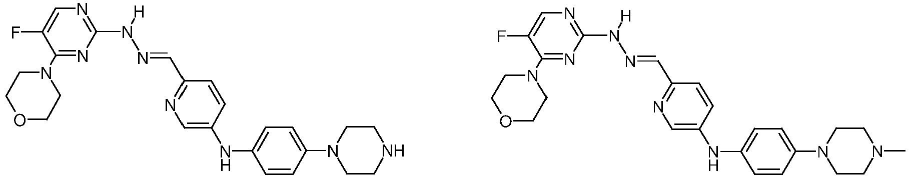

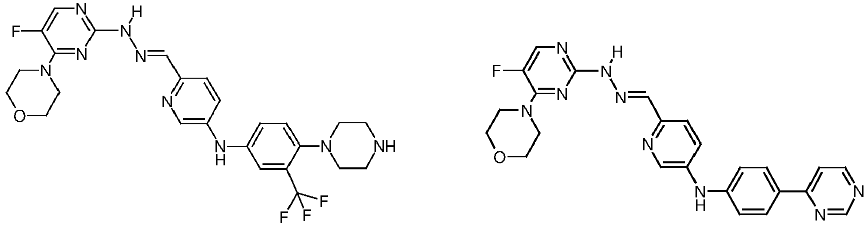

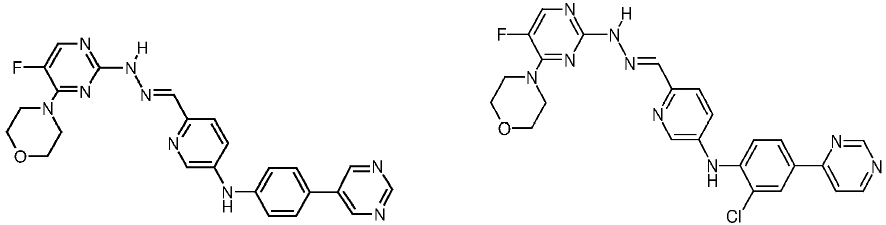

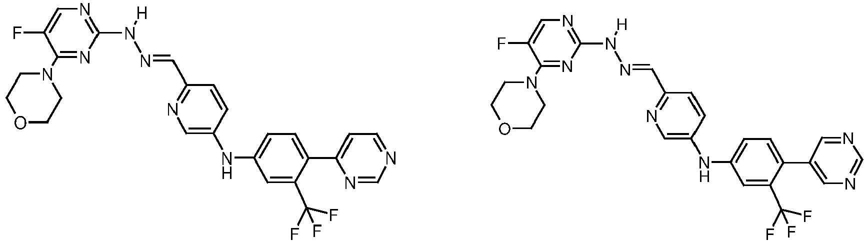

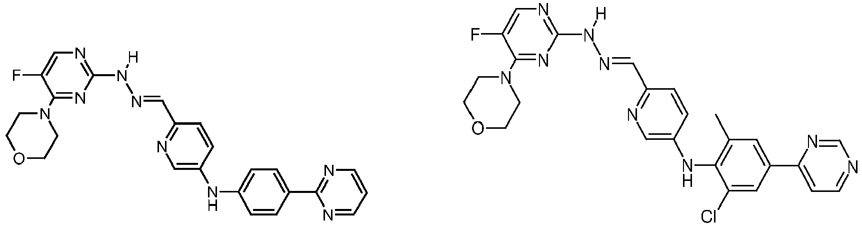

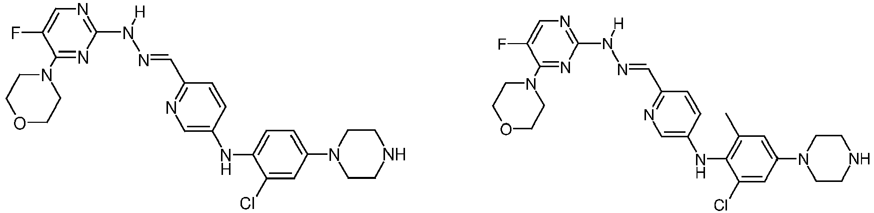

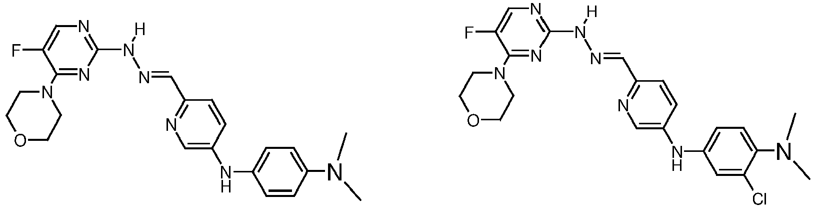

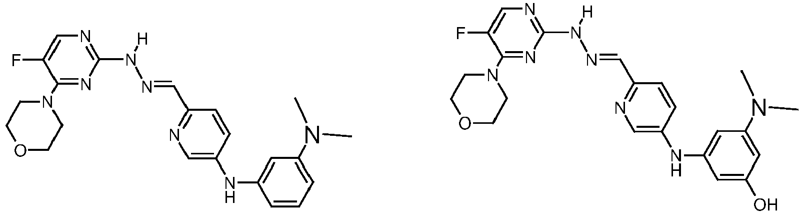

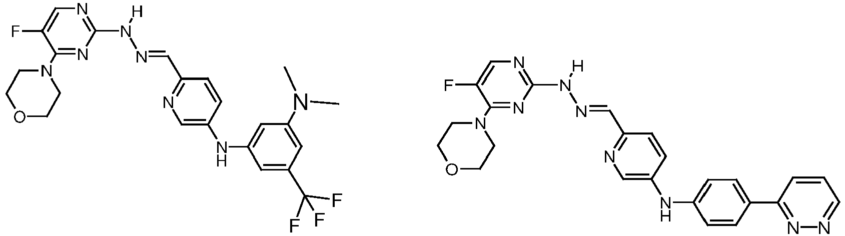

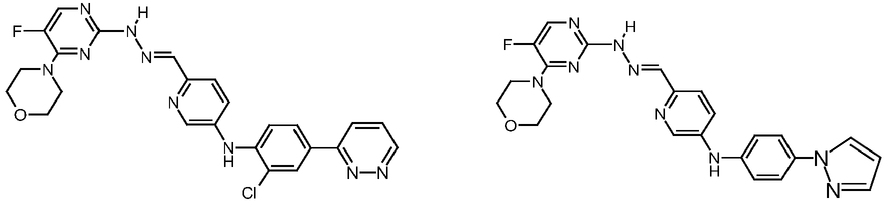

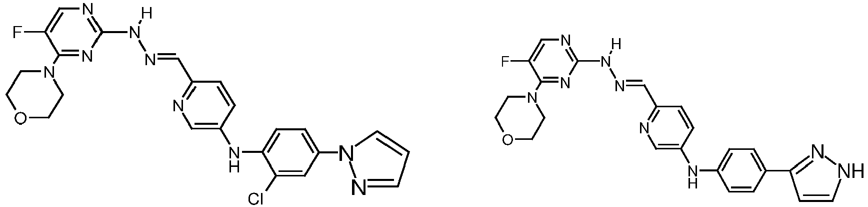

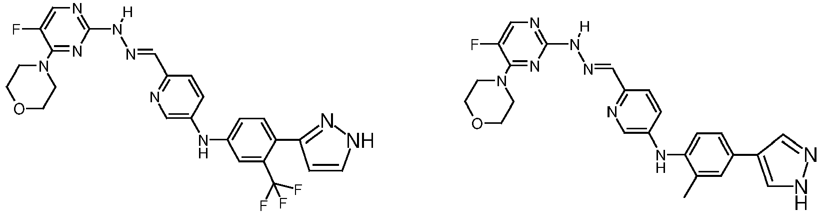

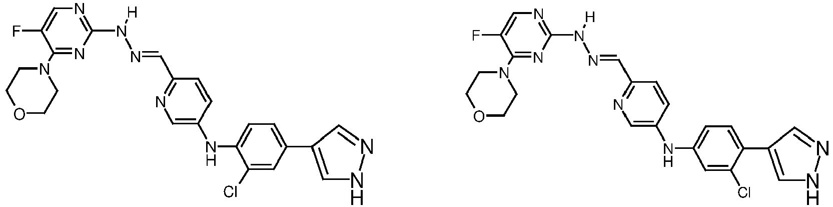

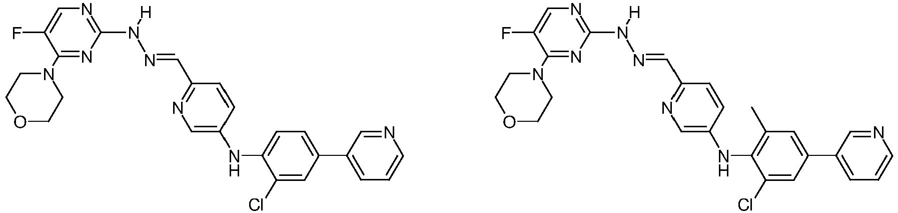

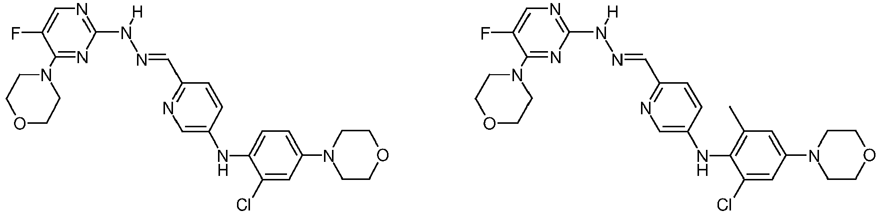

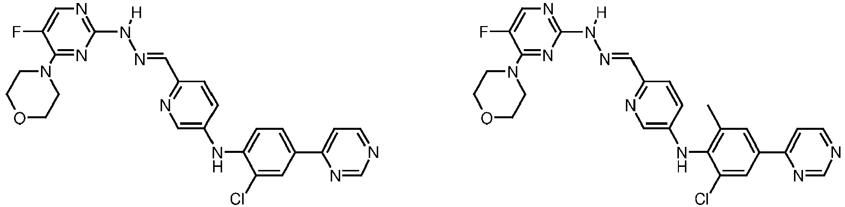

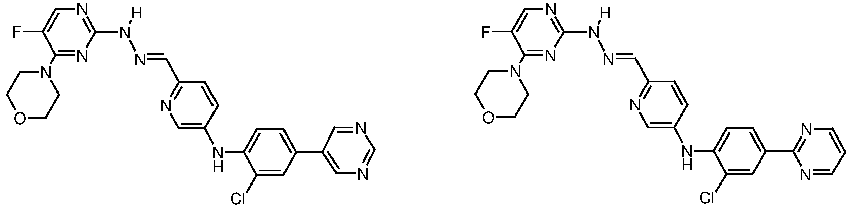

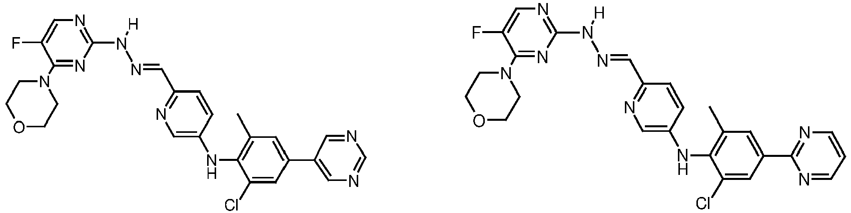

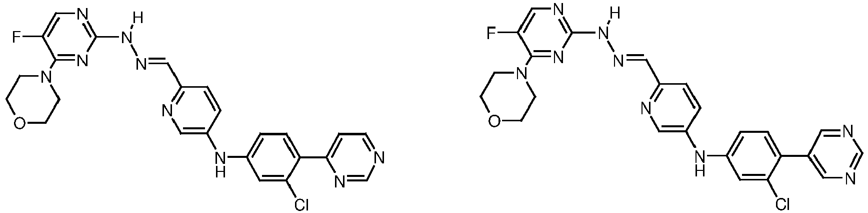

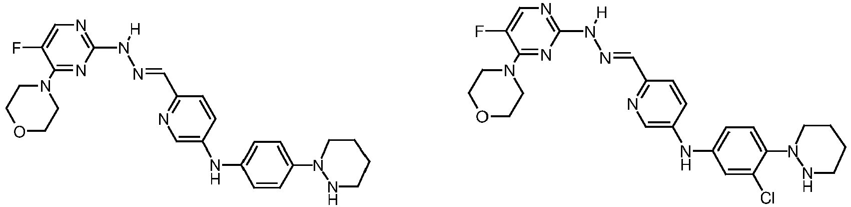

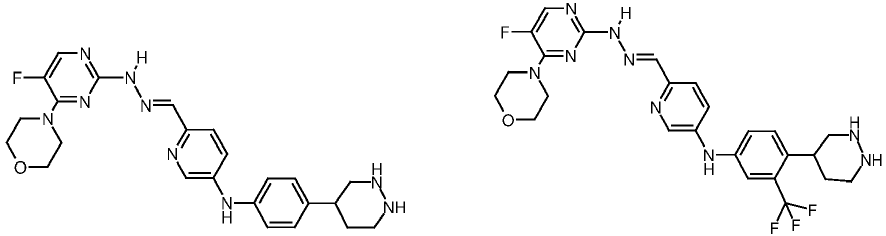

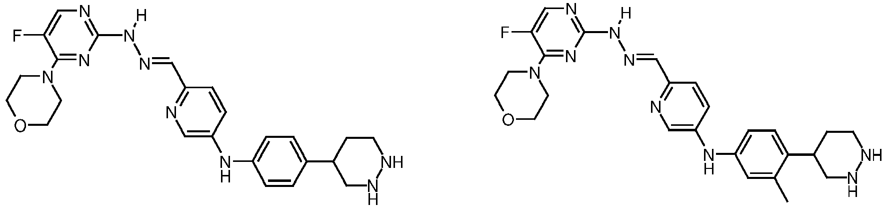

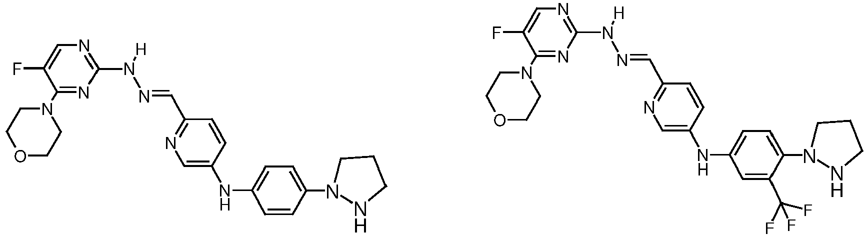

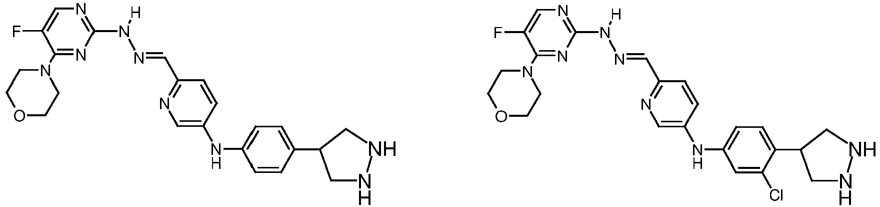

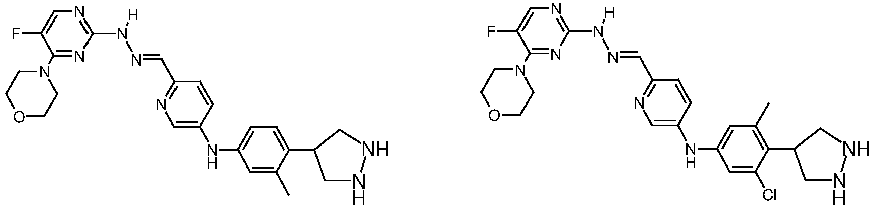

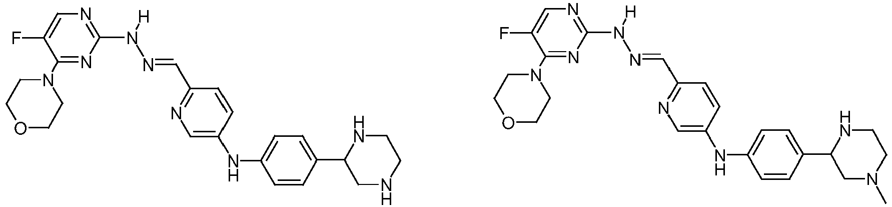

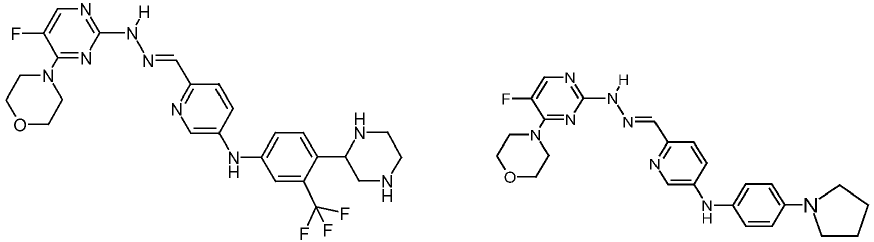

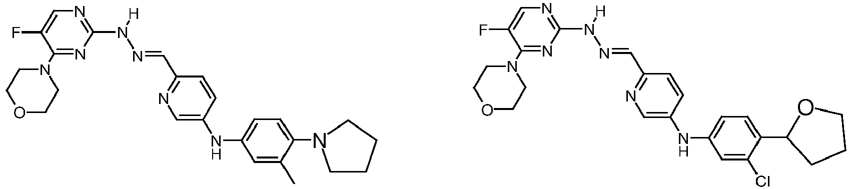

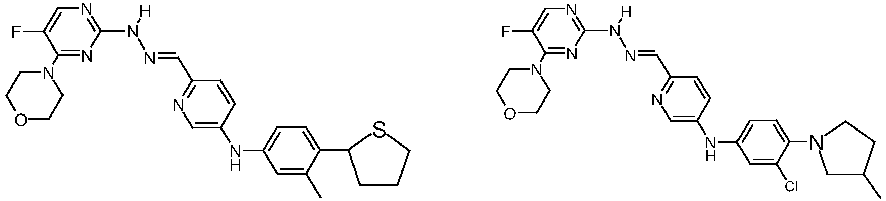

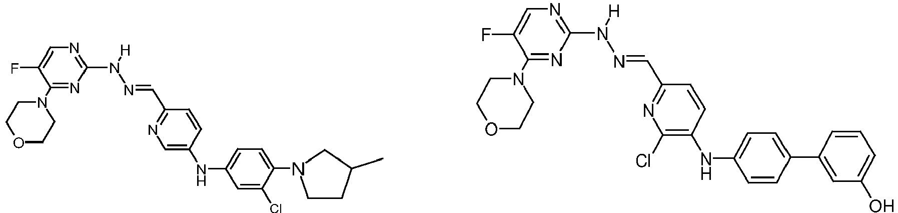

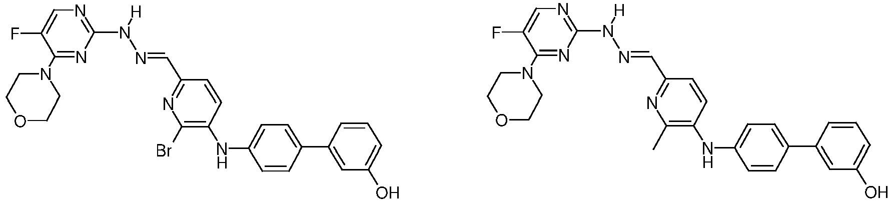

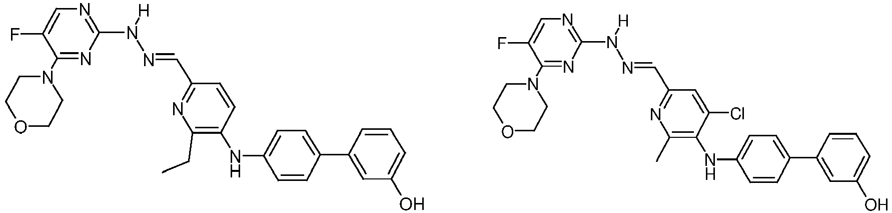

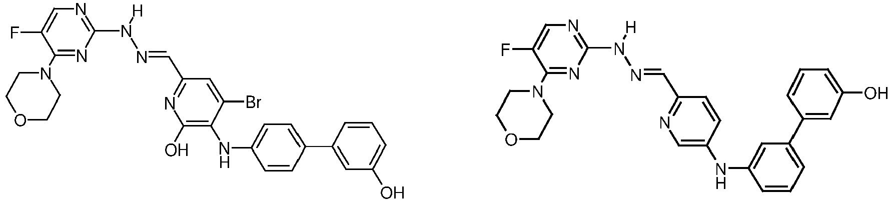

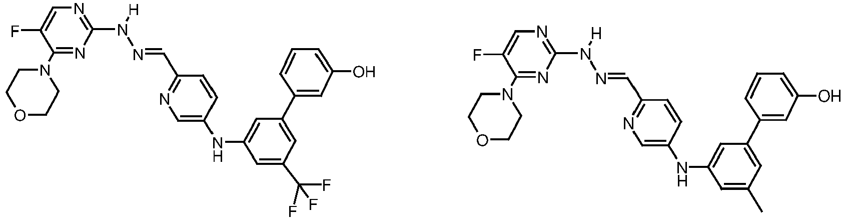

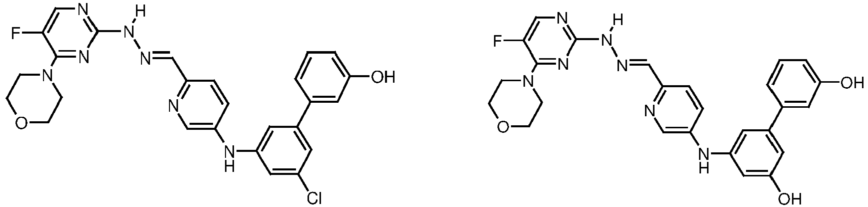

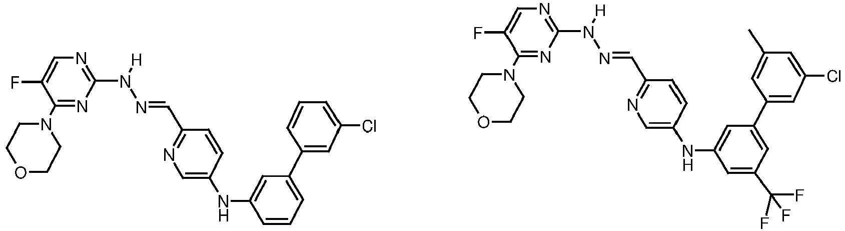

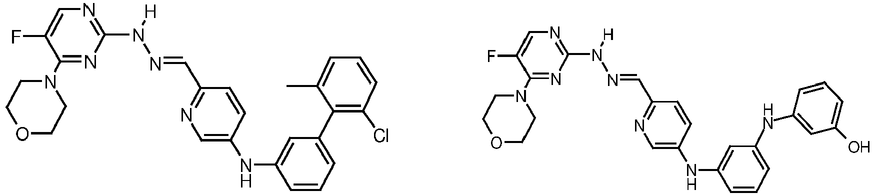

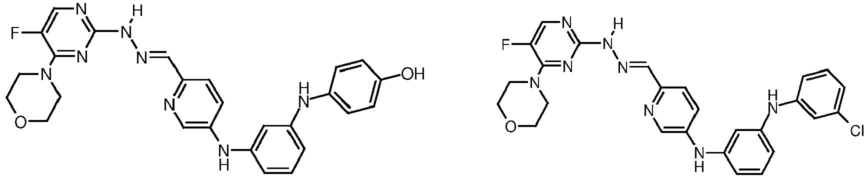

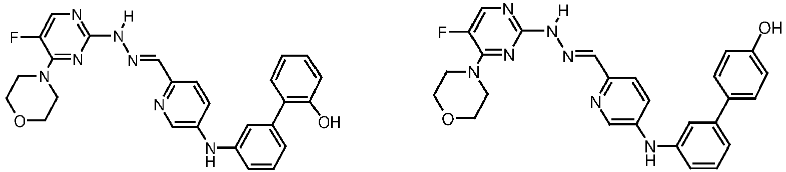

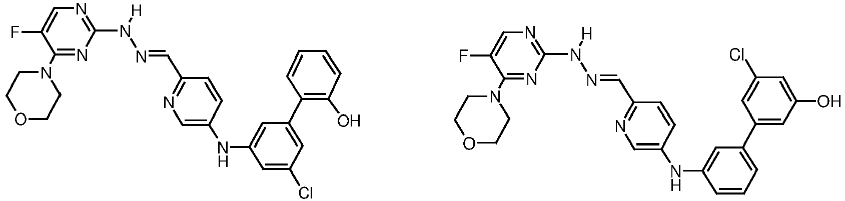

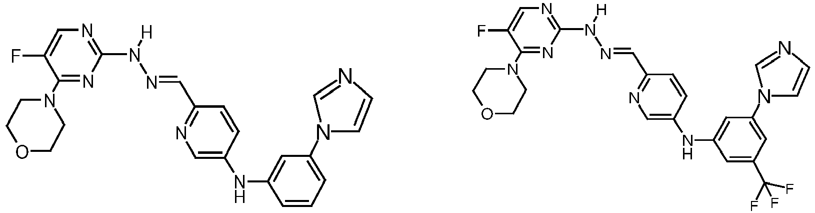

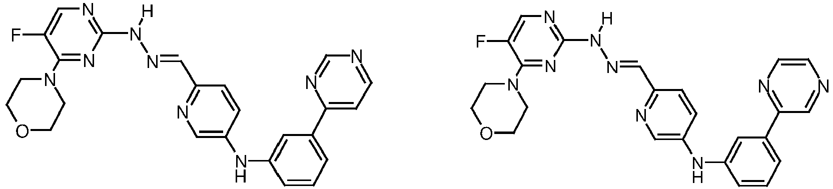

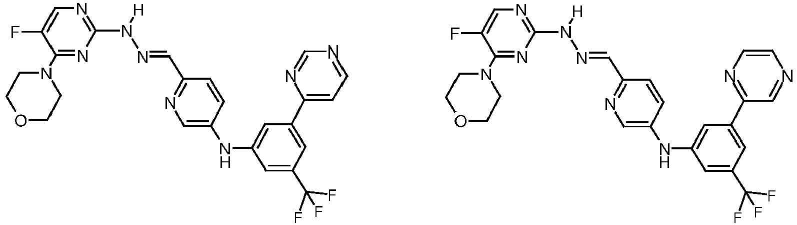

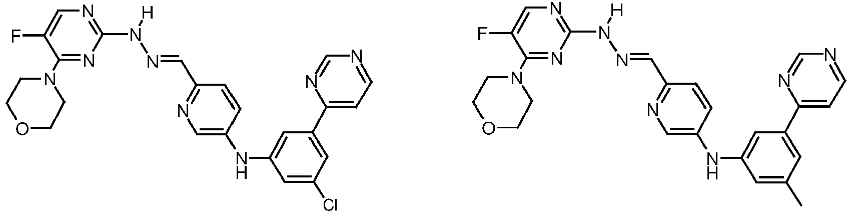

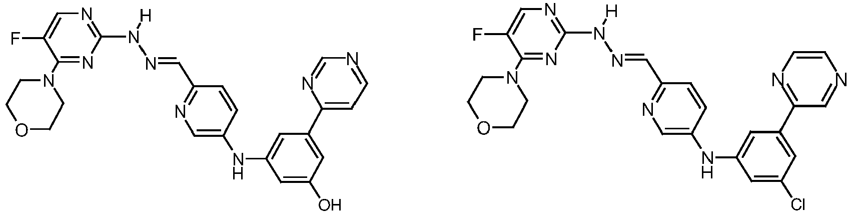

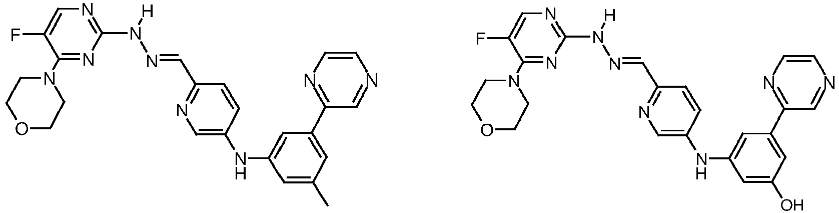

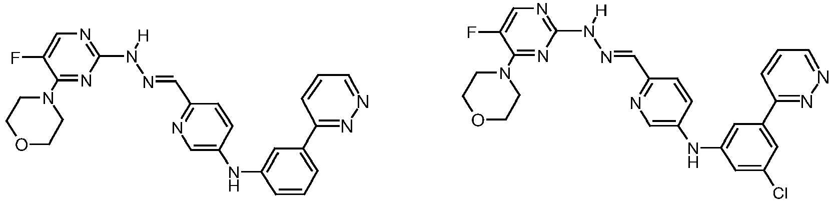

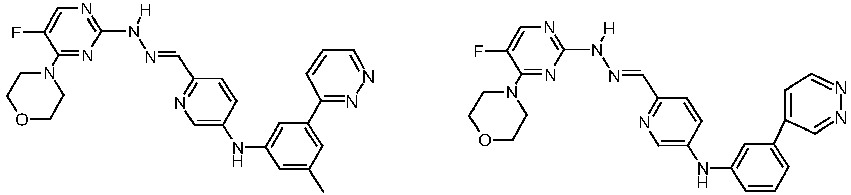

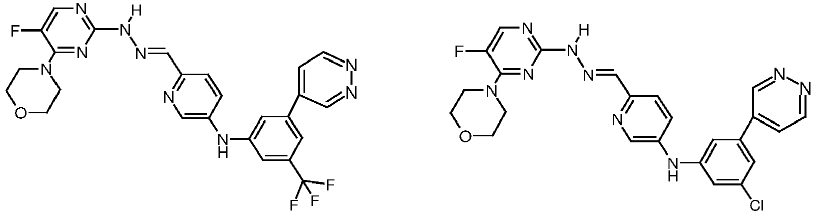

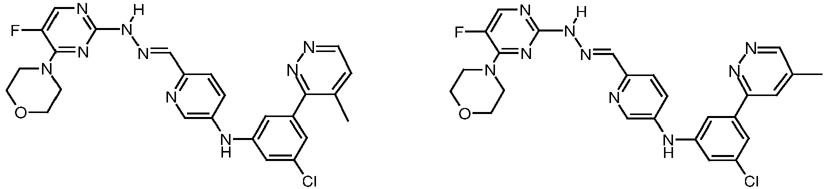

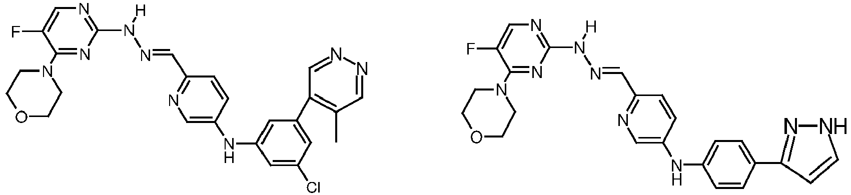

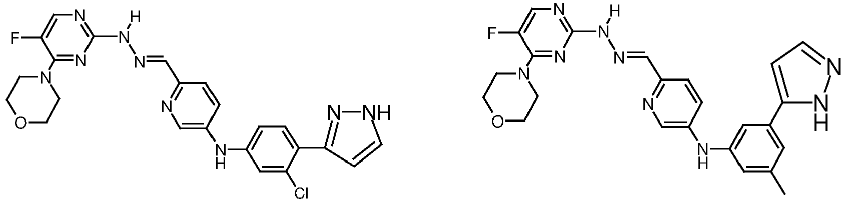

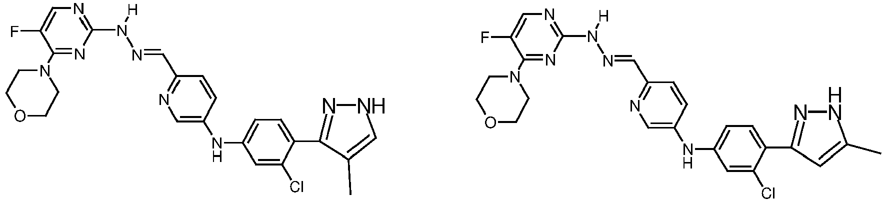

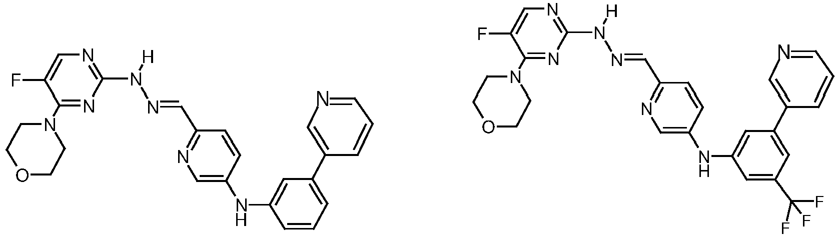

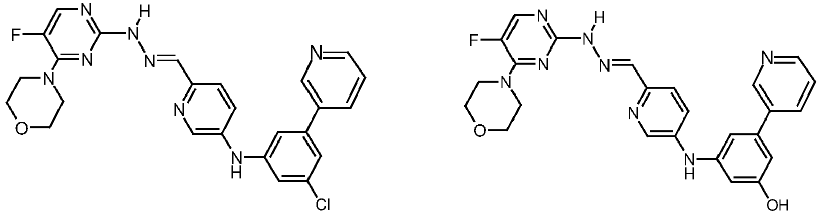

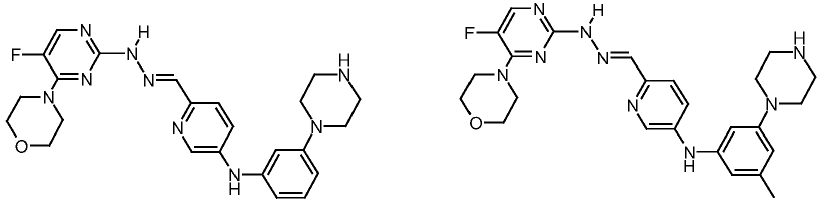

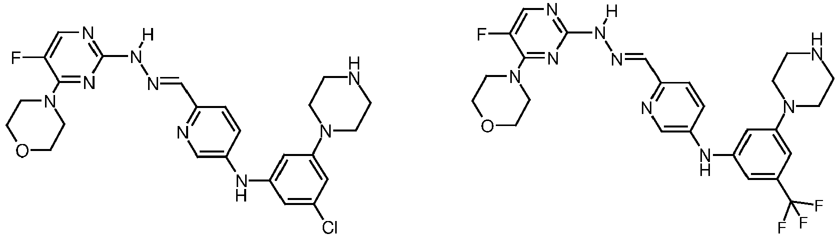

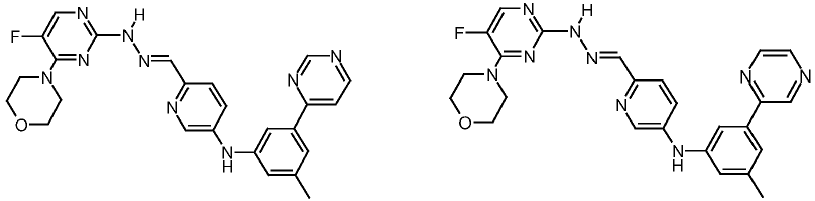

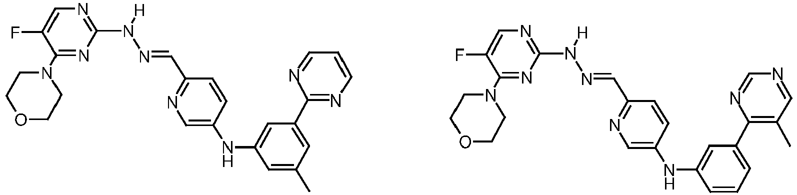

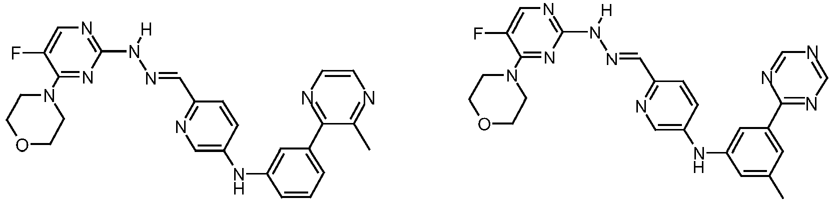

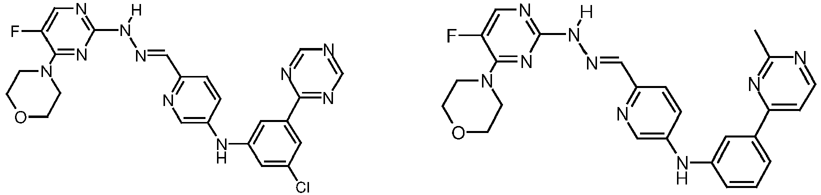

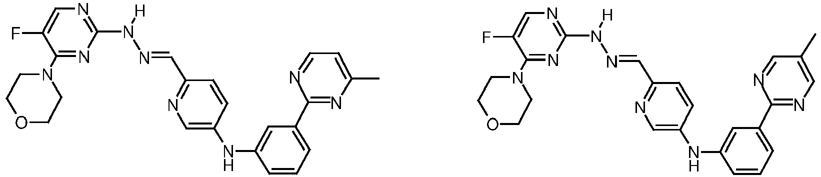

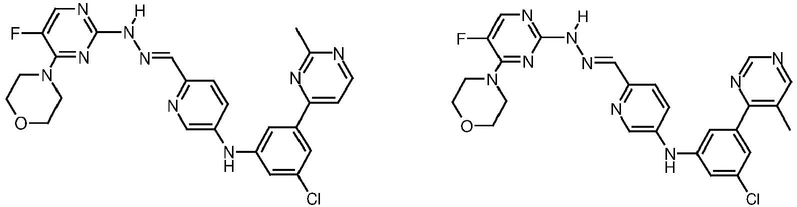

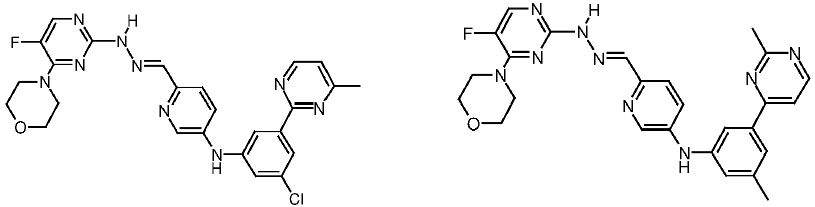

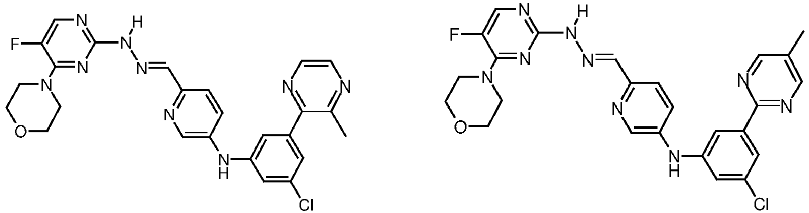

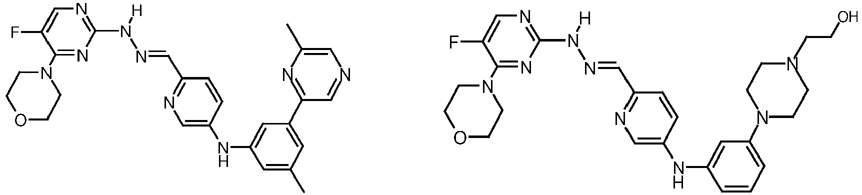

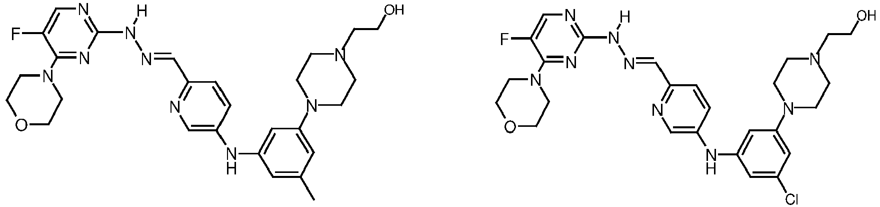

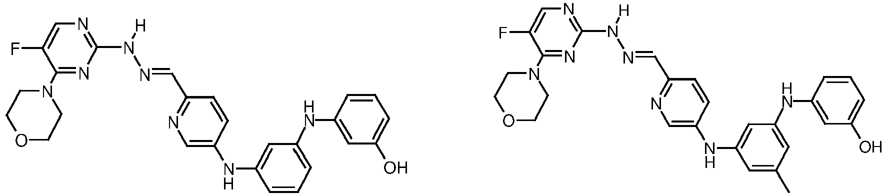

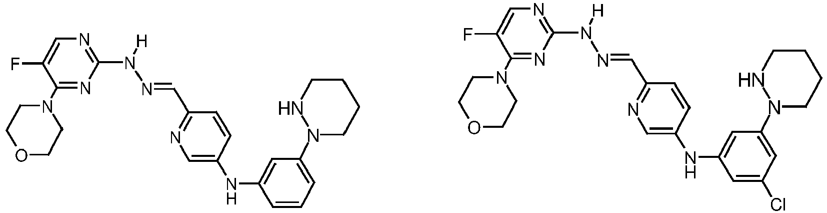

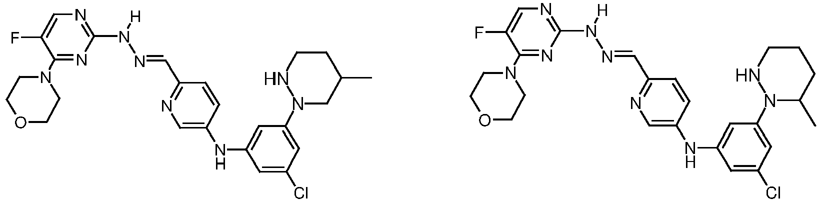

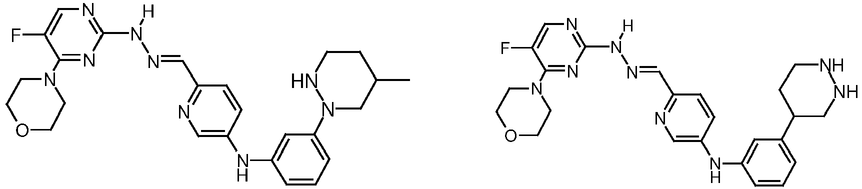

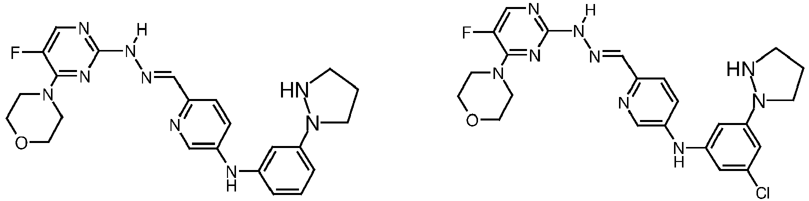

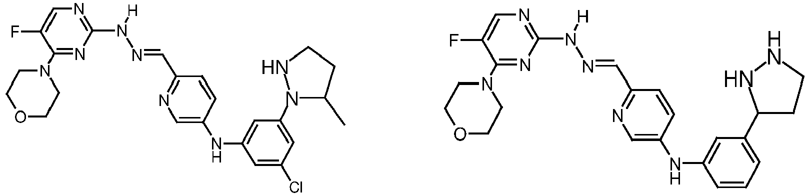

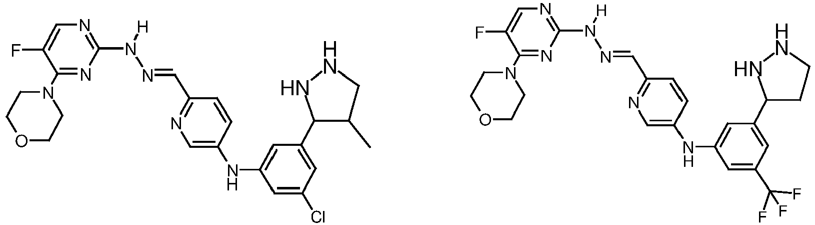

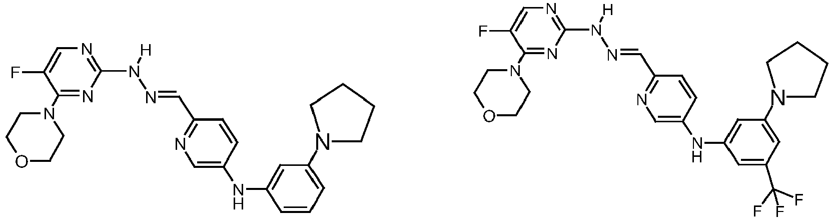

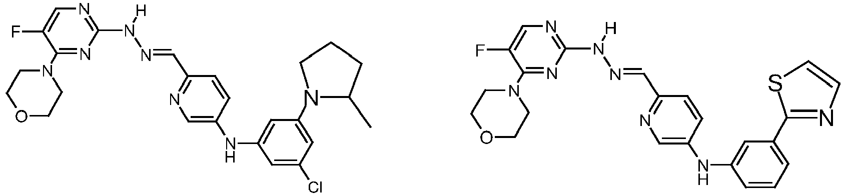

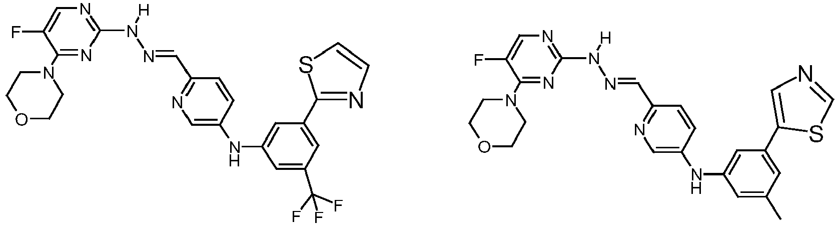

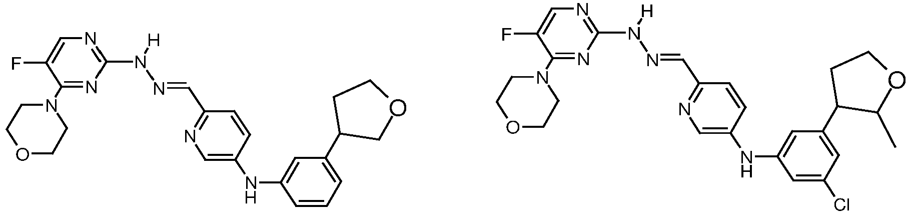

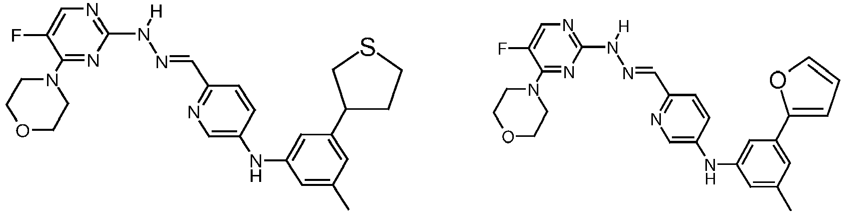

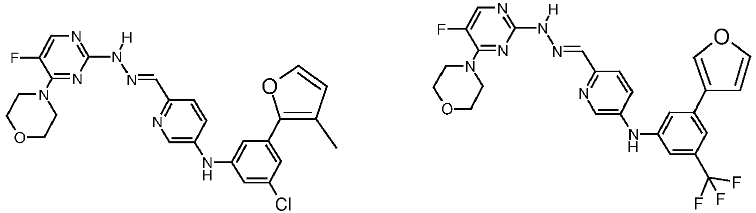

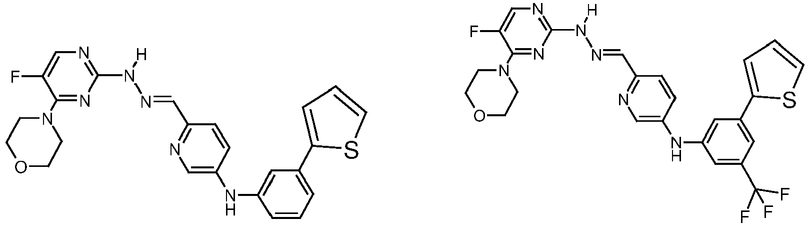

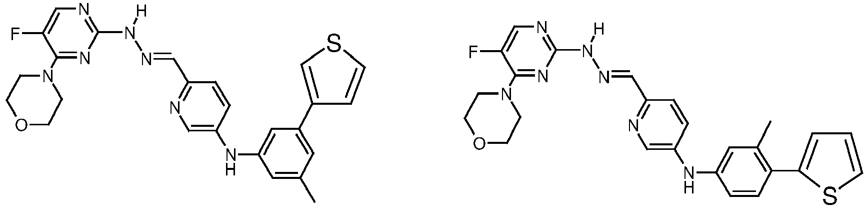

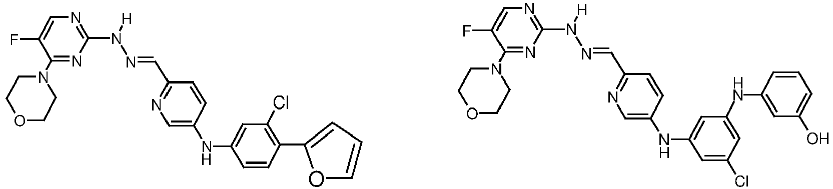

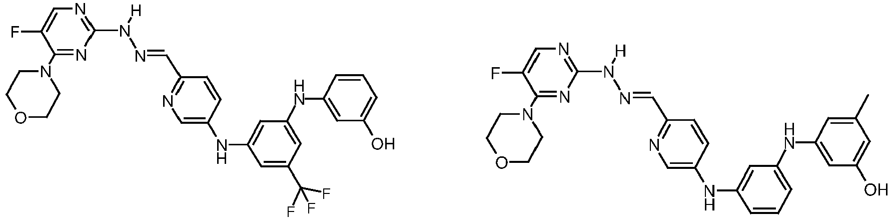

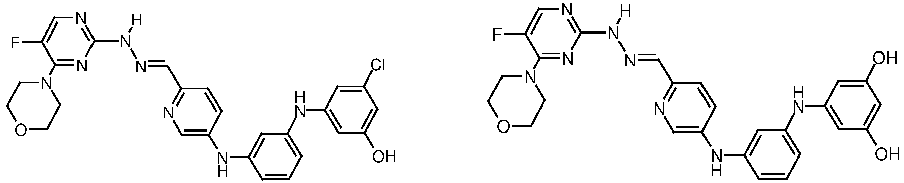

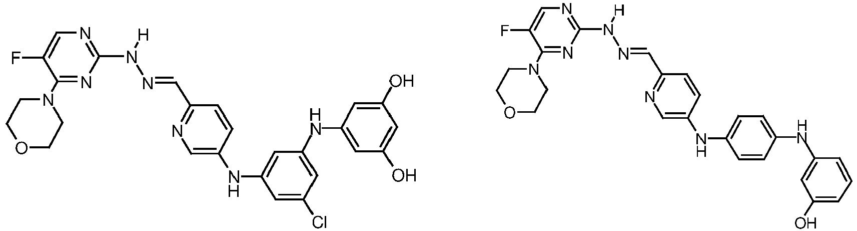

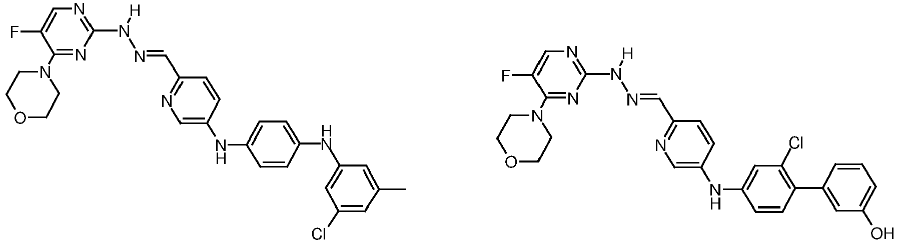

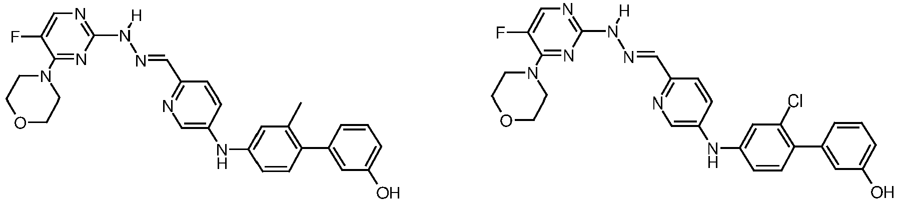

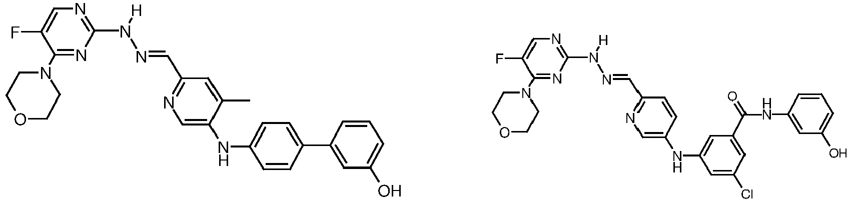

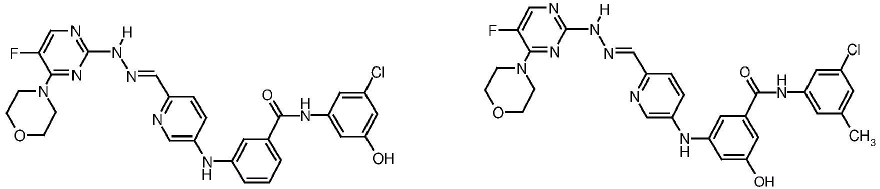

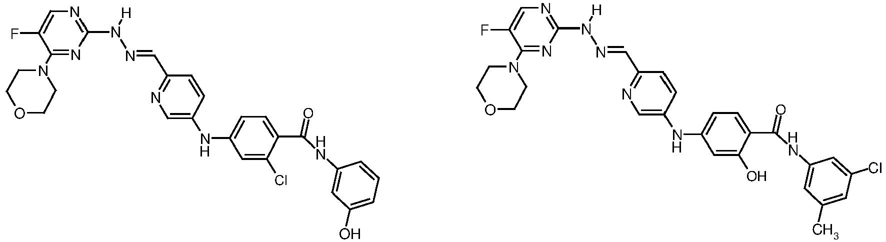

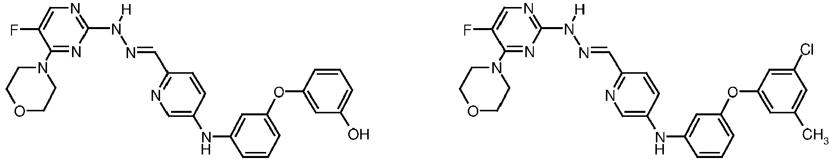

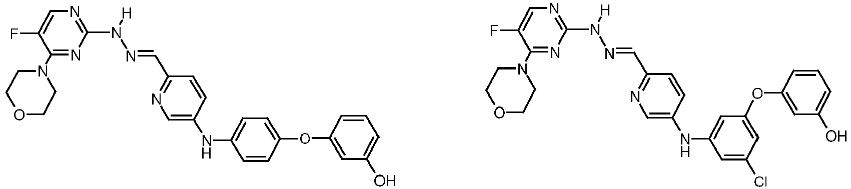

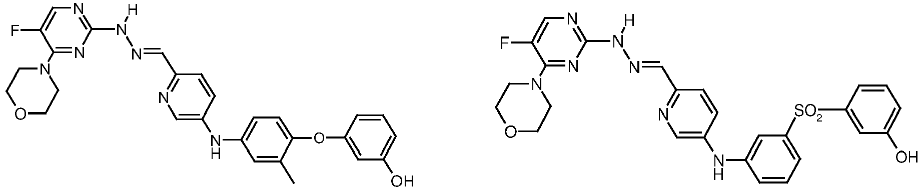

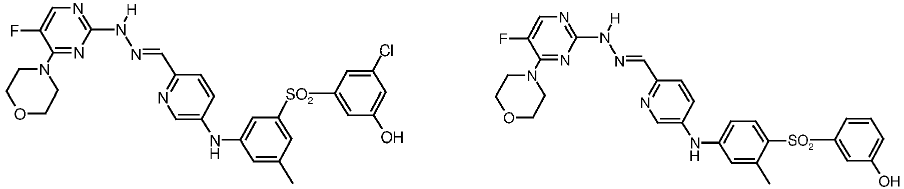

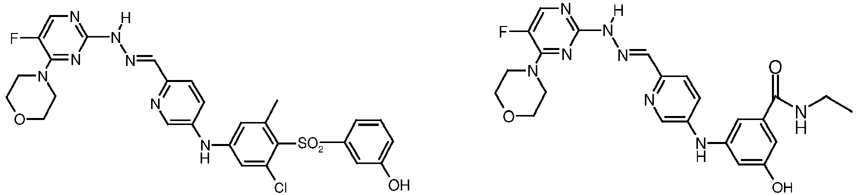

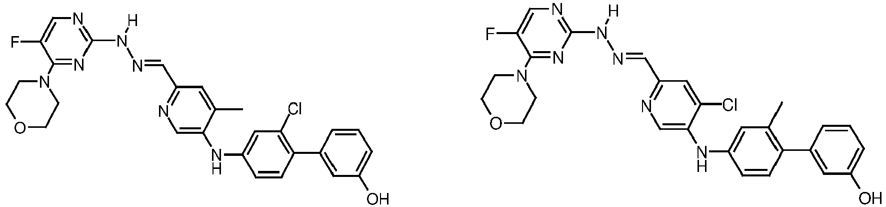

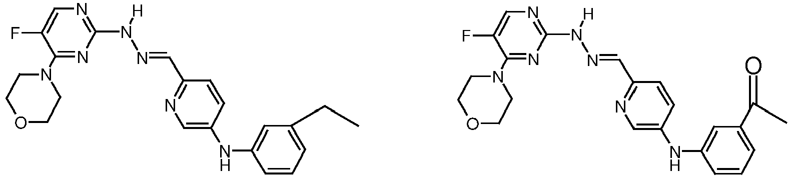

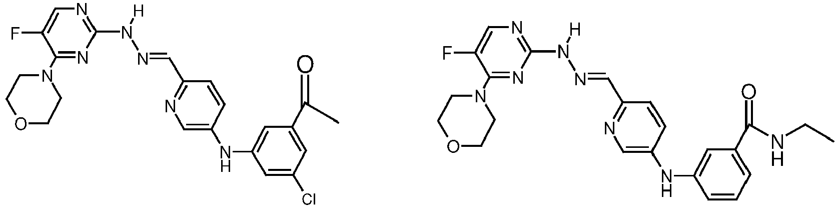

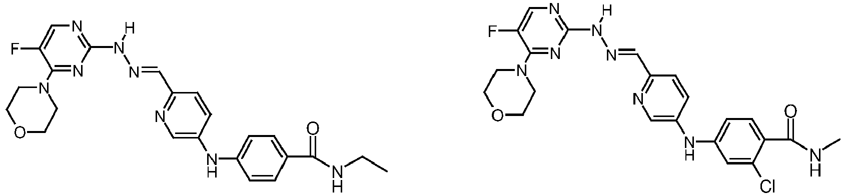

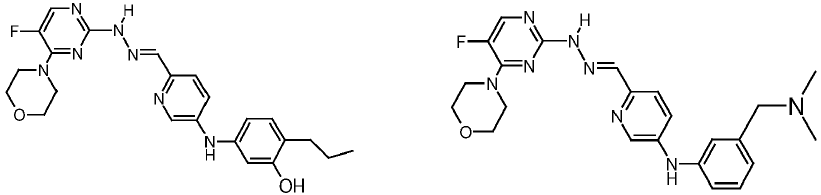

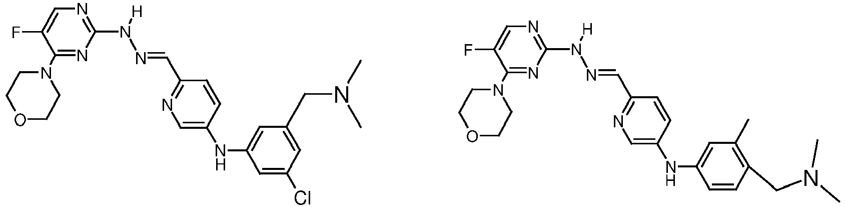

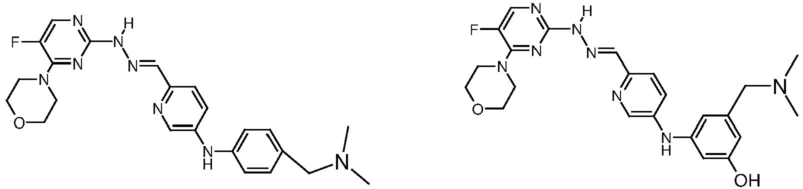

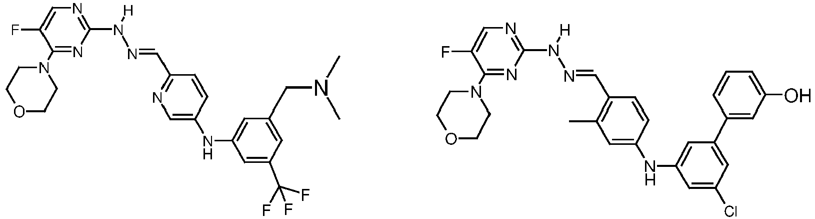

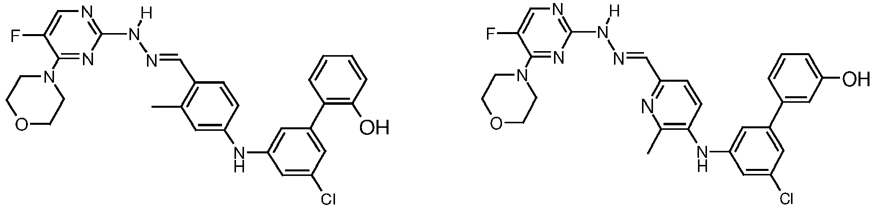

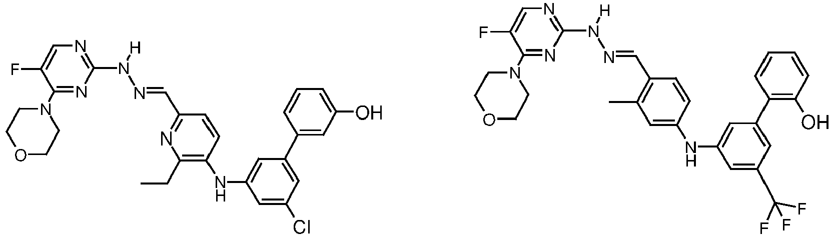

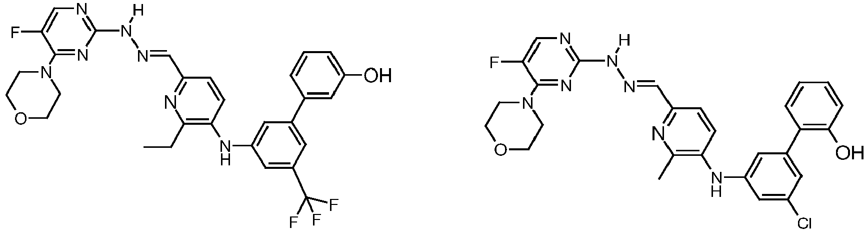

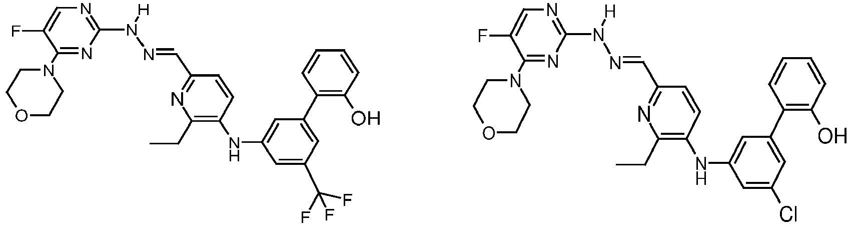

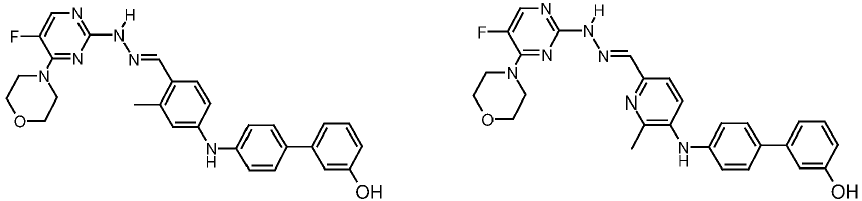

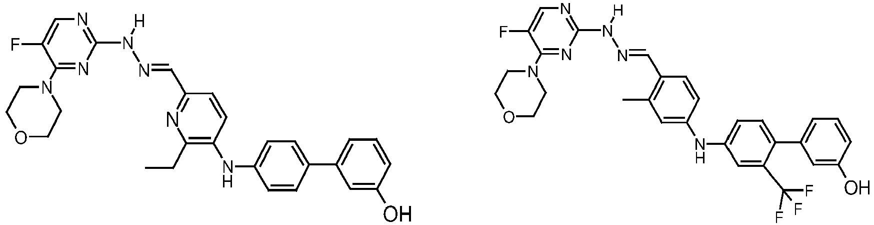

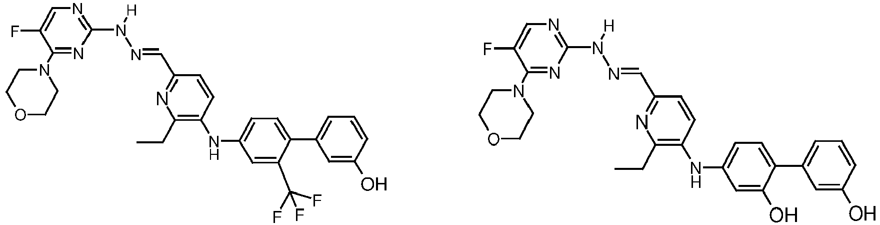

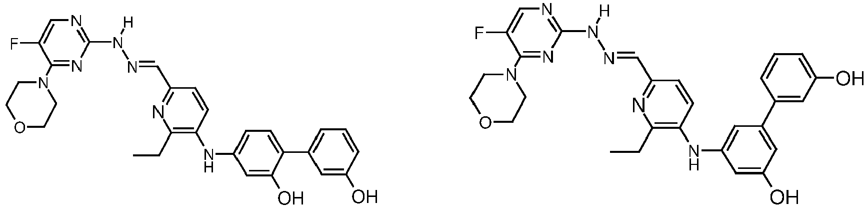

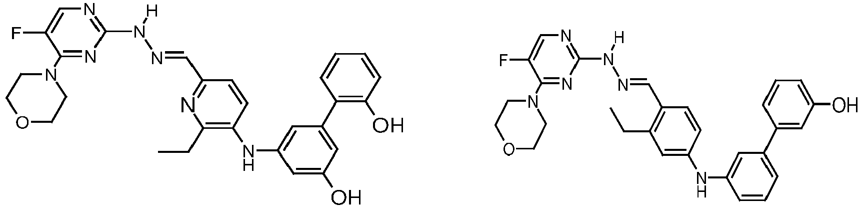

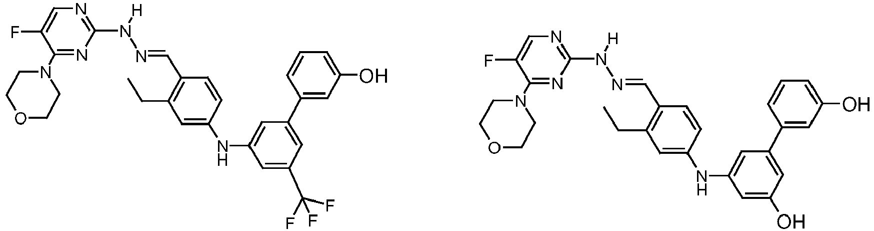

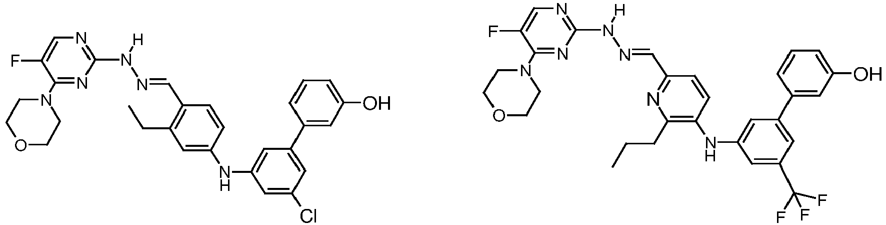

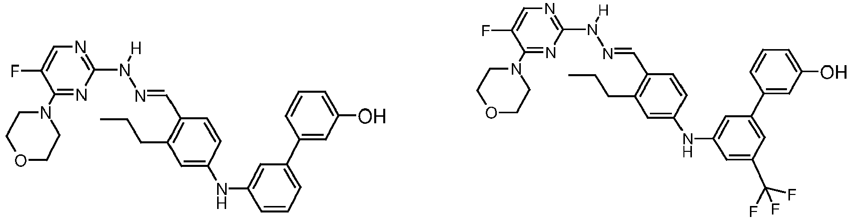

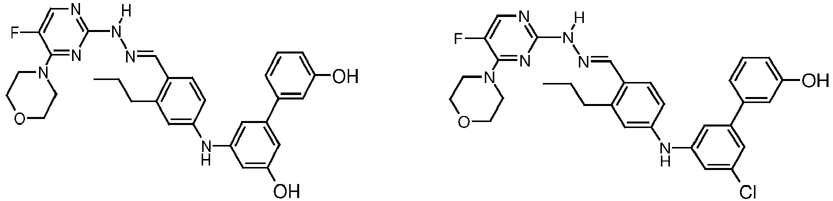

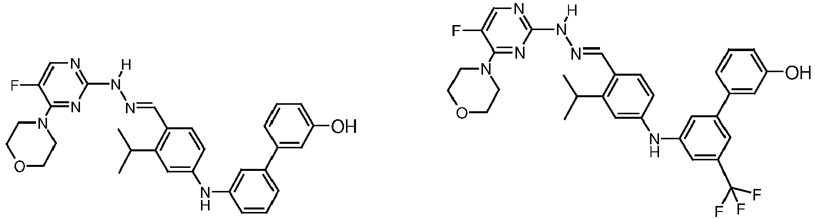

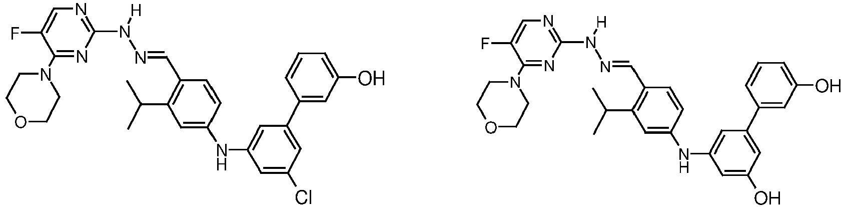

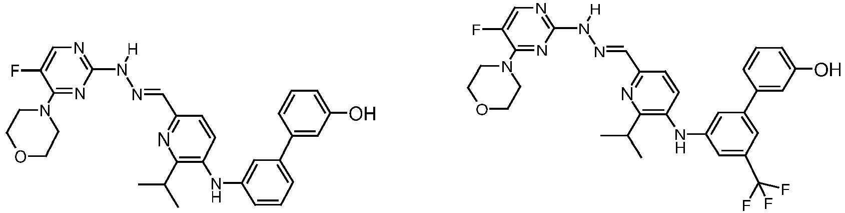

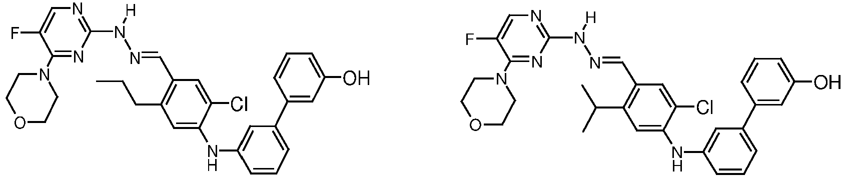

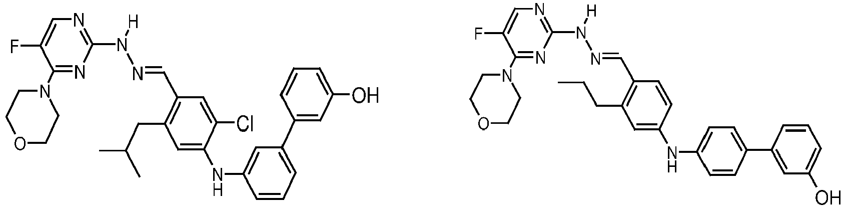

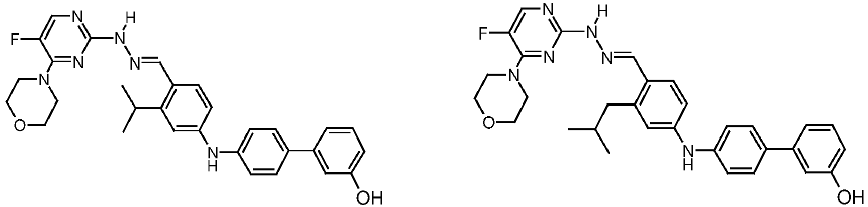

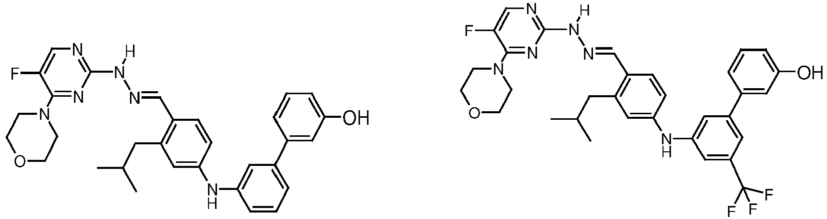

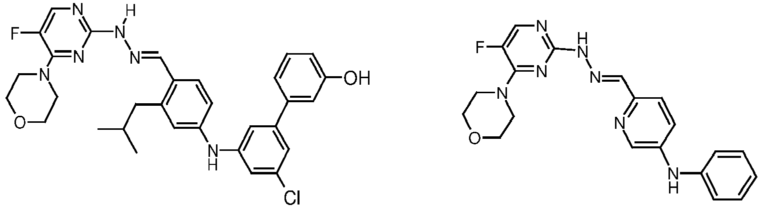

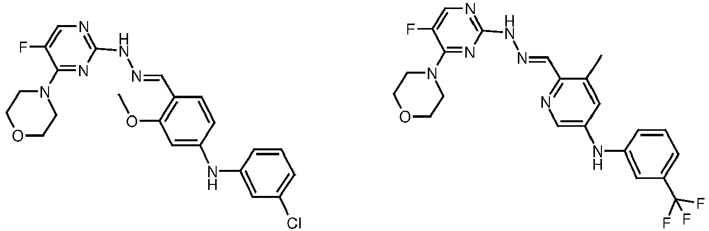

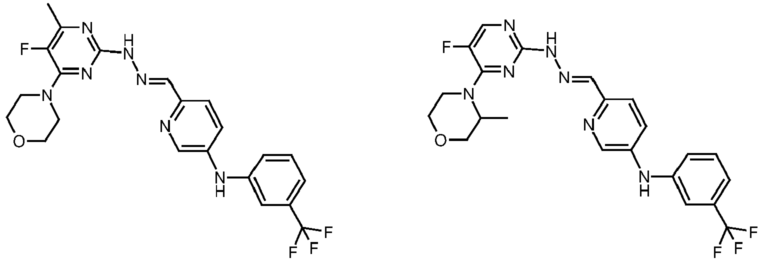

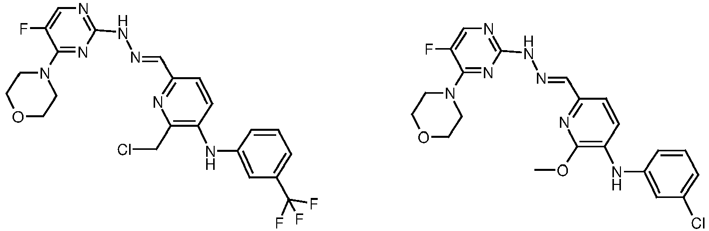

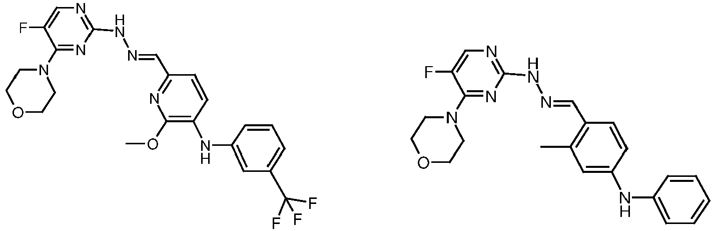

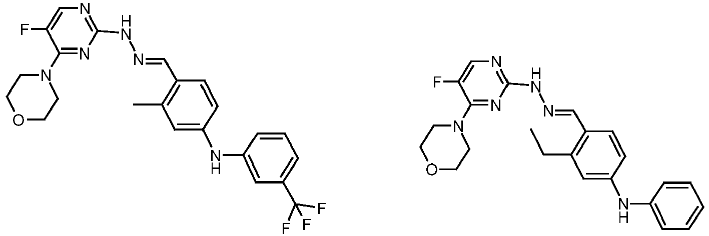

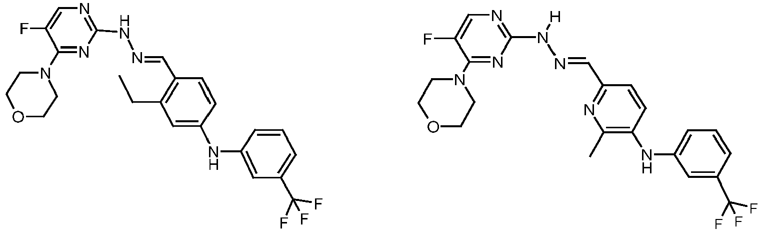

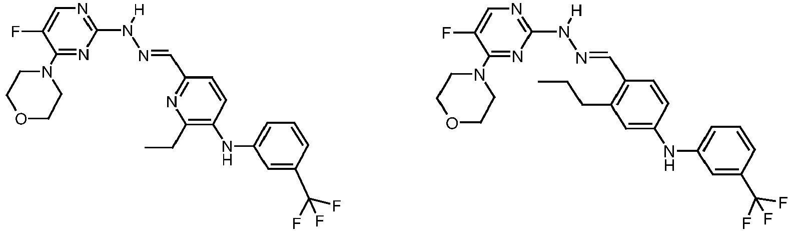

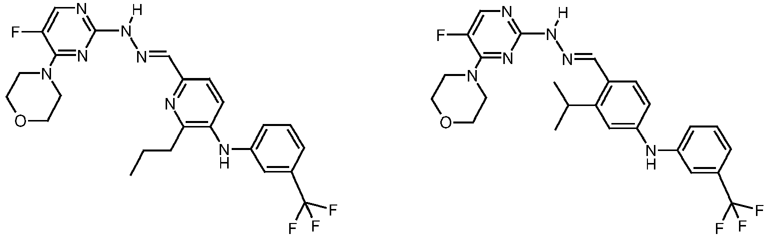

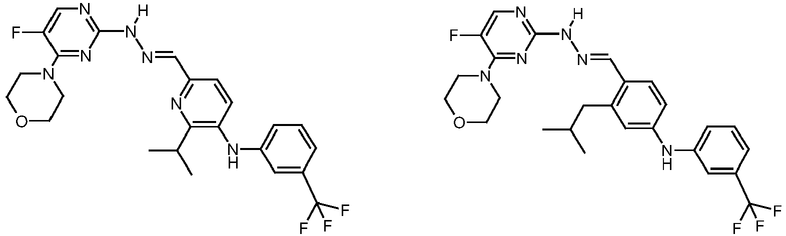

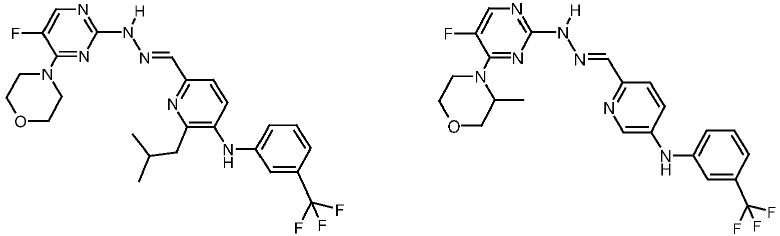

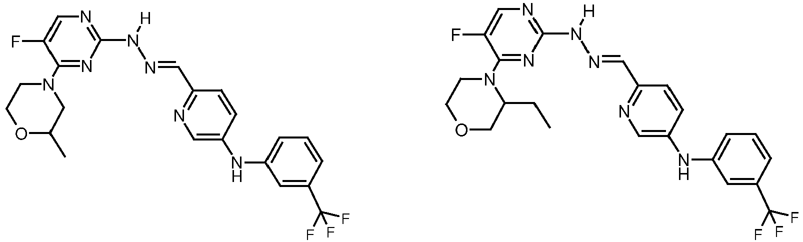

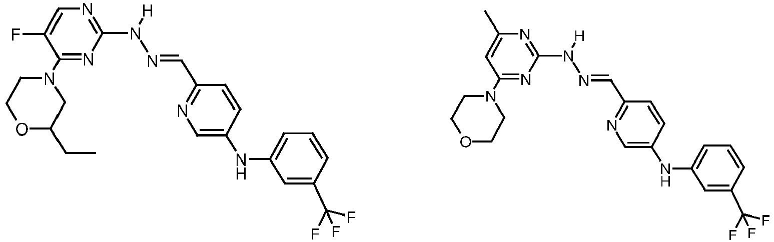

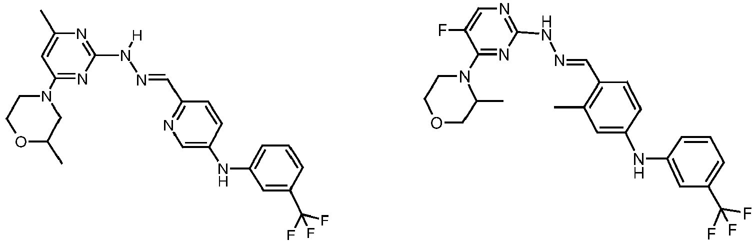

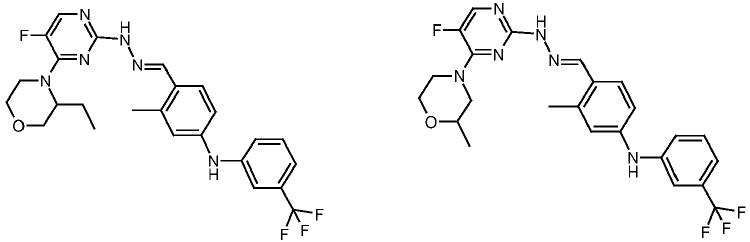

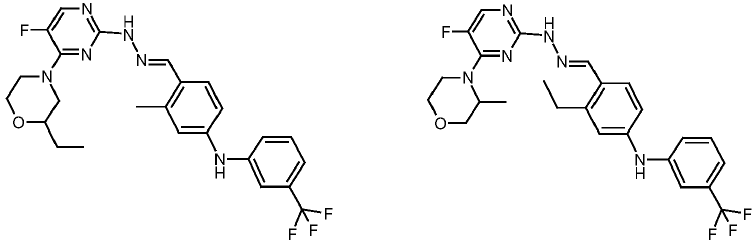

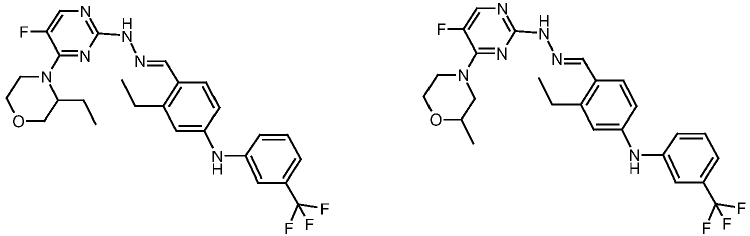

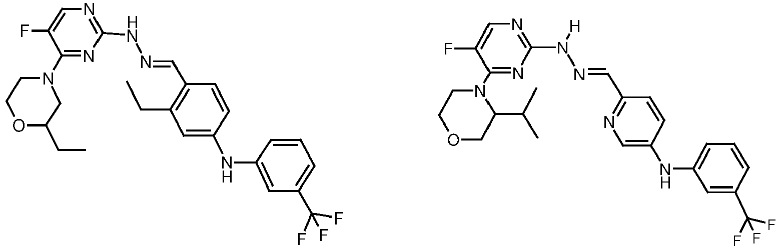

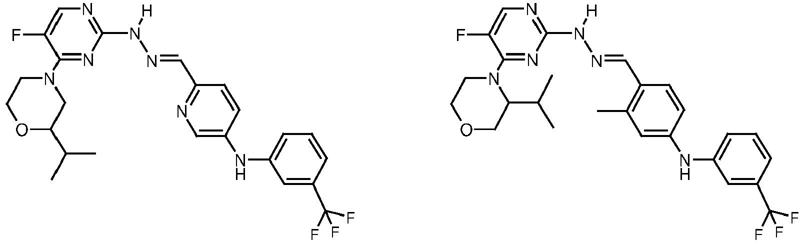

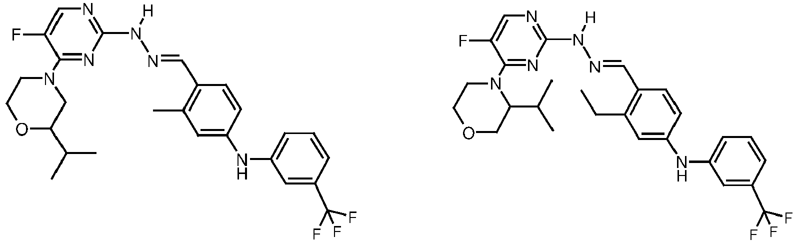

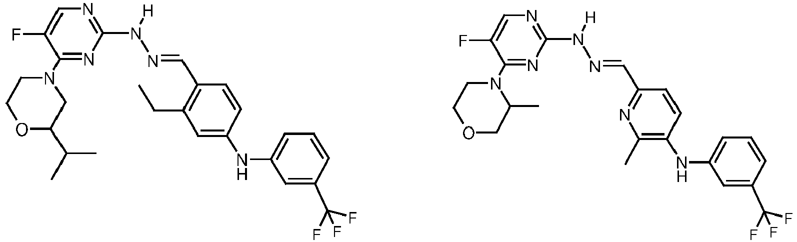

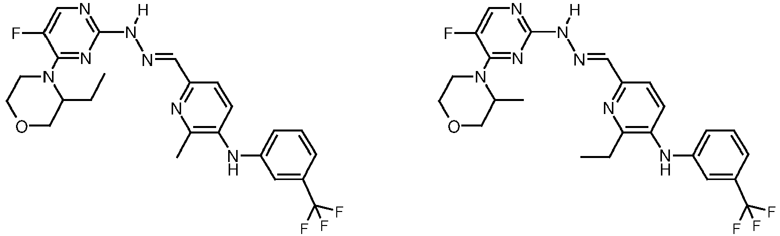

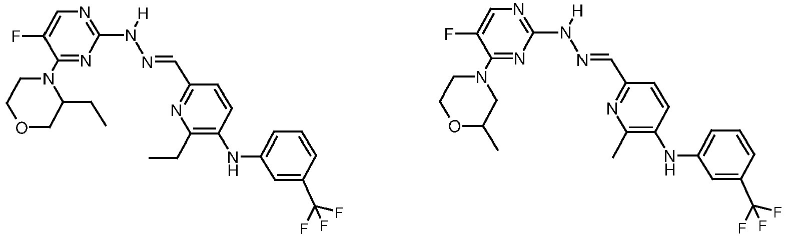

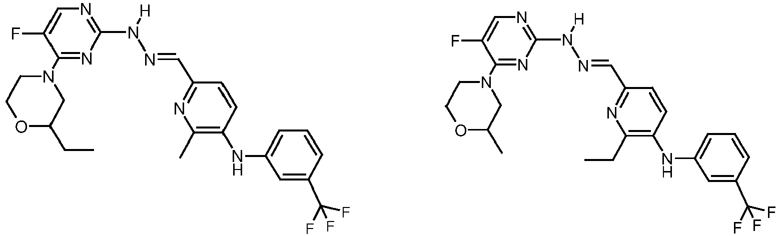

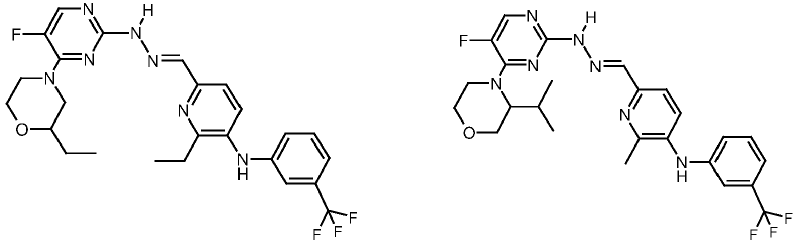

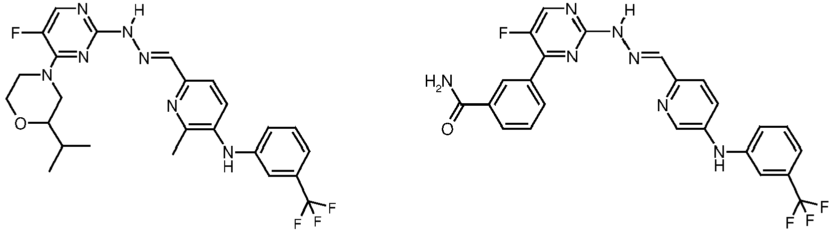

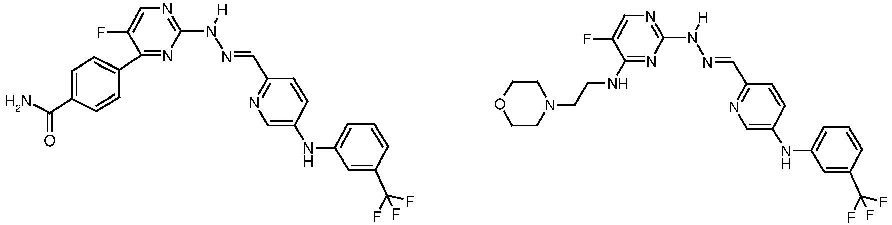

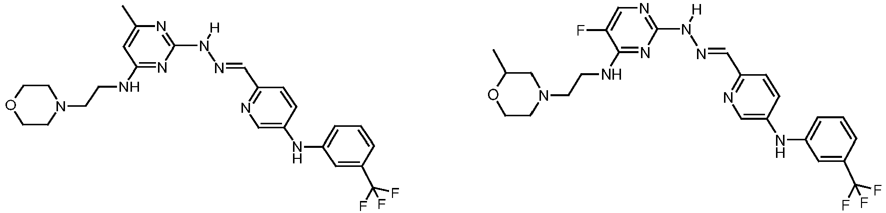

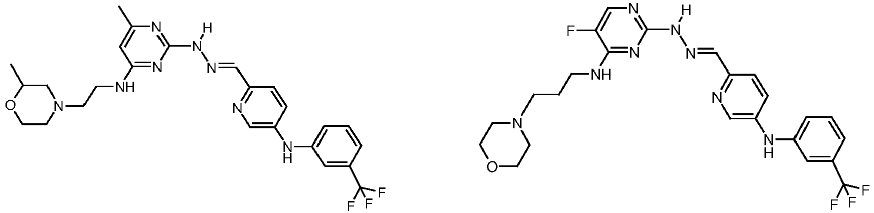

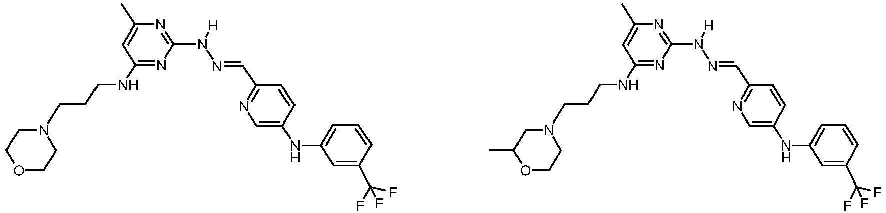

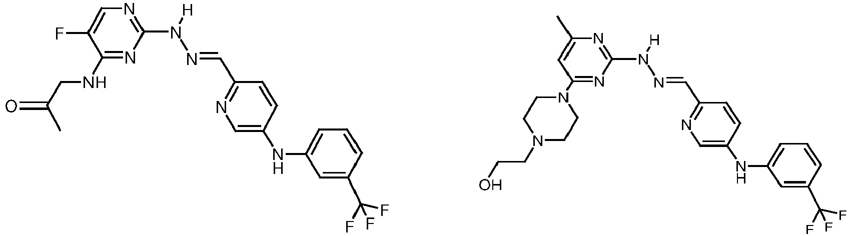

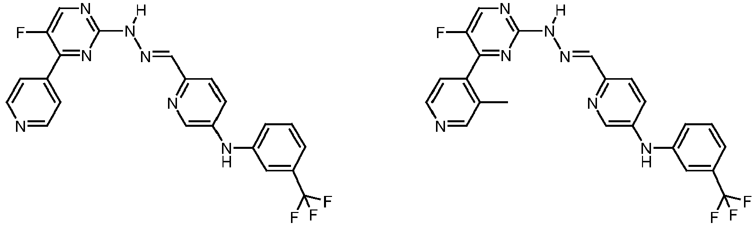

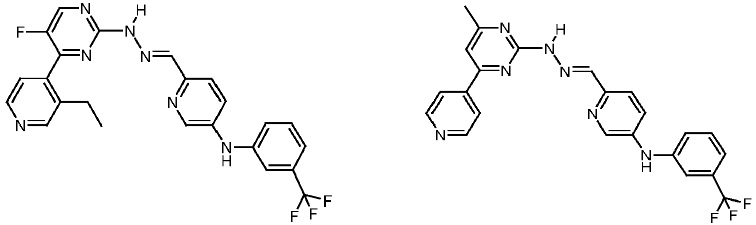

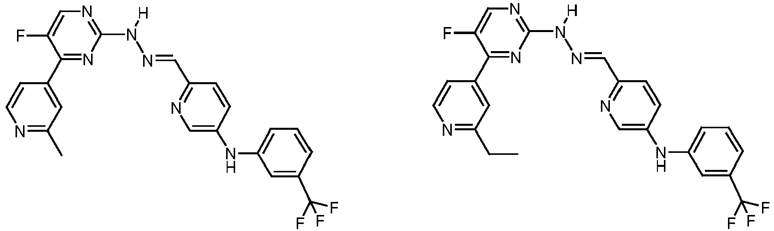

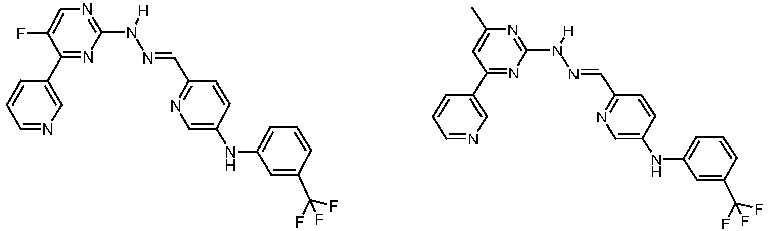

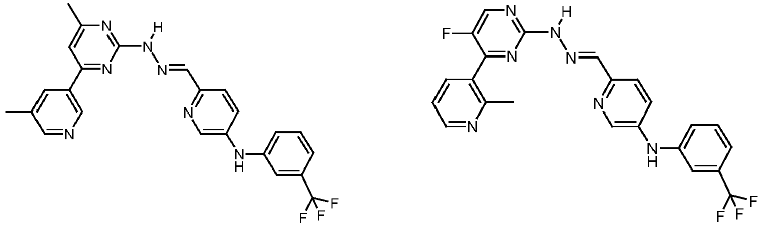

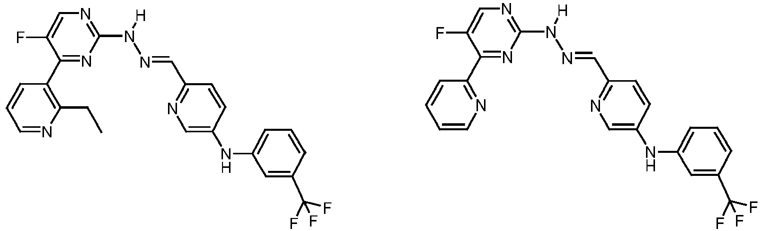

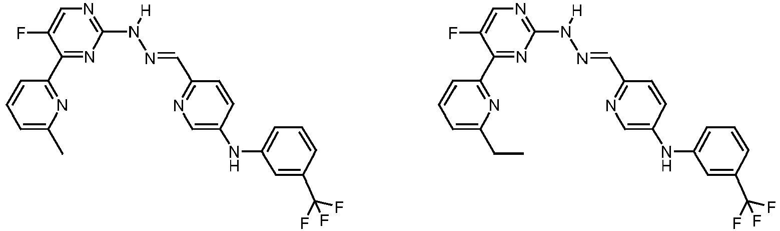

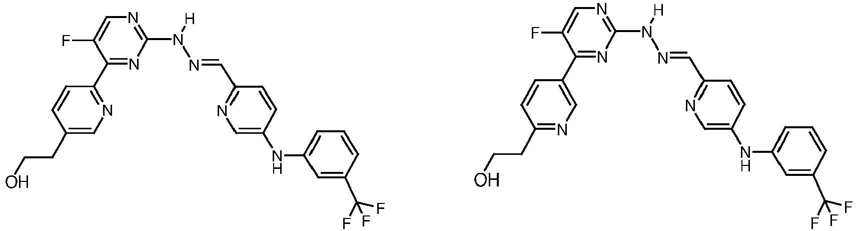

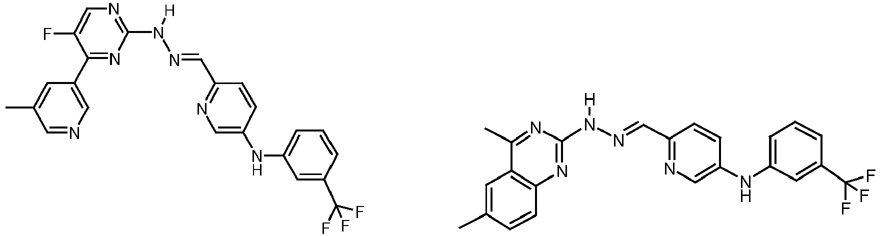

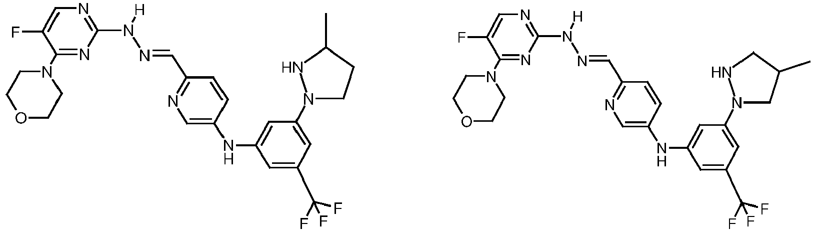

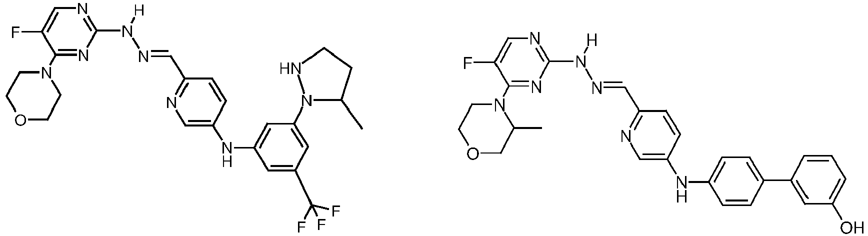

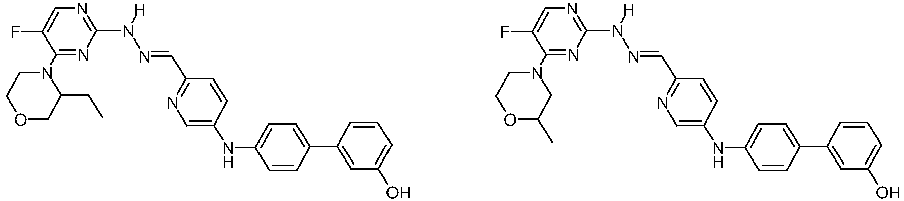

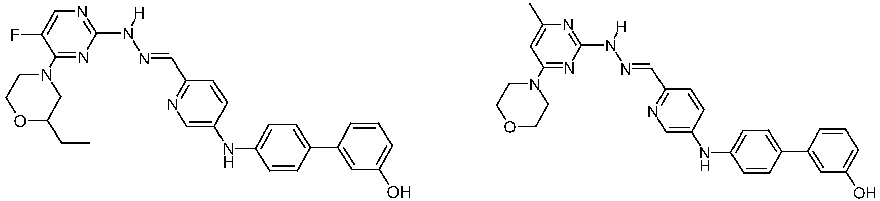

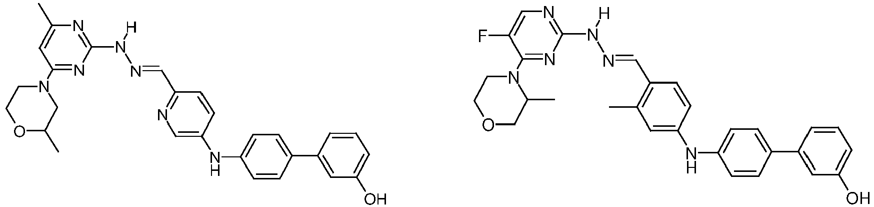

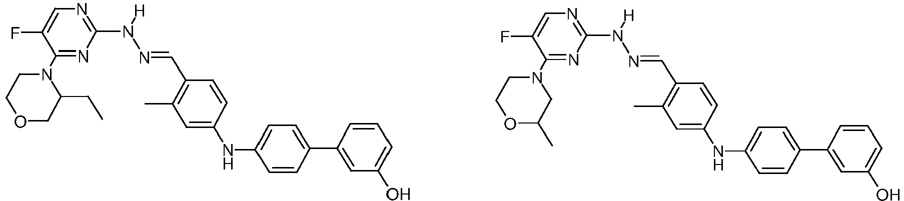

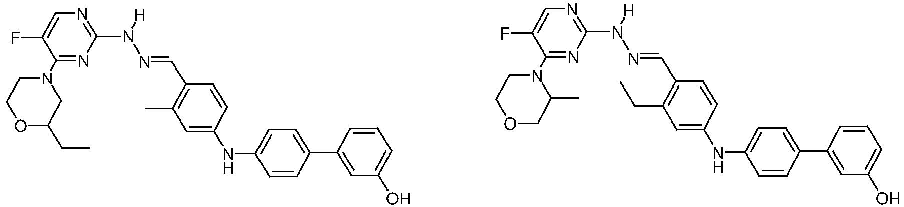

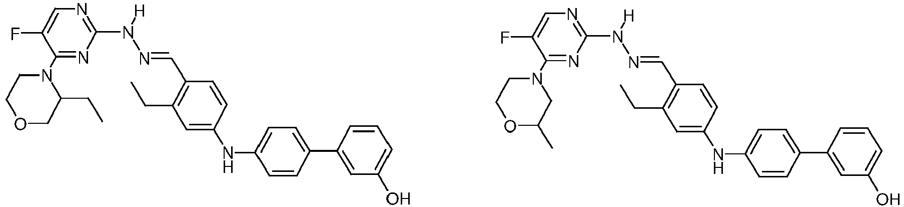

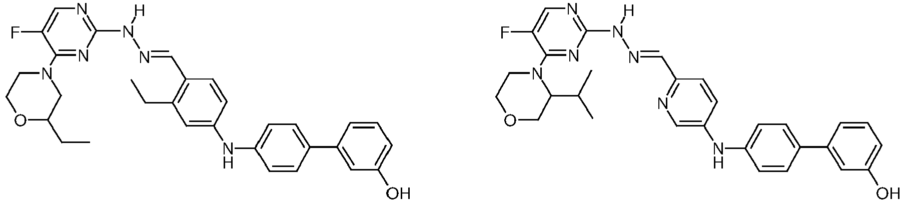

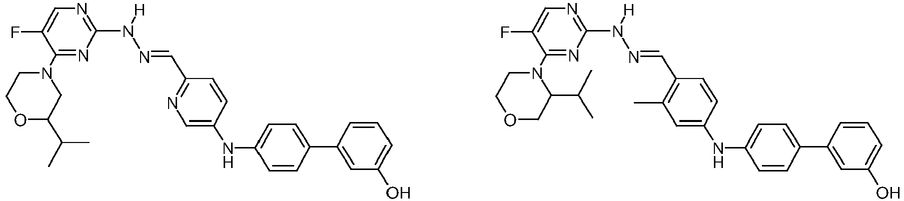

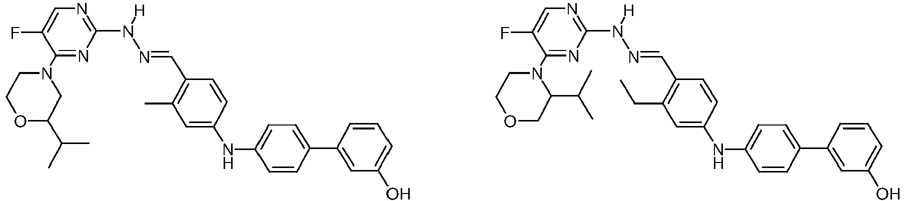

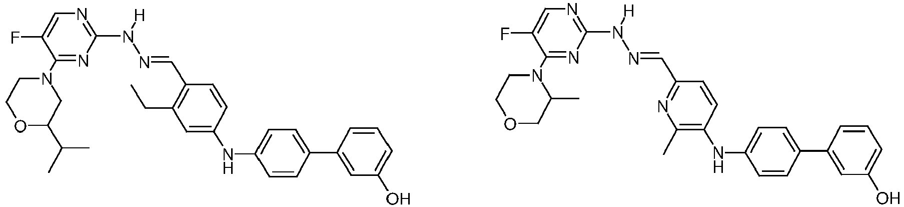

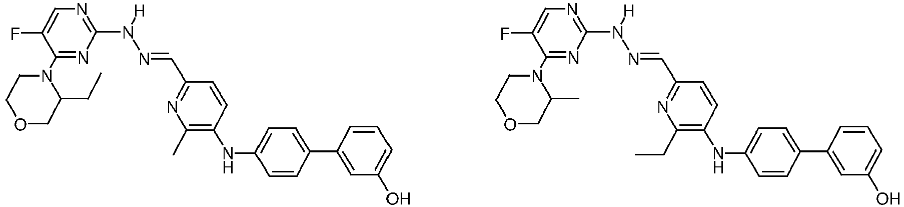

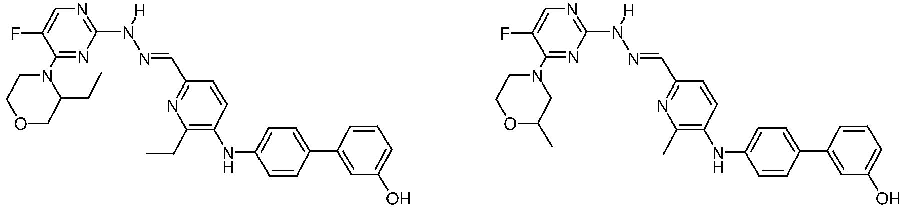

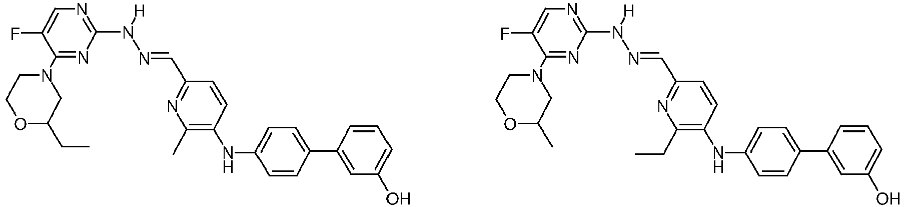

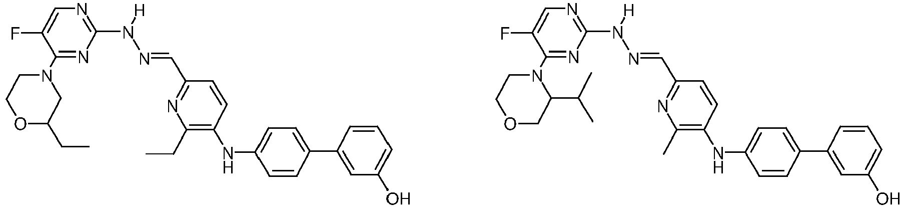

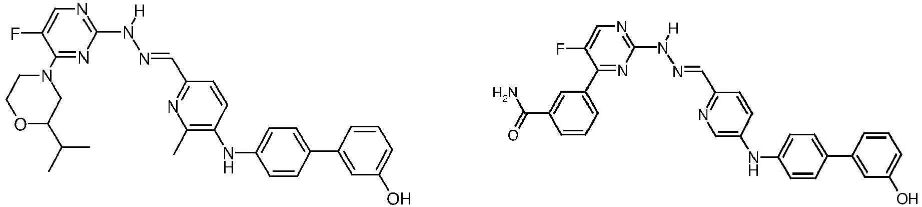

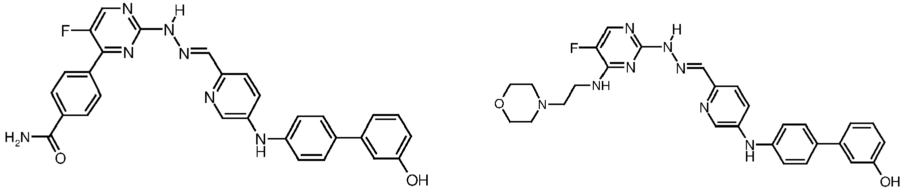

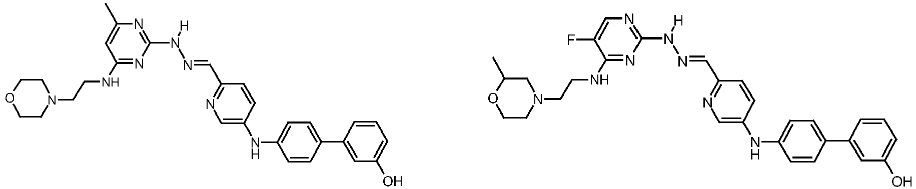

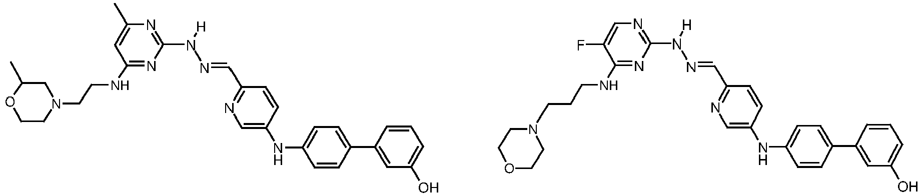

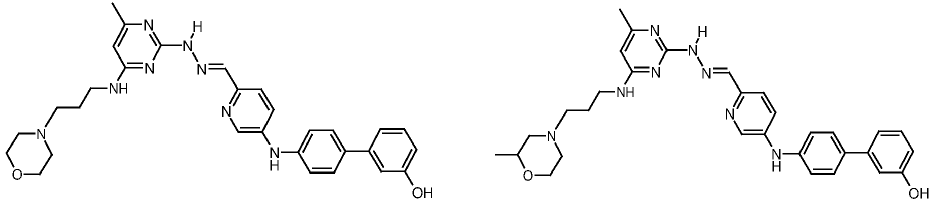

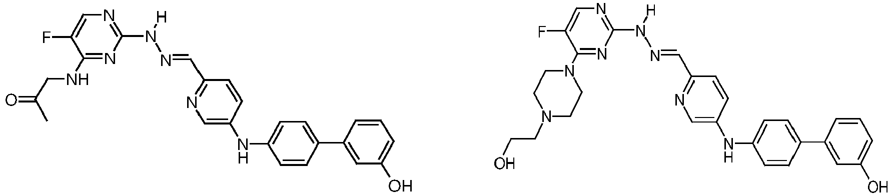

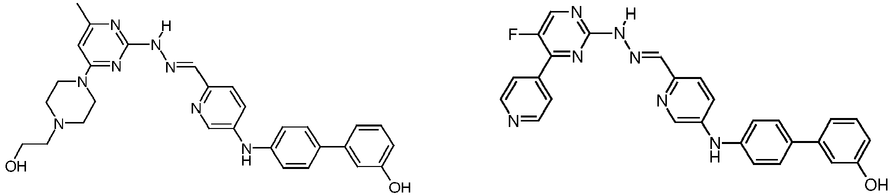

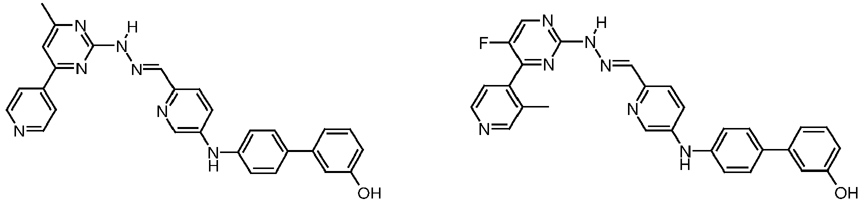

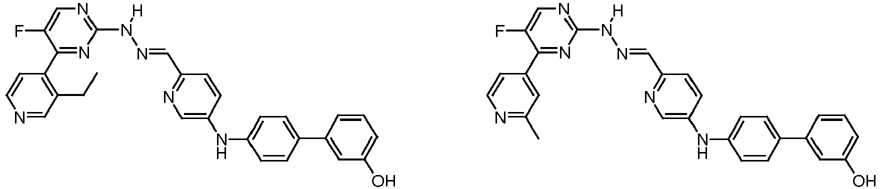

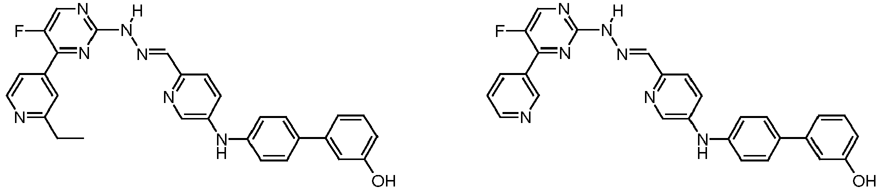

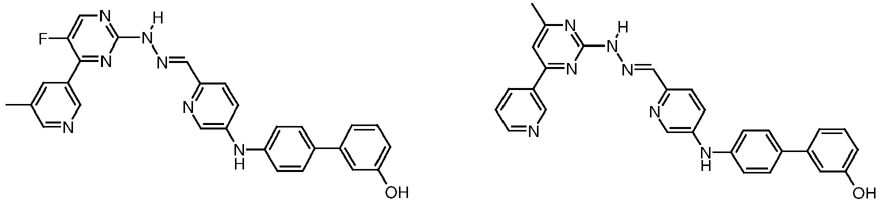

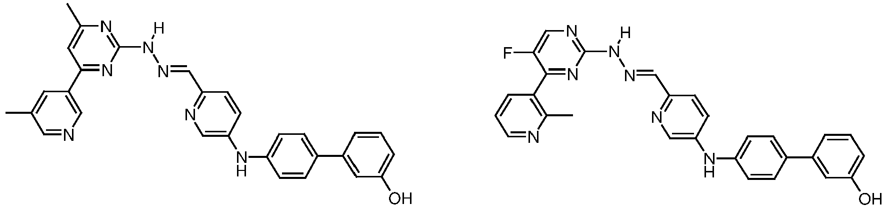

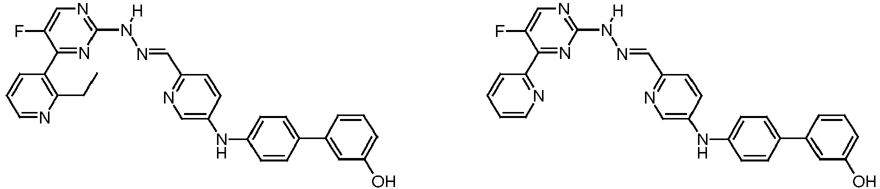

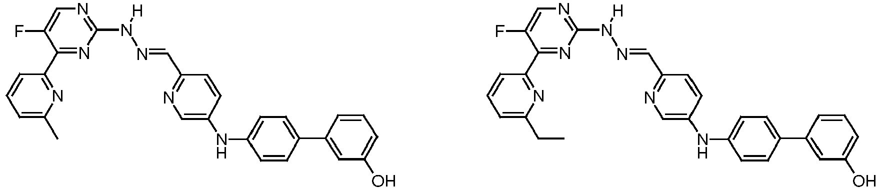

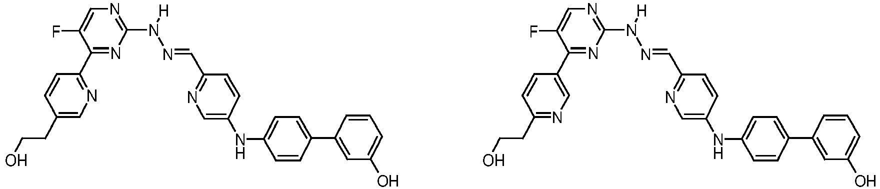

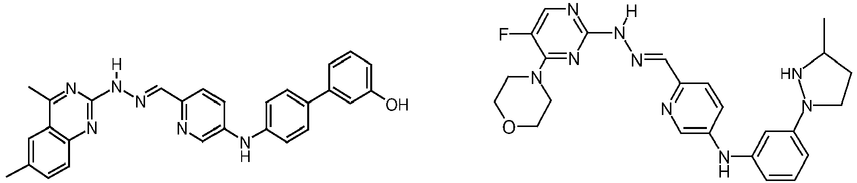

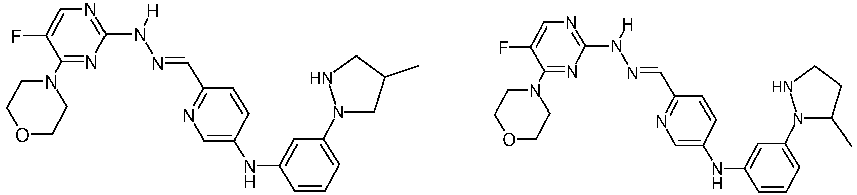

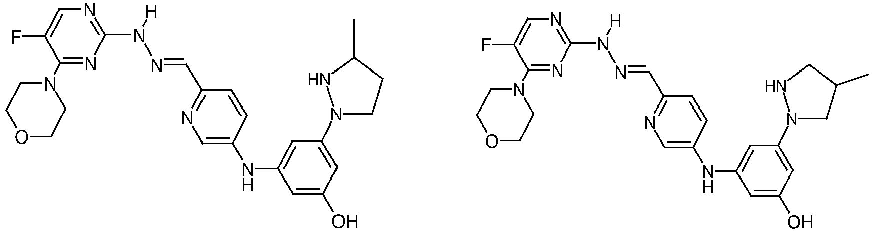

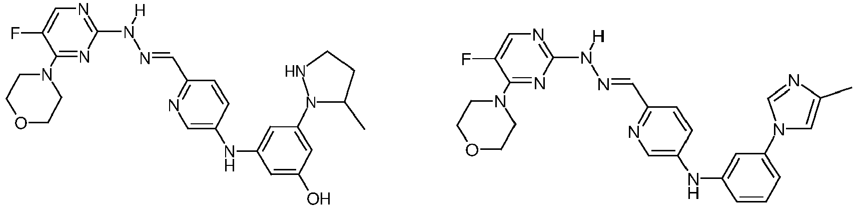

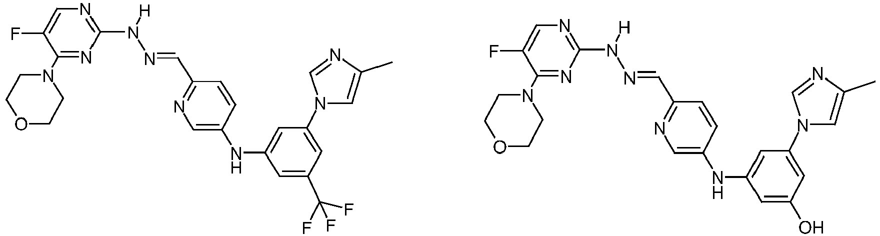

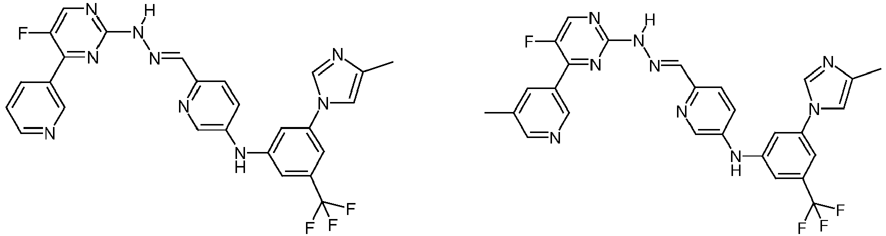

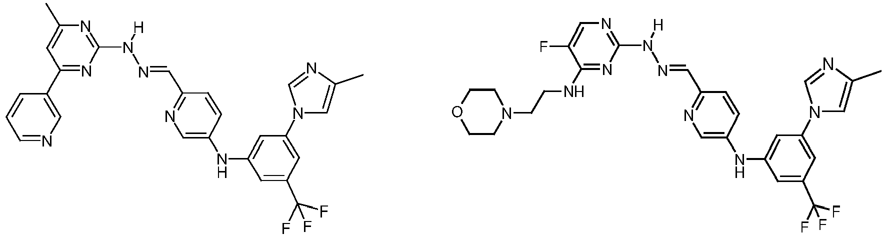

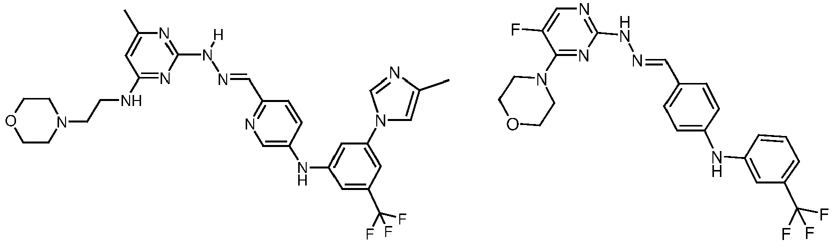

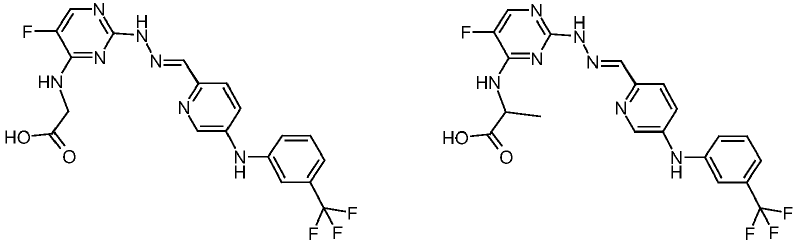

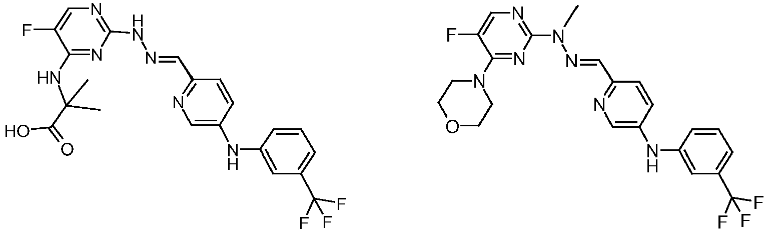

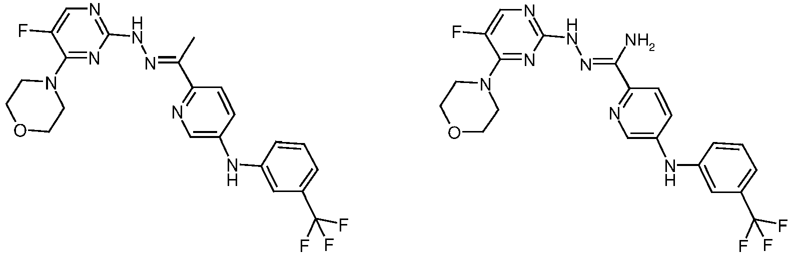

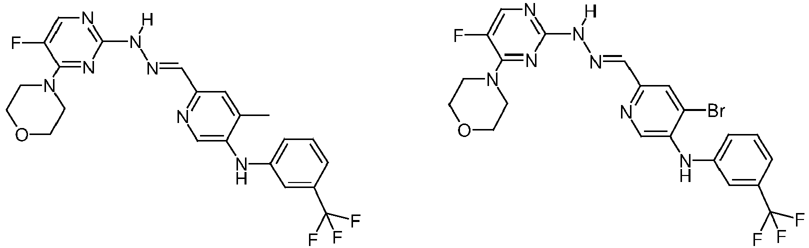

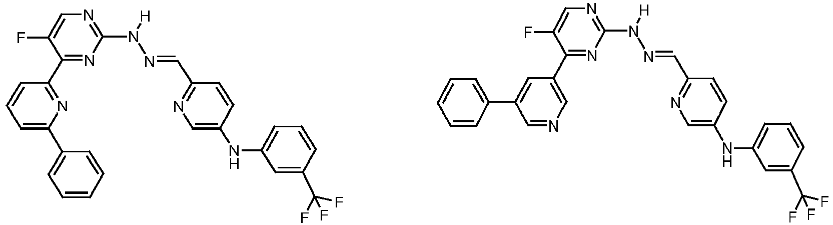

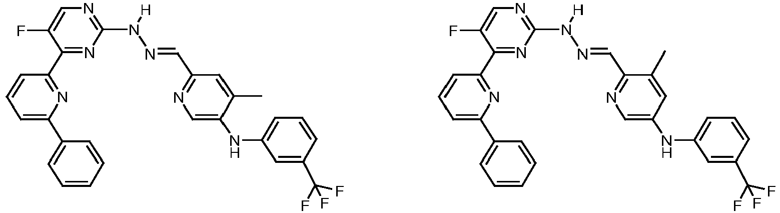

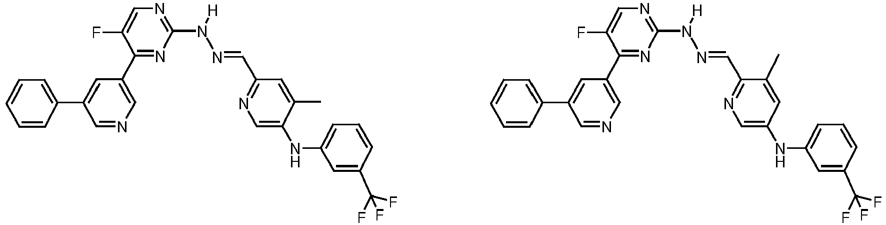

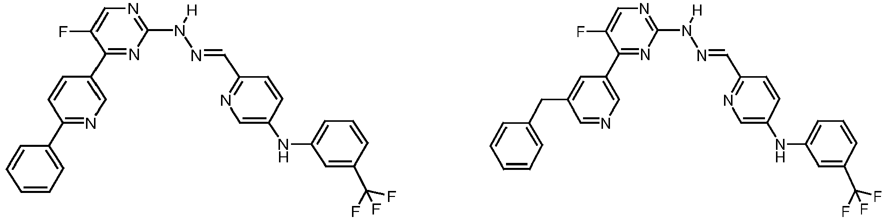

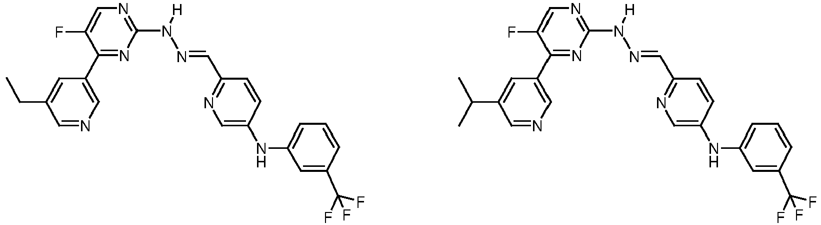

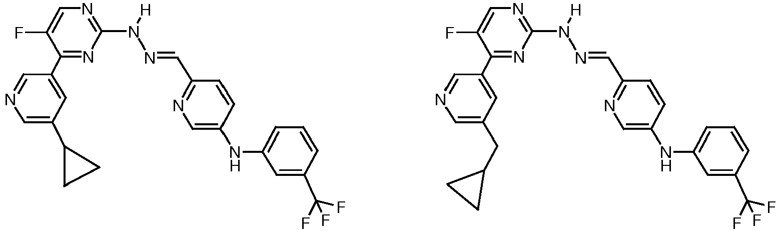

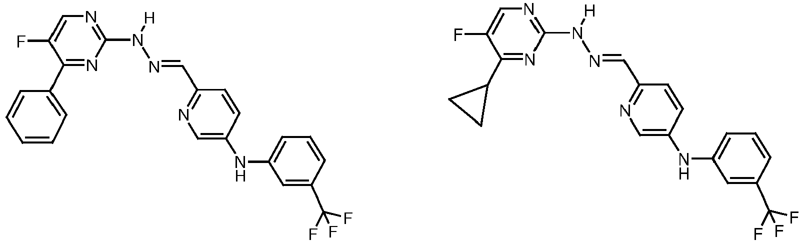

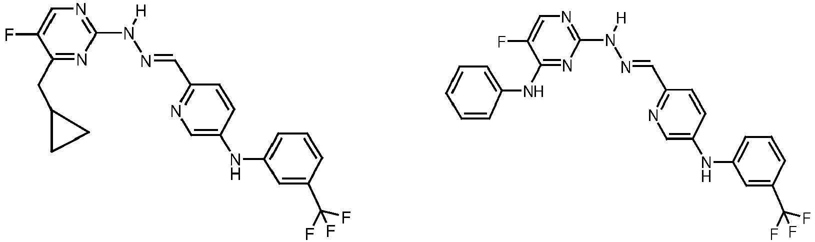

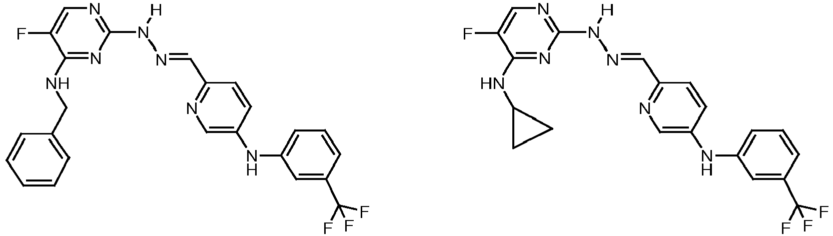

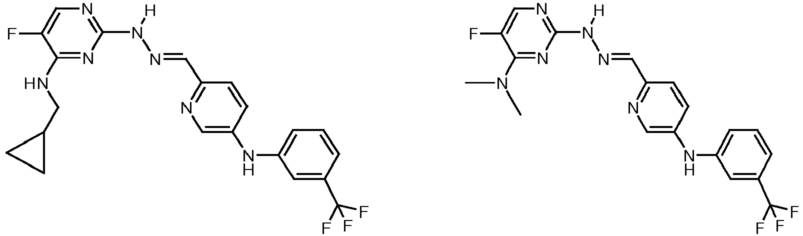

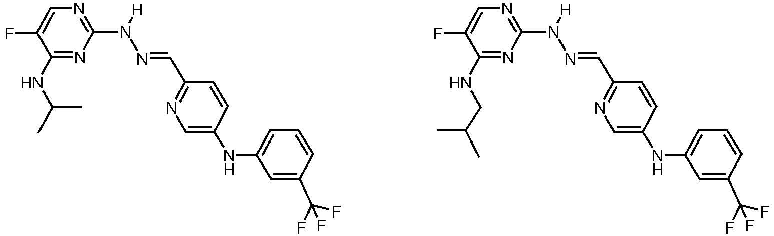

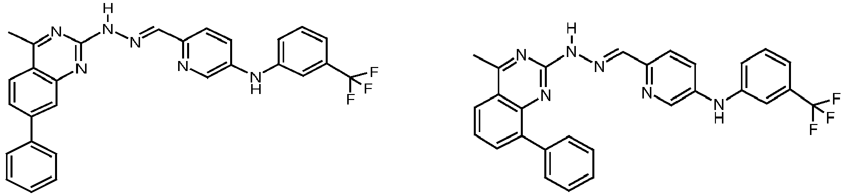

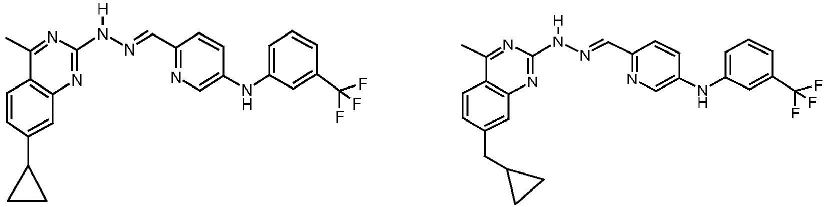

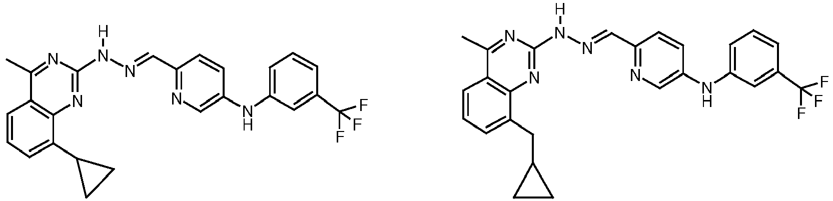

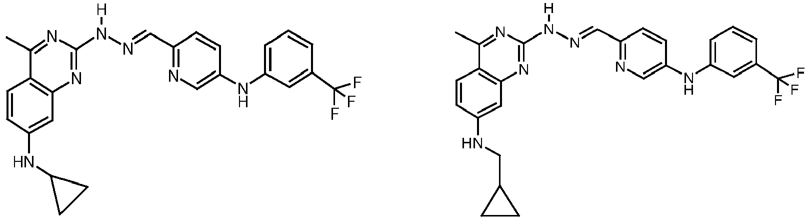

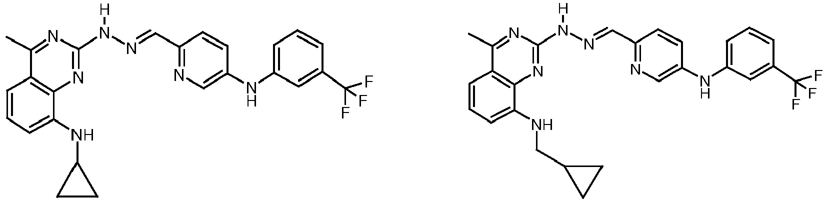

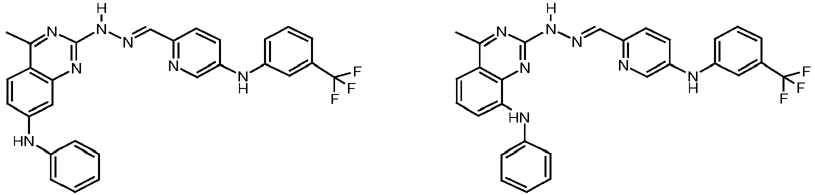

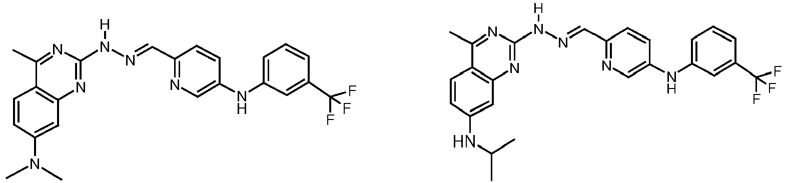

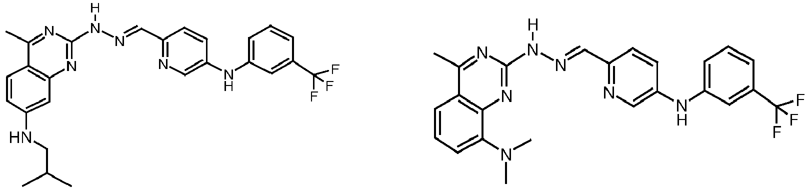

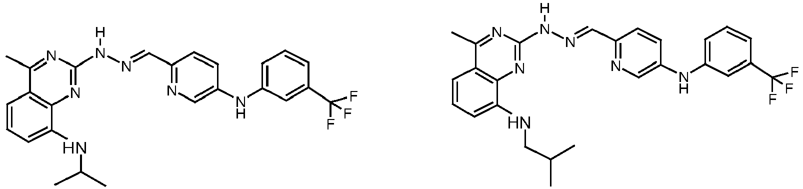

The present application further discloses inhibitors of the P210^{BCR-ABL-T315I} theramutein having the formula III 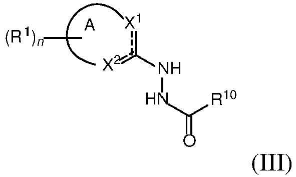 wherein
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹, -(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom; wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
R¹⁰ is selected from -Y'-R¹⁸;
Y' is selected from a chemical bond, O, NR⁰-, and a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
R¹⁸ is selected from the group consisting of H, alkyl, cycloalkyl, alkenyl, alkynyl, aralkyl, CF₃, aryl, and a heterocyclic ring; and
each R⁰ is independently selected from H, alkyl, cycloalkyl, aralkyl, aryl and a heterocyclic ring.

The present application further discloses inhibitors of the P210^{BCR-ABL-T315I} theramutein having the formula IIIₐ 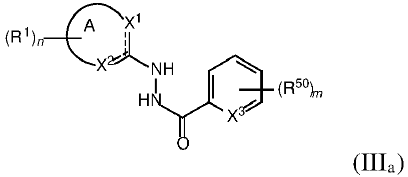 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R1¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;;
   *p* is 0 to 4;
   *q* is 0 to 4;
X³ is N, CH or C-R⁵⁰;
each R⁵⁰ is independently selected from the group consisting of alkyl, cycloalkyl, alkenyl, alkynyl, aralkyl, CN, CF₃, NO₂, OR⁵¹, -(CH₂)*ᵣ*C(O)(CH₂)*ₛ*R⁵¹, -(CH₂)*ᵣ*C(O)N(R⁵²)(R⁵³), -(CH₂)*ᵣ*C(O)O(CH₂)*ₛ*R⁵¹,-(CH₂)*ᵣ*N(R⁵¹)C(O)R⁵¹,-(CH₂)*ᵣ*N(R⁵²)(R⁵³),-N(R⁵¹)SO₂R⁵¹, -OC(O)N(R⁵²)(R⁵³), -SO₂N(R⁵²)(R⁵³), halo, aryl, and a heterocyclic ring, and additionally or alternatively, two R⁵⁰ groups on adjacent ring atoms form a 5- or 6-membered fused ring which contains from 0 to 3 heteroatoms;
   R⁵¹ is selected from H, alkyl, cycloalkyl, alkenyl, alkynyl, aralkyl, aryl, and a heterocyclic ring;
   R⁵² and R⁵³ are independently selected from H, alkyl, cycloalkyl, alkenyl, alkynyl, aralkyl, aryl, and a heterocyclic ring; or R⁵² and R⁵³ may be taken together with the nitrogen to which they are attached form a 5- to 7- membered ring which may optionally contain a further heteroatom, wherein the 5- to 7- membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *r* is 0 to 4;
   *s* is 0 to 4;
*m* is 0 to 4; and
each R⁰ is independently selected from H, alkyl, cycloalkyl, aralkyl, aryl and a heterocyclic ring.

The present application further discloses inhibitors of the P210^{BCR-ABL-T315I} theramutein having the formula III_{b} 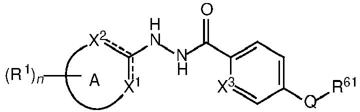 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹ -(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
X³ is N or CH;
R⁶¹ is selected from aryl and a heterocyclic ring;
Q is selected from a chemical bond or a group having the formula -O-, -(CH₂)*ᵢ*-, -(CH₂)*ᵢ*C(O)(CH₂)*ⱼ*-, -(CH₂)*ᵢ*-N(R ⁶²)-(CH₂)*ⱼ*-, -(CH₂)*ᵢ*C(O)-N(R ⁶²)-(CH₂)*ⱼ*-, -(CH₂)*ᵢ*C(O)O(CH₂)*ⱼ*-, -(CH₂)*ᵢ*N(R⁶²)C(O)-(CH₂)*ⱼ*-, -(CH₂)*ᵢ*OC(O)N(R⁶²)-(CH₂)*ⱼ*-, and -O-(CH₂)*ᵢ*-C(O)N(R⁶²)-(CH₂)*ⱼ*-;
R⁶² is selected from H, alkyl, aryl, and a heterocyclic ring;
each R⁰ is independently selected from H, alkyl, cycloalkyl, aralkyl, aryl and a heterocyclic ring;
*h* is 0 to 4;
*i* is 0 to 4; and
*j* is 0 to 4.

The present application further discloses inhibitors of the P210^{BCR-ABL-T315I} theramutein having the formula III_{c} 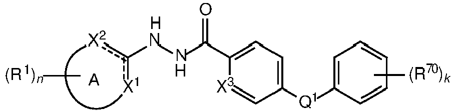 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)₉R¹¹, -(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
X³ is N or CH;
each R⁰ is independently selected from H, alkyl, cycloalkyl, aralkyl, aryl and a heterocyclic ring;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
each R⁷⁰ is selected from halo, alkyl, CN, N(R⁷¹)₂, cyclic-amino, NO₂, OR⁷¹, and CF₃,
each R⁷¹ is selected from H, alkyl, aryl, aralkyl and a heterocyclic ring; and
*k* is 0 to 4.

The present application further discloses inhibitors of the P210^{BCR-ABL-T315I} theramutein having the formula III_{d} 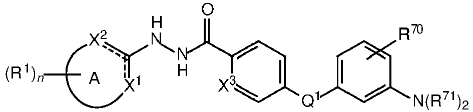 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)₉R¹¹, -(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom, wherein the 5- to 7-membered ring may optionally be substituted with one to three substituents that are independently selected from alkyl, cycloalkyl, alkenyl, alkynyl, aralkyl, CN, CF₃, NO₂, OR⁰, CO₂R⁰, C(O)R⁰, halo, aryl, and a heterocyclic ring;
   *p* is 0 to 4;
   *q* is 0 to 4;
each R⁰ is independently selected from H, alkyl, cycloalkyl, aralkyl, aryl and a heterocyclic ring;
R⁸ is selected from H and CH₃;
X³ is N or CH;
Q¹ is selected from a chemical bond or a group having the formula -O-, -CH₂-, -NH-, -C(O)-NH-, -C(O)O-, -NH-C(O)-, -OC(O)NH-, and -O-C(O)NH-;
each R⁷⁰ is selected from halo, alkyl, CN, N(R⁷¹)₂, cyclic-amino, NO₂, OR⁷¹, and CF₃; and
each R⁷¹ is selected from H and alkyl.

The present application further discloses inhibitors of the P210^{BCR-ABL-T315I} theramutein having the formula IIIₑ 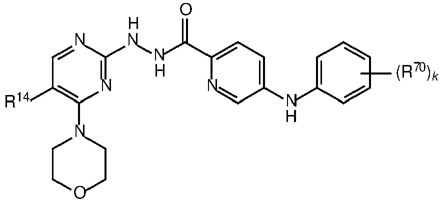 wherein
R¹⁴ is selected from H and F;
each R⁷⁰ is selected from halo, alkyl, CN, N(R⁷¹)₂, cyclic-amino, NO₂, OR⁷¹, and CF₃,
each R⁷¹ is selected from H, alkyl, aryl, aralkyl and a heterocyclic ring; and
*k* is 0 to 4.

Exemplary compounds of the formula III, 111*ₐ*, III*_{b}*, III*_{c}*, III*_{d}*, or III*ₑ* includes the following structures:

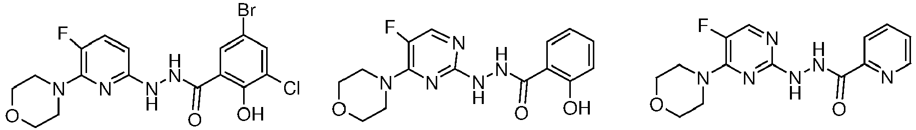

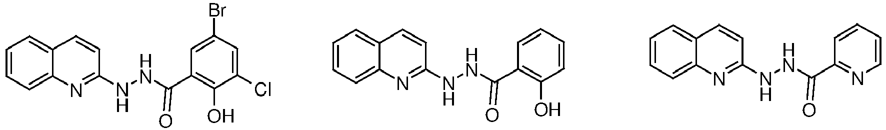

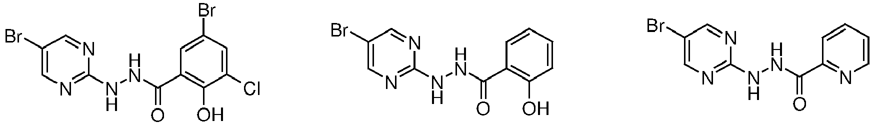

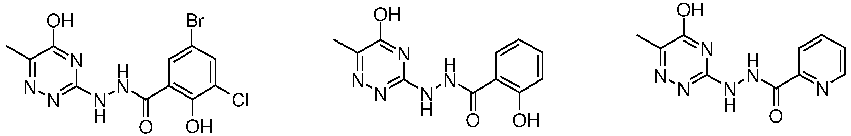

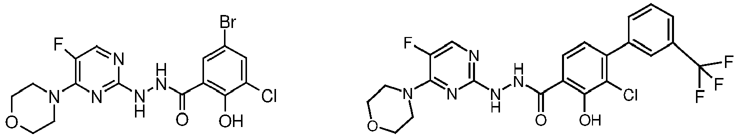

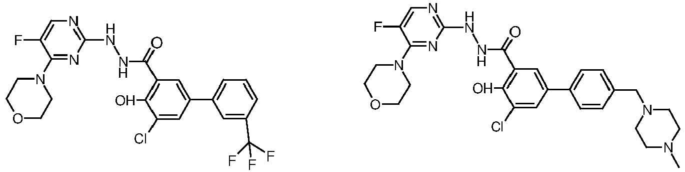

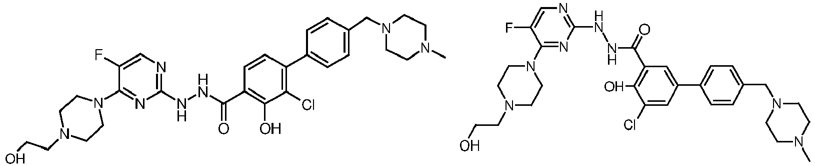

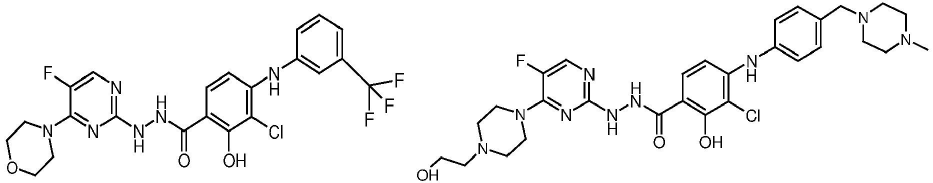

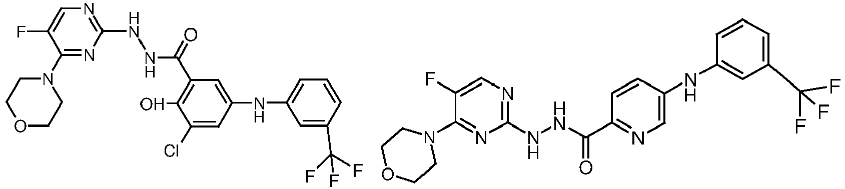

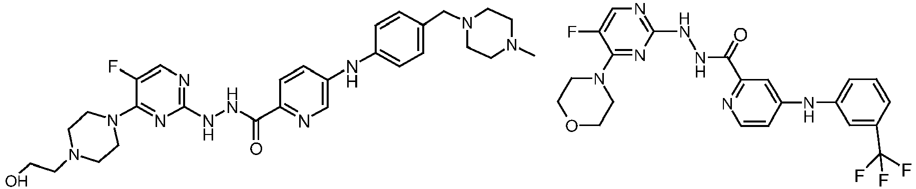

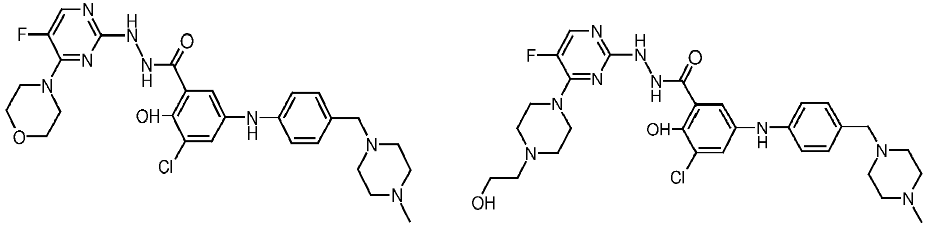

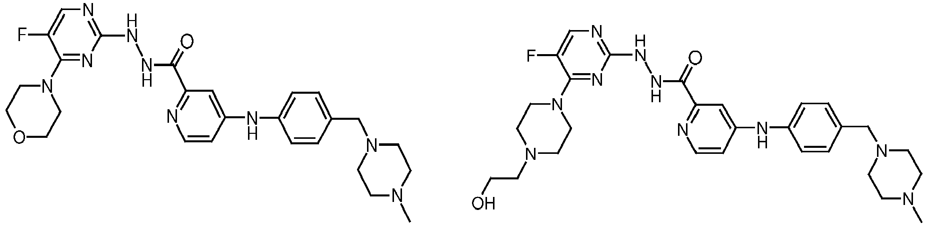

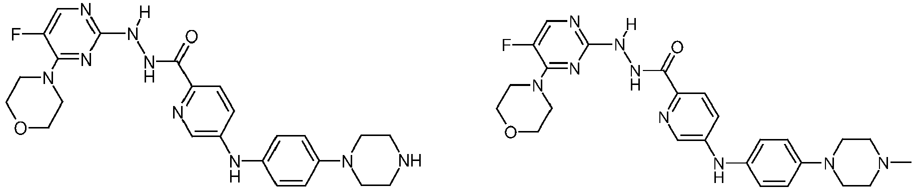

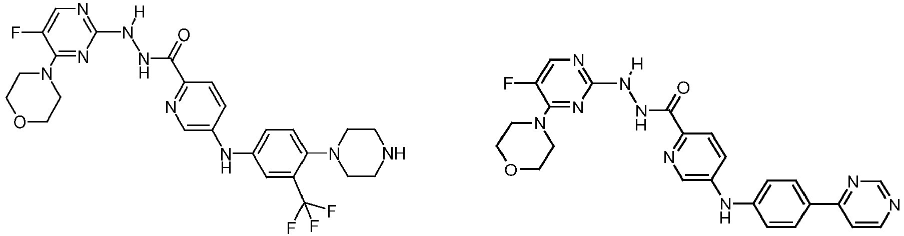

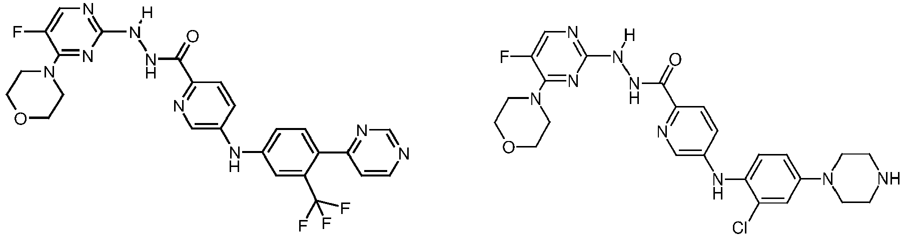

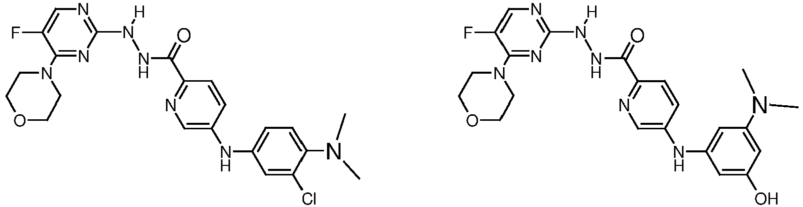

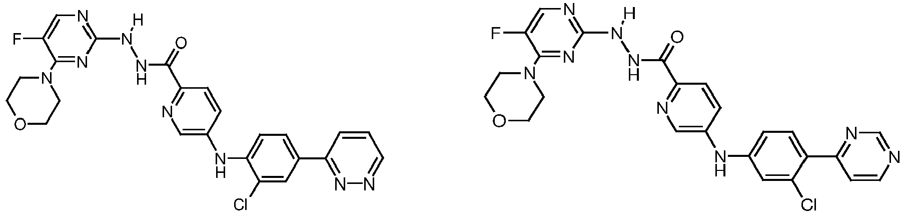

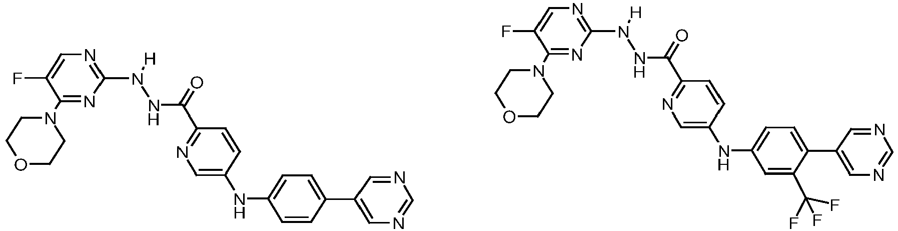

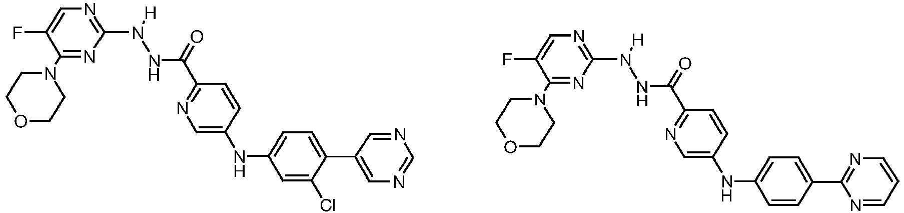

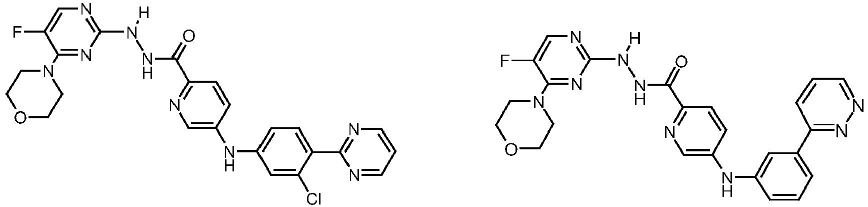

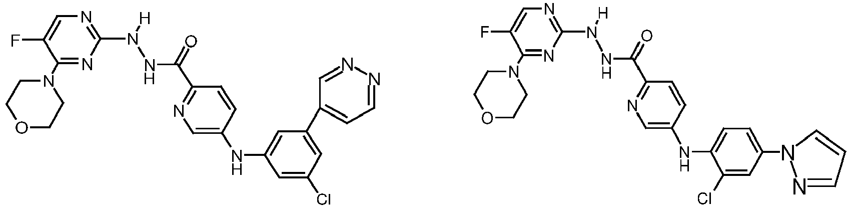

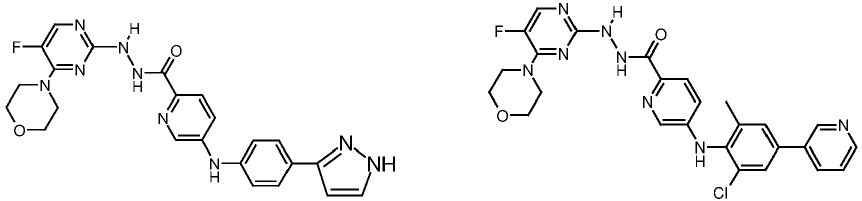

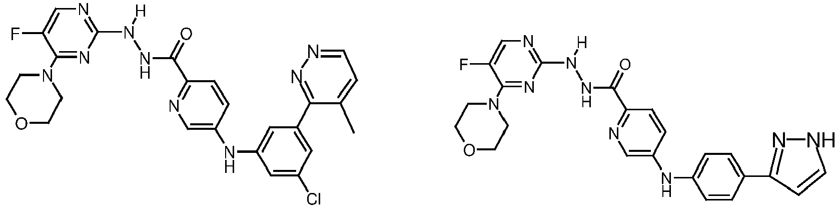

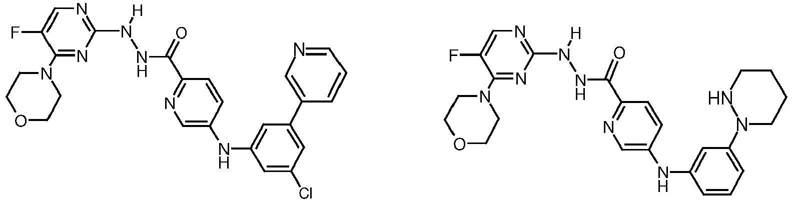

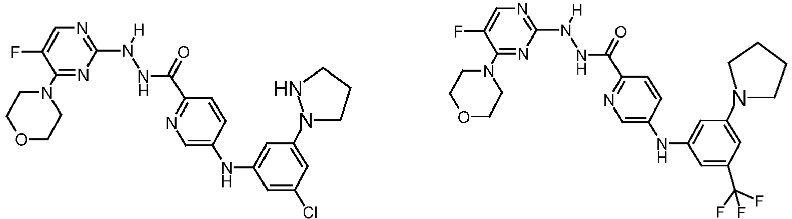

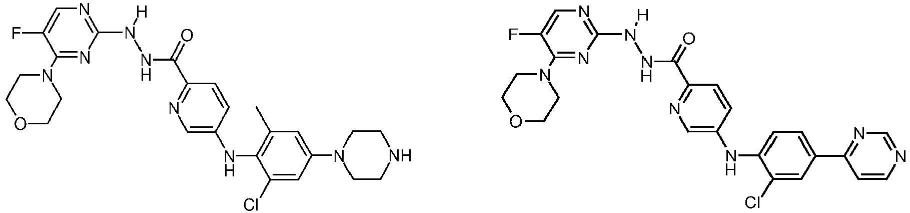

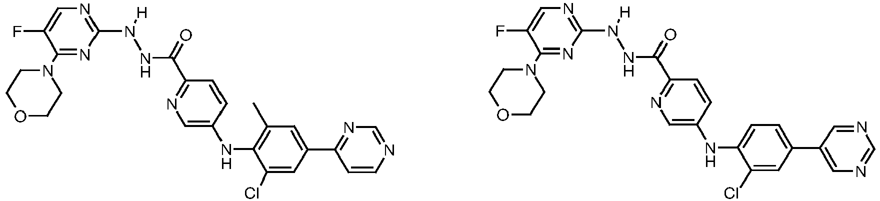

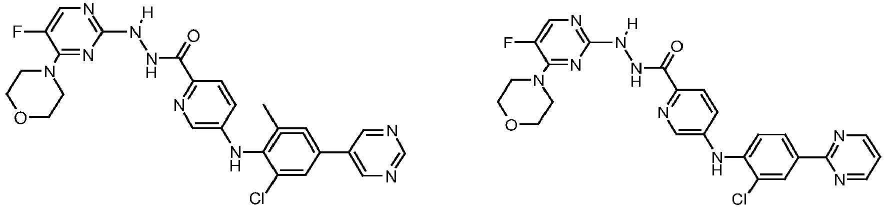

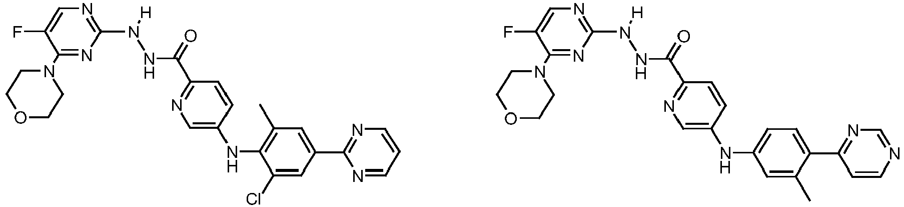

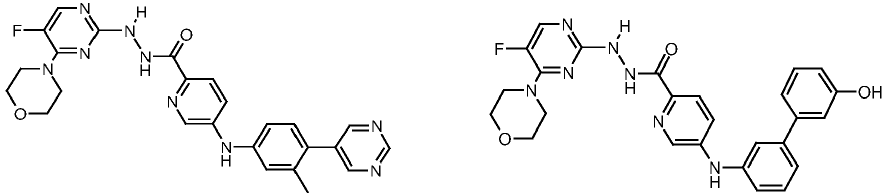

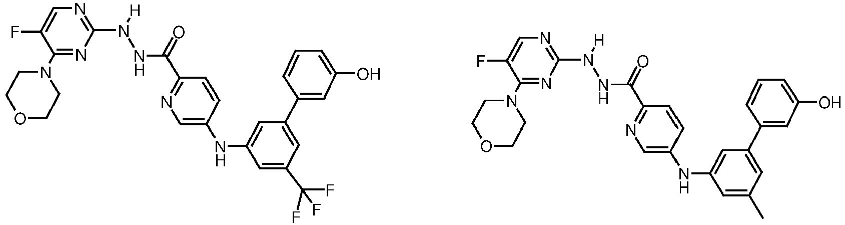

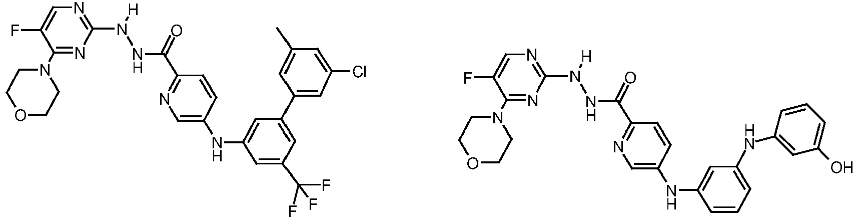

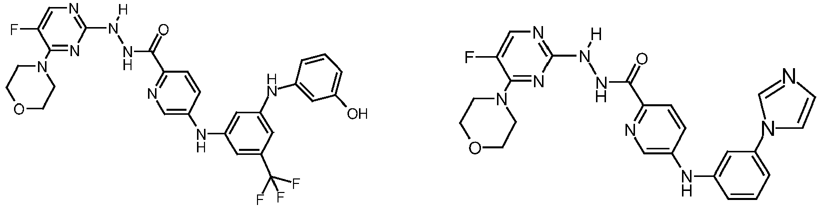

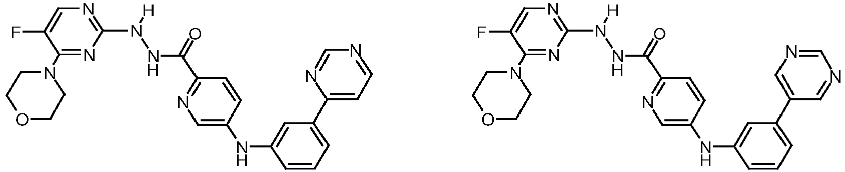

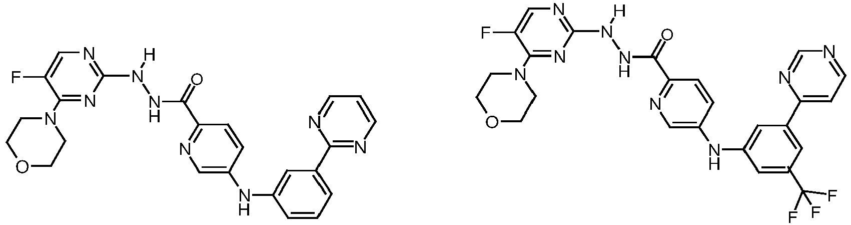

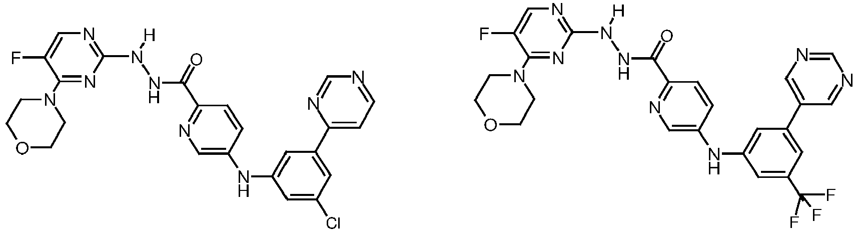

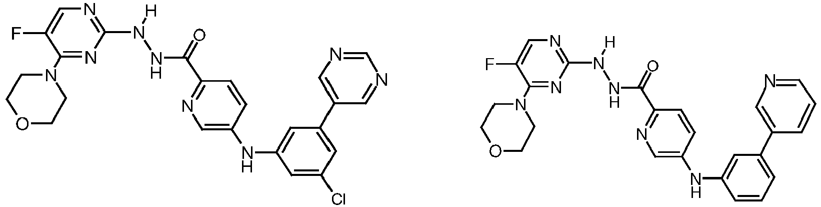

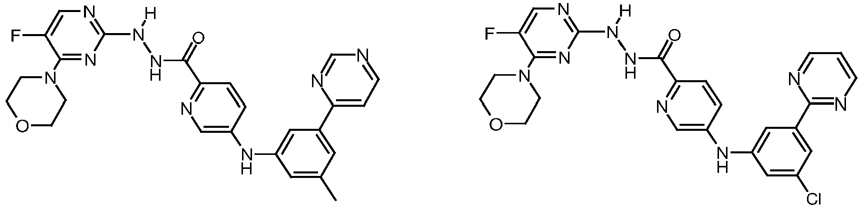

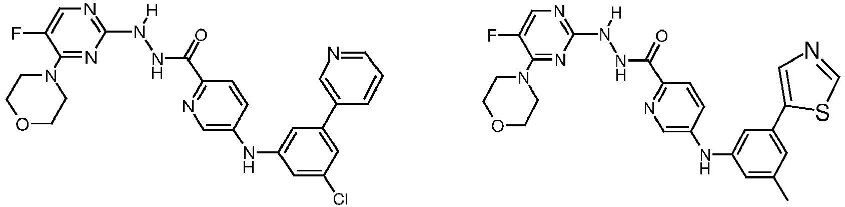

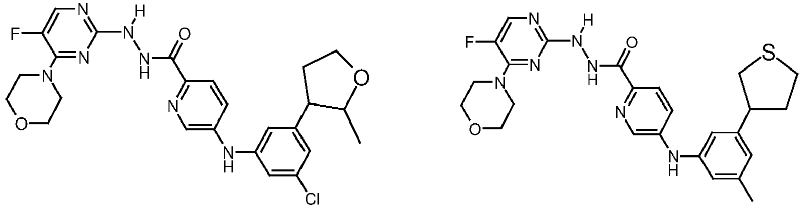

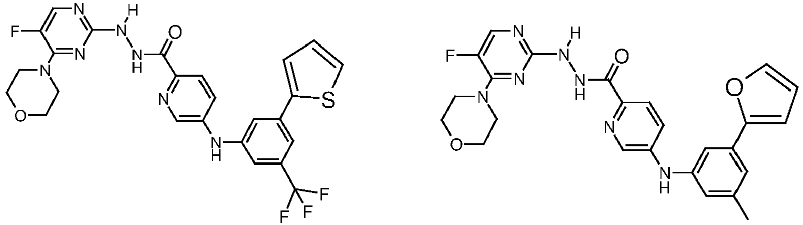

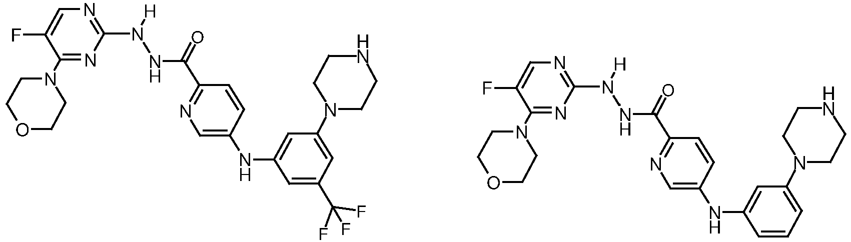

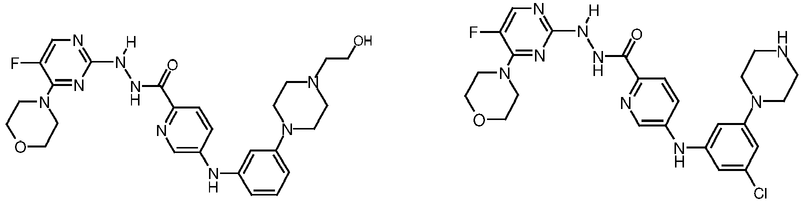

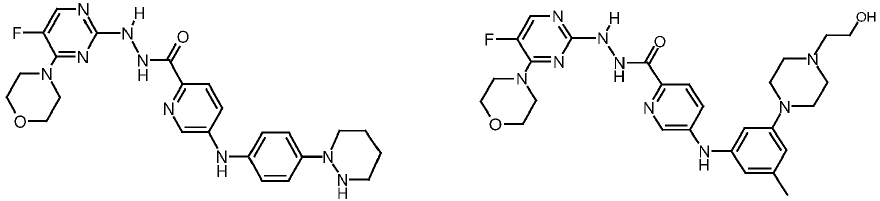

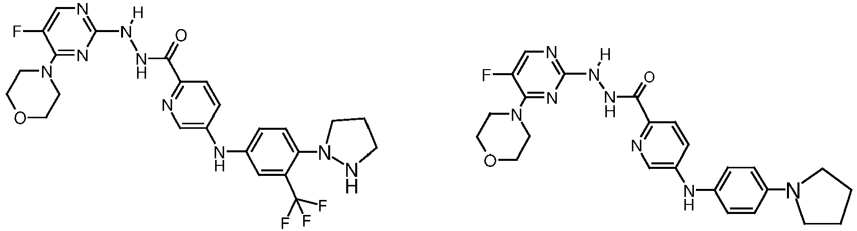

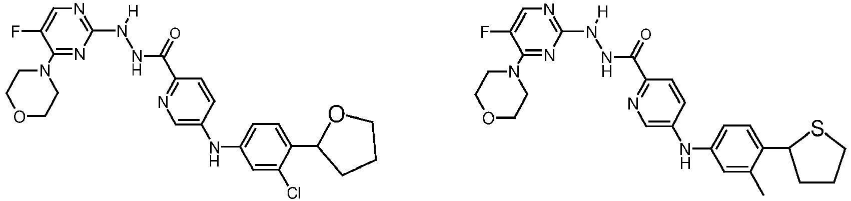

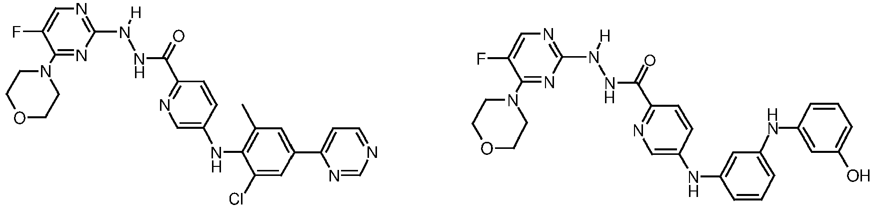

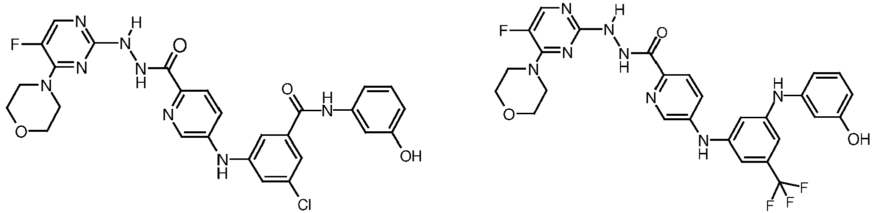

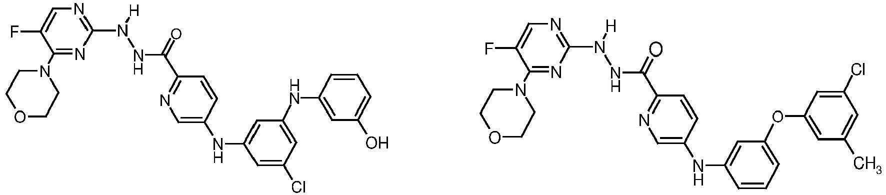

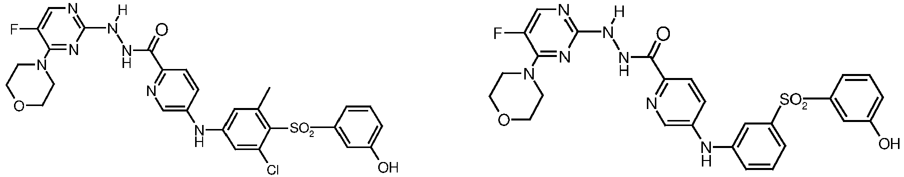

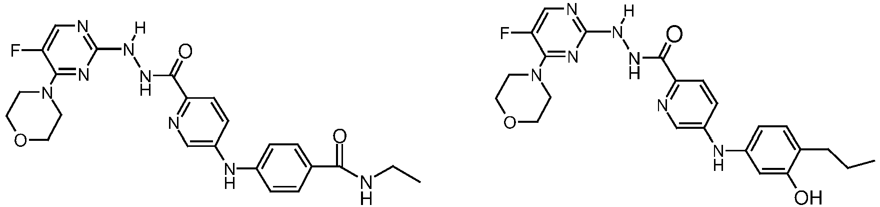

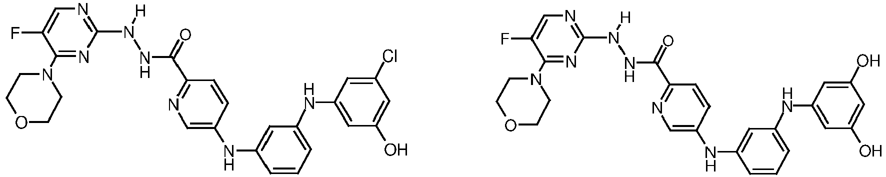

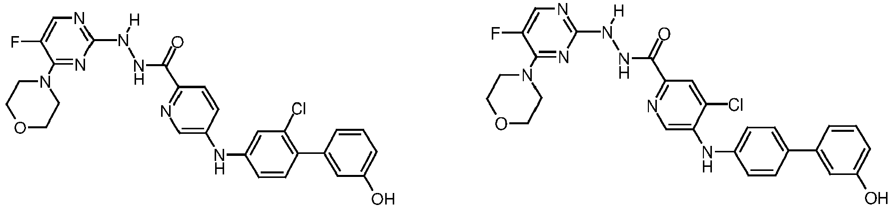

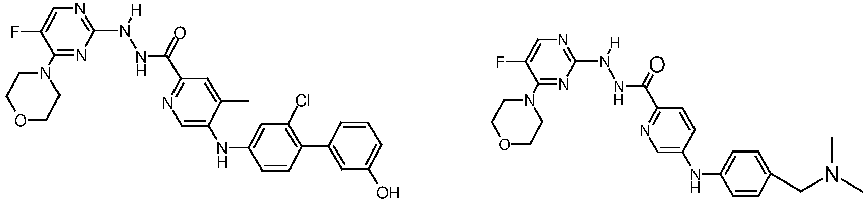

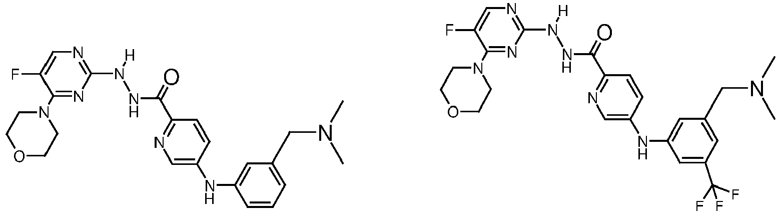

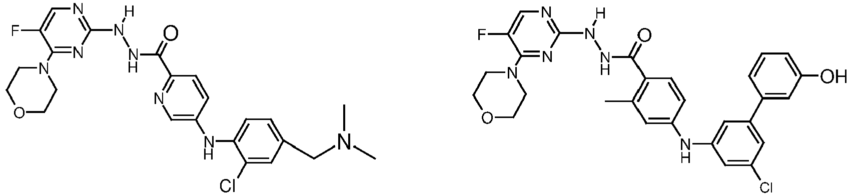

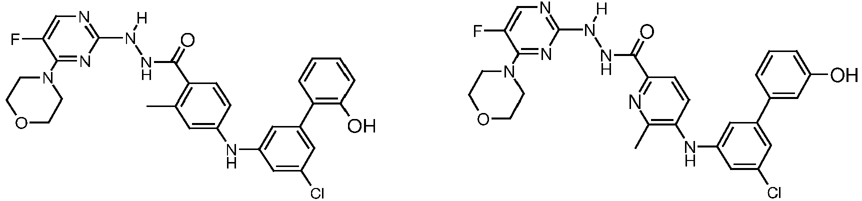

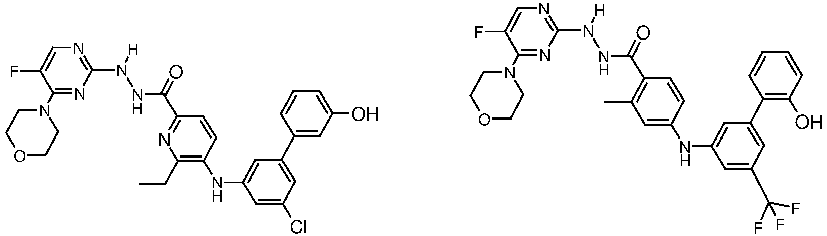

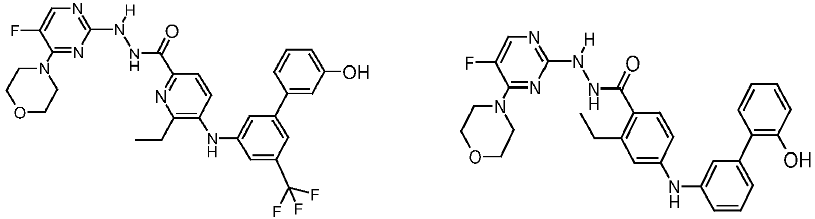

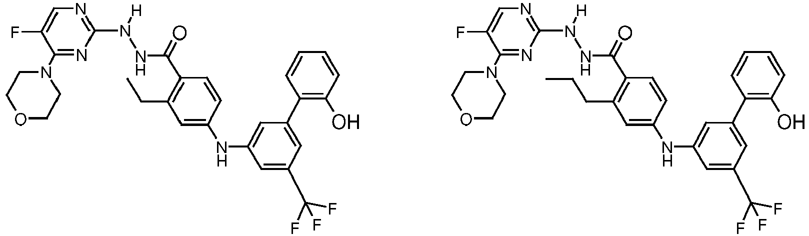

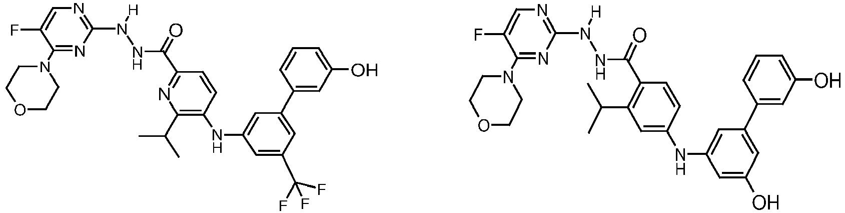

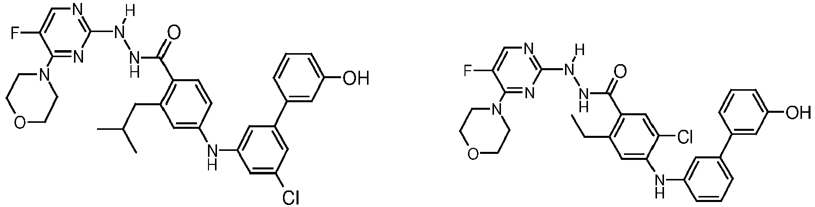

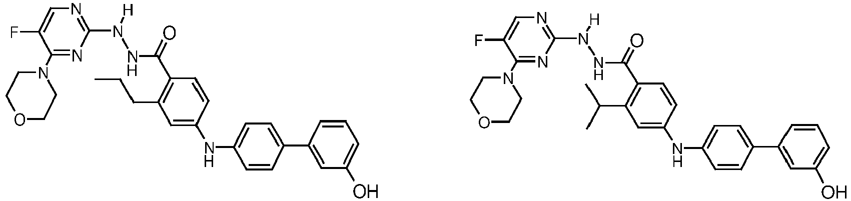

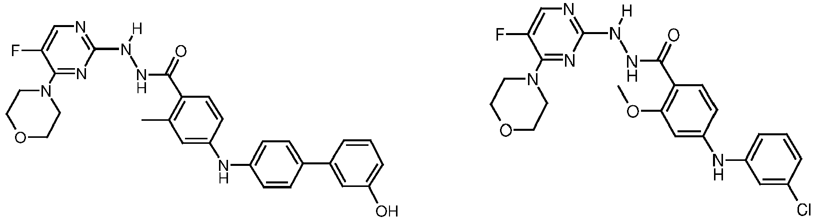

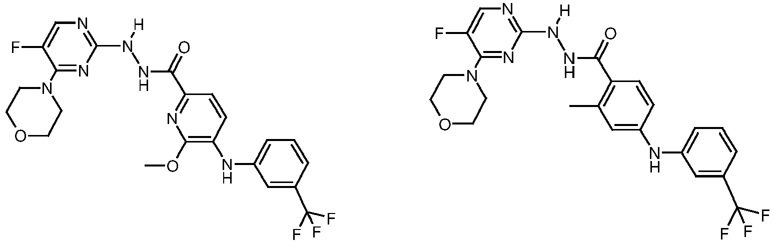

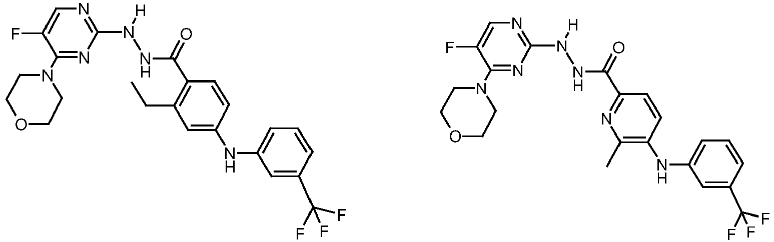

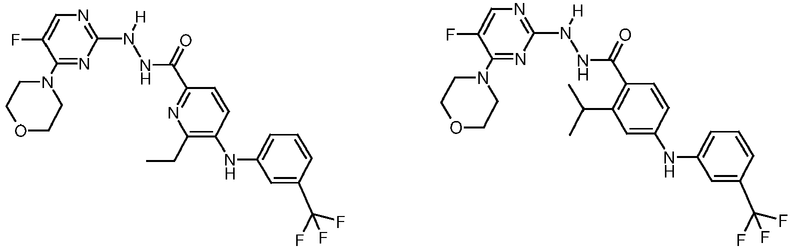

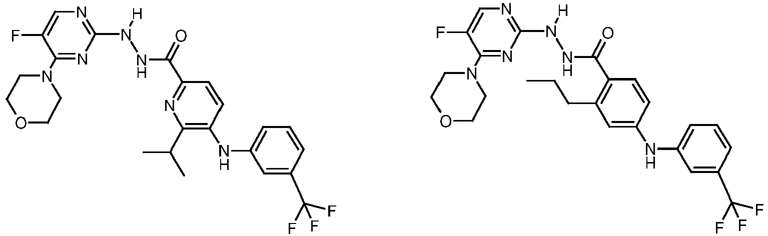

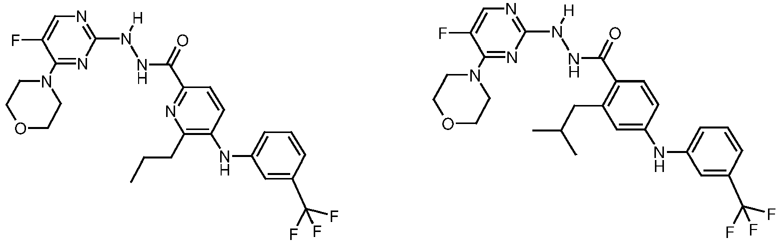

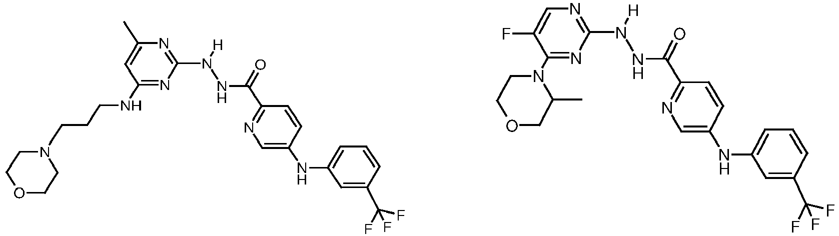

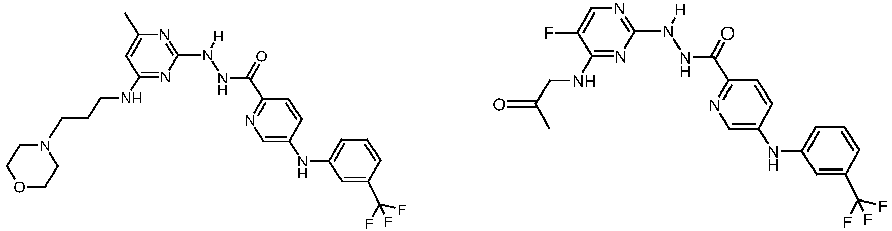

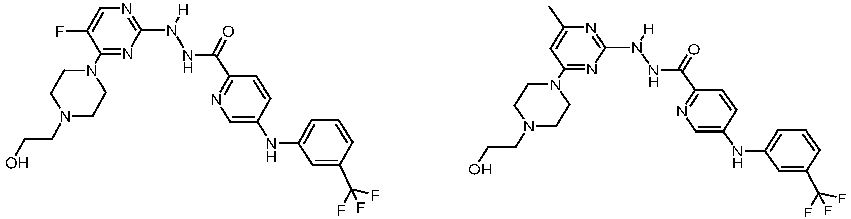

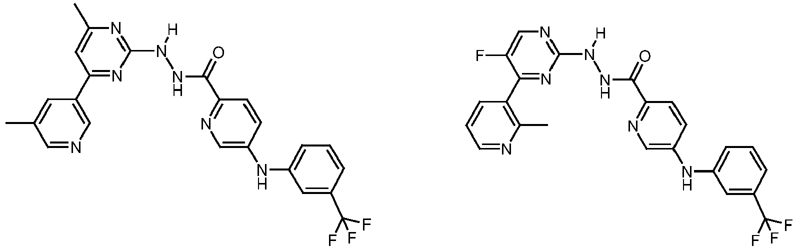

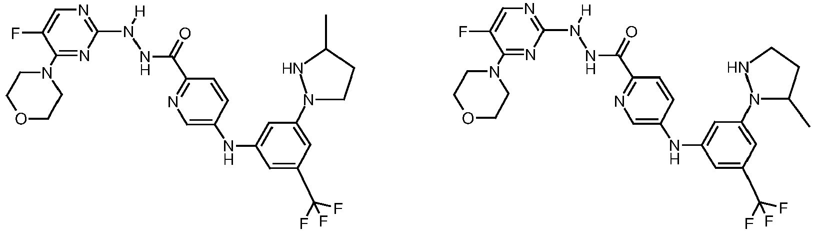

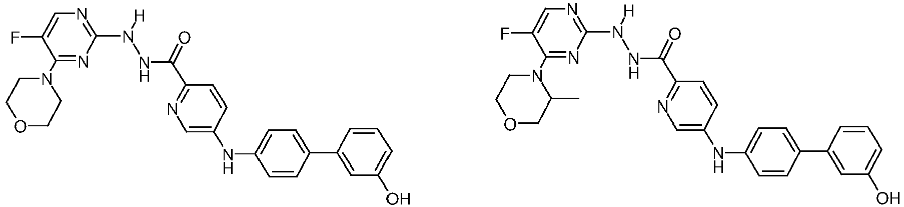

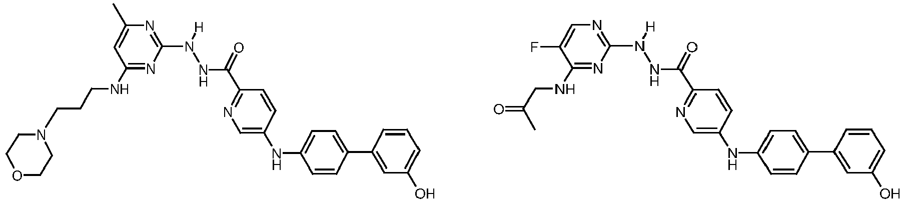

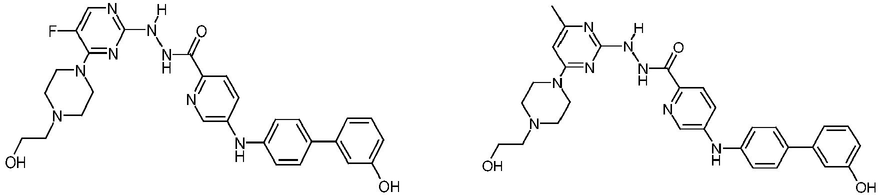

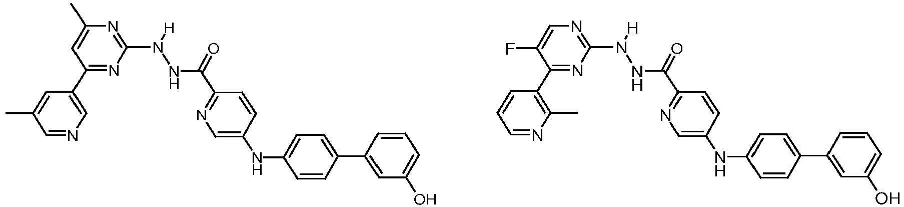

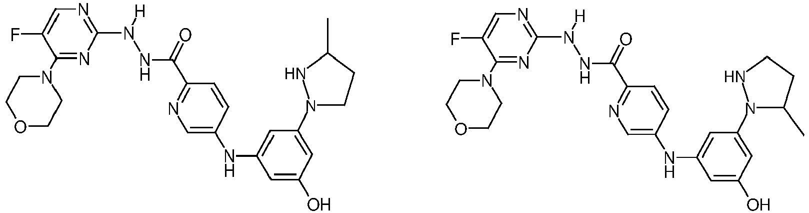

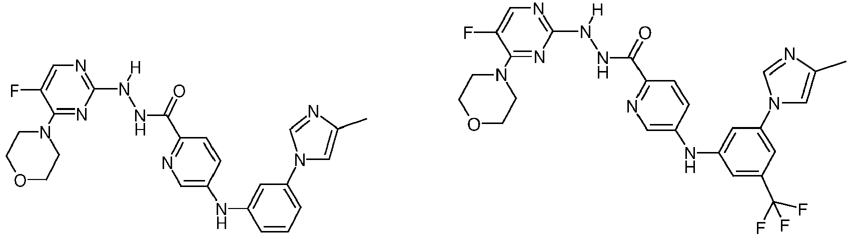

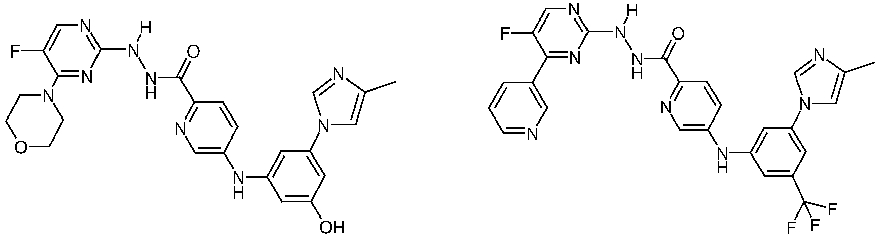

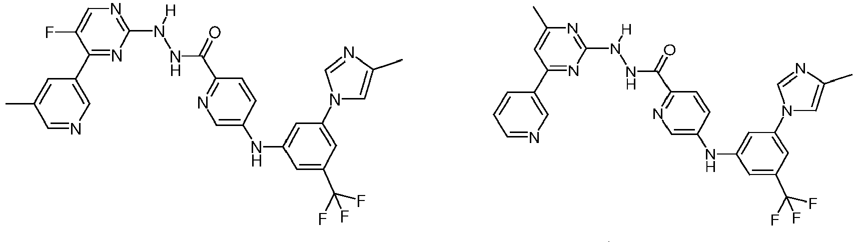

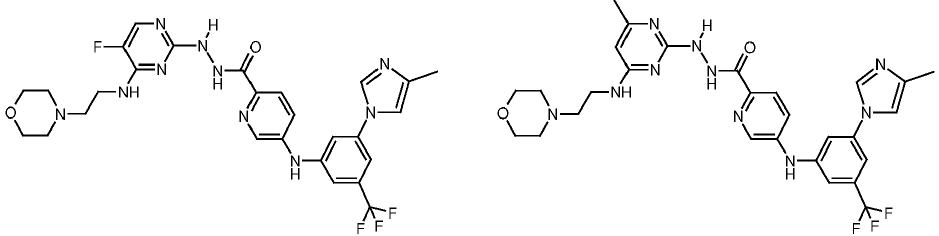

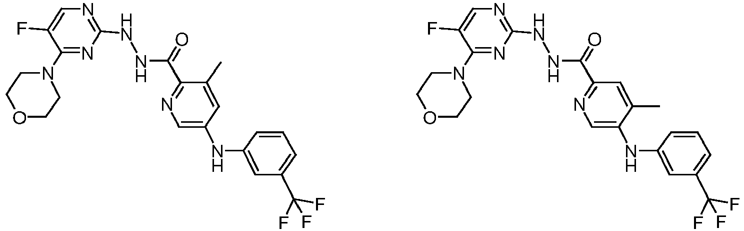

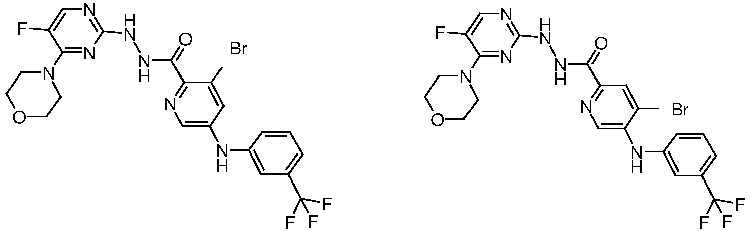

The present application further discloses inhibitors of the P210^{BCR-ABL-T315I} theramutein having the formula IV 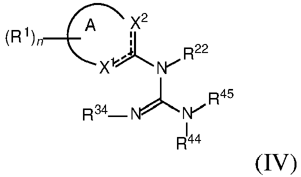 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
R²² is selected from H and C₁₋₃ alkyl;
R³⁴ is selected from H, NO₂, CN, alkyl, cycloalkyl, alkenyl, alkynyl, aralkyl, aryl and a heterocyclic ring;
R⁴⁴ is selected from H, alkyl, cycloalkyl, -(C=O)R⁰, alkenyl, alkynyl, aralkyl, aryl, and a heterocyclic ring;
R⁴⁵ is selected from -Y"-R¹⁹,
Y" is selected from a chemical bond, O, NR⁰-, and a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
R¹⁹ is selected from the group consisting of H, alkyl, cycloalkyl, alkenyl, alkynyl, aralkyl, CF₃, aryl, and a heterocyclic ring; and
each R⁰ is independently selected from H, alkyl, cycloalkyl, aralkyl, aryl and a heterocyclic ring.

Exemplary compounds of the formula IV include the following structures:

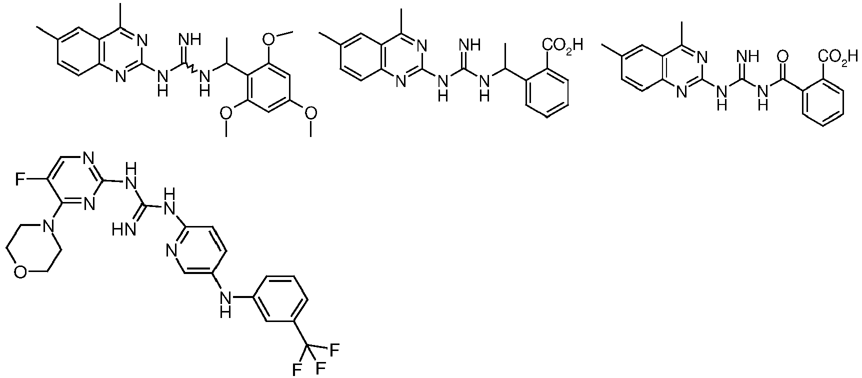

The present application further discloses inhibitors of the P210^{BCR-ABL-T315I} theramutein having the formula V 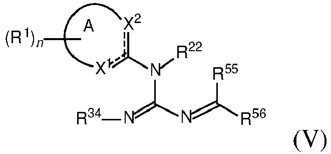 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
R²² is selected from H and C₁₋₃ alkyl;
R³⁴ is selected from H, NO₂, CN, alkyl, cycloalkyl, alkenyl, alkynyl, aralkyl, aryl and a heterocyclic ring;
R⁵⁵ is selected from H, alkyl, cycloalkyl, alkenyl, alkynyl, aralkyl, aryl, and a heterocyclic ring;
R⁵⁶ is selected from -Y"-R¹⁹,
Y" is selected from a chemical bond, O, NR⁰-, and a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
R¹⁹ is selected from the group consisting of H, alkyl, cycloalkyl, alkenyl, alkynyl, aralkyl, CF₃, aryl, and a heterocyclic ring; and
each R⁰ is independently selected from H, alkyl, cycloalkyl, aralkyl, aryl and a heterocyclic ring.

The present aplication further discloses inhibitors of the P210^{BCR-ABL-T315I} theramutein having the formula Vₐ 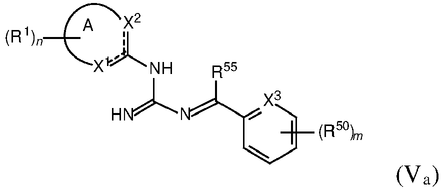 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
R⁵⁵ is selected from H, alkyl, cycloalkyl, alkenyl, alkynyl, aralkyl, aryl, and a heterocyclic ring;
X³ is N or C-R⁵⁰;
each R⁵⁰ is independently selected from the group consisting of alkyl, cycloalkyl, alkenyl, alkynyl, aralkyl, CN, CF₃, NO₂, OR⁵¹, -(CH₂)*ᵣ*C(O)(CH₂)*ₛ*R⁵¹, -(CH₂)*ᵣ*C(O)N(R⁵²)(R⁵³), -(CH₂)*ᵣ*C(O)O(CH₂)*ₛ*R⁵¹,-(CH₂)*ᵣ*N(R⁵¹)C(O)R⁵¹, -(CH₂)*ᵣ*N(R⁵²)(R⁵³), -N(R⁵¹)SO₂R⁵¹, -OC(O)N(R⁵²)(R⁵³), -SO₂N(R⁵²)(R⁵³), halo, aryl, and a heterocyclic ring, and additionally or alternatively, two R⁵⁰ groups on adjacent ring atoms form a 5- or 6-membered fused ring which contains from 0 to 3 heteroatoms;
   R⁵¹ is selected from H, alkyl, cycloalkyl, alkenyl, alkynyl, aralkyl, aryl, and a heterocyclic ring;
   R⁵² and R⁵³ are independently selected from H, alkyl, cycloalkyl, alkenyl, alkynyl, aralkyl, aryl, and a heterocyclic ring; or R⁵² and R⁵³ may be taken together with the nitrogen to which they are attached form a 5- to 7- membered ring which may optionally contain a further heteroatom;
   *r* is 0 to 4;
   *s* is 0 to 4;
m is 0 to 4; and
each R⁰ is independently selected from H, alkyl, cycloalkyl, aralkyl, aryl and a heterocyclic ring.

Exemplary compounds of the formula V or V*ₐ* include the following structures:

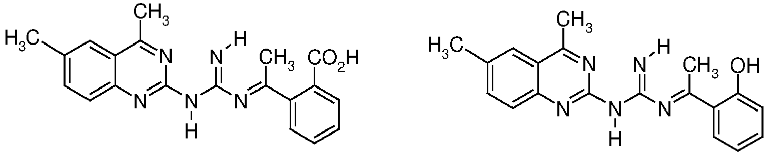

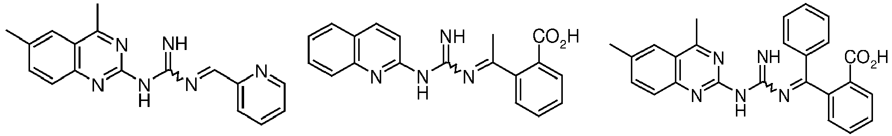

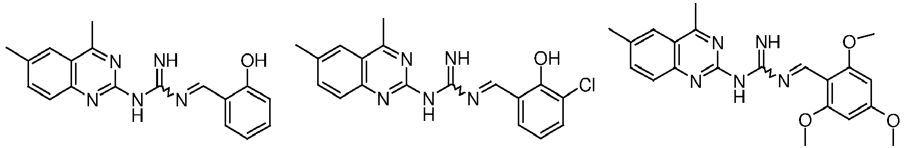

The present application further discloses inhibitors of the P210^{BCR-ABL-T315I} theramutein having the formula VI 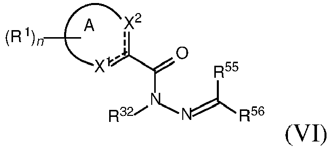 wherein:
ring A is a 5-, 6-, or 7- membered ring or a 7- to 12-membered fused bicyclic ring;
X¹ is selected from N, N-R° or C-R¹;
X² is selected from N, N-R⁰ or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
R⁵⁵ is selected from H, alkyl, cycloalkyl, alkenyl, alkynyl, aralkyl, aryl, and a heterocyclic ring;
R⁵⁶ is selected from -Y"-R¹⁹,
Y" is selected from a chemical bond, O, NR⁰-, and a hydrocarbon chain having from 1 to 4 carbon atoms, and optionally substituted with one or more of halo, alkyl, cycloalkyl, alkenyl, alkynyl, aralkyl, CO₂R⁰, C(O)R⁰, C(O)N(R⁰)₂, CN, CF₃, N(R⁰)₂, NO₂, and OR⁰;
R¹⁹ is selected from the group consisting of H, alkyl, cycloalkyl, alkenyl, alkynyl, aralkyl, CF₃, aryl, and a heterocyclic ring; and
each R⁰ is independently selected from H, alkyl, cycloalkyl, aralkyl, aryl and a heterocyclic ring.

The present application further discloses inhibitors of the P210^{BCR-ABL-T315I} theramutein having the formula VI*ₐ* 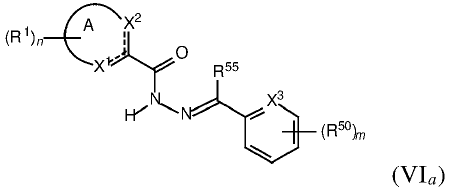 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*R¹¹, -(CH₂)*ₚ*C(O)N(R¹²)(R¹³), -(CH₂)*ₚ*C(O)O(CH₂)₉R¹¹, -(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
R⁵⁵ is selected from H, alkyl, cycloalkyl, alkenyl, alkynyl, aralkyl, aryl, and a heterocyclic ring;
X³ is N or C-R⁵⁰;
each R⁵⁰ is independently selected from the group consisting of alkyl, cycloalkyl, alkenyl, alkynyl, aralkyl, CN, CF₃, NO₂, OR⁵¹, -(CH₂)*ᵣ*C(O)(CH₂)*ₛ*R⁵¹, -(CH₂)*ᵣ*C(O)N(R⁵²)(R⁵³), -(CH₂)*ᵣ*C(O)O(CH₂)*ₛ*R⁵¹, -(CH₂)*ᵣ*N(R⁵¹)C(O)R⁵¹, -(CH₂)*ᵣ*N(R⁵²)(R⁵³), -N(R⁵¹)SO₂R⁵¹, -OC(O)N(R⁵²)(R⁵³), -SO₂N(R⁵²)(R⁵³), halo, aryl, and a heterocyclic ring, and additionally or alternatively, two R⁵⁰ groups on adjacent ring atoms form a 5- or 6-membered fused ring which contains from 0 to 3 heteroatoms;
   R⁵¹ is selected from H, alkyl, cycloalkyl, alkenyl, alkynyl, aralkyl, aryl, and a heterocyclic ring;
   R⁵² and R⁵³ are independently selected from H, alkyl, cycloalkyl, alkenyl, alkynyl, aralkyl, aryl, and a heterocyclic ring; or R⁵² and R⁵³ may be taken together with the nitrogen to which they are attached form a 5- to 7- membered ring which may optionally contain a further heteroatom;
   *r* is 0 to 4;
   *s* is 0 to 4;
*m* is 0 to 4; and
each R⁰ is independently selected from H, alkyl, cycloalkyl, aralkyl, aryl and a heterocyclic ring.

Exemplary compounds of the formula VI or VI*ₐ* include the following structures:

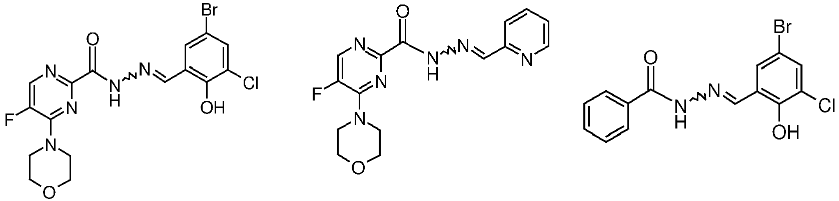

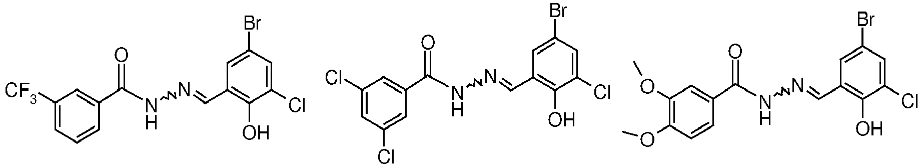

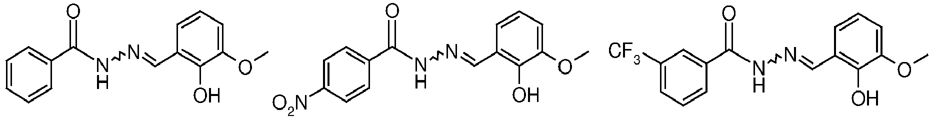

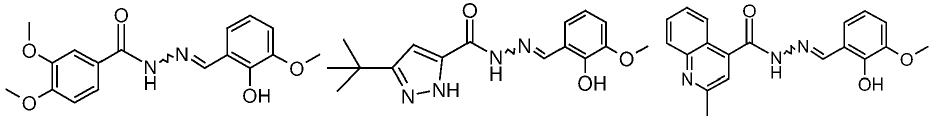

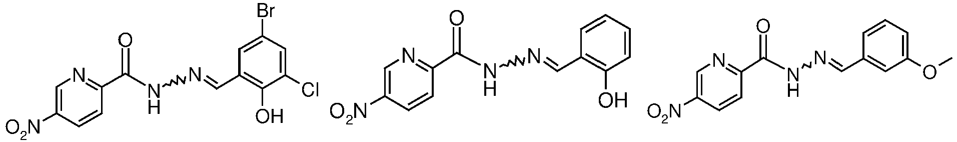

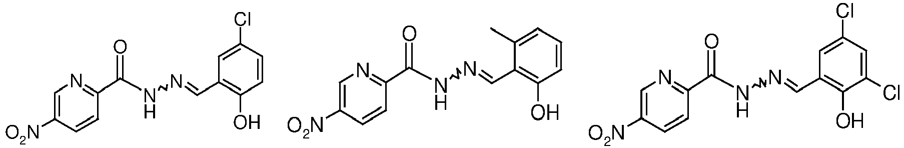

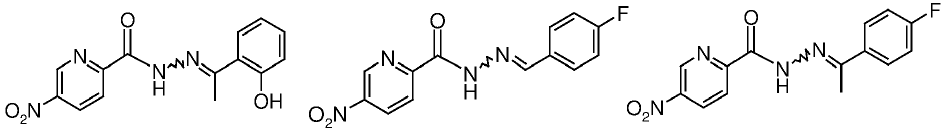

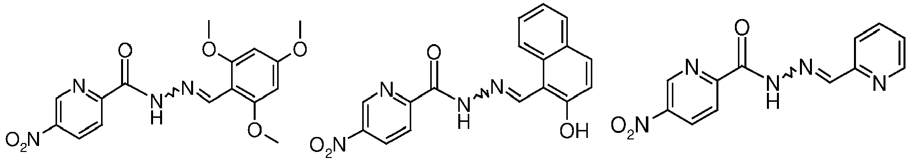

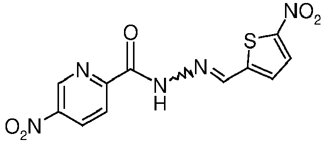

The present application further discloses inhibitors of the P210^{BCR-ABL-T315I} theramutein having the formula VII 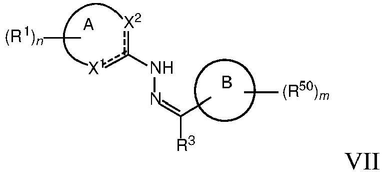 wherein:
ring A is a 5-, 6-, or 7- membered ring or a 7- to 12-membered fused bicyclic ring;
X¹ is selected from N, N-R° or C-R¹;
X² is selected from N, N-R° or C-R¹;
the dotted lines represent optional double bonds;
each R¹ is independently selected from the group consisting of H, alkyl, cycloalkyl, alkenyl, alkynyl, aralkyl, CN, CF₃, NO₂, OR¹¹, -(CH₂)*ₚ*C(O)(CH₂)*_{q}*Rⁿ, -(CH₂)*ₚ*C(O)N(R¹²)(R¹³), -(CH₂)*ₚ*C(O)O(CH₂)*_{q}*R¹¹,-(CH₂)*ₚ*N(R¹¹)C(O)R¹¹, -(CH₂)*ₚ*N(R¹²)(R¹³), -N(R¹¹)SO₂R¹¹, -OC(O)N(R¹²)(R¹³), -SO₂N(R¹²)(R¹³), halo, aryl, and a heterocyclic ring, and additionally or alternatively, two R¹ groups on adjacent ring atoms form a 5- or 6-membered fused ring which contains from 0 to 3 heteroatoms;
*n* is 0 to 6,
   each R¹¹ is independently selected from H, alkyl, cycloalkyl, alkenyl, alkynyl, aralkyl, aryl, and a heterocyclic ring;
   each R¹² and R¹³ are independently selected from H, alkyl, cycloalkyl, alkenyl, alkynyl, aralkyl, aryl, and a heterocyclic ring; or R¹² and R¹³ may be taken together with the nitrogen to which they are attached form a 5- to 7- membered ring which may optionally contain a further heteroatom;
   *p* is 0 to 4;
   *q* is 0 to 4;
ring B is selected from a cycloalkyl group having 5 or 6 ring atoms, and a heterocyclic group containing 5 or 6 ring atoms which includes one to three hetero atoms;
each R⁵⁰ is independently selected from the group consisting of alkyl, cycloalkyl, alkenyl, alkynyl, aralkyl, CN, CF₃, NO₂, OR⁵¹, -(CH₂)*ᵣ*C(O)(CH₂)*ₛ*R⁵¹, -(CH₂)*ᵣ*C(O)N(R⁵²)(R⁵³), -(CH₂)*ᵣ*C(O)O(CH₂)*ₛ*R⁵¹,-(CH₂)*ᵣ*N(R⁵¹)C(O)R⁵¹,-(CH₂)*ᵣ*N(R⁵²)(R⁵³),-N(R⁵¹)SO₂R⁵¹, -OC(O)N(R⁵²)(R⁵³), -SO₂N(R⁵²)(R⁵³), halo, aryl, and a heterocyclic ring, and additionally or alternatively, two R⁵⁰ groups on adjacent ring atoms form a 5- or 6-membered fused ring which contains from 0 to 3 heteroatoms;
   R⁵¹ is selected from H, alkyl, cycloalkyl, alkenyl, alkynyl, aralkyl, aryl, and a heterocyclic ring;
   R⁵² and R⁵³ are independently selected from H, alkyl, cycloalkyl, alkenyl, alkynyl, aralkyl, aryl, and a heterocyclic ring; or R⁵² and R⁵³ may be taken together with the nitrogen to which they are attached form a 5- to 7- membered ring which may optionally contain a further heteroatom;
   *r* is 0 to 4;
   *s* is 0 to 4;
*m* is 0 to 4; and
each R⁰ is independently selected from H, alkyl, cycloalkyl, aralkyl, aryl and a heterocyclic ring.

Exemplary compounds of the formula VII include the following structures:

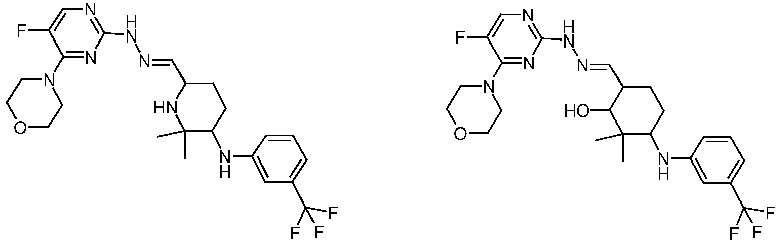

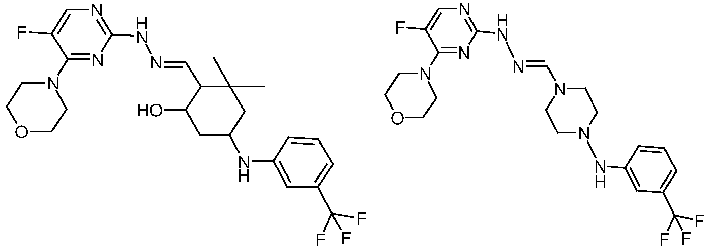

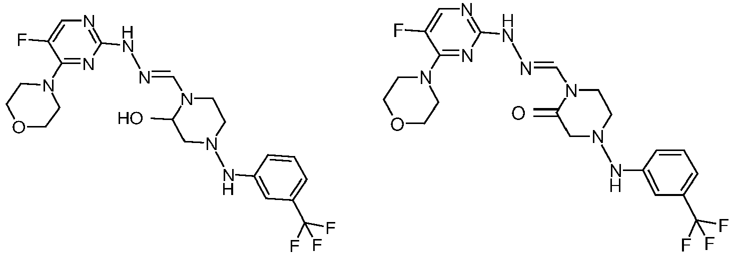

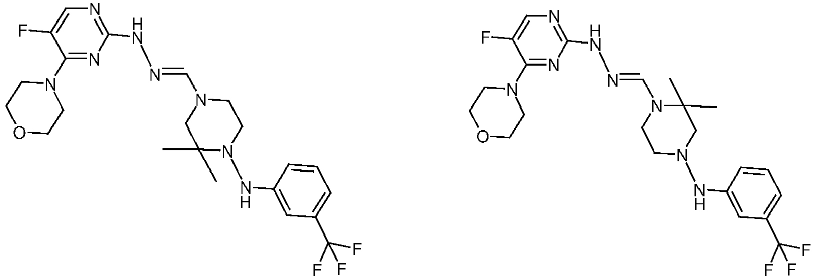

As used herein, the definition of each expression, e.g. alkyl, m, n, R, R' etc., when it occurs more than once in any structure, is intended to be independent of its definition elsewhere in the same structure.

For each of the above descriptions of compounds of the structures I, Iₐ, I_{b}, II, IIₐ, etc., each recitation of the terms halo, alkyl, cycloalkyl, alkenyl, alkynyl, aralkyl, aryl, heterocyclic group or heterocyclic ring, are independently selected from the definitions of these terms as provided in the beginning of this section.

It will be understood that chemical structures provided herein include the implicit proviso that substitution is in accordance with permitted valence of the substituted atom and the substituent(s), and that the substitution results in a stable compound, e.g., which does not spontaneously undergo transformation such as by rearrangement, cyclization, elimination, etc.

When one or more chiral centers are present in the compounds, the individual isomers and mixtures thereof (e.g., racemates, etc.) are intended to be encompassed by the formulae depicted herein.

When one or more double bonds are present in the compounds, both the cis- and trans- isomers are intended to be encompassed by the formulae depicted herein. Although chemical structures (such as, for example, structures II, IIₐ, V, Vₐ, VI, and VIₐ) are depicted herein in either cis of trans configuration, both configurations are meant to be encompassed by the each of the formulae.

In certain embodiments, compounds may exist in several tautomeric forms. Accordingly, the chemical structures depicted herein encompass all possible tautomeric forms of the illustrated compounds.

The compounds may generally be prepared from commercially available starting materials and known chemical techniques. Embodiments of the application may be synthesized as follows. One of skill in the art of medicinal or synthetic chemistry would be readily familiar with the procedures and techniques necessary to accomplish the synthetic approaches given below.

Compounds of the formula II may be prepared by reaction of an appropriate hydrazine compound, such as ***A***, and an appropriate aldehyde, such as ***B***, under conditions similar to those described on p. 562 of Gineinah, et al. (Arch. Pharm. Med. Chem. 2002, 11, 556-562). 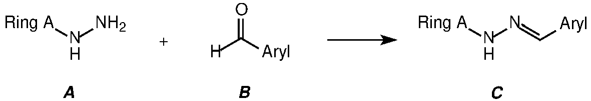 For example, heating ***A*** with 1.1 equivalents of ***B*** for 1 to 24 hours in a protic solvent such as a C₁ to C₆ alcohol, followed by cooling and collection of the precipitate, would afford ***C*.** Alternatively, product ***C*** may be isolated by evaporation of the solvent and purification by chromatography using silica gel, alumina, or C₄ to C₁₈ reverse phase medium. Similar methodology would be applicable in the cases where "Aryl" is replaced by other groups defined under R⁵.

Compounds of the formula III ring may be prepared by reaction of an appropriate hydrazine compound, such as ***D***, and an activated carboxylic acid such as ***E***, wherein LG is a leaving group such as halo, 1-oxybenztriazole, pentafluorophenoxy, *p-*nitrophenoxy, or the like, or Compound ***E*** may also be a symmetrical carboxylic acid anhydride, whereby conditions similar to those described on p. 408 of Nair and Mehta (Indian J. Chem. 1967 5, 403-408) may be used.

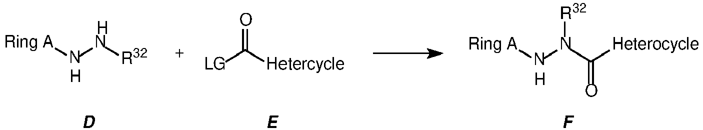

For example, treatment of ***D*** with an active ester such as Aryl-C(O)-OC₆F₅ in an inert solvent such as dichloromethane, 1,2-dichloroethane, or N,N-dimethylformamide, optionally in the presence of a base such as pyridine or another tertiary amine, and optionally in the presence of a catalyst such as 4-*N*,*N*-dimethylaminopyridine, at an appropriate temperature ranging from 0° C to the boiling point of the solvent, would afford ***F***, which may be isolated by evaporation of the solvent followed by chromatography using silica gel, alumina, or C₄ to C₁₈ reverse phase medium. The above active ester example of ***E*** would be readily prepared from the corresponding carboxylic acid and pentafluorophenol using a carbodiimide such as dicyclohexylcarbodiimide as a condensing agent.

Precursors such as ***A*** and ***D*** may be prepared by reaction of an appropriate nucleophile, for example, a hydrazine derivative, with a heteroaromatic compound bearing a halo substituent at a position adjacent to a nitrogen atom. For example, using methods analogous to those described by Wu, et al. (J. Heterocyclic Chem. 1990, 27, 1559-1563), Breshears, et al. (J. Am. Chem. Soc. 1959, 81, 3789-3792), or Gineinah, et al. (Arch. Pharm. Med. Chem. 2002, 11, 556-562), examples of compounds ***A*** and ***D*** may be prepared starting from, for example, a 2,4-dihalopyrimidine derivative, many of which are commercially available or are otherwise readily prepared by one skilled in the art. Thus, treatment of an appropriate 2,4-dihalopyrimidine derivative ***G*** with an amine or other nucleophile (Z), optionally in the presence of an added base, selectively displaces the 4-halo substituent on the pyrimidine ring. Subsequent treatment of the product with a second nucleophilic reagent such as hydrazine or a hydrazine derivative, optionally in a solvent such as a C₁ to C₆ alcohol and optionally in the presence of an added base, displaces the 2-halo substituent on the pyrimidine ring, to afford compounds that are examples of structures ***A*** and ***D*** above.

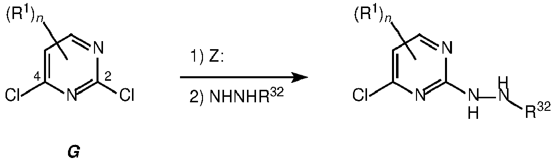

Embodiments wherein R² is -NR²² and R³ is -C(=R³³) can be synthesized by methods such as the following, or straightforward modifications thereof. The synthesis may be conducted starting from an appropriate ring A derivative ***J*** that bears a leaving group (LG) adjacent to the requisite ring nitrogen. Structure ***G*** above and the product of reaction of structure ***G*** with nucleophile Z, as illustrated above, are examples of such appropriate Ring A derivatives ***J***. Suitable LG' groups are halo, alkylthio, alkylsulfonyl, alkylsulfonate or arylsulfonate. Treatment of ***J*** with an amine R¹²NH₂ effects displacement of LG' to afford intermediates ***K***. An example of this chemical transformation wherein R¹² is H and LG' is CH₃SO₂- is reported by Capps, et al. J. Agric. Food Chem. 1993, 41, 2411-2415, and an example wherein R¹² is H and LG' is Cl is reported in Marshall, et al. J. Chem. Soc. 1951, 1004-1015.

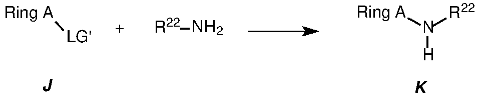

Intermediates of structure ***K*** are transformed to the compounds by simultaneous or sequential introduction of the elements, of R³, R⁴, and R⁵. For example, treatment of intermediates of structure ***K*** with individual isocyanates R⁶-N=C=O affords in a single step compounds of structure ***M***, which are compounds wherein R² = -NR²²-, R³ = - C=O- , R⁴ = -NH-, and R⁵ = -chemical bond-R⁶. Alternative methods to convert compounds of structure ***K*** to compounds of structure ***M*** are well known to those skilled in the art, wherein R³ together with a leaving group (for example *p*-nitrophenoxy or chloro) is first introduced, followed by subsequent displacement of the leaving group by, for example, an amine R⁶-NH₂, to introduce R⁵ and R⁶.

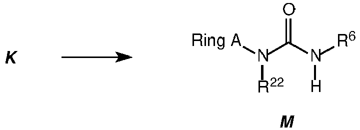

Alternatively, treatment of intermediates of structure ***K*** with a reagent such as cyanamide (NH₂-CN), typically under conditions of heating and optionally in the presence of acid in a solvent such as ethyl acetate or dioxane, affords intermediates ***N***. Alternatives to cyanamide are nitroguanidine or amidinosulfonic acid (NH₂-C(=NH)-SO₃H). An example of such a transformation using cyanamide is reported by Latham et al., J. Org. Chem. 1950, 15, 884. An example using nitroguanidine is reported by Davis, Proc. Natl. Acad. Sci. USA 1925, 11, 72. Use of amidinosulfonic acid was reported by Shearer, et al. Bioorg. Med. Chem. Lett. 1997, 7, 1763.

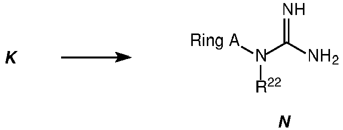

In analogy to the conversion of intermediates ***A*** or ***D*** to embodiments represented by ***C*** or ***F***, intermediates ***K*** are converted, respectively, to compounds represented by ***P*** or ***Q**,* which are further embodiments of the application.

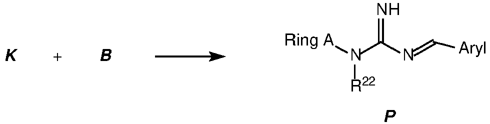

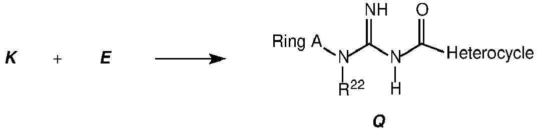

Treatment of ***A*** or ***K*** with a ketone ***S***, wherein R is as defined above, in place of an aldehyde ***B*** in the schemes above, affords compounds of structure ***T*** or ***U**,* respectively, which are further embodiments of the application.

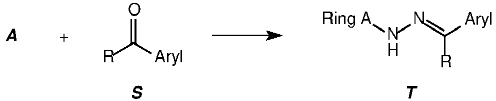

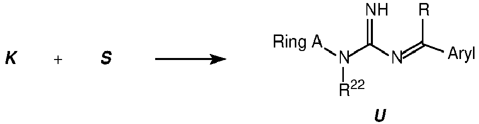

The non-guanidino carbon-nitrogen double bond of U can be selectively reduced by an appropriate reducing agent such as a metal (boron, aluminum, silicon, etc.) hydride reagents, preferably one with basic properties, to afford compounds **V** of the application.

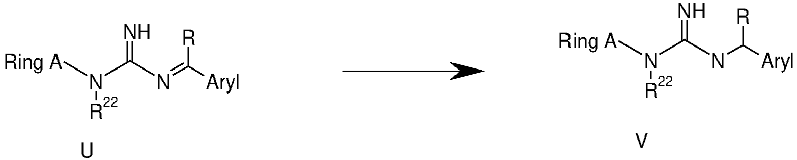

Embodiments wherein R² = CO, R³ = -NR³²⁻, R⁴ = N-, and R⁵ = ZR⁷, wherein Z is a hydrocarbon chain and R⁷ is as defined above, may be prepared as follows. When R³² = H, a Ring A-derived carboxylic acid **W** is activated by conversion to the corresponding acid chloride, or alternatively to an active ester, or to an analogous activated derivative, many of which are well known in the art. Treatment of the activated carboxylic acid with hydrazine affords the corresponding hydrazide **Y.** Treatment of **Y** with an aldehyde or ketone (under conditions of heating and/or mild acid catalysis if necessary) affords the desired final product **Z.**

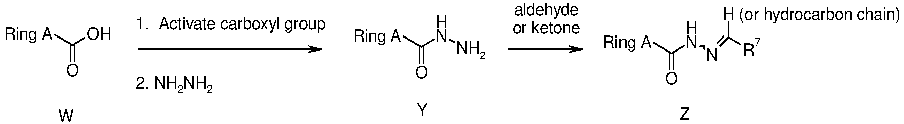

If not commercially available, Ring A-derived carboxylic acids **W** may be prepared by treatment of starting material **J** above with cyanide ion, optionally with heating or transition metal catalysis, to replace the leaving group LG' with a cyano residue. Basic or acidic hydrolysis of the cyano group affords the desired carboxylic acid intermediate **W.**

When R³² is not H, then a protected form of monosubstituted hydrazine may be used in the above scheme in place of hydrazine,. Thus, treatment of the activated carboxylic acid from **W** with R³²NHNH-PG, where PG is a nitrogen protecting group such as benzyloxycarbonyl or t-butyloxycarbonyl, followed by deprotection and treatment with an appropriate aldehyde or ketone as above affords **Z',** a further embodiment of the application.

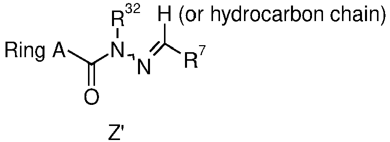

It will be apparent to a practitioner skilled in the art of organic molecule synthesis that the reaction processes illustrated above are representative of a broader set of methods that are logical extensions of the illustrated processes. Thus, additional embodiments that incorporate additional variants in R², R³, R⁴, and R⁵ are prepared by obvious modifications of the above processes.

As would be recognized by a person of ordinary skill, it may be advantageous to employ a temporary protecting group in achieving the final product. The phrase "protecting group" as used herein means temporary modifications of a potentially reactive functional group which protect it from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T. W.; Wuts, P. G. M. Protective Groups in Organic Synthesis, 2nd ed.; Wiley: New York, 1991).

One embodiment of this application is directed to any endogenously occurring mammalian target protein selected by the skilled investigator to be of interest for the identification and/or optimization of a compound as an inhibitor or activator of said protein. In general such selected proteins will already be known to be involved in the etiology or pathogenesis of a human disease. In another embodiment, the application is also directed toward mutant forms of such mammalian proteins. A "mutein" is a protein having an amino acid sequence that is altered as a result of a mutation that has occurred in its corresponding gene (Weigel et al, 1989). Such mutations may result in changes in one or more of the characteristics of the encoded protein. For example, an enzyme variant that has modified catalytic activity resulting from a change in one or more amino acids is a mutein.

This invention is concerned with proteins harboring an alteration of at least one amino acid residue (the terms "amino acid sequence change" or "amino acid sequence alteration" include changes, deletions, or additions, of at least one amino acid residue, or any combination of deletions, additions, changes) such that the resulting mutein has become (as a result of the mutation) resistant to a known therapeutic agent relative to the sensitivity of the non-mutated version of said protein to the therapeutic agent. This specialized class of muteins is hereinafter referred to as a ***theramutein**,* and the corresponding protein lacking the mutation is referred to herein as a ***prototheramutein**.*

As used herein, "prototheramutein" refers to an endogenously occurring protein in a cell that is susceptible to mutation that confers relative insensitivity (i.e. resistance) to a therapeutic compound which otherwise inhibits or activates the protein. Accordingly, "theramutein" refers to an endogenously occurring protein or portion of a protein in a cell that contains at least one amino acid sequence alteration relative to an endogenous form of the protein, wherein the amino acid sequence change is or was identified or becomes identifiable, and is or has been shown to be clinically significant for the development or progression of a given disease, *following exposure of at least one human being to a substance that is known to inhibit or activate the prototheramutein.* Solely for the purposes of defining the preceding sentence, a substance need not be limited to a chemical agent for the purposes of first defining the existence of a theramutein. Thus, ***by definition***, a theramutein is a protein which harbors a mutation in its corresponding endogenous gene, wherein said mutation is associated with the development of clinical resistance in a patient to a drug that is normally able to activate or inhibit the non-mutated protein. With respect to a given theramutein, the term "corresponding prototheramutein" refers to the prototheramutein which, through mutation, gives rise to said theramutein. Similarly, with respect to a given prototheramutein, the "corresponding theramutein" refers to the theramutein which has arisen by mutation from said prototheramutein.

Accordingly, it is apparent to a skilled artisan that, as the genes which encode theramuteins are limited to endogenously occurring genes, the definition of a theramutein excludes proteins encoded by disease-causing infectious agents such as viruses and bacteria. As used herein, the term "endogenous gene" refers to a gene that has been present in the chromosomes of the organism at least in its unmutated form, since inception. The term "cell" as used herein refers to a living eukaryotic cell whether in an organism or maintained under appropriate laboratory tissue or organ culture conditions outside of an organism.

In one embodiment of the invention, the target protein (POI) may be any endogenously encoded mammalian protein. In another aspect of the invention, the POI is a theramutein, which is a protein that is altered for the first time with respect to a commonly occurring "wild type" form of the protein (*i.e.,* a wild type protein is the prototheramutein from which the theramutein arises). In yet another aspect of the invention, a theramutein is a variant of a protein that is, itself, already a mutein (*i.e.,* a mutein is the prototheramutein from which the theramutein arises). In still another embodiment, a theramutein may be further mutated as compared to a previously existing theramutein. In such instances, the first theramutein (such as the T315I mutant of p210 BCR-ABL (see below), may be thought of as a "primary" theramutein, whereas subsequent mutations of the (already mutated) T315I variant may be termed a secondary theramutein, tertiary theramutein, etc. As exemplified below, a mutein of the invention is a variant of Bcr-Abl tyrosine kinase that escapes inhibition by an inhibitor of the "wild type" Bcr-Abl. Such a Bcr-Abl mutein is altered with respect to a more common or "wild type" form of Bcr-Abl (which is also a mutein as well) in such a way that a property of the protein is altered.

It is understood that a protein of interest (POI) is an endogenously encoded mammalian protein. It will also be understood that a mutein of primary interest is a theramutein that may have the same, increased, or decreased specific activity relative to its prototheramutein, and that it is not inhibited or is poorly inhibited by an agent that is used to inhibit the prototheramutein. Likewise, another theramutein of primary interest is one that has the same, increased or decreased specific activity (relative to its prototheramutein) and that is not activated or is poorly activated by an agent that is used to activate the prototheramutein. Other variations are obvious to the skilled artisan. It will be further appreciated that theramuteins can include naturally occurring or commonly observed variants of a protein, for example, variants that are expressed from different alleles of a particular gene. In some cases such variants may be unremarkable with respect to their normal cellular function, with functional differences becoming apparent only in the presence of agents that differentially inhibit or activate the cellular function of the variants. For example, naturally occurring variants of a particular enzyme may have activity profiles that are not substantially different, but a therapeutic agent that modulates one may be ineffective in modulating the other.

It will be appreciated that one aspect of the invention is the identification of an agent that is active against a selected POI whose cellular function contributes to a given disease state such that activators or inhibitors of said POI would be expected to be therapeutically effective during the course of treatment for the disease. No limitation of any kind or nature is intended on the type of disease that may be treated, nor on the type of protein that may be targeted for modulation according to the teachings herein, provided that all other limitations stated herein are met, including the fact that any such protein that is selected for targeting must be an endogenous protein. Obviously, the skilled investigator may use non-endogenously occurring nucleic acids such as cDNAs in order to practice the method taught herein provided that the amino acid sequence corresponds to an endogenously occurring POI.

It will also be appreciated that, whereas one aspect of the invention is the identification of an agent that is active against a protein or theramutein that arises or becomes dominant (by any mechanism) prior to or during the course of a treatment for a given disease, another aspect is the identification of an agent that is active against a mutein that is common within a population of unafflicted individuals, but wherein said mutein is less susceptible to modulation by an approved drug, and where the variation in the activity profile of the mutein becomes important (and is therefore first identified as being a theramutein) in a disease state such as where it is overexpressed or participates in a signaling process which has otherwise become abnormally regulated. For example, a neoplastic disease may be caused by abnormal regulation of a cellular component other than the theramutein or its prototheramutein, and still be treatable with an inhibitor of the prototheramutein, whereas the same treatment would be less effective or ineffective where the theramutein was present. This can be an issue where it is observed that the response of a particular tumor type to an anticancer agent varies among individuals that express different variants of an enzyme against which the anticancer agent is directed (Lynch et al., 2004). Here, the variants would not have arisen or become predominant during the course of treatment of the disease, but are preexisting in the healthy population and are detected only by their altered responsiveness to a particular course of established therapeutic treatment.

As used herein, the terms "agonist" and "activator" of a protein are used interchangeably. An activator (agonist) is limited to a substance that binds to and activates the functioning of a given protein. Unless explicitly stated otherwise, an "activator", an "agonist", and an "activator of a protein" are identical in meaning. The activation by an activator may be partial or complete. Likewise, as used herein, the terms "antagonist" and "inhibitor" of a protein are used interchangeably. An inhibitor (antagonist) is limited to a substance that binds to and inhibits the functioning of a given protein. To state that a substance "inhibit(s)" a protein means the substance binds to the protein and reduce(s) the protein's activity in the cell without materially reducing the amount of the protein in the cell. Similarly, to state that a substance "activate(s)" a protein, such as a prototheramutein or theramutein, is to state that the substance increases the defined function of the protein in the cell without substantially altering the level of the protein in the cell. Unless explicitly stated otherwise, an "inhibitor", an "antagonist" and an "inhibitor of a protein" are also synonymous. The inhibition by an inhibitor may be partial or complete. A modulator is an activator or an inhibitor. By way of example, an "activator of PKC_{β1}" should be construed to mean a substance that binds to and activates PKC_{β1}. Similarly, an "inhibitor of p210^{Bcr-Ab1}" is a substance that binds to and inhibits the functioning of p210^{Bcr-Ab1}. To state that a substance "inhibits a protein" requires that the substance bind to the protein in order to exert its inhibitory effect. Similarly, to state that a substance "activates protein X" is to state that the substance binds to and activates protein X. The terms "bind(s)," "binding," and "binds to" have their ordinary meanings in the field of biochemistry in terms of describing the interaction between two substances (*e.g*., enzyme-substrate, protein-DNA, receptor-ligand, etc.). As used herein, the term "binds to" is synonymous with "interacts with" in the context of discussing the relationship between a substance and its corresponding target protein. As used herein, to state that a substance "acts on" a protein, "affects" a protein, "exerts its effect on" a protein, etc., and all such related terms uniformly mean (as the skilled investigator is well aware) that said substance activates or inhibits said protein.

The concept of inhibition or activation of a mutated form of an endogenous protein to a greater extent than the corresponding non-mutated counterpart protein is defined for the first time and referred to herein as a positive "***specificity gap***." In general terms, *and using an inhibitor case as an example*, the ***specificity gap*** refers to the difference between the ability of a given substance, under comparable conditions to inhibit the theramutein in a cell-based assay system of the invention as compared to either:
a) the ability of the same substance under comparable conditions to inhibit the prototheramutein; or
b) the ability of a second substance (usually a known inhibitor of the prototheramutein) to inhibit the theramutein under comparable conditions; or
c) the ability of the second substance to inhibit the prototheramutein under comparable conditions.

When the comparison is made between the effects of two distinct substances (tested individually) on the theramutein alone, the result is termed a ***homologous specificity gap*** determination.

Alternatively, when a comparison is made between the effects of two distinct substances (generally, but not always), one of which is tested on the theramutein and the other on the prototheramutein, respectively, the result is termed a ***heterologous specificity ga**p* (SG) determination. Thus, (a) and (c) as given above are examples of heterologous specificity gap (SG) determinations (although (a) uses the same substance in both instances), whereas (b) is an example of a homologous specificity gap determination.

Reference to Figure 3 is informative in understanding and elucidating these concepts.

Analogous issues apply when the case concerns an activator. It will be immediately obvious to the skilled artisan that the term "comparable conditions" includes testing two different compounds, for example, at the same concentration (such as comparing two closely related compounds to determine relative potency), or by comparing the effects of two different compounds tested at their respective IC₅₀ values on the corresponding prototheramutein and theramutein. Accordoing to the method of the invention as characterized in the claims, the CSG is quantified by dividing the IC₅₀ of the compounds on the phenoresponse of the control cell by the IC₅₀ of the compounds on the phenoresponse of the test cell. The skilled investigator will easily recognize other useful variations and comparable conditions.

Thus, in one embodiment of the application of this approach, substances that are more effective against a theramutein have a "positive specificity gap." A "zero, null or no" specificity gap indicates that there is no significant measurable difference between the effect of a substance on the theramutein as compared to its effect on the prototheramutein (however such compounds may be quite useful in their ability to inhibit or activate both a theramutein and its corresponding prototheramutein), and a "negative specificity gap" indicates a substance that at a given concentration is less effective against the given theramutein than against a form of the corresponding prototheramutein or other comparative form of the theramutein (such as one that may harbor a different mutation). The latter category is generally of lesser interest than the former categories of compounds, except in the case where the compound is so potent that its relatively lesser effect on the theramutein is of no real concern from the perspective of therapeutic efficacy. The skilled investigator can easily recognize a variety of approaches to quantifying the specificity gap assessment in a manner tailored to his or her needs. Such an analysis may assist the skilled investigator in classifying various compounds into discrete categories that may be helpful in guiding further lead optimization or biological profiling studies on such compounds.

The invention also provides a means for identifying compounds that exhibit a desired specificity gap. Such compounds can be identified and their ability to inhibit or activate the theramutein determined using an *in vitro* cell-based assay system where the effect of a substance on the cellular functioning of the mutated endogenous form of the protein is compared to the effect of the same drug on the cellular functioning of a non-mutated endogenous form of the protein.

Thus, the system enables the discovery of compounds capable of binding to a theramutein and exerting a greater modulatory effect on the cellular functioning of said theramutein than on its corresponding prototheramutein. Further, the system enables the discovery of compounds capable of binding to a theramutein and exerting at least as great or greater modulatory effect on the cellular functioning of a theramutein than previously known compounds are able to exert on the corresponding prototheramutein. In a particular embodiment of the invention, a compound may be screened for and identified that 1) is at least as effective against the theramutein as the original drug is against the prototheramutein, and/or 2) is similarly effective against the prototheramutein as against the theramutein (*i.e.,* displays a small or essentially zero specificity gap).

In an embodiment of the invention, cells that overexpress a theramutein of interest are used to identify chemical agents that are inhibitors or activators of (*i.e.,* that bind to and inhibit or that bind to and activate) at least the selected theramutein. The chemical agents may also be inhibitors or activators of the prototheramutein or even other theramuteins of the same prototheramutein. As used herein, the terms "chemical agent" and "compound" are used interchangeably, and both terms refer exclusively to substances that have a molecular weight up to, but not including, 2000 atomic mass units (Daltons). Such substances are sometimes referred to as "small molecules." Unless otherwise stated herein, the term substance as used herein refers exclusively to chemical agents/compounds, and does not refer to ***biological agents**.* As used herein, "***biological agents***," are molecules which include proteins, polypeptides, and nucleic acids, and have molecular weights equal to or greater than 2000 atomic mass units (Daltons).

In one embodiment of the invention, a theramutein is selected and used in a phenoresponse-based cellular assay system of the present invention designed to identify agents that are inhibitors or activators of the theramutein. Where two or more distinct theramuteins originating from the same prototheramutein are known, it is preferable to select the most resistant theramutein available for use in the assay system. In general, the degree of resistance of a theramutein to a given chemical agent is determined relative to its non-mutated counterpart (prototheramutein) using the drug that was first administered and known to inhibit or activate the prototheramutein and against which the theramutein "arose." The methods of determining the degree of such resistance, for example by analysis of IC₅₀ or AC₅₀ values, are well known and standard in the art and will not be reiterated herein. However, no causal relationship is necessary or should be inferred between the treatment of the patient with a given therapeutic agent *per se* and the subsequent appearance of a theramutein. Rather, what is required in order to practice the invention as it pertains to theramuteins is that a true theramutein be properly selected according to the teachings herein.

Thus, for example, randomly generated site directed mutants of known proteins that are created in the laboratory but that have ***not*** been shown to be clinically relevant are not appropriate muteins for use within this invention. Such muteins would not, of course, be properly classified as theramuteins either.

For example, in an effort to obtain potential inhibitors of mutants of p210^{Bcr-Abl}, Huron et al. (2003) used a recombinant c-abl preparation and screened a series of compounds known to inhibit c-src tyrosine kinase activity. The authors performed c-abl kinase assays on their compounds and identified the most potent compound as an 8 nM inhibitor against c-abl. When this compound (PD166326) was tested against various p210^{Bcr-Abl} theramuteins, however, it showed activity against some of the mutants such as p210^{Bcr-Abl-E255K}, but the p210^{Bcr-Abl-T315I} theramutein was found to remain 10 fold more resistant (Huron et al. 2003, Table 3). Furthermore, in each case the compound was still markedly ***less effective*** on the p210^{Bcr-Abl} theramuteins than it was against the wild-type p210^{Bcr-Abl}. When the compound was tested against p210^{Bcr-Abl-T315I} mutant activity, it was unable to inhibit the activity to any appreciable extent (p. 1270, left hand column, second paragraph; see also Fig. 4.). Wolff et al. (2005) described that PD166326 had greater antileukemic activity than imatinib mesylate in a murine model of chronic myeloid leukemia and leukemia and confirmed that P210/T315I murine CML was resistant to either PD166326 or imatinib mesylate treatment. Thus, the disclosed compound was able to inhibit a theramutein that is partially resistant to STI-571, but had no activity against the T315I mutant of Bcr-Abl., which was already known at that time to be the theramutein that exhibited the most resistance to STI-571. Hence purely and simply, the Huron methodology failed to identify an effective inhibitor of the p210^{Bcr-AblT315I} theramutein. La Roseé et al. (2002) described the activity of a similar pyrido-[2,3-d]pyrimidine compound, PD180970 using isolated kinase domains and cell lines expressing Bcr-Abl with various mutations identified in relapsed patients. Also here, the p210^{Bcr-Abl-T315I} mutant was shown to be resistant to both imatinib and PD180970. Bubnoff et al. (2003) examined further pyrido-[2,3-d]pyrimidine derivatives, including PD166326 and PD180970, whether they perform superior to imatinib in the potency of inhibition of Bcr-Abl and whether activity is maintained against clinically relevant mutant forms of BCR-ABL, which cause resistance toward imatinib. Again, it was confirmed that the p210^{Bcr-AblT315I} theramutein is not affected by that class of compounds.

Indeed, prior to the disclosure of this invention, including both the detailed methodology described for the first time herein as well as the compositions provided herein, *no one anywhere in the world* has been successful in identifying a chemical agent, let alone a methodology that is capable of identifying a chemical agent that effectively inhibits the p21O^{Bcr-AblT315I} theramutein to an equal or greater extent than STI-571 is able to do with respect to the wild type p210^{Bcr-Abl} protein. (See Shah et al., Science, July, 2004; O'Hare et al., Blood, 2004; Tipping et al., Leukemia, 2004; Weisberg et al., Leukemia, 2004).

It cannot be overemphasized that such compounds would be immensely useful, because at the present time there is no alternative for patients who progress to p210^{Bcr-Abl-T315I} theramutein-mediated imatinib mesylate-resistant status. Once patients develop such resistance, there is no other effective alternative treatment available, and death is certain. The method described herein provides the first reported approach to identify, pharmacologically characterize and chemically synthesize effective inhibitors of the p210^{Bcr-Abl-T315I} theramutein. Moreover, the skilled investigator will immediately recognize the applicability and generalizability of this approach to any highly drug-resistant theramutein. Finally, the skilled investigator will further recognize that linking a phenoresponse as defined herein to the increased presence and functional activity of a particular POI in the cell under appropriate conditions allows one to utilize the method with any given endogenous target protein for which a therapeutically effective compound is sought.

In the present invention, a test cell is used that displays a carefully selected phenotypic characteristic (as defined below) which is linked to the presence and functional activity of the particular protein-of-interest (POI) or theramutein-of-interest (TOI) in the cell under appropriate conditions. With respect to a theramutein, this should be qualitatively the same as the phenotypic characteristic displayed by a cell that expresses the prototheramutein. A phenotypic characteristic (i.e. a non-genotypic characteristic of the cell) is a property which is observed (measured), selected and/or defined for subsequent use in an assay method as described herein. Expression of the phenotypic characteristic is responsive to the total activity of the protein in the cell, and is a result of the absolute amount of the protein and its specific activity. Often, the phenotypic characteristic is observable as a result of elevated levels of protein activity and is not apparent in cells that express low amounts of the protein, or if the protein is also a theramutein, then the phenotypic characteristic will often not be apparent in cells expressing low amounts of either the theramutein or its corresponding prototheramutein. Further, it can often be demonstrated that the phenotypic characteristic is modulated by modulating the specific activity of the protein with an inhibitor or activator of the theramutein, although this is not always the case since an inhibitor or activator of the TOI may not always be available at the time the skilled investigator undertakes such a project. (However, clearly a known inhibitor or activator of a given prototheramutein will always exist as a result of the intrinsic definition of the nature of a theramutein itself.) Thus, for the purpose of defining the phenotypic characteristic to be subsequently used with a given test cell for assay purposes, the skilled investigator may also use a substance capable of increasing or decreasing the expression of the gene encoding a given POI (such as a theragene in the case of a theramutein), which will in turn lead to increases or decreases of the level of the corresponding theramutein. This allows the skilled investigator to simulate the effects of certain types of activators or inhibitors of the theramutein (such as a suicide inhibitor of the theramutein, which is a class of chemical agent which binds irreversibly and covalently modifies the TOI, rendering it permanently inactive), without actually having access to such a compound, for the purposes of refining the appropriate phenotypic characteristic for subsequently establishing a useful cellular assay system. Examples known to one of ordinary skill that would be helpful for such purposes include the use of anti-sense DNA oligonucleotides, small interfering RNAs, other RNA interference-based methodologies, and vector constructs containing inducible promoter systems. In this manner, the selected phenotypic characteristic is linked to the activity of the theramutein in the test cell. Notably for theramuteins, the selected phenotypic characteristic is usually also displayed by a cell that overexpresses the prototheramutein and in which the phenotypic characteristic is modulated by known inhibitors or activators of the prototheramutein.

A phenotypic characteristic is simply a characteristic of a cell other than a genotypic characteristic of the cell. Except for the specific requirements of a properly defined phenotypic characteristic as disclosed herein for the purposes of creating useful cellular assay systems according to the teachings of certain of the embodiments of the invention, no other limitation of the term phenotypic characteristic of any kind or nature is intended or appropriate in order to properly and effectively practice the invention. Indeed, the skilled artisan must be able to select any characteristic of the cell that maximizes the utility of establishing the proper cell-based assay for his or her needs. The phenotypic characteristic can be quantitative or qualitative and be observable or measurable directly (e.g., observable with the naked eye or with a microscope), but most commonly the characteristic is measured indirectly using standard automated laboratory equipment and assay procedures which are known to those of skill in the art. The term "observable" means that a characteristic may be measured or is otherwise detectable under appropriate conditions by any means whatsoever, including the use of any type of laboratory instrumentation available. The term "detectable" is not the same as "detected." A characteristic may be detectable to a skilled artisan without being detected at any given time, depending upon how the investigator chooses to design the assay system. For example, in searching for activators of a POI such as a prototheramutein (or theramutein), it may be desirable to have the relevant phenotypic characteristic detected only after the addition of a known activator or test substance capable of activating the POI. This provides the ability to maximize the intensity of the signal that is generated by the test cell in the assay.

Phenotypic characteristics include but are not limited to growth characteristics, transformation state, differentiation state, substrate phosphorylation state, catalytic activity, ion flux across the cell membrane (calcium, sodium, chloride, potassium, hydrogen ions, etc.), pH changes, fluctuations of second messenger molecules or other intracellular chemical species such as cAMP, phosphoinositides, cyclic nucleotides, modulations of gene expression, and the like. The characteristic of the cell may be observable or measurable continuously (*e.g*., growth rate of a cell), or after a period of time (*e.g*., terminal density of a cell culture), or transiently (*e.g*., modulation of a protein causes a transient change in phosphorylation of a substrate of the protein, or a transient flux in ion flow across the membrane, or elevations or reductions in intracellular cAMP levels). In certain embodiments, a selected phenotypic characteristic may be detected only in the presence of a modulator of the protein. No limitations are intended with respect to a characteristic that may be selected for measurement. As used herein, the terms "characteristic of a cell" and "phenotypic characteristic", and simply "characteristic", when used to refer to the particular measurable property of the intact cell or a subcellular fraction of the cell following the treatment of a test cell with a substance, are identical. For example, a phenotypic characteristic can be focus formation that becomes observable when a cell that over expresses a selected protein is cultured in the presence of an activator of the protein, or it may be a transient increase or decrease in the level of an intracellular metabolite or ion, such as cAMP, calcium, sodium, chloride, potassium, lithium, phosphatidylinositol, cGMP, bicarbonate, etc. It is obvious to one of ordinary skill in the art that after a cell is exposed to a test substance, the characteristic so measured (assayed) may be determined on a sub-cellular fraction of the cell. However, the initial treatment of the cell with a substance, which thereby causes the substance to come into contact with the cell, must be performed on the intact cell, not a sub-cellular fraction.

The characteristic selected for measurement within the cell must not be an intrinsic physical or chemical property of the protein (or theramutein or prototheramutein) itself (such as the mere amount (mass) of the protein inside the cell), but rather must be a characteristic that results from the activity of the protein (or theramutein or prototheramutein) inside the cell, thus affecting a characteristic of the cell which is distinct from the theramutein itself, as discussed in detail above. For example, where the theramutein is a protein kinase that is capable of undergoing autophosphorylation, a process whereby the enzyme is capable of catalyzing the phosphorylation of itself by transferring a terminal phosphate group from ATP onto itself, it would NOT be appropriate to select the phosphorylation state of the TOI as an appropriate phenotypic characteristic of the cell for measurement. This is because such a characteristic does not reflect the activity of the TOI on other cellular components. As the skilled investigator knows, autophosphorylation is not necessarily reflective of the activity of a protein kinase in a cell, since mutants of protein kinases are known that retain enzymatic activity sufficient to undergo autophosphorylation, yet have lost the capability to engage in signal transduction events within the cell. The classic paper by White et al. (1988) is both educational and noteworthy in this respect.

The term "responsive phenotypic characteristic" means a characteristic of the cell which is responsive to inhibitors or activators of a given protein (including, *e.g.,* a prototheramutein or theramutein). The term "known therapeutic agent" is defined as any agent that has been administered to a human being for the treatment of a disease in a country of the world.

A useful phenotypic characteristic, as exemplified herein in association with p210^{Bcr-Abl} and theramuteins thereof, is disregulation of cell growth and proliferation. It is noted that the same or similar assay may be appropriate for use with many different proteins of interest. For example, disregulations of growth, proliferation, and/or differentiation are common phenotypic characteristics that may result from overexpression of a variety of different cellular proteins. It is an important teaching of this invention that by overexpressing a selected protein in order to cause the appearance of such a phenotypic characteristic, the characteristic becomes linked to the presence, amount, and specific activity of that selected protein under suitable conditions, and this linkage allows the skilled investigator to identify inhibitors or activators of a protein of interest (POI) as desired. Accordingly, the phenotypic characteristic is responsive to changes in the level and/or specific activity of the selected protein. Such a responsive phenotypic characteristic, when also demonstrated to be responsive to a known modulator of the POI is referred to herein as a "***phenoresponse***." In the special case of a theramutein which has no known modulator, a modulator of the prototheramutein must be utilized to establish a phenorespone to be used with the theramutein. The conception and recognition of this highly useful property of a cell represents one of the substantial advances of this invention over the prior art, including Applicant's own prior original work in the general area of cell-based assays (U.S. Pat. Nos. 4,980,281; 5,266,464; 5,688,655; 5,877,007). The identification and selection of the phenoresponse provides the skilled investigator with a cellular assay system that is extremely sensitive in terms of its ability to identify inhibitors or activators of the POI, and therefore identifies such chemical agents with a much higher degree of assurance than any other related assay method disclosed in the prior art.

Though not always necessary, it will often be advantageous to employ cells that express high levels of the POI, and to select a phenotypic characteristic that results from overexpression of the POI. This is because phenotypic characteristics linked to the functioning of the POI generally become more distinguishable (easier to measure) as a POI is overexpressed to a greater extent. Further, phenoresponses that are observed in response to modulators of the POI are often amplified as the functional level of the POI is increased. Expressed another way, the selected phenoresponse observed in cells that overexpress the protein (or theramutein) is particularly sensitive to modulators of the protein (or theramutein).

Preferably, the protein is stably expressed in a test cell. Stable expression results in a level of the protein in the cell that remains relatively unchanged during the course of an assay. For example, stimulation or activation of a component of a signaling pathway may be followed by a refractory period during which signaling is inhibited due to down-regulation of the component. For proteins of the invention, such down-regulation is usually sufficiently overcome by artificially overexpressing the protein. Expressed another way, the expression is sufficiently maintained that changes in a phenotypic characteristic that are observed during the course of an assay are due primarily to inhibition or activation of the protein, rather than a change in its level, even if down-modulation of the protein subsequently occurs. For these reasons, although stable expression of the protein is preferred, transfection followed by transient expression of the protein may be employed provided that the selected phenotypic characteristic is measurable and the duration of the assay system is short relative to the progressive decline in the levels of the transiently expressed protein that is to be expected in such systems over time. For these reasons, stably expressing cell lines are preferred (U.S. Patent No. 4,980,281).

The term "cellular specificity" means the ability of a compound, at a given concentration, to modulate a selected phenoresponse of the Test cell without affecting the Control Cell to the same extent, if at all. The term "cellular specificity gap" ("CSG") means a measurement of the ability of a selected compound to modulate the selected phenoresponse corresponding to a given target protein (not limited to a theramutein) in a test cell relative to the ability of said compound to modulate the same phenoresponse in a corresponding control cell. For the purposes of applying the CSG technique to non-theramutein endogenous target proteins, the selected phenoresponse must have been previously defined using a known inhibitor or activator of the target protein.

Determination of the CSG provides the skilled investigator with a method of comparing the relative potential therapeutic value of different compounds within a group of compounds (two or more) by comparing their relative cross-reactivity with control cells irrespective of the potency against the target protein of any given compound within the group. In accordance with the method of the present invention as characterized in the claims, the CSG is quantified by dividing the IC₅₀ of the compounds on the phenoresponse of the control cell by the IC₅₀ of the compounds on the phenoresponse of the test cell.Compounds that exhibit the greatest "specificity" in their activity against test cells relative to control cells are generally the most desirable compounds, since a "wide" CSG will assist in selecting a compound that may reasonably be assumed to have minimal potential side effects in patients as compared to other compounds within the aforementioned group that have "narrow" CSGs. The effects of the CSG measurement are seen most easily when comparing cell-based assay generated dose-response curves in their entirety, however the following hypothetical example is also instructive.

Consider the following table of hypothetical compounds and their corresponding IC₅₀ values using a cell-free assay system. This example uses a protein kinase as the target protein. This is the sort of situation that investigators skilled in the art are faced with on a daily basis when dealing with the problem of trying to perform lead optimization on a selected compound or group of compounds for the purpose of identifying a potential optimized lead candidate compound for subsequent pre-clinical (animal) and clinical studies.

| **Table 1. Cell-Free Purified Protein Kinase Inhibition Assay.** | | | |
|---|---|---|---|
| **Compound** | **IC₅₀ against Target Protein Kinase (nM)** | **IC₅₀ against a Non-Target Protein Kinase (nM)** | **IC₅₀ Ratio** |
| A | 0.2 | 10,000 | 50,000 |
| B | 3 | 10,000 | 3,333 |
| C | 250 | 10,000 | 40 |
| D | 500 | 10,000 | 20 |

A standard approach in the art at the present time is to identify compounds that exhibit a high degree of *potency* with respect to inhibition of the target protein kinase's enzymatic activity in a cell-free assay system without showing significant inhibitory activity against a distinct but closely related protein kinase. As the results of the cell-free assay system shown above in Table 1 indicate, compound A is the most potent of the series of compounds (A,B,C,D) and also shows the largest difference between its IC₅₀ against the target protein relative to its effect on the non-target protein. For example, if one were interested in identifying inhibitors of the Abl kinase, one might use another protein kinase, such as the EGF receptor, c-kit, or c-Src, as a "negative" control kinase in such an assay. As with c-Abl, all of these latter enzymes are tyrosine protein kinases. Indeed, it is commonplace in the field at the present time to use so-called "panels" of protein kinases, including serine/threonine kinases, tyrosine kinases, and dual-specificity kinases, in order to identify compounds that inhibit as few protein kinases as possible (other than the target protein kinase itself). The reasoning behind this approach is that the fewer the number of kinases that are inhibited in a cell-free system by a given compound, the less likely the compound is to have untoward side effects in the patient. However, there is very little clinical evidence that actually supports this view.

Furthermore, in some cases it has been argued by others that compounds that target more than one kinase may have additional therapeutic effects as compared to those compounds that are highly specific for only a single target protein. There is some evidence for this being true in the case of imatinib, whose cross-reactivity with c-kit has resulted in beneficial effects for patients with certain histologic types of carcinoma of the small intestine, as discussed previously herein. Despite this cross-reactivity with c-kit, however, imatinib displays a high degree of cellular specificity in the assay systems of the present invention, which is consistent with its high degree of clinical efficacy and relatively modest side effect profile within the first three years of treatment. However, as the specificity of a given compound drops *in the cellular systems of the present invention*, the increased cross-reactivity with other targets such as (in this example) other protein kinase family members may result in untoward side effects in the patient. This is discussed in further detail below.

| **Table 2. Results from Applying the Method(s) of the Invention by Utlilizing a *Phenoresponse-Based* Cellular Assay System and Measuring the *Cellular Specificity Gap (CSG)*** | | | |
|---|---|---|---|
| **Compound** | **IC₅₀ against Test Cells (nM)** | **IC₅₀ against Control Cells (nM)** | **CSG** |
| A | 1 | 1 | 1 |
| B | 10 | 100 | 10 |
| C | 500 | 20,000 | 40 |
| D | 10 | 200 | 20 |

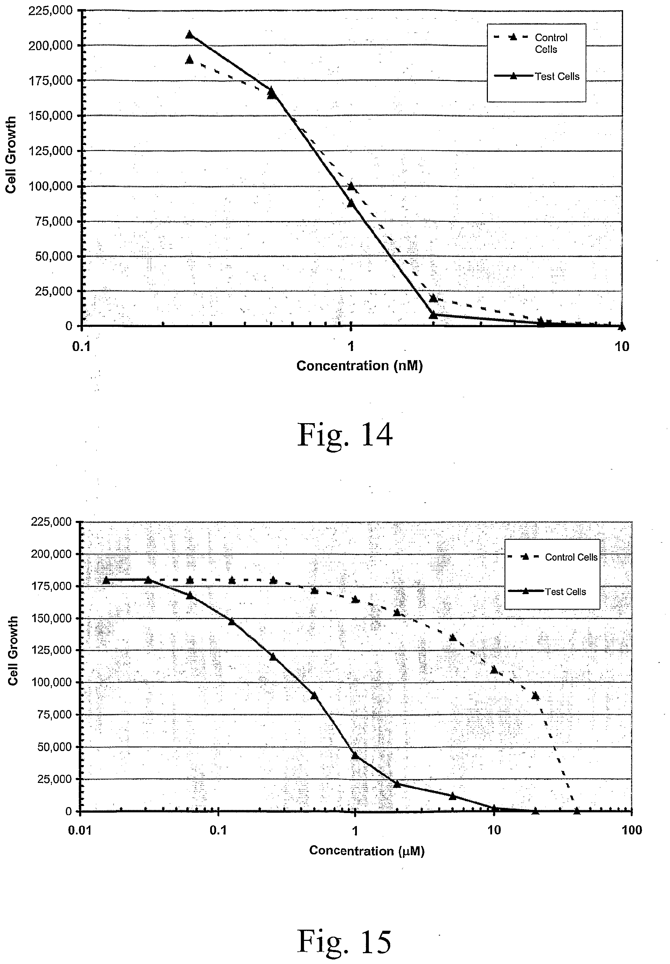

The conclusion that would be drawn by the investigator from the results of the cell-free assay shown in Table 1 above is that compound A is the most potent compound, showing a 50% inhibitory concentration (IC₅₀) value of 0.2 nanomolar (0.2 nM). As shown in Table 2 and Fig. 14, however, this compound, shows no specificity for the Test cells relative to the Control Cells, since it's IC₅₀ for the Control cells is also 1 nanomolar. Similarly, compounds B and D are still quite potent, with both having IC₅₀'s of 10 nM against the Test cells, whereas compound D is more specific than B since its IC₅₀ for the Control cells is higher (200 nM). The CSG measurements of compounds B and D immediately reflect that compound D would be the preferred compound between these two, all other considerations being equal. Most importantly, however, is that compound C is shown in this example to be the best compound of the group in terms of its CSG, at 40 (Table 2, Fig. 15). This means that compound C shows the greatest specificity for the Test cells relative the the Control cells, and would be expected to have the lowest incidence of inducing unwanted side effects in patients, since this finding is derived from a direct testing of the compound in a living cellular system, the ultimate point of pharmacological action for the overwhelming majority of medicines. The important point in this example is that the CSG measurement allows the skilled investigator to rank order the potential therapeutic value of a series of compounds *independently* of their potency in cell-free systems. Thus, although compound C is the least potent compound, it is the most specific in its ability to inhibit the Test cells while leaving the Control cells relatively unaffected over wide concentration range. This is reflected in its CSG of 40, and demonstrates that compound C, rather than compound A, is the compound that should be given the highest priority for further pre-clinical and clinical development efforts.

Use of the method of present invention in this manner provides for an ability to rank order and prioritize the pharmaceutical discovery and development process in a manner which was not possible previously. Through iterative application of the approach given above, the skilled investigator working together with medicinal chemists may synthesize analogus of compounds that score positively in the assay systems described herein, test such compounds in the assay methods of the invention, rank order the compounds according to their CSG values, select the best ones for further development, and repeat the process as many times as necessary in order to fully develop and optimize compounds that exhibit a high degree of specificity against a given Test cell relative to its corresponding Control cell. Once a given compound has been optimized using the system described herein, which generally means that the CSG between Control and Test cells (if measured using the cellular IC₅₀ ratio method described above) is at least three to five fold, the skilled investigator can then proceed to complete the lead optimization process through additional chemical modifications of the compound selected in this way and tested for properties such as plasma half-life, oral bioavailability, and related parameters using appropriate animal models. Of course, the likelihood of such compounds having the desired effects on the target protein in the cellular environment is virtually assured as a result of the process of optimizing said compounds according to the methods described herein, rather than with older, cell-free methods.

It will be immediately apparent to the skilled investigator that analogous approaches may also be utilized with activators of a given target protein. It will also be apparent to the skilled investigator that there are other ways of determining the CSG. For example, using the inhibitor example given above, another useful approach to determine the CSG is to measure the ratio of the highest concentration of a compound which results in 50% growth inhibition of the control cell line, divided by the lowest concentration of the compound at which at least 90% of the test cell line is inhibited. Other obvious modifications of this approach may be utilized as well, including computing the logarithm of the concentrations at which compounds show a given percentage of activity, normalization of either the control or test cell responses relative to one another, etc. No limitation is intended on the nature of the computed or observed comparison of the control cell responsiveness to the test cell responsiveness for the purposes of determining the CSG.

If one uses the IC₅₀-ratio of control cells/test cells method as described above, then compounds with CSG values less than or equal to 1 would not generally be considered to be good clinical candidate compounds, whereas compounds with CSG values of greater than approximately 10 would be quite promising and worthy of further consideration.

This example also highlights the distinctions between the effects of a given compound in a cell-free system versus the more medically and physiologically relevant cell-based system of the present invention.

In an embodiment of the invention, compound profiling is used to identify and/or minimize side effects associated with administration of a compound to a patient. The cell-based lead optimization method allows early identification of potential side effects as compared to the cell-free approach. For example, imatinib shows a wide cellular specificity gap according to the methods of the invention described herein. This is consistent with imatinib's significant advance in the area of anti-cancer drugs. However, it is not without side effects. Recent evidence demonstrates that imatinib is associated with cardiac toxicity in a small percentage of patients (Kerkelä et al., 2006). This group may increase over time as patients take imatinib for longer periods of time.

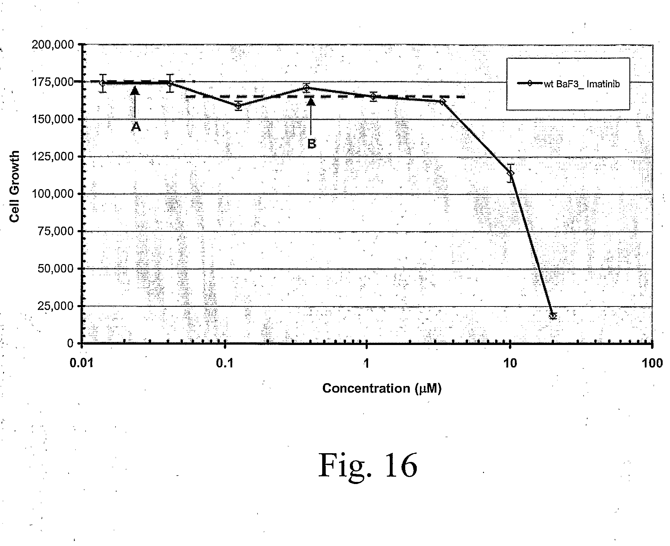

Through the use of the methods of the invention, Figure 16 shows that imatinib tested at various concentrations on the wild type BalF3 cell line shows a slight but significant growth inhbitory effect at concentrations that are substantially below the apparent IC₅₀ for cellular toxicity (about 10 µM) on the control cell line that imatinib exhibits at markedly higher concentrations. Such results become even more evident when the comparison of the effects of other compounds on the Control cell line are compared to those of imatinib.

In still another implementation of the method, compounds which may show promising activity in a cell-free system but have small CSG values (as discussed above) would be expected to have higher potential side effects in patients, especially over longer treatment periods. Compounds having low CSG values have been reported by others with respect to certain targets such at p210 Bcr-Abl^{T315I} mutant (Carter et al., 2005), and still other groups have even entered such compounds into clinical trials. However, based upon the teachings of this invention it may be expected that such compounds will have an increased incidence of untoward side effects in patients.

A preferred drug screening method of the present invention involves the following:
1) Identification of a protein of interest (POI), such as a theramutein for which a novel inhibitor or activator is desired. Often, the POI is implicated or suspected to be implicated in establishment or maintenance of a disease state, perhaps due to inappropriate expression or a mutation-induced change in specific activity. Identification of an appropriate theramutein, for example, may be performed using standard techniques (See, Gorre et al., Science, 2001; see also PCT/US02/18729). Briefly, patients that have been given a course of a therapeutically effective treatment using an activator or inhibitor of a known or suspected prototheramutein and have subsequently shown clinical signs and symptoms consistent with disease relapse are identified, and cells or tissue samples derived from such patients are obtained. Using standard laboratory techniques such as RT-PCR, the sequence of the prototheramutein is determined and compared to the previously determined nucleic acid sequence of the known prototheramutein gene or cDNA sequence. Mutations, if present, are identified and are correlated with functional resistance of the prototheramutein's function either in cell-based or, more commonly, cell-free assay systems, again using standard methodology. Once resistance-inducing mutations are confirmed, then said one or more confirmed mutants comprise a defined theramutein which may be used in the subsequent methods as described herein.
2) Provision of a test cell that expresses the POI and displays an observable (measurable) phenotypic characteristic that is linked to expression of the POI. In the case of a theramutein, the phenotypic characteristic is usually one which has been previously shown to be responsive to inhibitors or activators of the theramutein or, more commonly, the corresponding prototheramutein. Such a phenotypic characteristic that is linked to expression of the POI and has been previously shown to be responsive to inhibitors or activators of the POI (or the prototheramutein-of-interest (pTOI)) is defined herein as a "phenoresponse." One embodiment of this invention is the definitive use of the phenoresponse for the purpose of identifying compounds that are likely to be inhibitors or activators of the TOI. This may be accomplished through the use of a high-throughput screen using a cell line overproducing a given TOI and for which an appropriate phenoresponse has been identified and characterized. Alternatively, one may utilize a high-throughput primary screen using a more generic phenotypic characteristic of a cell line (that does not qualify as a phenoresponse according to the teachings herein) and then utilize a secondary screen according to the teachings herein to distinguish between compounds that are true positive "hits", i.e. inhibitors or activators of the theramutein of interest, from false positive compounds that are not inhibitors or activators of the theramutein of interest. In one embodiment, a cell is selected that naturally expresses the theramutein such that a responsive phenotypic characteristic is present under suitable culture conditions which are obvious to one of ordinary skill in the art. In other embodiments, the theramutein is overexpressed, in some instances in a host cell that does not otherwise express the theramutein at all. This usually involves construction of an expression vector from which the theramutein can be introduced into a suitable host cell and overexpressed using standard vector systems and methodology. (Gorre et al., 2001; Housey et al., 1988). In one embodiment, overexpression results in a level of the theramutein that is at least about 3 times the amount of the protein usually present in a cell. Alternatively, the amount is at least about 10 times the amount usually present in a cell. In another embodiment, the amount is at least about 20 times or more preferably at least about 50 times the amount usually present in a cell.
3) Provision of a control cell that expresses the POI to a lesser extent or not at all (e.g., an unmodified host cell or host cell harboring an expression vector that does not express the POI). In the case of a theramutein of interest, the control cell can also be a cell expressing the prototheramutein corresponding to the theramutein of interest.

As some of the muteins that are described herein are also enzymes, they usually retain catalytic activity, and therefore the control cell usually displays substantially the same phenotypic characteristic as the test cell. The phenotypic characteristic need not be quantitatively alike in both cells, however. For example, a mutation that leads to reactivation of the prototheramutein may also increase, decrease, or otherwise affect its specific activity with respect to one or more of its substrates in the cell. As a result, it may exhibit the selected phenotypic characteristic to a greater or lesser extent. Accordingly, it may be desirable in some cases to adjust expression of either or both of the prototheramutein and the theramutein such that test and control cells exhibit the phenotypic characteristic to approximately the same degree. This may be done, for example, by expressing the proteins from promoters whose activity can be adjusted by adjusting the amount of inducer present, all using standard methodology (see, for example, Sambrook et al. 1989 and 2001).

It will be obvious to one of ordinary skill in the art that a properly defined phenoresponse may be *quantitatively* different between the prototheramutein- and the theramutein-expressing cell lines as a result of differences in the specific activity (if any) between the theramutein and its corresponding prototheramutein. Theramutein-inducing mutations may increase or decrease the specific activity of said theramutein relative to the corresponding prototheramutein. When comparing a theramutein expressing cell line with a prototheramutein expressing cell line, it is preferable that the selected phenoresponse is qualitatively the same in both cell types. Thus, the skilled investigator may choose to normalize the activity of the theramutein-expressing cell line to that of the prototheramutein-expressing cell line, or vice versa. Such normalization methods are standard in the art. See, for example, Bolstad et al. (2003).

Alternatively, the skilled investigator may also wish to use unmodified host cells or host cells harboring the expression vector only as control cells for certain experimental procedures. (The host cells are the cells into which an expression vector encoding the theramutein was introduced in order to generate the test cells.) This may be the case where the investigator is only interested in identifying a specific inhibitor or activator of the theramutein of interest, irrespective of whether or not said compound is also effective against the prototheramutein of interest (pTOI).

4) The test and control cells are then maintained or propagated (although not necessarily at the same time) in growth media (or even in intact animals) under suitable conditions such that the phenoresponse may be expressed and assayed. Control cells that are expressing the prototheramutein may be treated with a known modulator of the prototheramutein, or with a test substance, and test cells are treated with test compounds to determine whether they are active against the theramutein, as measured by the ability of said substances to modulate the phenoresponse in the expected manner. Alternatively, control cells not expressing the prototheramutein may also be substituted, depending upon the particular phenoresponse that the skilled investigator has chosen for study. Substances may then be assayed on the test cells and, optionally, on the control cells at the same time, or at another time, and the results compared.

In one embodiment of the invention, substances that are active with regard to the test cells can be rapidly identified by their ability to modulate the phenoresponse of the test cells in the same manner as, for example, the known modulator of the prototheramutein alters the phenoresponse of prototheramutein-expressing control cells. In another embodiment, active substances may be identified by their ability to modulate the activity of the theramutein in the test cells while having little or no effect on the unmodified (prototheramutein and/or theramutein non-expressing) control cells. The skilled investigator will readily appreciate the many variations of this approach that may be utilized to identify, for example, modulators that are more effective against the theramutein, or that are equally effective against both the prototheramutein and one or more corresponding specific theramuteins.

Other phenoresponses can be observed and/or measured and include, for example, detection of substrates of the prototheramutein, and detection of gene expression changes that are regulated by the activity of the theramutein. In the simplest terms, any characteristic of the cell that the skilled investigator has previously correlated with the functional activity of the theramutein may be suitable for use with such methods. However, in selecting a given characteristic, the skilled investigator must first verify that said characteristic fulfills the criteria of being a phenoresponse according the teachings as given in detail herein. The skilled investigator may also wish to normalize the phenoresponse with the theramutein expressing cells to that of the prototheramutein expressing cells.

Characteristics suitable for detection may be measured by a variety of methods very well known to those of skill in the art. Such methods include, but are not limited to, detection of fluorescence of suitably labeled proteins (FACS), immunohistochemistry (IHC) for detection of protein expression, competitive radioligand binding assays, solid matrix blotting techniques, such as Northern, Southern, and Western blots of cell extracts, reverse transcriptase polymerase chain reaction (RT-PCR), enzyme linked immunosorbent assays (ELISA), phosphorylation assays, gel retardation assays, membrane potential perturbations, and the like. The relevant phenotypic characteristic may be detected either on the intact cell after treatment with a test substance or, alternatively, on a subcellular fraction of the cell after treatment of the intact cell with a test substance.

Once compounds are identified that have the desired effect on the theramutein expressing test cells, it may be desirable (but not necessary) to independently verify that the compounds identified are exerting their effects on the theramutein through a direct binding mechanism, i.e. that the compounds fulfill the criteria of being inhibitors or activators (as desired) of the theramutein according to the teachings of the invention (the reader is referred to the definitions of the terms "activator" and "inhibitor" as given above). This may be accomplished with numerous standard binding assays that are known to one of ordinary skill in the art, involving either purified protein samples or intact cellular binding assays using cells transfected with the appropriate prototheramutein or theramutein together with appropriate controls as dictated by sound scientific methods. Since such methods are well established in the art they will not be reiterated here. Numerous reference texts comprehensively discuss such techniques (see, for example, Foreman and Johansen, 2002; Enna S.J. et al. (1991) Current Protocols in Pharmacology, Wiley & Sons, Incorporated; Bonifacino, J.S. et al. (1999) Current Protocols in Cell Biology, Wiley & Sons, Incorporated). See also Housey, G.M. 1988, Chapter 4, and references therein; see also Horowitz et al., 1981.

In a particular embodiment of the invention, the method is used to identify substances that are inhibitors of the p210^{Bcr-Abl-T315I} theramutein. The prototheramutein and theramutein are each expressed in BalF3 (murine) cells using standard methodology and the phenoresponses that are observed are growth characteristics (terminal cell density for a carefully defined cell culture, and growth in the absence of Interleukin-3 (IL-3). Unmodified host cells, or host cells containing the expression vector only or both, may optionally also be used. In still another embodiment, the test cells alone may be used with or without reference to a known inhibitor or activator.

Another useful assay is the determination of the state of phosphorylation of a direct substrate of p210^{Bcr-Abl-T315I}. One such substrate is Crkl (Gorre et al., Science 293:876-80 (2001)), an adapter protein which mediates the connection between Bcr-Abl and Ras. The phosphorylation state of CRKL is representative of the signaling activity of p210^{Bcr-Abl} in a cell. Another downstream substrate is p62DOK. Any such substrate would suffice for these purposes, provided of course that phosphorylation of said substrate has been shown to occur inside the cell, and is not simply an autophosphorylation event of the TOI or PTOI as discussed above. Other signal transduction cascade components may also be monitored, including src family kinases, STATS, PI3 Kinase, raf kinase, RAS, MEK, ERK1 and ERK2, JNK1, 2 and 3, MLK1, 2 and 3, MKK4, MKK7, AKT, mTOR, HSP90, and others.

As exemplified herein, inhibitors of the T315I theramutein have been identified. Furthermore, these inhibitors are also active to differing extents against the wild type prototheramutein p210^{Bcr-Abl-wt}.

According to the present application, a therapeutically effective amount of one or more compounds that modulate the functional activity of a p210^{Bcr-Abl} theramutein is administered to a mammal in need thereof. The term "administering" as used herein means delivering the compounds to a mammal by any method that may achieve the result sought. They may be administered, for example, orally, parenterally (intravenously or intramuscularly), topically, transdermally or by inhalation. The term "mammal" as used herein is intended to include, but is not limited to, humans, laboratory animals, domestic pets and farm animals. "Therapeutically effective amount" means an amount of a compound that, when administered to a mammal, is effective in producing the desired therapeutic effect, such as inhibiting kinase activity, inhibiting cancer cell growth and division, etc.

The application discloses a method of treating disease in a mammal by administering to the mammal an effective amount of a modulator of a theramutein. Suitable diseases to be treated include, but are not limited to, relapsing neoplastic or other proliferative disorders that have become resistant to previously administered drugs. The method is also useful for overcoming variation among individuals with respect to susceptibility to drug treatment that results from allelic differences among therapy targets. For example, the role of p210^{Bcr-Abl} tyrosine kinase signaling in CML has been extensively demonstrated, as has the role of theramuteins of p210^{Bcr-Abl} in drug resistant recurrence of CML. Further, different muteins of p210^{Bcr-Abl} exhibit varying sensitivity to inhibitors of p210^{Bcr-Abl}. Although some theramuteins arise during drug therapy, others may preexist in the population. These latter examples will not be recognized as theramuteins until such time as the disease state ensues and is followed by treatment with a known class of therapeutic agents. Only after said treatment will such preexisting theramuteins reveal themselves as being clinically significant in terms of relative non-responsiveness leading to the progression of the disease in the patient harboring the theramutein.

In an embodiment theramutein modulators are administered in combination with one or more other anti-neoplastic agents. Any suitable anti-neoplastic agent can be used, such as a chemotherapeutic agent, radiation or combinations thereof. The anti-neoplastic agent can be an alkylating agent or an anti-metabolite. Examples of alkylating agents include, but are not limited to, cisplatin, cyclophosphamide, melphalan, and dacarbazine. Examples of anti-metabolites include, but not limited to, doxorubicin, daunorubicin, and paclitaxel, gemcitabine, and topoisomerase inhibitors irinotecan (CPT-11), aminocamptothecin, camptothecin, DX-8951f, topotecan (topoisomerase I inhibitor), and etoposide (VP-16; topoisomerase II inhibitor) and teniposide (VM-26; topoisomerase II inhibitor). When the anti-neoplastic agent is radiation, the source of the radiation can be either external (external beam radiation therapy - EBRT) or internal (brachytherapy - BT) to the patient being treated. The dose of anti-neoplastic agent administered depends on numerous factors, including, for example, the type of agent, the type and severity of the tumor being treated and the route of administration of the agent. There is no limitation to any particular dose, route of administration, or combination of chemotherapeutic agents or other therapeutic regimens that are combined with the administration of protein modulators.

Anti-neoplastic agents which are presently known in the art or being evaluated can be grouped into a variety of classes including, for example, mitotic inhibitors, alkylating agents, anti-metabolites, intercalating antibiotics, growth factor inhibitors, cell cycle inhibitors, enzymes, topoisomerase inhibitors, anti survival agents, biological response modifiers, anti-hormones, and anti-angiogenesis agents, all of which can be administered with inhibitors or activators of theramuteins.

A modulator of a theramutein can be administered with antibodies that neutralize other receptors involved in tumor growth. Further, a modulator of a theramutein can be administered with a compound that otherwise modulates a component of a signal transduction pathway, preferably a component of the signal transduction pathway in which the theramutein is active and which is common to one or more other signal transduction pathways. In an embodiment, a theramutein modulator is used in combination with a receptor antagonist that binds specifically to the Epidermal Growth Factor Receptor (EGFR). Particularly preferred are antigen-binding proteins that bind to the extracellular domain of EGFR and block binding of one or more of its ligands and/or neutralize ligand-induced activation of EGFR. An EGFR antagonist can be an antibody that binds to EGFR or a ligand of EGFR and inhibits binding of EGFR to its ligand. Ligands for EGFR include, for example, EGF, TGF-α, amphiregulin, heparin-binding EGF (HB-EGF) and betacellulin. EGF and TGF-α are thought to be the main endogenous ligands that result in EGFR-mediated stimulation, although TGF-α has been shown to be more potent in promoting angiogenesis. It should be appreciated that the EGFR antagonist can bind externally to the extracellular portion of EGFR, which can or can not inhibit binding of the ligand, or internally to the tyrosine kinase domain in the case of chemical agents. Examples of EGFR antagonists that bind EGFR include, without limitation, biological agents such as antibodies (and functional equivalents thereof) specific for EGFR, and chemical agents (small molecules), such as synthetic kinase inhibitors that act directly on the cytoplasmic domain of EGFR.

Other examples of growth factor receptors involved in tumorigenesis are the receptors for vascular endothelial growth factor (VEGFR-1 and VEGFR-2), platelet-derived growth factor (PDGFR), nerve growth factor (NGFR), fibroblast growth factor (FGFR), and others.

In a combination therapy, the theramutein inhibitor is administered before, during, or after commencing therapy with another agent, as well as any combination thereof, *i.e.,* before and during, before and after, during and after, or before, during and after commencing the anti-neoplastic agent therapy. For example, the theramutein inhibitor can be administered between 1 and 30 days, preferably 3 and 20 days, more preferably between 5 and 12 days before commencing radiation therapy. In a preferred embodiment, chemotherapy is administered prior to, concurrently with or, more preferably, subsequent to antibody therapy.

Any suitable method or route can be used to administer theramutein inhibitors, and optionally, to co-administer anti-neoplastic agents and/or antagonists of other receptors. The anti-neoplastic agent regimens utilized, include any regimen believed to be optimally suitable for the treatment of the patient's neoplastic condition. Different malignancies can require use of specific anti-tumor antibodies and specific anti-neoplastic agents, which will be determined on a patient to patient basis. Routes of administration include, for example, oral, intravenous, intraperitoneal, subcutaneous, or intramuscular administration. The dose of antagonist administered depends on numerous factors, including, for example, the type of antagonists, the type and severity of the tumor being treated and the route of administration of the antagonists..

Suitable carriers include, for example, one or more of water, saline, phosphate buffered saline, dextrose, glycerol, ethanol and the like, as well as combinations thereof. Carriers can further comprise minor amounts of auxiliary substances, such as wetting or emulsifying agents, preservatives or buffers, which enhance the shelf life or effectiveness of the theramutein modulator as the active ingredient. The compositions can, as is well known in the art, be formulated so as to provide quick, sustained or delayed release of the active ingredient after administration to the mammal.

The compositions can be in a variety of forms. These include, for example, solid, semi-solid and liquid dosage forms, such as tablets, pills, powders, liquid solutions, dispersions or suspensions, liposomes, suppositories, injectable and infusible solutions. The preferred form depends on the intended mode of administration and therapeutic application.

Such compositions are prepared in a manner well known in the pharmaceutical art. In making the composition the active ingredient will usually be mixed with a carrier, or diluted by a carrier and/or enclosed within a carrier which can, for example, be in the form of a capsule, sachet, paper or other container. When the carrier serves as a diluent, it can be a solid, semi-solid, or liquid material, which acts as a vehicle, excipient or medium for the active ingredient. Thus, the composition can be in the form of tablets, lozenges, sachets, cachets, elixirs, suspensions, aerosols (as a solid or in a liquid medium), ointments containing, for example, up to 10% by weight of the active compound, soft and hard gelatin capsules, suppositories, injection solutions, suspensions, sterile packaged powders and as a topical patch.

It should be appreciated that the methods and compositions can be administered to any suitable mammal, such as a rabbit, rat, or mouse. More preferably, the mammal is a human.

The compounds may also be present as salts. In this context, preference is given to pharmaceutically acceptable salts. Pharmaceutically acceptable salts refers to an acid addition salt or a basic addition salt of a compound in which the resulting counter ion is understood in the art to be generally acceptable for pharmaceutical uses. Pharmaceutically acceptable salts can be salts of the compounds with inorganic or organic acids. Preference is given to salts with inorganic acids, such as, for example, hydrochloric acid, hydrobromic acid, phosphoric acid or sulfuric acid, or to salts with organic carboxylic or sulfonic acids, such as, for example, acetic acid, maleic acid, fumaric acid, malic acid, citric acid, tartaric acid, lactic acid, benzoic acid, or methanesulfonic acid, ethanesulfonic acid, phenylsulfonic acid, toluenesulfonic acid or naphthalenedisulfonic acid. Pharmaceutically acceptable salts can also be metal or ammonium salts of the compounds. Particular preference is given to, for example, sodium, potassium, magnesium or calcium salts, and also to ammonium salts which are derived from ammonia or organic amines, such as, for example, ethylamine, di- or triethylamine, di- or triethanolamine, dicyclohexylamine, dimethylaminoethanol, arginine, lysine, ethylenediamine or 2-phenylethylamine. (see, Berge et al. J. Pharm. Sci. 1977, 66, 1-19).

Throughout this application, various publications, reference texts, textbooks, technical manuals, patents, and patent applications have been referred to. The teachings and disclosures of these publications, patents, patent applications and other documents describe the state of the art to which the present invention pertains.

It is to be understood and expected that variations in the principles of invention herein disclosed may be made by one skilled in the art.

The following examples further illustrate the invention. Detailed descriptions of conventional methods, such as those employed in the construction of vectors and plasmids, the insertion of genes encoding polypeptides into such vectors and plasmids, the introduction of plasmids into host cells, and the expression and determination thereof of genes and gene products can be obtained from numerous publications, including Sambrook, J et al., (1989) Molecular Cloning: A Laboratory Manual, 2nd ed., Cold Spring Harbor Laboratory Press; Coligan, J. et al. (1994) Current Protocols in Immunology, Wiley & Sons, Incorporated; Enna, S.J. et al. (1991) Current Protocols in Pharmacology, Wiley & Sons, Bonifacino, J.S. et al. (1999) Current Protocols in Cell Biology, Wiley & Sons, and U.S. Patent 4,980,281..

### EXAMPLES

It is to be understood and expected that variations in the principles of the invention herein disclosed may be made by one skilled in the art.

Examples of the invention which follow are set forth to further illustrate the invention.

### EXAMPLE 1: IDENTIFICATION OF A PROTEIN MODULATOR

p210^{Bcr-Abl-T315I} is a theramutein of the p210Bcr-Abl protein (p210^{Bcr-Abl}) that is resistant to inhibition by imatinib mesylate (Gleevec, STI-571). The mutation at position 315 converts a threonine to an isoleucine residue and is one of several mutations that are observed among resistant or relapsed patients. This particular mutant, however, is the most resistant such theramutein yet identified.

A phenoresponse was determined for a BalF3 cell line engineered to overexpress the p210^{Bcr-Abl-T315I} theramutein. The phenoresponse was determined relative to non-transformed BalF3 cells and BalF3 cells that express the p210^{Bcr-Abl-wt} prototheramutein. The phenoresponse was the ability of the T315I mutants to grow to a higher cell saturation density under analogous culture conditions as compared to the control non-transformed BalF3 cell line, and to grow in the absence of interleukin 3 (IL-3), which is required for maintenance of the control non-transformed BalF3 cell line. The phenoresponse was defined and characterized according to the teachings given above.

The detection system utilized was a high speed cell imaging and counting system in which 3 µl sample volumes of cells were sequentially injected through a 5 µl optical microcell, digitally imaged and electronically stored, scanned, and then counted, all under a microcomputer-based control system. The system has the capacity to perform direct cell counts on samples from cultures as small as 500 µl and provides statistically significant total cell counts from culture samples containing as few as 12,500 cells. All of the figures displaying cell count and viability assays utilized this system for data acquisition and analysis. Simultaneously with the cell count performed, the system is also capable of determining overall cell viability by distinguishing counted, imaged cells that have excluded trypan blue (counted as "viable" cells) from cells which have taken up the trypan blue dye (counted as "non-viable" cells). Injection of trypan blue into the cell sample occurs immediately prior to the sample being sequentially injected into the microcell for simultaneous cell counting and imaging.

The system may be integrated into the workflow of high-throughput screening devices to provide a sensitive and precise cell counting and cell viability assay system that is more reliable and less prone to confounding effects of metabolic viability-based cellular assays such as XTT or Alamar blue.

Initially, approximately 113,000 compounds were screened at concentrations generally ranging from 10 to 20µM to identify a subset that was capable of affecting growth of BalF3 cells (BalF3 T315I cells) overexpressing the p210^{Bcr-Abl-T315I} theramutein by any means.

A total of approximately 11,760 compounds showed greater than 50% growth inhibition, which were thought to correspond to approximately 4500 distinct chemical classes. Retesting of these compounds with the same cell line yielded a database of compound responsiveness which was then sorted and rank ordered according to those compounds exhibiting the highest overall growth inhibition. From this rank ordered database, the highest scoring 130 compounds (based upon the greatest degree of growth inhibition observed at the lowest concentrations that compounds were tested) were then rescreened in a defined cell-based assay system using BalF3 T315I as test cells and wild type BalF3 as control cells according to the methods of the present invention. Compounds of interest were those that differentially inhibited growth of BalF3 cells expressing the p210^{Bcr-Abl-T3151} theramutein relative to non-transformed wild type BalF3 cells. Six compounds were identified that fulfilled the desired criteria, and some of these compounds were analyzed in further detail using the BalF3 p210^{Bcr-Abl-wt} cells line (BalF3 P210 cells) as well. One compound was unavailable for further testing due to lack of availability of additional material from the chemical supplier. The remaining five compounds were independently evaluated in additional cell-based assays using the aforementioned cell lines as well as in a cell-free purified protein kinase assay using human recombinantly produced 120 Kd kinase domain fragments isolated from both wild type P210 Bcr-Abl as well as P210 T315I mutant kinase domain.

All five compounds inhibited p210^{Bcr-Abl-T3151} 120 Kd activity as measured by inhibition of autophosphorylation activity. Thus, of the 6 highest scoring compounds out of more than 113,000 compounds screened, at least 5 of the six directly inhibited the p210^{Bcr-AblT3151} mutant directly. One compound appeared to spread the recombinant protein band out on the SDS page gel. This was also evident on the silver-stained gel (data not shown). It is possible that this compound may actually be a "suicide" inhibitor that is able to covalently cross-link the POI in order to permanently inhibit its activity, but this will require further study.

Taken together, the teachings and the results described herein provide conclusive proof that the system is capable of identifying inhibitors or activators of the selected theramutein, and the skilled investigator will immediately recognize that such a system may be easily applied to any other theramutein or other protein with only obvious, minor modifications.

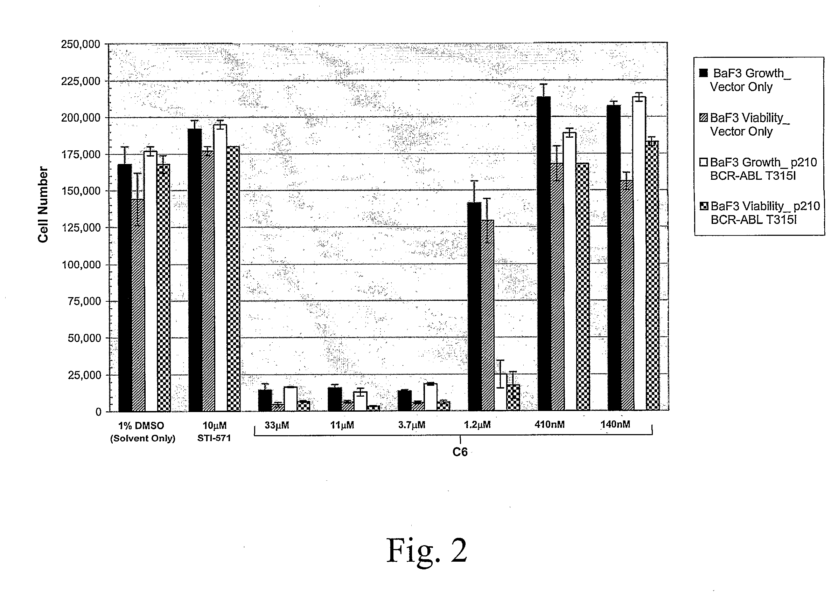

Representative examples of the cell-based assay results demonstrating selective inhibition of growth of the Ba/F3 T315I cell line relative to the wild type non-transformed BalF3 cells are shown in Figures 1 and 2. The compounds inhibited growth and reduced the viability of cells expressing the T315I theramutein at concentrations under which the growth and viability of the wild type BalF3 non-transformed cells (not expressing either p210^{Bcr-Abl-wt} or p210^{Bcr-Abl-T315I}) were relatively unaffected, whereas cells expressing both the prototheramutein as well as the theramutein were substantially inhibited. In some instances, the T315I expressing cells were inhibited to an even greater extent than the P210 prototheramutein expressing cells. (See, for example, Figure 3, right hand side, Compound 3 results against P210 and T315I cells.

In summary, the methods presented herein provide a fundamental advance in the form of a generalizable approach for creating or identifying modulators of any given theramutein. The results demonstrate conclusively the power of the method to identify critically needed compounds to overcome a specific type of acquired drug resistance that is uniformly fatal in certain patient populations and is presently untreatable. Furthermore, it is evident to one of skill in this art that the techniques and methods described herein may, using obvious modifications, be straighforwardly generalized to any potential theramutein or other disease associated protein of clinical significance.

It is remarkable that out of a primary screen of more than 100,000 compounds where approximately 10,000 compounds exhibited some degree of growth inhibition, when the most potent growth inhibitory substances were rescreened using the Method described in detail herein, 6 distinct compounds were identified and all of the compounds that were subsequently tested exhibited inhibitory activity in a cell-free purified protein kinase assay using the T315I mutant (one compound was unavailable for further testing). Based upon such remarkable results, it becomes immediately clear to the skilled artisan that *the method may be effectively applied toward the identification of inhibitors or activators of any protein* based upon the proper selection and definition of the phenoresponse according to the teachings in the sections given above. For example, with knowledge of the foregoing, one of ordinary skill in the art could easily design an assay system to identify inhibitors of theramuteins derived from other prototheramuteins known to exhibit mutations that confer drug resistance such as the c-kit gene product or the Epidermal Growth Factor (EGF) Receptor (EGFR), or the Platelet Derived Growth Factor (PDGF) Receptor α and β. No limitation should be inferred upon the utility of the method with respect to its ability to be utilized with any given protein, including theramuteins and protothermuteins, expressed in any mammalian cell type for which a corresponding phenoresponse is detectable.

### EXAMPLE 2: PHENORESPONSE-BASED OPTIMIZATION OF A PROTEIN MODULATOR

In this example, a compound previously identified according to the teachings of this invention is optimized for activity against its selected protein target. However, unlike methods typically used in the prior art, the optimization process herein is also performed entirely through the use of the phenoresponse-based cellular assay system. For completeness sake and to demonstrate the power of the methodology to refine , a cell-free assay system using recombinantly produced target enzyme is also used to independently demonstrate that the compounds that score positively in the phenoresponse-based cellular assay indeed also score positively in a cell-free assay system format that is standard in the art and uses recombinantly produced enzyme.

Compound C2 which was originally identified as an inhibitor of the T315I theramutein was subjected to a novel lead optimization program as follows. Various chemical modifications were introduced into the basic scaffold structure of compound C2 using standard medicinal chemistry synthetic methods. Once synthesized, the various analogues (chemical variants) were tested using the phenoresponse-based cellular assay system described above in Example 1.

Based upon the original structure of compound C2, the contribution(s) to pharmacological activity arising from the phenyl ring that contained the bromo, chloro, and hydroxyl substituents were analyzed. An initial series of analogues were synthesized that consisted of either the unsubstituted phenyl ring (C2-01), or various substituents on the phenyl ring, such as bromo, chloro, and hydroxyl, etc. located various positions around the phenyl ring. Detailed chemical structures are shown in Table 3.

| **Table 3: Optimization of C2 Compounds** | | |
|---|---|---|
| | 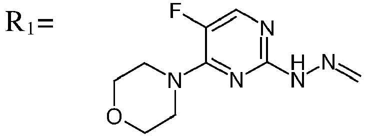 | |
| C2 | 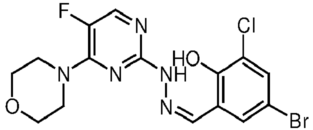 | 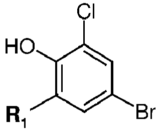 |
| C2-27 | 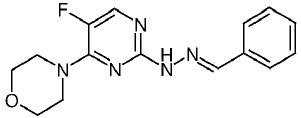 | 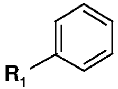 |
| C2-21 | 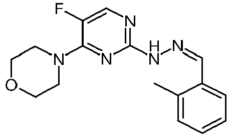 | 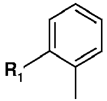 |
| C2-01 | 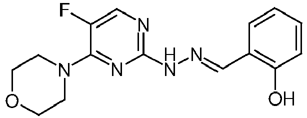 | 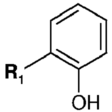 |
| C2-02 | 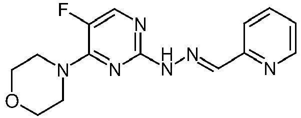 | 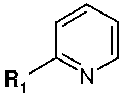 |
| C2-05 | 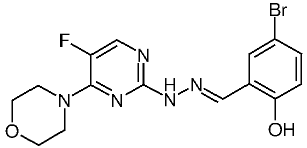 | 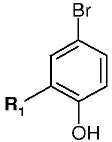 |
| C2-86 | 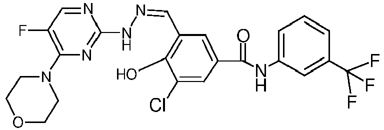 | 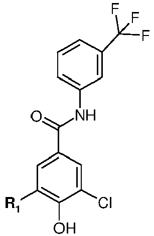 |
| C2-87 | 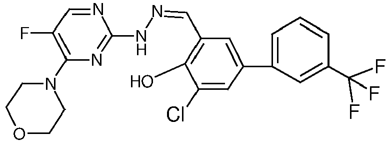 | 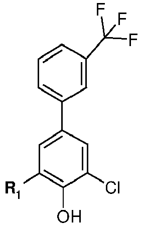 |
| C2-109 | 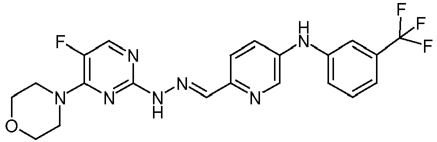 | 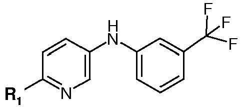 |
| C2-112 | 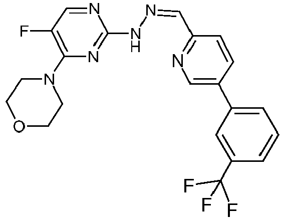 | 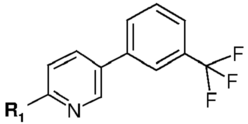 |
| C2-121 | 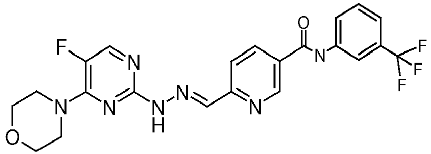 | 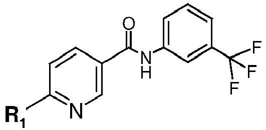 |
| C2-122 | 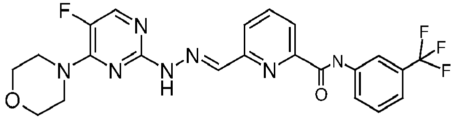 | 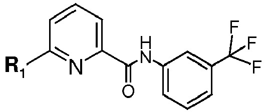 |
| C2-128 | 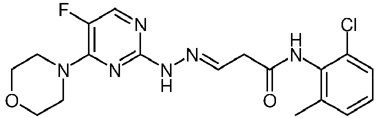 | 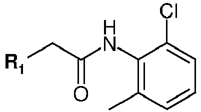 |

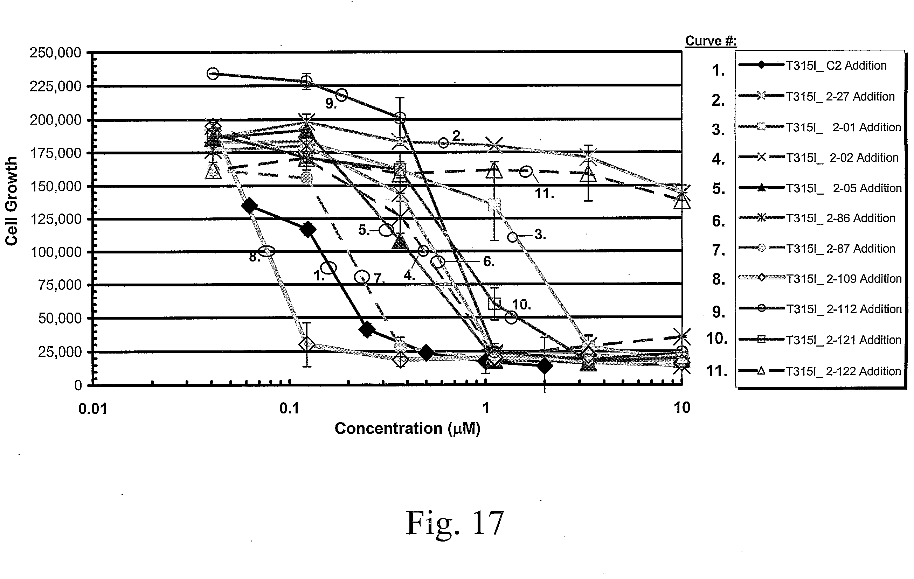

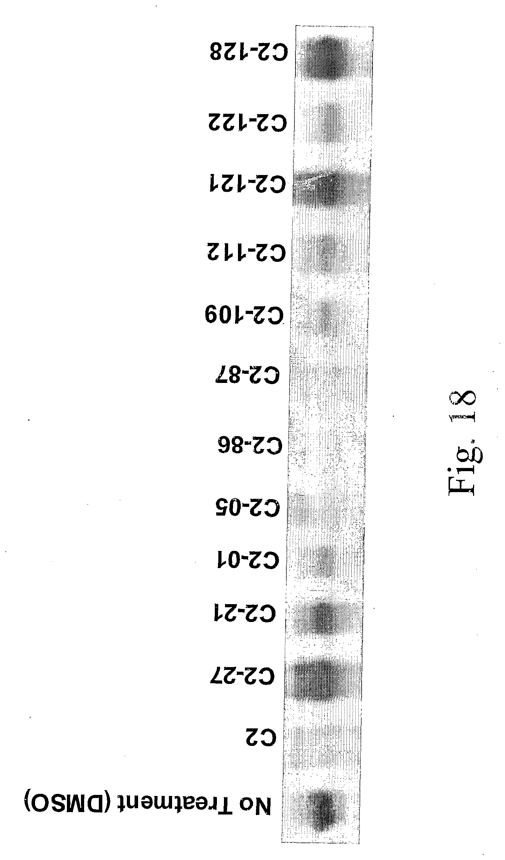

These compounds were then tested in the phenoresponse-based cellular assay system, as shown in Figure 17. Each of the compounds was also tested in a standard cell free protein kinase autophosphorylation assay at a concentration of 20 µM as shown in Figure 18. As a comparison of Figure 17 and Figure 18 will indicate, there was a striking, essentially complete qualitative correlation between the activity of the compounds in the phenoresponse-based (cellular) assay system and their corresponding activity in the cell free purified protein kinase autophosphorylation assay.

Additional chemical modifications were made to the same phenyl ring discussed above for a variety of medicinal chemistry purposes, but were related to enhancing potency againt the p210^{Ber-Abl-T315I} target while simultaneously limiting cross-reactivity with the control wild type BalF3 non-transformed cells (not expressing either p210^{Bcr-Abl-wt} or p210^{Bcr-Abl-T315I}), as well as to improve selectivity, minimize potential side effects in the patient, etc. As shown in Figure 17 and 18 taken together with Table 3, additional compounds synthesized and tested included C2-109, C2-112, C2-122, and C2-128. A detailed comparison of Figures 17 and 18 reveals once again that all of the compounds that score positively in the cellular assay system also exhibited protein kinase inhibitory activity in the cell free system, whereas those that were essentially inactive in the phenoresponse-based cellular assay were also essentially inactive in the cell-free system. These results conclusively demonstrate that the compounds showing inhibitory activity in the phenoresponse-based assay system were entirely consistent qualitatively with the results obtained in the cell free protein kinase autophosphorylation assay.

Furthermore, where there are modest differences in relative potency between the cell free assay results and the phenoresponse-based assay results (see for example, compound C2-122 which appears to be more potent in the cell-free assay than in the phenoresponse-based cellular system, such distinctions point out the enhanced ability of the cellular assay system to predict in vivo efficacy of a given compound as compared to classical cell-free assay systems. Using a classical cell-free screen, one might have considered C2-122 to be an important compound, yet the phenoresponse-based assay immediately rules it out as being less potent than several of the other compounds tested. This type of lead optimization strategy employing a cellular system without dependence and reliance upon a cell-free radioligand or other binding assay has not been reported before in the prior art.

In summary, the present invention provides the skilled investigator with a powerful and rapid method for lead optimization which supplants the necessity for repeated cell free in vitro verifications of the ability of a compound to hit its corresponding target. Thus the optimization process may be performed essentially entirely with reliance upon the phenoresponse-based assay systems results, obviating the need for repetitive confirmatory cell free assay determinations. While such confirmatory experiments may be performed if the skilled investigator chooses to do so, they are generally unnecessary with this method, as the results given above unequivocally demonstrate.

The skilled investigator is well aware that no assay system of any kind or nature, whether it be a radioligand binding assay, ELISA, ligand binding assay, or cellular assay, is free of false-positive results. This assay system, while surprisingly robust, will not be free of the possibility of false positive results either, and the skilled investigator knows that independent verification of unusual results is simply good science and should be taken into consideration where appropriate.

### EXAMPLE 3: PHENORESPONSE-BASED PROFILING OF A PROTEIN MODULATOR

The phenoresponse-based assay system of the invention can be used to profile the biological activity of a given compound with respect to its ability to inhibit or activate multiple distinct protein targets to differing extents. For example, in certain instances the skilled artisan may be interested in identifying or optimizing modulators of a given target protein where additional proteins are known that are distinct but highly related to the target POI. Such protein families may consist of two or more members that share a high degree of homology at both the DNA and amino acid sequence levels, yet the family members may have distinct functions within the cell. Through iterative application of the phenoresponse-based system described herein, one could create individual Test Cells expressing each of the distinct family members and then utilize three or four or more distinct Test Cell lines with corresponding defined phenoresponses to identify or optimize compounds that are selective for one particular family member.

In yet another embodiment of the present invention, the skilled artisan may also choose to express two or three or even four distinct protein targets in a single Test Cell (or Test Cell line) and create a phenoresponse-based assay system useful for identifying compounds that are NOT selective among individual isozymes of a given protein family. In certain therapeutic situations, lack of selectivity among individual family members may be preferable. Ibuprofen, for example, is an established, low-cost safe and effective non-steroidal anti-inflammatory drug that does not significantly discriminate between the cyclooxygenase type 1 (COX-1) and COX-2 family members. Such lack of discrimination may in some instances be beneficial and may reduce the likelihood of certain unwanted side effects that may occur with an overly selective chemical agent.

Profiling the biological effects of a given compound with respect to its ability to inhibit or activate certain related protein targets, whether or not such targets are members of the same protein family, also has substantial value from the perspective of understanding the molecular and cellular mechanism(s) of action of a given chemical agent. For example, in the case of imatinib, not only does the compound inhibit the wild type version of the P210 Bcr/Abl protein (p210^{Bcr-Abl-wt}), it also cross reacts with and is capable of inhibiting the c-kit oncoprotein as well. As discussed above in the background of the invention, this cross-reactive inhibition of the kit oncoprotein is serendipitous, because gastrointestinal stromal tumors (GIST), a type of tumor arising in the small intestine, are driven by kit activity and are thus responsive to imatinib treatment as well (NEJM paper). Thus, such cross reactivity with other related proteins need not always be associated with toxicity of a drug. In some instances such cross-reactivities can be therapeutically effective.

Representative Compounds of the application corresponding to the various chemical formulae given above were tested in the cellular assay system described elsewhere herein (see Example 1) and assigned activity categories as shown in Table 4. The assigned activity categories are represented by the following designations, wherein the IC₅₀ for a given cell line is the concentration at which a given compound inhibits the growth of that cell line by 50% in the cellular assay system. Compounds tested on a given cell line that exhibited an IC₅₀ value that was < 300 nM (less than 300 nanomolar) were designated as Category "A" compounds. Compounds tested on a given cell line that exhibited an IC₅₀ value that was <1µM (less than 1micromolar) were designated as Category "B" compounds. Compounds tested on a given cell line that exhibited an IC₅₀ value that was < 10µM (less than 10 micromolar) were designated as Category "C" compounds. Compounds tested on a given cell line that exhibited an IC₅₀ value that was ≥ 10µM (greater than or equal to 10 micromolar) were designated as Category "D" compounds.

**Table 4.**

| **Structure:** | **wt BaF3** | **T315I** |
|---|---|---|
| | | |
| 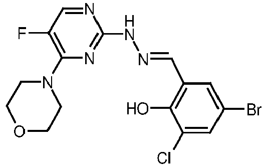 | B | A |
| 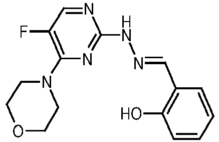 | C | C |
| 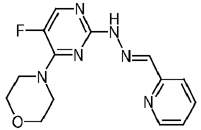 | B | B |
| 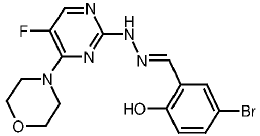 | B | B |
| 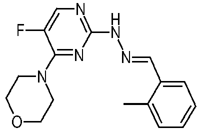 | D | D |
| 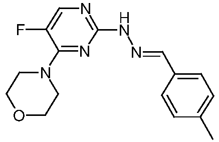 | D | D |
| 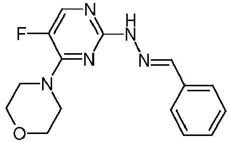 | D | D |
| 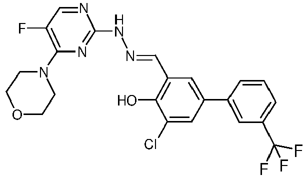 | B | A |
| 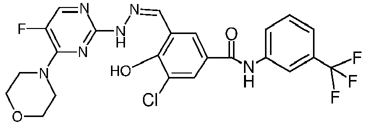 | B | B |
| 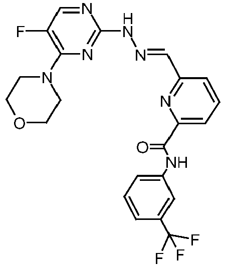 | D | D |
| 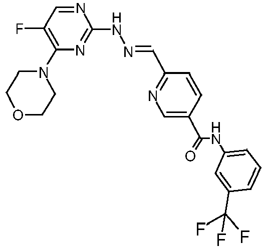 | C | B |
| 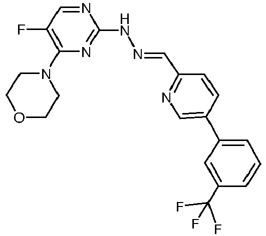 | B | B |
| 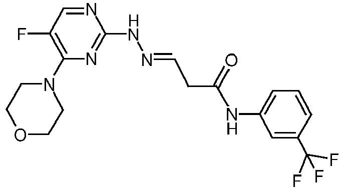 | D | D |
| 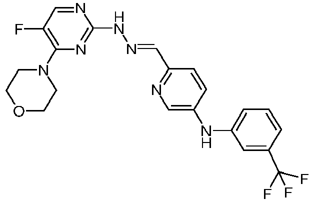 | A | A |
| 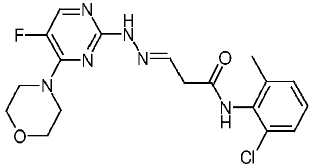 | D | D |
| 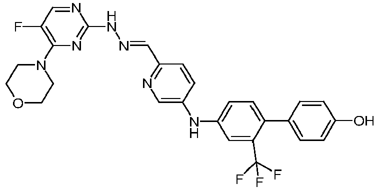 | A | A |
| 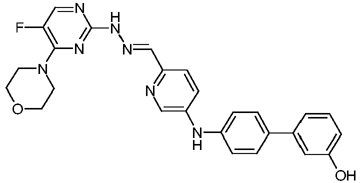 | A | A |
| 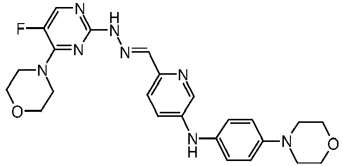 | B | B |
| 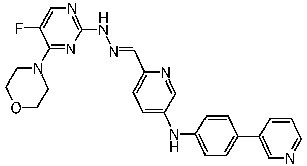 | B | A |
| 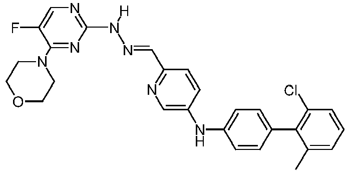 | A | A |
| 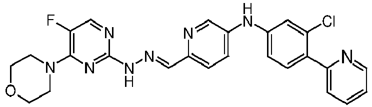 | B | B |
| 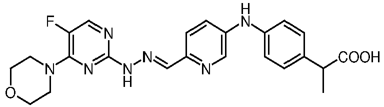 | D | D |
| 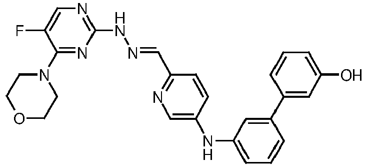 | B | A |
| 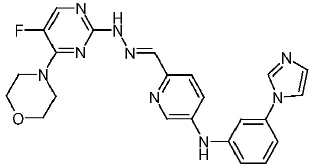 | B | B |
| 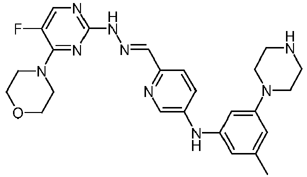 | C | B |
| 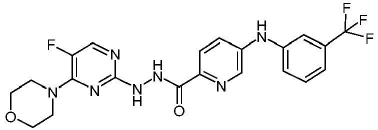 | B | B |
| 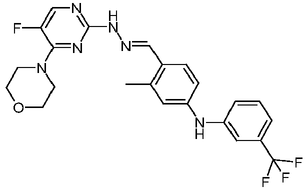 | D | D |
| 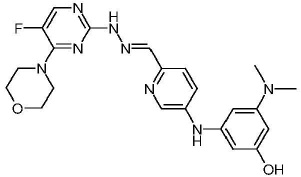 | C | B |
| 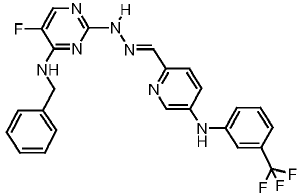 | A | A |
| 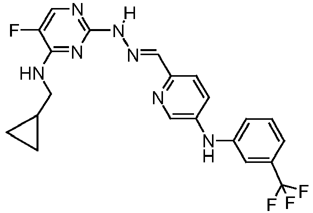 | A | A |
| 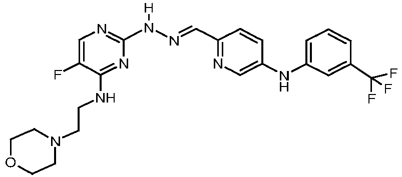 | A | A |
| 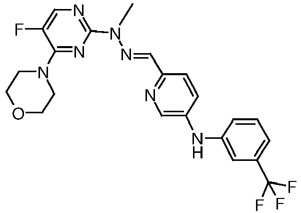 | A | A |
| 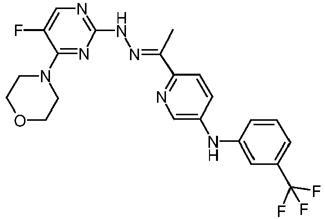 | A | A |
| 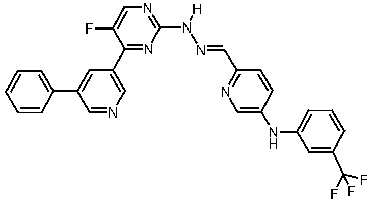 | B | B |
| 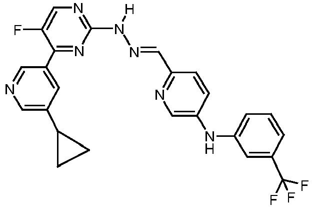 | C | C |
| 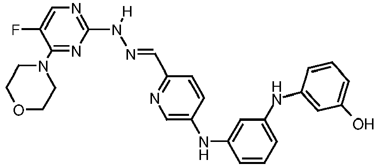 | B | B |

### EXAMPLE 4:

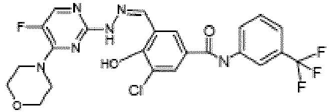

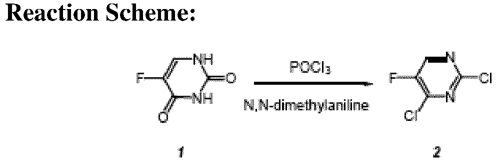

**Experimental Details:** The mixture of **compound 1** (25 g) and N, N-dimethylaniline (24.2 g) in POCl₃ (110 mL) was refluxed for 5 h. POCl₃ was removed by evaporation at reduced pressure and the residue was poured into ice-water (500 g) cautiously and stirred for 1h. The mixture was then filtered and the solid was washed with water to give **compound 2** as a yellow solid.

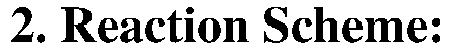

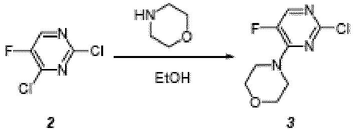

**Experimental Details:** To a solution of **compound 2** (1.04 g) in 15 ml of ethanol, 1.08 g (2 eq) of morphine was added dropwise at -10°C in 15 min. The mixture was stirred for 0.5 h and heated at 50 °C for 15 min. After cooling and dilution with water (50 ml), **compound 3** was obtained as a yellow solid powder after filtration.

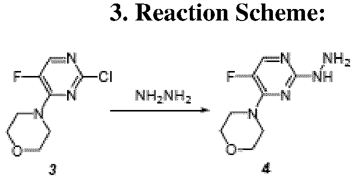

**Experimental Details:** To 1.1 g of **compound 3** was added 8 ml of NH₂NH₂.H₂O. The mixture was refluxed for 2h. After cooling, the crude product was obtained after filtration. Purification by column chromatography gave pure **compound 4** as a light yellow solid.

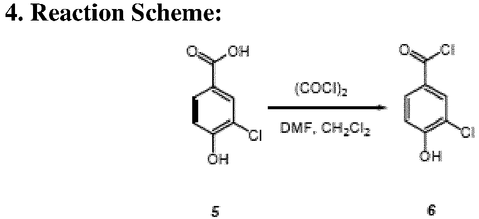

**Experimental Details:** To the solution of **compound 5** (1.0 g, 1.0 eq) and DMF (0.05 g, cat. amount) in 20 mL of dichloromethane was added (COCl)₂ (0.81 g, 1.1 eq) dropwise. The reaction mixture was stirred at r.t. for 2 h and then concentrated to give 1.2 g of crude product of **compound 6,** which was used for the next step without further purification.

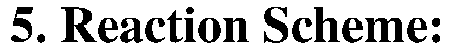

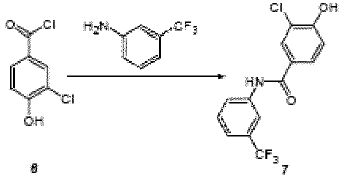

**Experimental Details:** To the crude product of **compound 6** (1.2 g, 1.0 eq) in 20 mL of dichloromethane was added 3-trifluoromethyl-phenylamine (0.94 g, 1.0 eq) and triethylamine (0.71 g, 1.2 eq). The reaction mixture was stirred at r.t. overnight, washed with 1 N NaOH solution, 1 N HCl solution and brine. The organic layer was collected, dried over Na₂SO₄, and concentrated to give crude product of **compound 7.** After purification by column chromatography, 1.1 g of **compound 7** was obtained.

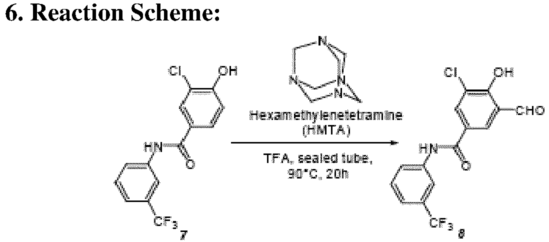

**Experimental Details:** To the mixture of **compound 7** (0.3 g, 1.0 eq) and 3 mL of trifluoroacetic acid was added hexamethylenetetramine (0.53 g, 4.0 eq). The reaction mixture was immediately sealed and heated to 90°C for 20 h. After cooling, the reaction mixture was adjusted to pH 8 with 1 N NaOH solution, extracted with dichloromethane and the organic phase was dried, concentrated to give a brown solid. Purification by preparative TLC gave **compound 8** as a yellow solid.

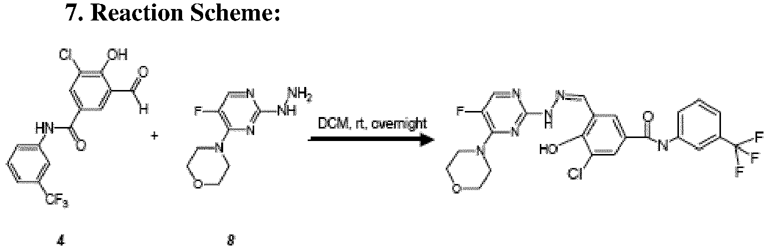

**Experimental Details:** The mixture of **compound 4** (30 mg, 1.0 eq) and **compound 8** (19 mg, 1.0 eq) in 5 mL of dichloromethane was stirred at r.t. overnight. The precipitates were collected and washed with dichloromethane thoroughly, dried under vacuum to give the desired compound.

### EXAMPLE 5:

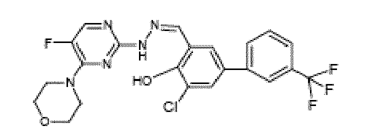

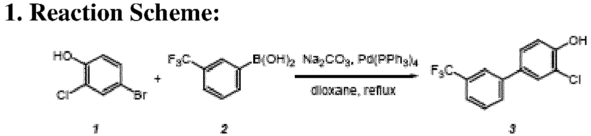

**Experimental Details:** To the mixture of **compound 1** (1.0 g, 1.0 eq), **compound 2** (0.92 g, 1.0 eq), Na₂CO₃ (0.77 g, 1.5 eq) in 15 mL of dioxane was added Pd(PPh₃)₄ (0.56 g, 0.1 eq), and the reaction mixture was refluxed under N₂ for 16 h. After cooling, the mixture was filtered and the filtrate was evaporated to dryness and purified by column chromatography to give **compound 3.**

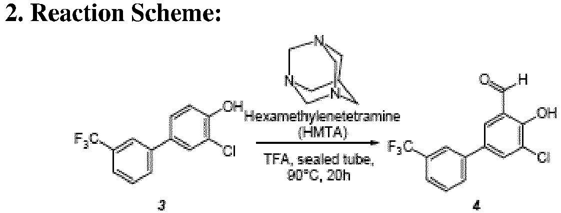

**Experimental Details:** To the mixture of **compound 3** (0.3 g, 1.0 eq) and 3 mL of trifluoroacetic acid was added hexamethylenetetramine (0.62 g, 4.0 eq). The reaction mixture was immediately sealed and heated to 90°C for 20 h. After cooling, the reaction mixture was adjusted to pH 8 with 1 N NaOH solution, extracted with dichloromethane and the organic phase was dried, concentrated to give a brown solid. Purification by preparative TLC gave **compound 4** as a yellow solid.

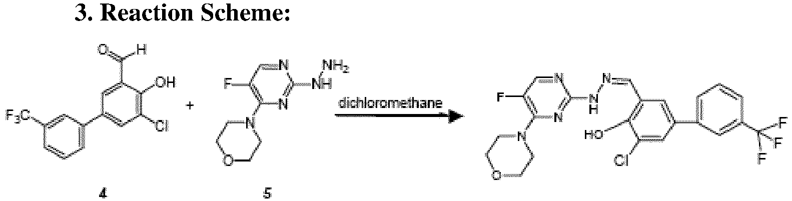

**Experimental Details:** The mixture of **compound 4** (30 mg, 1.0 eq) and **compound** 5 (19 mg, 1.0 eq) in 5 mL of dichloromethane was stirred at r.t. overnight. The precipitates were collected and washed with dichloromethane, dried under vacuum to give the desired compound.

### EXAMPLE 6.

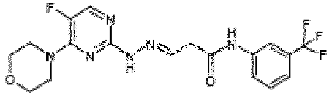

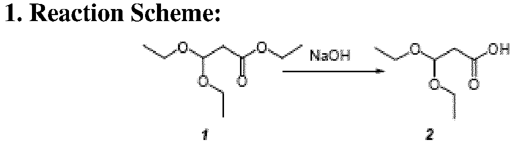

**Experimental Details:** To the solution of **compound 1** (0.6 g, 1.0 eq) in 10 mL of methanol was added 4 mL of 1 N NaOH solution, and the mixture was stirred at r.t. overnight. Solvent was evaporated and the residue was acidified to pH 6 with 5 % citric acid, extracted with dichloromethane. The organic layer was dried, concentrated to give **compound 2.**

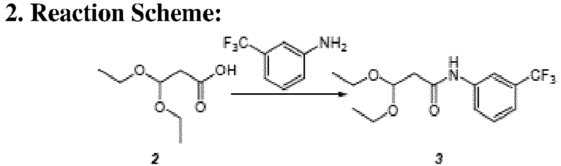

**Experimental Details:** The mixture of **compound 2** (0.4 g, 1.0 eq), trifluoromethyl phenylamine (0.39 g, 1.0 eq), EDC (0.71 g, 1.5 eq), HOBt (33 mg, 0.1 eq) in 10 mL of dichloromethane was stirred at r.t. overnight. The mixture was washed with 1 N NaOH solution, water, extracted with dichloromethane. The organic layer was dried over Na₂SO₄, concentrated to dryness, and purified by column chromatography to give **compound 3.**

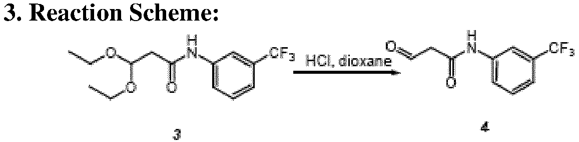

**Experimental Details:** The solution of **compound 3** (0.2 g, 1.0 eq) in 10 mL of dioxane was treated with 4 mL of 1 N HCl, and the mixture was heated to 60°C for 2 h. After cooled, pH was adjusted to 8 by addition of NaHCO₃. The mixture was extracted with dichloromethane, washed the organic layer with water, dried with Na₂SO₄ and evaporated to dryness. The crude product was purified by column chromatography to give **compound 4.**

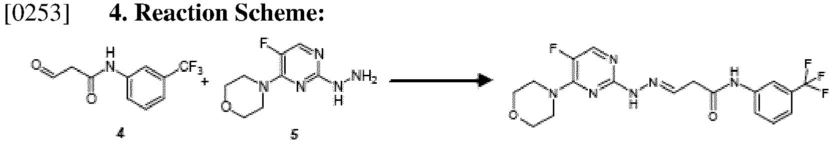

**Experimental Details:** The mixture of **compound 4** (40 mg, 1.0 eq) and **compound 5** (30 mg, 1.0 eq) in 5 mL of dichloromethane was stirred at r.t. overnight. The mixture was concentrated to dryness and purified by preparative HPLC to give the desired compound.

### EXAMPLE 7.

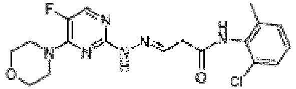

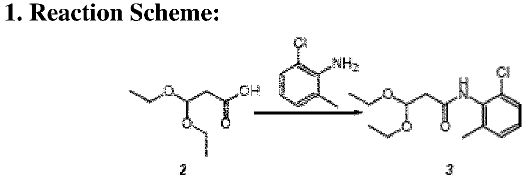

**Experimental Details:** The mixture of **compound 2** (0.3 g, 1.0 eq), 2-chloro-6-methyl-phenylamine (0.26 g, 1.0 eq), EDC (0.53 g, 1.5 eq), HOBt (25 mg, 0.1 eq) in 10 mL of dichloromethane was stirred at r.t. overnight. The mixture was washed with 1 N NaOH solution, water, and extracted with dichloromethane. The organic layer was dried over Na₂SO₄, concentrated to dryness, purified by column chromatography to give **compound 3.**

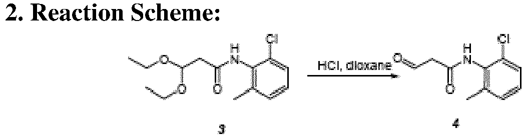

**Experimental Details:** The solution of **compound 3** (0.2 g, 1.0 eq) in 10 mL of dioxane was treated with 4 mL of 1 N HCl, and the mixture was heated to 60oC for 2 h. After cooling, the pH was adjusted to 8 by addition of NaHCOs. The mixture was extracted with dichloromethane, washed the organic layer with water, dried with Na₂SO₄ and evaporated to dryness. The crude product was purified by column chromatography to give

### compound 4.

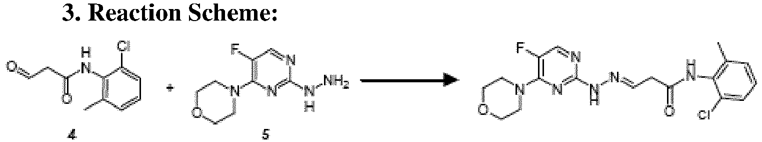

**Experimental Details:** The mixture of **compound 4** (40 mg, 1.0 eq) and **compound 5** (30 mg, 1.0 eq) in 5 mL of dichloromethane was stirred at r.t. overnight. The mixture was concentrated to dryness and purified by preparative HPLC to give the desired compound.

### EXAMPLE 8.

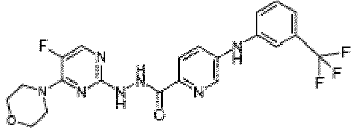

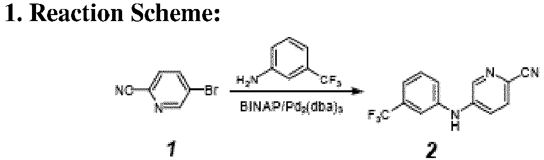

**Experimental Details:** To a solution of 1g (5.5mmol) of 5-bromo-2-cyanopyridine, 0.97g of (6.05mmol 1.1eq ) 3-(trifluoromethyl)aniline in 100ml of toluene was added 3eq of t-BuONa, 0.2eq of BINAP and O.leq of Pd₂(dba)₃. Then the solution was heated to reflux overnight. The reaction was monitored by LC/MS. The volatiles were removed under reduced pressure. The crude product was purified by flash chromatography to afford **compound 2.**

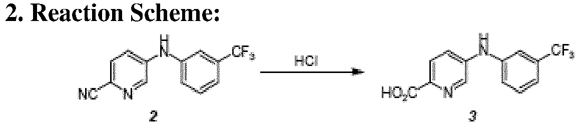

**Experimental Details:** 250mg (0.95mmol) of **compound 2** was added to 20mL of concentrated HCl , then the solution was heated to reflux until the starting material disappeared. The mixture was concentrated under reduced pressure to obtain **compound 3** as a yellow solid without purification.

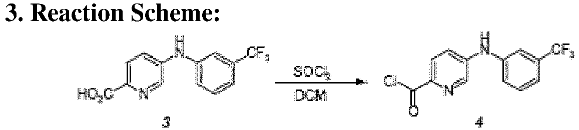

**Experimental Details:** A solution of 50mg (0.18mmol) of **compound 3** in 3mL of dichloromethane was added to 0.5mL of thionyl dichloride. The mixture was heated and stirred for 3h. Finally the solution was evaporated under reduced pressure. **Compound 4** was obtained and used in next step without purification.

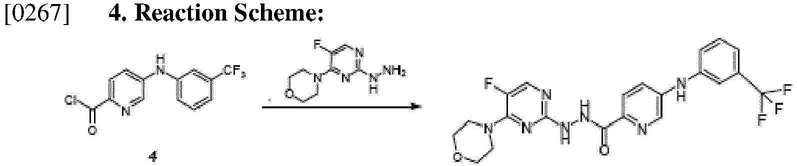

**Experimental Details:** A solution of 50mg of **compound 4** and 43mg(1.2eq) of hydrazine in 5mL of DCM was stirred at 25°C for 3 hours. The reaction mixture was concentrated and the residue was purified by preparative HPLC to give the desired compound.

### EXAMPLE 9.

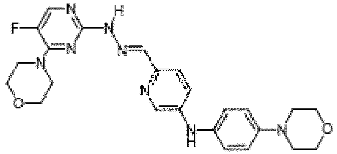

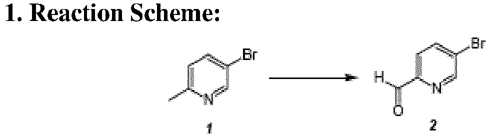

**Experimental Details:** A suspension of compound **1** (25 g, 0.145 mol) and SeO₂ (27.5 g, 0.247 mol) in acetic acid (1200 mL) was heated to reflux for 12 h. The reaction mixture was concentrated under reduced pressure to dryness. The residue was dissolved into water and brought to pH = 9 by adding K₂CO₃. The resulted mixture was extracted with EA (100 mL × 3). The combined EA was dried over Na₂SO₄. After filtrating off the Na₂SO₄, the filtrate was concentrated under reduced pressure to give the crude product **2,** which was used in next step without purification.

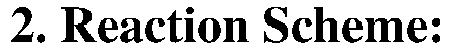

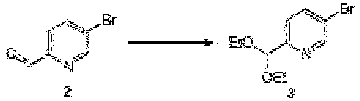

**Experimental Details:** A solution of **2** above prepared in ethanol triethyl orthoformate (10 mL) was refluxed 4 h. After removing off the solvent, the residue was separated by column to give the product **3**
as oil.

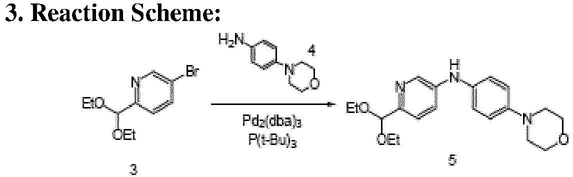

**Experimental Details:** To a stirred and degassed mixture of compound **3** (73 mg, 0.28 mmol) and compound **4** (50 mg, 0.28 mmol), and tBuONa (27 mg, 0.56 mmol) and BINAP (70.4 mg, 1.12 mol) in toluene (15 mL) was added Pd₂(dba)₃ (26 mg, 0.028 mmol) under N₂ atmosphere and stirred at 80 _{°}C for 48 h. After filtering off the solid, the filtrate was concentrated to give the crude product **5** which was used in the next step without purification.

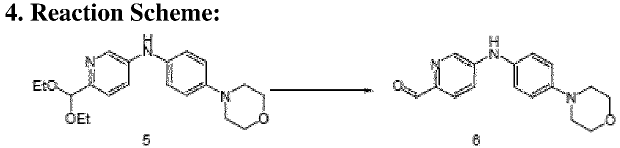

**Experimental Details:** A solution of compound **5** (335 mg, 0.1 mmol) in dichloromethane (10 mL) was treated with BBr₃ (146 mg, 0.6 mmol) at - 30°C under N₂ atmosphere, then was stirred at room temperature for 4 h. The reaction was poured unto ice-water and then was brought by adding Na₂CO₃. The resulting mixture was extracted with dichloromethane (25 mL × 3), the combined organic layer was dried over Na₂SO₄. After filtrating off the Na₂SO₄, the filtrate was concentrated to give the crude product **6** which was done next step without purification.

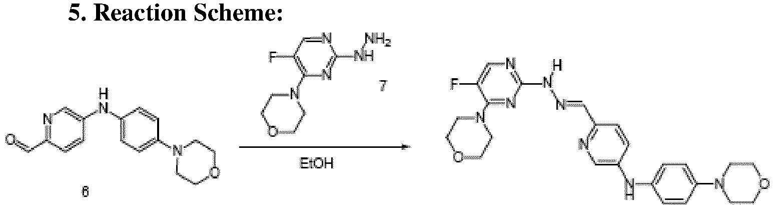

**Experimental Details:** A solution of compound **6** (27.74 mg, 0.1 mmol) and compound **7** (21 mg, 0.1 mmol) in anhydrous CH₂Cl₂ (300 mL) was stirred under reflux for 6 h. The solvent was removed under reduced pressure. The residue was separated by prep-TLC to give the desired compound.

### EXAMPLE 10.

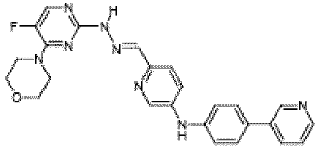

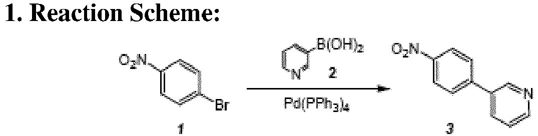

**Experimental Details:** To a stirred and degassed mixture of compound **1** (5 g, 25 mmol) and compound **2** (3.4g, 27 mmol) in DMF (50 mL) and aqueous Na₂CO₃ (20 mL, 2 M) was added Pd₂(dbbf)₃ (26 mg, 0.028 mmol) under N₂ atmosphere and stirred at 100 °C for 18 h. After cooling to room temperature and filtrating off the solid, the filtrate was extracted with EA (200 mL). The organic layer was concentrated to dryness. The residue was purified by column to give the crude product **3.**

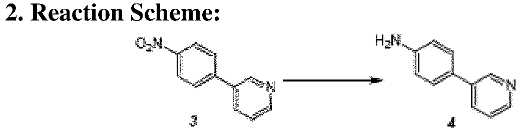

**Experimental Details:** A mixture of compound **3** (2 g, 0.1 mol) and Na₂S₂O₄ (5.2 g, 0.3 mol) in methanol (80 mL) and H₂O (20 mL) was heated to reflux for 3 h. The reaction was concentrated to dryness under reduced pressure. The residue was dissolved into water and then was extracted with EA (150 mL). The organic layer was washed with brine twice and dried over Na₂SO₄. After filtrating off the Na₂SO₄, the filtrate was concentrated to give the product **4.**

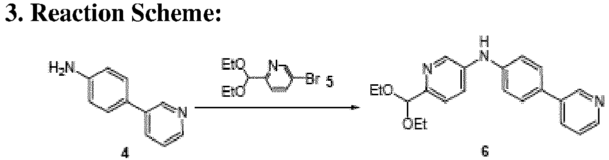

**Experimental Details:** To a stirred and degassed mixture of compound **5** (228 mg, 88 mmol) and compound **4** (150 mg, 88 mmol), and tBuONa (170 mg, 176 mmol) and BINAP (210 mg, 176 mmol) in toluene (25 mL) was added Pd₂(dba)₃ (79 mg, 0.88 mmol) under N₂ atmosphere and stirred at 80 °C for 48 h. After filtering off the solid, the filtrate was concentrated to give the crude product **6.**

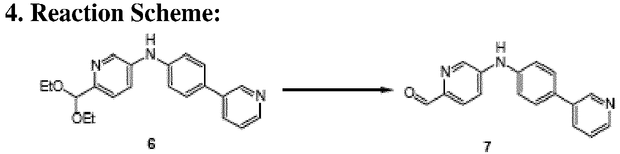

**Experimental Details:** A solution of compound 6 (140 mg, 4 mmol) in dichloromethane (20 mL) was treated with BBr₃ (600 mg, 10.6 mmol) at - 30°C under N₂ atmosphere, then was stirred at room temperature for 4 h. The reaction was poured unto ice-water and then was brought pH = 9 by adding Na₂CO₃. The resulting mixture was extracted with dichloromethane (25 mL × 3), the combined organic layer was dried over Na₂SO₄. After filtrating off the Na₂SO₄, the filtrate was concentrated to dryness. The residue was purified by column to give the product **7.**

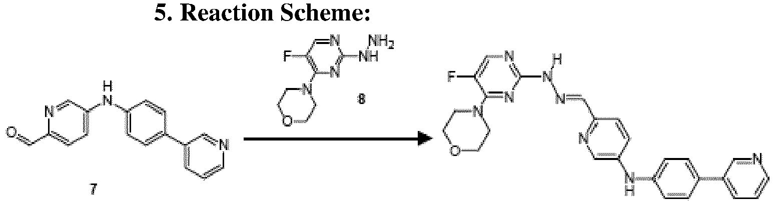

**Experimental Details:** A solution of compound **7** (98 mg, 0.36 mmol) and compound **8** (64 mg, 0.3 mmol) in anhydrous CH₂Cl₂ (300 mL) was stirred under reflux for 6 h. The solvent was removed under reduced pressure. The residue was separated by prep-TLC to give the desired compound.

### EXAMPLE 11.

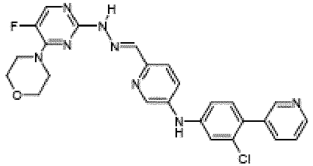

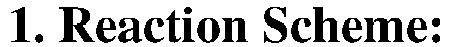

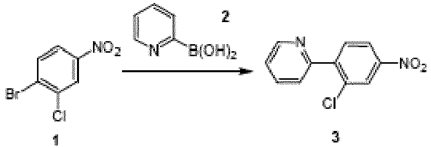

**Experimental Details:** To a stirred and degassed mixture of compound **1** (5.9 g, 25 mmol) and compound **2** (3.3 g, 27 mmol) in DMF (50 mL) and aqueous Na₂CO₃ (20 mL, 2 M) was added Pd₂(dbbf)₃ (26 mg, 0.028 mmol) under N₂ atmosphere and stirred at 100 °C for 18 h. After cooling to room temperature and filtrating off the solid, the filtrate was extracted with EA (200 mL). The organic layer was concentrated to give the crude product **3** which is done next step without purification.

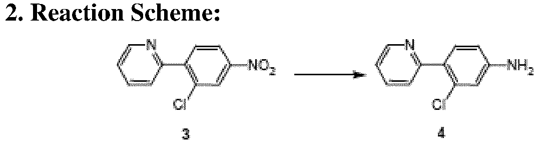

**Experimental Details:** A mixture of **3** (6.5 g, 27.9 mmol) and Pd(OH)₂ (10 %, 0.5 g) in ethanol (200 mL) was stirred under hydrogen atmosphere (20 psi) at room temperature for 2 hour. The catalyst was filtrated off, and the filtrate was removed under vacuum to afford the product **4** as a colorless oil.

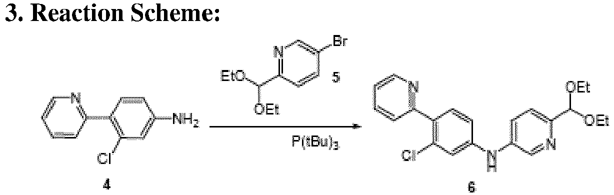

**Experimental Details:** To a stirred and degassed mixture of compound **4** (406 mg, 20 mmol) and compound **5** (520 mg, 20 mmol), and tBuONa (170 mg, 176 mmol) and BINAP (210 mg, 176 mmol) in toluene (25 mL) was added Pd₂(dba)₃ (79 mg, 0.88 mmol) under N₂ atmosphere and stirred at 80 °C for 48 h. After filtrating off the solid, the filtrate was concentrated to give the crude product **6** which is used in next step without purification.

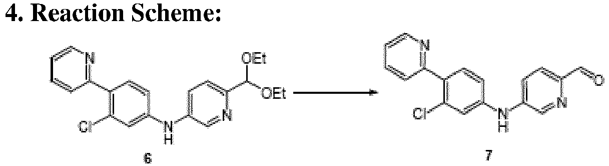

**Experimental Details:** A solution of compound **6** (383 mg, 10 mmol) in dichloromethane (20 mL) was treated with BBr₃ (600 mg, 10.6 mmol) at - 30°C under N₂ atmosphere, then was stirred at room temperature for 4 h. The reaction was poured unto ice-water and then was brought to pH 9 by adding Na₂CO₃. The resulting mixture was extracted with dichloromethane (25 mL × 3), the combined organic layer was dried over Na₂SO₄. After filtrating off the Na₂SO₄, the filtrate was concentrated to dryness. The residue was purified by column to give the product **7.**

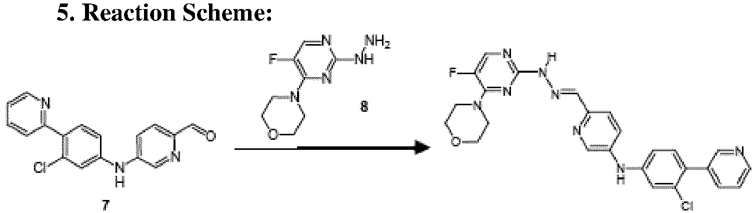

**Experimental Details:** A mixture of **7** (60 mg, 0.22 mmol) nicotinaldehyde (33 mg, 0.15 m mol) in dichloromethane (10 mL) was heated to reflux for 3 hr. After removing off solvent, the residue was purified by chromatography to give the desired compound.

### EXAMPLE 12.

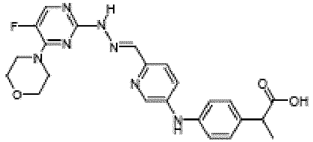

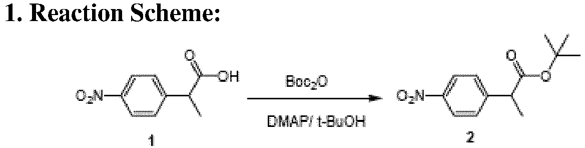

**Experimental Details:** A solution of DMAP (9.3 g, 0.077 mol) and compound **1** (10 g, 0.051 mol) and Boc₂O (12 g, 0.051 mol) in tBuOH (200 mL) was stirred at room temperature overnight. The solvent was removed under reduced pressure and the residue was purified by flash chromatography on silica gel.(ethyl acetate/ petroleum ether = 10:1) to give **2** as a colorless oil.

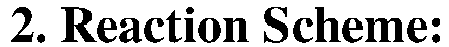

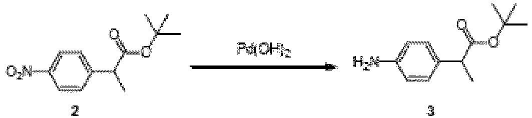

**Experimental Details:** A mixture **of 2** (6.17 g, 27.9 mmol) and Pd(OH)₂ (10 %, 1 g) in ethanol (200 mL) was stirred under hydrogen atmosphere (50 psi) at room temperature for 4 hour. The catalyst was filtrated off, and the filtrate was removed under vacuum to afford the product **3** as a colorless oil.

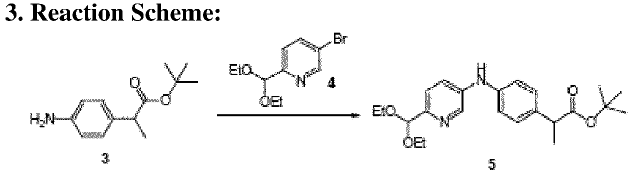

**Experimental Details:** A mixture of compound **3** (2.65 g, 12 mmol) and compound **4** (2.6 g, 10 mmol) and tBuONa (1.34 g, 14 mmol,) and Pd₂(dba)₃ (46.5 mg, 50 mmol, ) and DCHPB (70 mg, 0.2 mmol) in dry toluene (50 mL) was heated to 80-90 °C under N₂ for 24 hours. The precipitation was filtrated and the filtrate was removed in vacuo and the residue was purified by chromatography on silica gel (ethyl acetate/ petroleum ether = 10:1) to give afford **5** as a yellow oil.

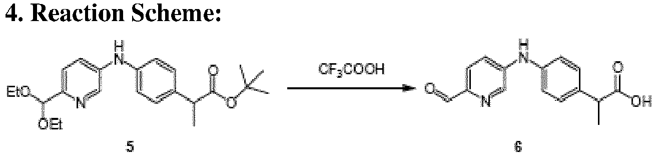

**Experimental Details:** To a solution of **5** (0.9 g, 2.24 mmol) in CHCl₃ (50 mL), CF₃COOH (40 mL) was added at 0°C. After addition complete, the resulting mixture was stirred at room temperature overnight. The solvent was removed under reduced pressure to dryness. The residue was recrystallized from ether to yield an off -white solid. The solid was dissolve in ammonia (10 mL). The mixture was brought the pH = 7.0 by adding 1M HCl and precipitated. The precipitate was collected and washed with cold water (5 mL), and dried under reduced pressure to afford **6** as a dark solid.

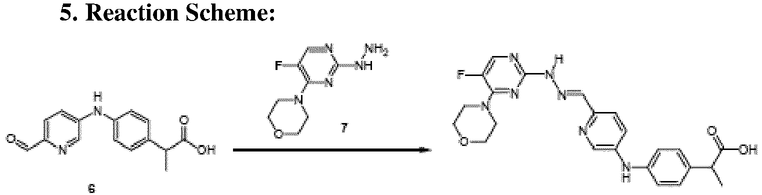

**Experimental Details:** A mixture of **6** (60 mg, 0.22 mmol), nicotinaldehyde (33 mg, 0.15 m mol) in dichloromethane (10 mL) was heated to reflux for 3 hr. After removing off solvent, the residue was purified by chromatography to give the desired compound as a yellow solid.

### EXAMPLE 13.

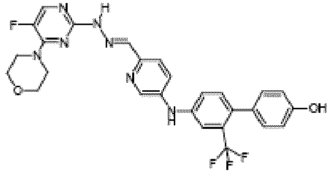

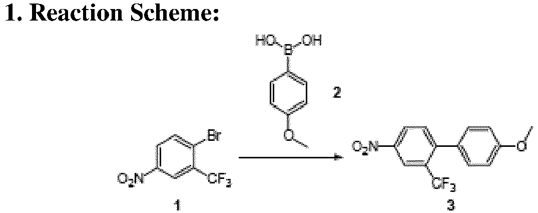

**Experimental Details:** To a stirred and degassed mixture of compound **1** (6.7 g, 25 mmol) and compound **2** (4.1 g, 27 mmol) in DMF (50 mL) and aqueous Na₂CO₃ (20 mL, 2 M) was added Pd₂(dbbf)₃ (26 mg, 0.028 mmol) under N₂ atmosphere and stirred at 100 °C for 18 h. After cooling to room temperature and filtrating off the solid, the filtrate was extracted with EA (200 mL). The organic layer was concentrated to give the crude product **3.**

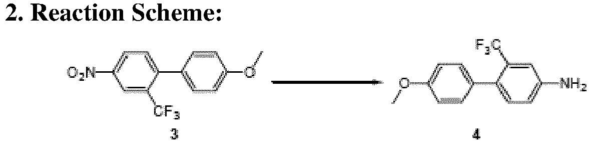

**Experimental Details:** A mixture of **3** (3.2 g, 10 mmol) and Pd(OH)₂ (10 %, 0.5 g) in ethanol (200 mL) was stirred under hydrogen atmosphere (20 psi) at room temperature for2 hour. The catalyst was filtrated off, and the filtrate was removed under vacuum to afford the product **4** as colorless oil.

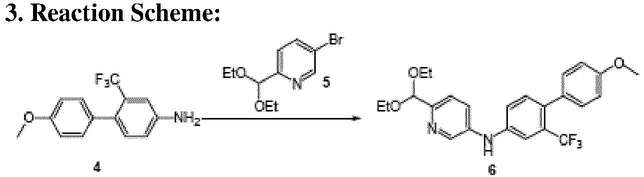

**Experimental Details:** To a stirred and degassed mixture of compound **4** (297 mg, 10 mmol) and compound **5** (259 mg, 10 mmol), and tBuONa (170 mg, 17.6 mmol) and BINAP (210 mg, 17.6 mmol) in toluene (25 mL) was added Pd₂(dba)₃ (79 mg, 0.88 mmol) under N₂ atmosphere and stirred at 80 °C for 48 h. After filtrating off the solid, the filtrate was concentrated to give the crude product **6** which is done next step without purification.

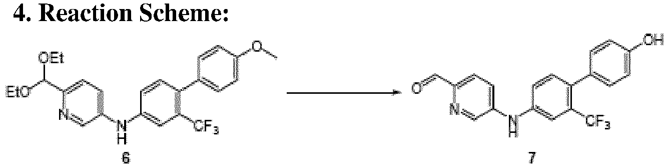

**Experimental Details:** A solution of compound **6** (446 mg, 10 mmol) in dichloromethane (20 mL) was treated with BBr₃ (600 mg, 10.6 mmol) at - 30oC under N₂ atmosphere, then was stirred at room temperature for 4 h. The reaction was poured unto ice-water and then was brought pH = 9 by adding Na₂CO₃. The resulting mixture was extracted with dichloromethane (25 mL × 3), the combined organic layer was dried over Na₂SO₄. After filtrating off the Na₂SO₄, the filtrate was concentrated to dryness. The residue was purified by column to give the product **7.**

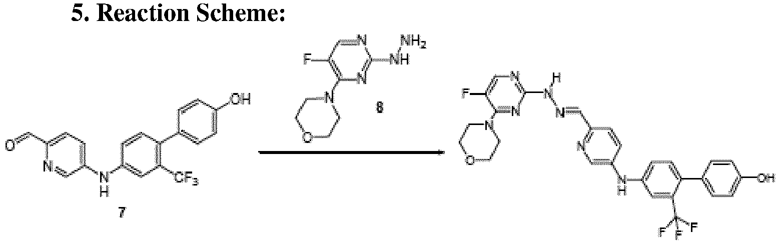

**Experimental Details:** A mixture **of 7** (67 mg, 0.15 mmol), nicotinaldehyde (33 mg, 0.15 m mol) in dichloromethane (10 mL) was heated to reflux for 3 hr. After removing off solvent, the residue was purified by chromatography to give the desired compound as a yellow solid.

### EXAMPLE 14.

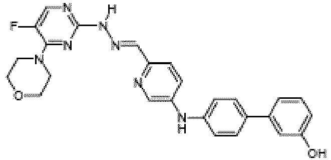

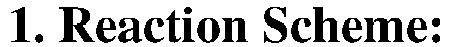

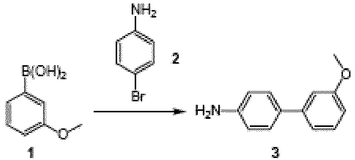

**Experimental Details:** To a stirred and degassed mixture of compound **1** (3 g, 0.02 mol) and compound **2** (3.4 g, 0.02 mol), and KOH (5.28 g, 0.1 mol) and TBBA (6.44 g, 0.02 mol) in anhydrous THF (100 mL) was added Pd (PPh₃)₄ (2.31 g, 2 mmol) under N₂ atmosphere and stirred under reflux for 12 h. After filtrating off the solid, the filtrate was concentrated to dryness. The residue was purified by column to give the product **3,** which is used in next step without purification.

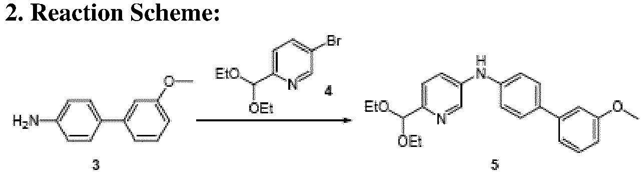

**Experimental Details:** To a stirred and degassed mixture of compound **3** (334 mg, 1.7 mmol) and compound **4** (434 mg, 1.7 mmol), and t-BuONa (322 mg, 3.4 mmol) and BINAP (420 mg, 0.67 mol) in toluene (60 mL) was added Pd₂(dba)₃ (156 mg, 0.017 mmol) under N₂ atmosphere and stirred at 80 °C for 48 h. After filtrating off the solid, the filtrate was concentrated to dryness. The residue was purified by column to give the product **5.**

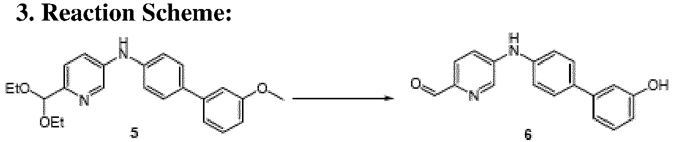

**Experimental Details:** A solution of compound **5** (100 mg, 0.3 mmol) in dichloromethane (10 mL) was treated with BBr₃ (393 mg, 0.6 mmol) at - 30°C under N₂ atmosphere, then was stirred at room temperature for 4 h. The reaction was poured unto ice-water and then was brought by adding Na₂CO₃. The resulting mixture was extracted with dichloromethane (25 mL × 3), the combined organic layer was dried over Na₂SO₄. After filtrating off the Na₂SO₄, the filtrate was concentrated to give the crude product **6.**

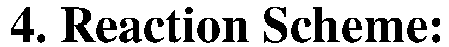

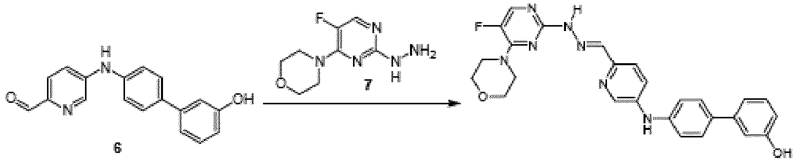

**Experimental Details:** A mixture of **6** (39 mg, 0.13 mmol), nicotinaldehyde (29 mg, 0.13 m mol) in dichloromethane (10 mL) was heated to reflux for 3 hr. After removing off solvent, the residue was purified by chromatography to give the desired compound as a yellow solid.

### EXAMPLE 15.

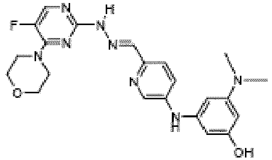

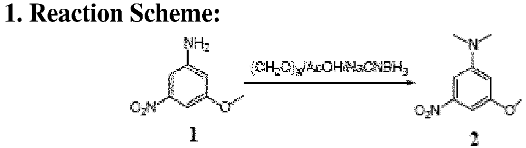

**Experimental Details:** Paraformaldehyde(1.8g, 59.3mmol) was added to a solution of compound **1** (1.0 g, 5.9 mmol) in acetic acid (40mL), followed by NaCNBH₃(1.8 g, 28.8 mmol) at 10°C. After stirring of 16h at room temperature, the solution was poured into ice/water (100mL), and the PH adjusted to 10 with concentrated NaOH. The solution was extracted with DCM(3 × 100mL). The combined organic layers were dried (MgSO₄), filtrated and concentrated in vacuo to give compound **2.**

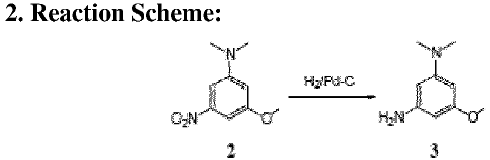

**Experimental Details:** 0.84g (4.3mmol) of compound **2** was hydrogenated under latm hydrogen with Pd/C for 16 hour. The reaction mixture was filtered and the filtrate was concentrated to afford compound **3** without further purification.

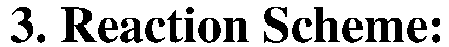

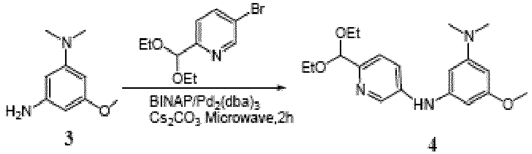

**Experimental Details:** A solution of 250mg (1.51mmol) of compound **3,** 0.2eq of BINAP, O.leq of Pd₂(dba)₃, 3eq of Cs₂CO₃ and 5-Bromo-2-diethoxymethyl-pyridine(0.783g, 3.01mmol) in lOmL of 1,4-dioxane was reacted at 150°C under microwave for 2 hours. The reaction was monitored by LC-Ms. The mixture was concentrated and the residue was purified by preparative TLC to afford compound **4.**

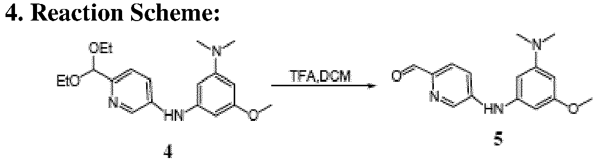

**Experimental Details:** A solution of 300mg(0.55mmol) of compound **4** in 5mL of DCM was added 4mL of TFA. The reaction mixture was stirred for 30min at r.t. The mixture was added ice/water and basified by NaHCOs to PH=10 and extracted with DCM (15mL*3). The combined organic layer was washed with water and brine, dried over MgSO₄, filtered and concentrated to afford compound **5.**

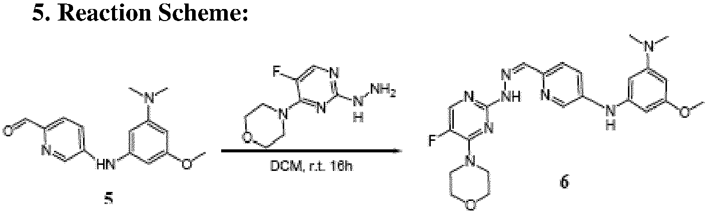

**Experimental Details:** A solution of 80mg(0.295mmol) of compound **5** and (5-Fluoro-4-morpholin-4-yl-pyrimidin-2-yl)-hydrazine (125mg, 0.59mmol) in lOmL of DCM was stirred at 25°C for 15 hours. The reaction mixture was concentrated and the residue was purified by preparative HPLC to afford compound **6.**

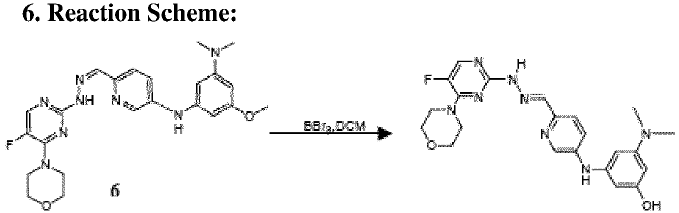

**Experimental Details:** To a solution of compound 6 (40mg, 0.086mmol) in dry DCM (5mL) was added drop wise BBr (22mg, 0.258mmol) at 0°C. The reaction mixture was stirred for 3h at r.t. The reaction was quenched with methanol, and the mixture was concentrated. The residue was purified by preparative HPLC to give the desired compound.

### EXAMPLE 16.

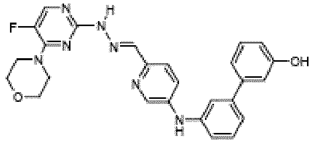

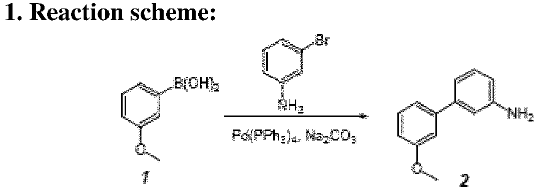

**Experimental details:** To a stirred solution of 3-Bromoaniline (0.86g) in 30ml of toluene were added O.leq of Pd(PPh₃)₄, 5ml of sat. aq. of Na₂CO₃ and a solution of 3-methoxyphenyl boronic acid (0.75g) in 10ml of EtOH under N₂ atmosphere. The mixture was vigorously stirred under reflux for 15h and cooled, 10ml of H₂O was added, and the mixture was extracted with CH₂Cl₂ (20ml×3). The combined organic layers were dried over Na₂SO₄ and concentrated. The residue was purified by column chromatography (PE/EA=10:1) to get pure **compound 2.**

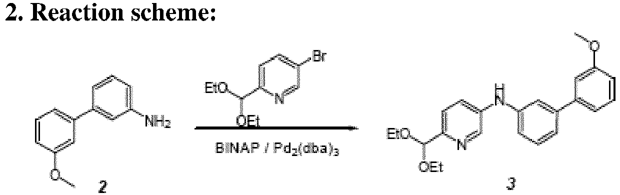

**Reaction details:** A mixture of **compound 2** (59.5mg), 5-bromo-2-diethoxymethyl-pyridine (116.7mg), t-BuONa (86.4mg), BINAP (36.7mg) and Pd₂(dba)₃ (27.4mg) in dioxane (2ml) was microwaved for 2hs at 150°C, the solution was filtered and concentrated. The residue was purified by preparative TLC to afford **compound 3.**

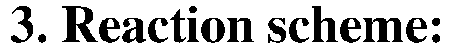

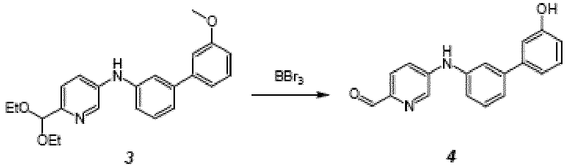

**Reaction details:** To a solution of **compound 3**(120mg) in 5ml of DCM was added 1ml of BBr₃, the reaction was stirred overnight at r.t. Then added 5ml of H₂O to the mixture, extracted with EtOAc and concentrated. The crude product was purified by pre. TLC to afford **compound 4.**

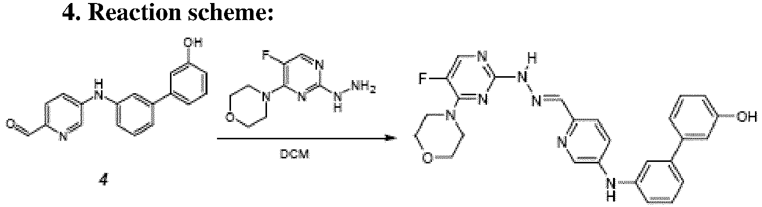

**Experimental details:** A mixture of 43.5mg of **compound 4** and 32mg of hydrazine in 5 ml DCM was stirred overnight at r.t. and concentrated. The crude product was purified by prereparative TLC to afford the desired compound.

### EXAMPLE 17.

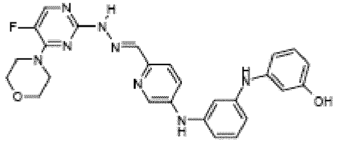

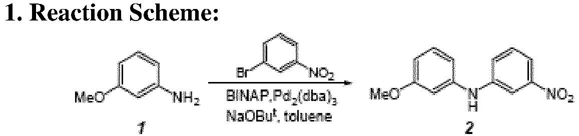

**Experimental Details:** A solution of 2.0 g (16.2 mmol, 1.0 eq) of **compound 1, 0.05** eq of BINAP, 0.05 eq of Pd₂(dba)₃, 1.2 eq of t-BuONa and 1-Bromo-3-nitro-benzene (3.28 g, 16.2 mmol, 1.0 eq) in 20 mL of anhydrous toluene was reacted at 100oC for 24 hours. The reaction was monitored by LC-MS. The mixture was concentrated and the residue was purified by column chromatography to afford **compound 2.**

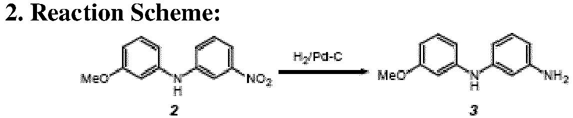

**Experimental Details:** 2.5 g of **compound 2** was hydrogenated under latm of hydrogen with Pd/C (0.25 g) for 16 hour. The reaction mixture was filtered and the filtrate was concentrated to afford **compound 3** without further purification.

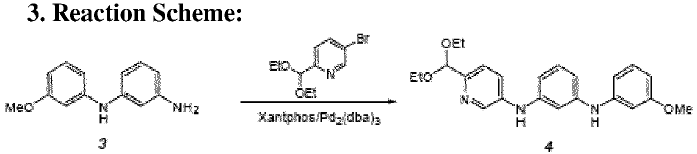

**Experimental Details:** A solution of 500 mg (2.33 mmol) of **compound 3,** 5-Bromo-2-diethoxymethylpyridine (607 mg, 2.33 mmol), 0.05 eq of xantphos, 0.05 eq of Pd₂(dba)₃ and 1.5eq of t-BuONa in 10 mL of toluene was refluxed at 100°C for 24 hours. The reaction was monitored by LC-MS. The mixture was concentrated and the residue was purified by column
chromatography to afford **compound 4.**

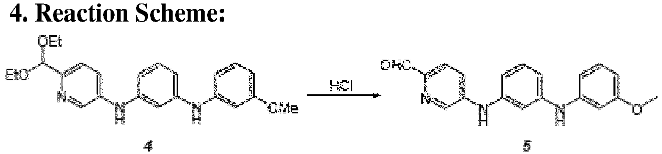

**Experimental Details:** 100 mg of **compound 4** was treated with 1 mL of HCl (1N aqueous solution) and 10 mL of dioxane. The mixture was stirred at rt for 4 h, adjusted to pH 8-9 with 0.5 N NaOH solution. After extracted with DCM, dried organic layer with Na₂SO₄ and concentrated to give **compound 5.**

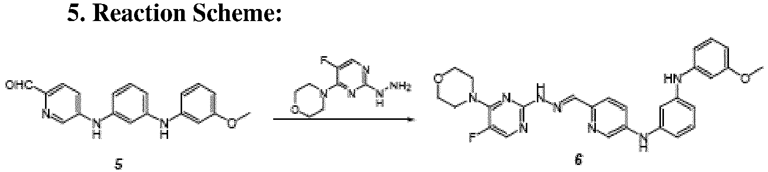

**Experimental Details:** A solution of 50 mg (0.16 mmol) of **compound 5** and (5-Fluoro-4-morpholin-4- ylpyrimidin-2-yl)-hydrazine (50 mg, 0.23 mmol) in 5 mL of DCM was stirred at 25°C for 15 hours. The reaction mixture was concentrated and the residue was purified by preparative HPLC to afford **compound 6.**

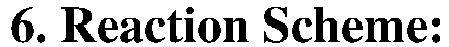

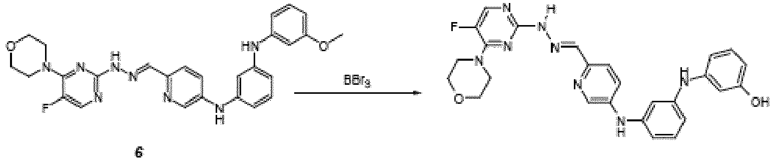

**Experimental Details:** To a solution of **compound 6** (50 mg, 0.09 mmol) in dry DCM(5mL) was added dropwise BBr₃ (20 mg, 0.25 mmol) at 0°C. The reaction mixture was stirred for 3hr at r.t. The reaction was quenched with methanol, and the mixture was concentrated. The residue was purified by preparative HPLC to give the desired compound.

### EXAMPLE 18.

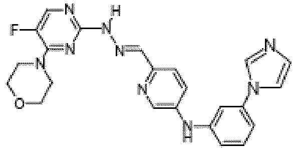

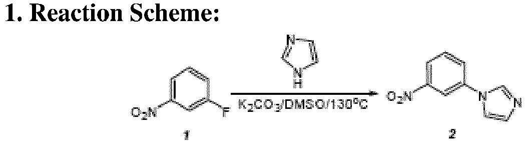

**Experimental Details:** A solution of 10 g (70.92mmol) of 3-fluoro-nitrobenzene, 1eq imidazole and 2eq K₂CO₃ in 100ml of DMSO was heated at 130°C for 5 hours. The reaction was monitored by LC-MS. Then 500mL of water was added and the precipitate was filtered and the solid was washed with water and dried to afford **compound 2** without further purification.

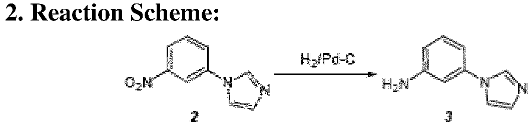

**Experimental Details:** 5g (31.4mmol) of **compound 2** was hydrogenated under latm hydrogen with Pd/C for 0.5 hour. The reaction mixture was filtered and the filtrate was concentrated to afford **compound 3** without further purification.

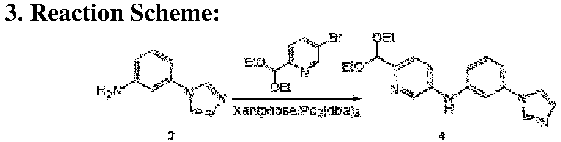

**Experimental Details:** A solution of 500mg (3.14mmol) of **compound 3,** O.leq of xantphose, O.leq of Pd₂(dba)₃ and 1.5eq of t-BuONa in lOmL of toluene was refluxed at 130°C for 15 hours. The reaction was monitored by LC-Ms and washed with water and extracted with EtOAc. The combined organic layer was washed with brine and dried over MgSO₄. Filtered and concentrated, residue was purified by preparative TLC to afford **compound 4.**

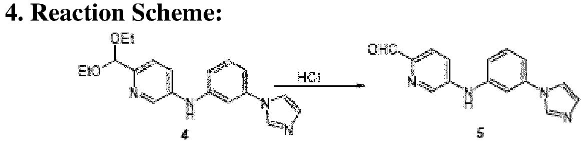

**Experimental Details:** A solution of 200mg of **compound 4** in 5mL of 1,4-dioxane was added 8mL of 4N HCl and heated at 80°C for 2 hours. The reaction mixture was basified by 2N NaOH to ph=10 and extracted with DCM (15mL*3). The combined organic layer was washed with water and brine, dried over MgSO₄, filtered and concentrated to afford 250mg of crude product. The crude product was purified by preparative TLC to afford **compound 5.**

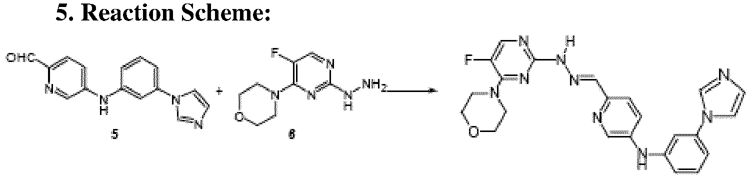

**Experimental Details:** A solution of 80 mg of **compound 5** and 1eq of **compound 6** in 5mL of DCM was stirred at 25°C for 15 hours. The reaction mixture was concentrated and the residue was purified by preparative HPLC to afford the desired compound.

### EXAMPLE 19.

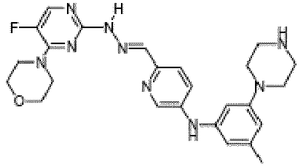

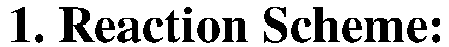

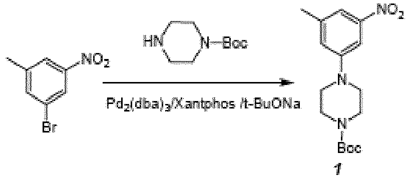

**Experimental Details:** A solution of 1.50g of 1-Bromo-3-methyl-5-nitrobenzene and 1.60g (1.2eq)of Piperazine-1-carboxylic acid tert-butyl ester , O.leq of xantphos, O.leq of Pd₂(dba)₃ and 1.5eq of t-BuONa in 20mL of toluene was refluxed at 130°C for 4 hours. The reaction was monitored by LC-Ms and washed with water and extracted with EtOAc. The combined organic layer was washed with brine and dried over Na₂SO₄. Filtered and concentrated, residue was purified with column chromatography on silica gel using 10:1 PA:EA as an eluant. The appropriate fractions were combined and concentrated under reduced pressure, to give intermediate **1.**

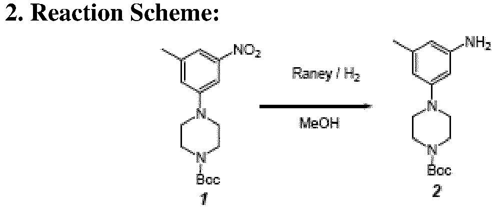

**Experimental Details:** 1.6g of intermediate of **1** was hydrogenated under latm hydrogen with Raney/Ni for 2 hours. The reaction mixture was filtered and the filtrate was concentrated to afford intermediate **2** without further purification.

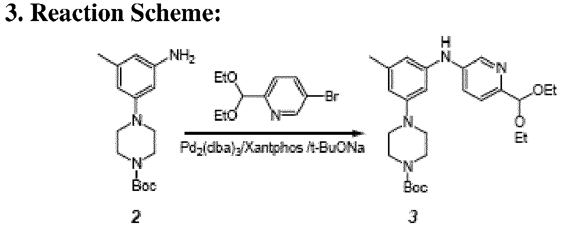

**Experimental Details:** A solution of 1.20g of Intermediate of **2,** and1.30g (1.20eq) of 5-Bromo-2-diethoxymethyl-pyridine, O.leq of xantphose, O.leq of Pd₂(dba)₃ and 1.5eq of t-BuONa in 20mL of toluene was refluxed at 130°C for 4 hours. The reaction was monitored by LC-MS and washed with water and extracted with EtOAc. The combined organic layer was washed with brine and dried over Na₂SO₄. Filtered and concentrated, residue was purified with column chromatography on silica gel using 4:1 PA:EA as an eluant. The appropriate fractions were combined and concentrated under reduced pressure to give intermediate **3.**

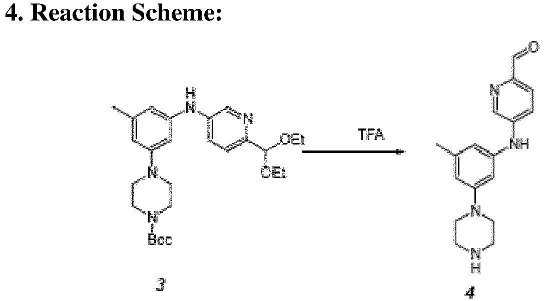

**Experimental Details:** 200mg of intermediate **3** was dissolved in 10ml of DCM. 130mg of TFA was added to the reaction mixture solution dropwise and stirred for 3h at r.t. The reaction mixture was based with NaHCOs solution to neutral and brine,dried over Na₂SO₄, filtered and concentrated to afford 220mg of crude product. The crude product was purified by preparative TLC to afford intermediate **4.**

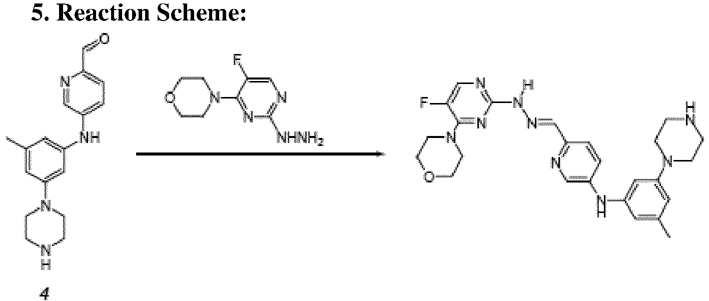

**Experimental Details:** A solution of 50mg of intermediate **4** and 1.0eq of (5-Fluoro-4-morpholin-4-ylpyrimidin-2-yl)-hydrazine in 5mL of DCM was stirred at r.t. for 3 hours. The reaction mixture was washed with water and brine, concentrated it to give the residue and purified by preparative HPLC to afford the desired compound.

### EXAMPLE 20.

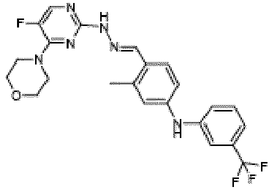

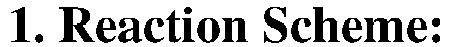

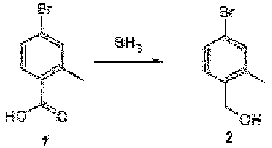

**Experimental Details:** 15ml of 1M BH₃/THF was added dropwise into a solution of 3g (13.95mmol) of 4- bromo-2-methyl-benzoic acid in 20ml of THF at 0°C. The reaction solution was allowed to reach room temperature for 1hour and quenched by the dropwise addition of 50ml of 50% aqueous THF. The mixture was treated with Na₂CO₃ and concentrated. The residue was extracted with Et₂O. The organic layer was dried to give **compound 2.**

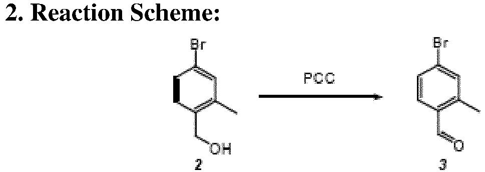

**Experimental Details:** A solution of 2.4g (11.9mmol) of **compound 2** in 20ml of DCM was added a slurry of 5.1g(23.8mmol) of PCC in 60ml of DCM. The reaction solution was stirred for 1hour at r.t. diluted with 300ml of Et₂O and filtered. The filtrate was concentrated to give **compound 3.**

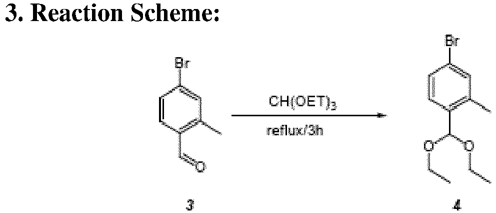

**Experimental Details:** A solution of 2.1g(14mmol) was added into a solution of ethanol which containing 1.9g (9.55mmol) of **compound 3.** The reaction solution was heated to reflux for 3hours, and then concentrated. The solid was washed with NaHCO₃ and extracted with acetic ether. The organic layer was dried to give **compound 4.**

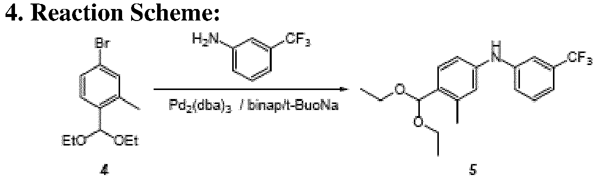

**Experimental Details:** 0.7g of **compound 4,** 0.41g of 3-trifluromethyl-phenylamine 0.32g of Pd₂(dba)₃, 0.21g of binap and 0.02g of t-BuONa were added into 35ml of toluene. The reaction solution was heated to reflux overnight, and concentrated. The crude product was purified by column chromatography (ethyl acetate/hexane=1:1) to give **compound 5.**

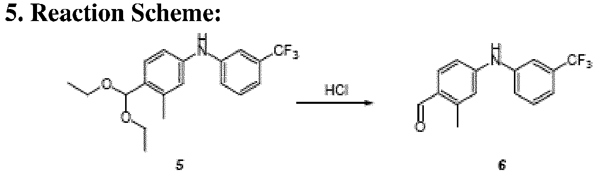

**Experimental Details:** The solution of **compound 5** (0.2 g, 1.0 eq) in 10 mL of dioxane was treated with 4 mL of 1 N HCl, and the mixture was heated to 60°C for 2 h. After cooling, the pH was adjusted to 8 by addition of NaHCOs. The mixture was extracted with dichloromethane, washed the organic layer with water, dried with Na₂SO₄ and evaporated to dryness. The crude product was purified by column chromatography to give **compound 6.**

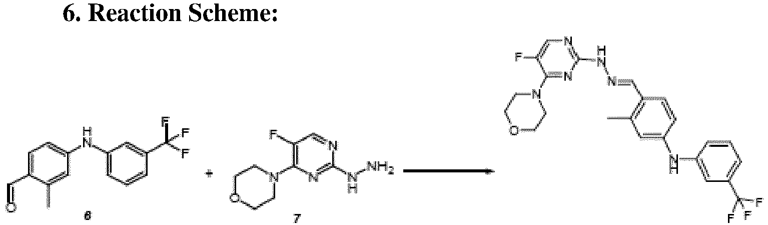

**Experimental Details:** A solution of 80mg of **compound 6** and 1 eq of **compound 7** in 5mL of DCM was stirred at 25°C for 15 hours. The reaction mixture was concentrated and the residue was purified by preparative HPLC to afford the desired compound.

### EXAMPLE 21.

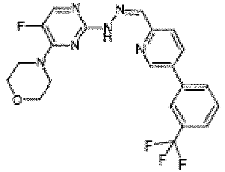

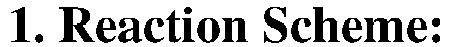

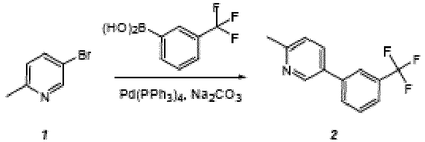

**Experimental Details:** To the mixture of **compound 1** (0.5 g, 1.0 eq), 3-trifluoromethyl phenyl boronic acid (0.63 g, 1.0 eq), Na₂CO₃ (0.46 g, 1.5 eq) in 15 mL of dioxane was added Pd(PPh₃)₄ (0.33 g, 0.1 eq), and the reaction mixture was refluxed under N₂ for 16 h. After cooling, the mixture was filtered and the filtrate was evaporated to dryness and purified by column chromatography to give **compound 2.**

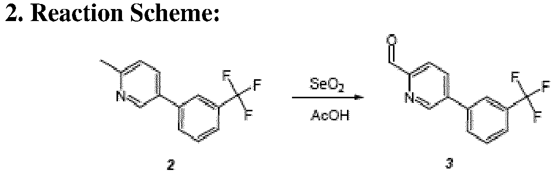

**Experimental Details:** A mixture of **compound 2** (0.2 g, 1.0 eq), SeO₂ (0.19 g, 2.0 eq) in 10 mL of acetic acid was refluxed under N₂ for 48 h. Solvent was removed by evaporation and the residue was dissolved in water and adjusted to pH 6 with saturated NaHCOs solution, extracted with dichloromethane. The organic layers were collected, dried and evaporated to dryness. The crude product was purified by column chromatography to give **compound 3.**

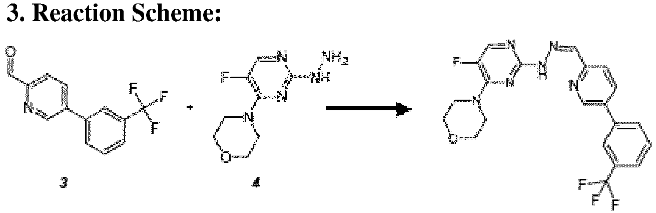

**Experimental Details:** The mixture of **compound 3** (30 mg, 1.0 eq) and **compound 4** (19 mg, 1.0 eq) in 5 mL of dichloromethane was stirred at r.t. overnight. The mixture was concentrated to dryness and purified by preparative HPLC to give the desired compound.

### EXAMPLE 22.

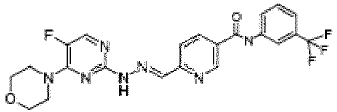

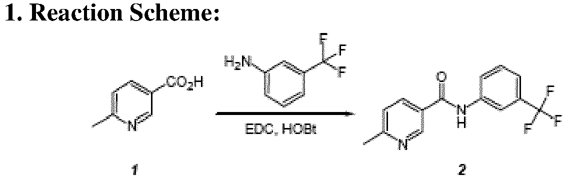

**Experimental Details:** The mixture of **compound 1** (0.5 g, 1.0 eq), trifluoromethyl phenylamine (0.58 g, 1.0 eq), EDC (1.05 g, 1.5 eq), HOBt (50 mg, 0.1 eq) in 15 mL of dichloromethane was stirred at r.t. overnight. The mixture was washed with 1 N NaOH solution, water, extracted with dichloromethane. The organic layer was dried over Na₂SO₄, concentrated to dryness, purified by column chromatography to give **compound 2.**

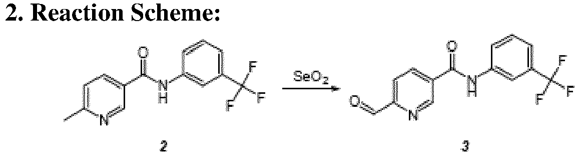

**Experimental Details:** A mixture of **compound 2** (0.2 g, 1.0 eq), SeO₂ (0.16 g, 2.0 eq) in 10 mL of acetic acid was refluxed under N₂ for 48 h. Solvent was removed by evaporation and the residue was dissolved in water and adjusted to pH 6 with saturated NaHCOs solution, extracted with dichloromethane. The organic layers were collected, dried and evaporated to dryness. The crude product was purified by column chromatography to give **compound 3.**

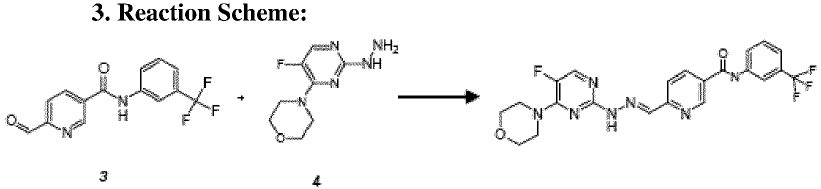

**Experimental Details:** The mixture of **compound 3** (40 mg, 1.0 eq) and **compound 4** (30 mg, 1.0 eq) in 5 mL of dichloromethane was stirred at r.t. overnight. The precipitates were collected and washed with dichloromethane, dried under vacuum to give the desired compound.

### EXAMPLE 23.

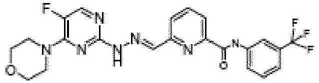

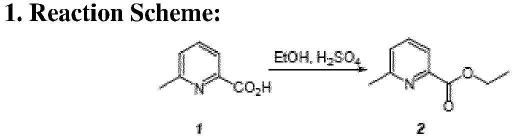

**Experimental Details:** The solution of **compound 1** (3.0 g, 1.0 eq) and 2 mL 98% H₂SO₄ in 10 mL of EtOH was refluxed for 4 h, cooled to r.t., evaporated to dryness, diluted with water, adjusted to pH 8 with NaHCO₃, extracted with dichloromethane. The organic layer was dried and concentrated to give **compound 2.**

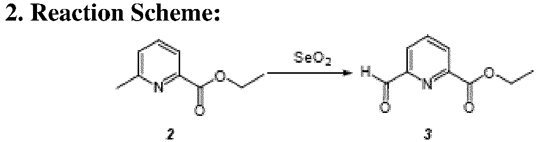

**Experimental Details:** A mixture of **compound 2** (1.7 g, 1.0 eq), SeO₂ (2.29 g, 2.0 eq) in 80 mL of acetic acid was refluxed under N₂ for 48 h. Solvent was removed by evaporation and the residue was dissolved in water and adjusted to pH 6 with saturated NaHCO₃ solution, extracted with dichloromethane. The organic layers were collected, dried and evaporated to dryness. The crude product was purified by column chromatography to give **compound 3.**

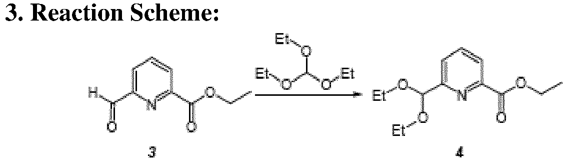

**Experimental Details:** The solution of **compound 3** (1.1 g, 1.0 eq), diethoxymethoxy-ethane (2.3 g, 2.5 eq) and TsOH.H₂O (0.12 g, 0.1 eq) in 20 mL of ethanol was refluxed for 5 h. The solvent was evaporated and the solid was dissolved in EtOAc, washed with water. The organic layer was dried over Na₂SO₄ and evaporated to give **compound 4.**

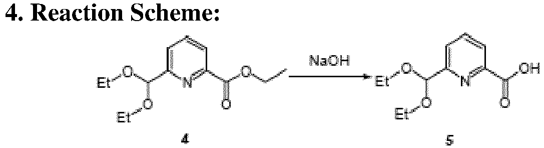

**Experimental Details:** To the solution of **compound 4** (0.6 g, 1.0 eq) in 10 mL of methanol was added 4 mL of 1 N NaOH solution, and the mixture was stirred at r.t. overnight. Solvent was evaporated and the residue was acidified to pH 6 with 5 % citric acid, extracted with dichloromethane. The organic layer was dried, concentrated to give **compound 5.**

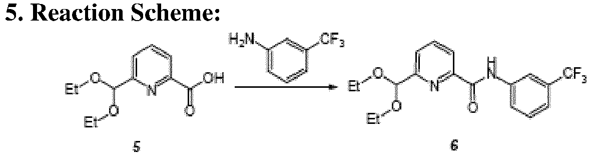

**Experimental Details:** The mixture of **compound 5** (0.4 g, 1.0 eq), trifluoromethyl phenylamine (0.29 g, 1.0 eq), EDC (0.51 g, 1.5 eq), HOBt (25 mg, 0.1 eq) in 10 mL of dichloromethane was stirred at r.t. overnight. The mixture was washed with 1 N NaOH solution, water, extracted with dichloromethane. The organic layer was dried over Na₂SO₄, concentrated to dryness, purified by column chromatography to give **compound 6.**

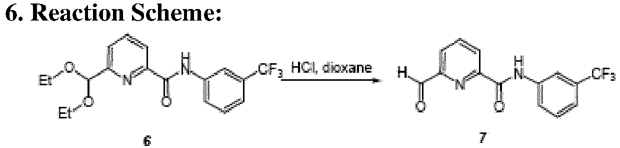

**Experimental Details:** The solution of **compound 6** (0.2 g, 1.0 eq) in 10 mL of dioxane was treated with 4 mL of 1 N HCl, and the mixture was heated to 60°C for 2 h. After cooling, pH was adjusted to 8 by addition of NaHCO₃. The mixture was extracted with dichloromethane, washed the organic layer with water, dried with Na₂SO₄ and evaporated to dryness. The crude product was purified by column chromatography to give **compound 7.**

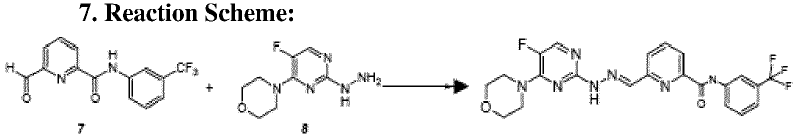

**Experimental Details:** The mixture of **compound 7** (30 mg, 1.0 eq) and **compound** 8 (19 mg, 1.0 eq) in 5 mL of dichloromethane was stirred at r.t. overnight. The precipitates were collected and washed with dichloromethane, dried under vacuum to give the desired compound.

### EXAMPLE 24.

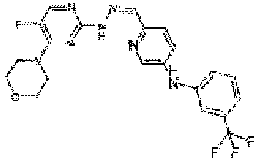

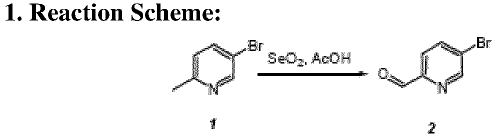

**Experimental Details:** A mixture of **compound 1** (2.0 g, 1.0 eq), SeO₂ (2.6 g, 2.0 eq) in 80 mL of acetic acid was refluxed under N₂ for 36 h. Solvent was removed by evaporation and the residue was dissolved in water and adjusted to pH 6 with saturated NaHCOs solution, extracted with dichloromethane. The organic layers were collected, dried and evaporated to dryness. The crude product was purified by column chromatography to give **compound 2.**

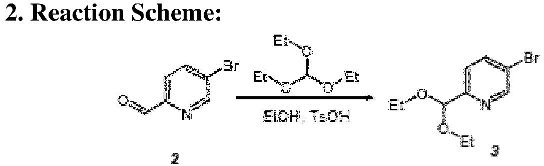

**Experimental Details:** The solution of **compound 2** (0.5 g, 1.0 eq), diethoxymethoxy-ethane (1.0 g, 2.5 eq) and TsOH.H₂O (0.05 g, 0.1 eq) in 8 mL of ethanol was refluxed for 3 h. The solvent was evaporated and the solid was dissolved in EtOAc, washed with water. The organic layer was dried over Na₂SO₄ and evaporated to give **compound 3.**

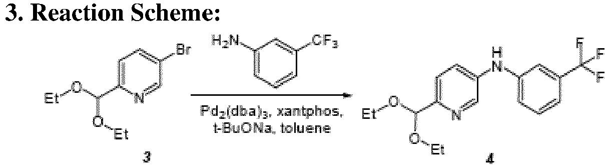

**Experimental Details:** To the mixture of **compound 3** (0.6 g, 1.0 eq), 3-trifluoromethyl-phenylamine (0.37 g, 1.0 eq), t-BuONa (0.26 g, 1.2 eq) in 15 mL of toluene was added under N₂ Pd₂(dba)₃ (42 mg, 0.02 eq) and xantphos (28 mg, 0.02 eq). The mixture was refluxed under N₂ for 16 h, cooled, filtered. The filtrate was concentrated and purified by column chromatography to give **compound 4.**

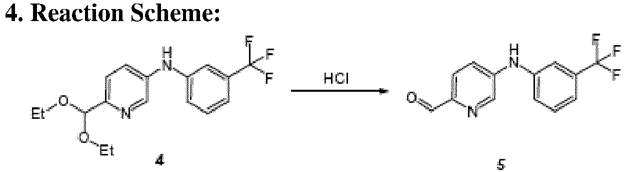

**Experimental Details:** The solution of **compound 4** (0.25 g, 1.0 eq) in 10 mL of dioxane was treated with 4 mL of 1 N HCl, and the mixture was heated to 60°C for 2 h. After cooling, the pH was adjusted to 8 by addition of NaHCOs. The mixture was extracted with dichloromethane, washed the organic layer with water, dried with Na₂SO₄ and evaporated to dryness. The crude product was purified by column chromatography to give **compound 5.**

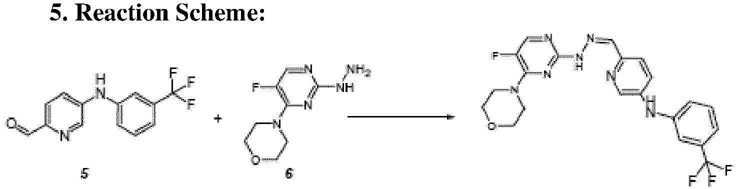

**Experimental Details:** The mixture of **compound 5** (30 mg, 1.0 eq) and **compound 6** (19 mg, 1.0 eq) in 5 mL of dichloromethane was stirred at r.t. overnight. The precipitates were collected and washed with dichloromethane, dried under vacuum to give the desired compound.

### EXAMPLE 25.

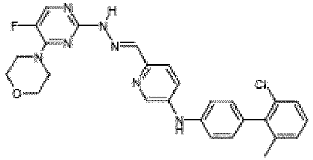

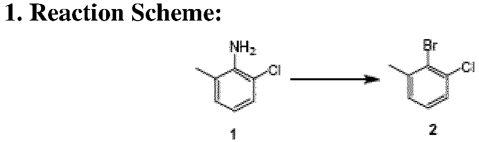

**Experimental Details:** To a solution of compound **1** (14 g, 0.1 mol) in aqueous HBr (30 mL) was added a solution of NaNO₂ (8.3 g 0.15 mol) in H₂O (10 mL) at 0 °C over a period of 30 min. After stirring for 60 min, the reaction mixture was added to a solution of CuBr (14 g, 0.1 mol) in aqueous HBr (16 mL) at 80 °C. After complete addition, the reaction mixture was stirred at the same temperature for 2 h. After cooling to room temperature, the reaction mixture was extracted with EA (100 mL × 3). The combined organic layer were washed with brine and dried over Na₂SO₄. After filtrating off the Na₂SO₄, the filtrate was concentrated to dryness. The residue was purified by column to give the product **2.**

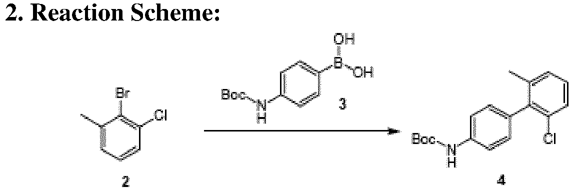

**Experimental Details:** To a stirred and degassed mixture of compound **2** (4.11 g, 0.02 mol) and compound **3** (4.74 g, 0.02 mol), and KOH (5.28 g, 0.1 mol) and TBBA (6.44 g, 0.02 mol) in anhydrous THF (100 mL) was added Pd (PPh₃)₄ (2.31 g, 2 mmol) under N₂ atmosphere and stirred under reflux for 12 h. After filtrating off the solid, the filtrate was concentrated to dryness. The residue was purified by column to give the product **4.**

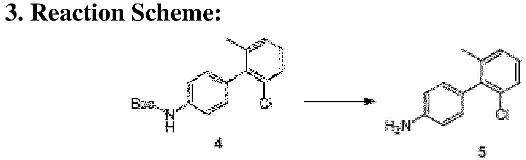

**Experimental Details:** A solution of **4** (1 g, 3 mmol) in dichloromethane (10 mL) was treated with TFA (1 mL) and then stirred for 6 h at room temperature. The solvent was removed under reduced pressure to give the product **5** which is done next step without purification.

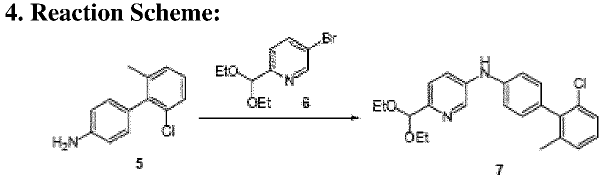

**Experimental Details:** To a stirred and degassed mixture of compound **5** (619 mg, 2.4 mmol) and compound **6** (520 mg, 2.4 mmol), and tBuONa (460 mg, 4.8 mmol) and BINAP (599 mg, 6.9 mol) in toluene (60 mL) was added Pd₂(dba)₃ (221 mg, 0.024 mmol) under N₂ atmosphere and stirred at 80 °C for 48 h. After filtrating off the solid, the filtrate was concentrated to dryness. The residue was purified by column to give the product **7.**

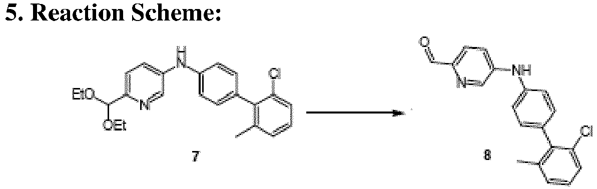

**Experimental Details:** A solution of compound **7** (396 mg, 0.1 mmol) in dichloromethane (10 mL) was treated with BBr₃ (146 mg, 0.6 mmol) at - 30 oC under N₂ atmosphere, then was stirred at room temperature for 4 h. The reaction was poured unto ice-water and then was brought by adding Na₂CO₃. The resulting mixture was extracted with dichloromethane (25 mL × 3), the combined organic layer was dried over Na₂SO₄. After filtrating off the Na₂SO₄, the filtrate was concentrated to give the crude product **8,** which was done next step without purification.

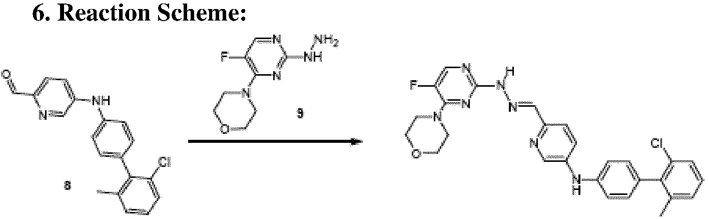

**Experimental Details:** A solution of compound **8** (32.2 mg, 0.1 mmol) and compound **9** (21 mg, 0.1 mmol) in anhydrous CH₂Cl₂ (300 mL) was stirred under reflux for 6 h. The solvent was removed under reduced pressure. The residue was separated by prep-TLC to give the desired compound.

### EXAMPLE 26.

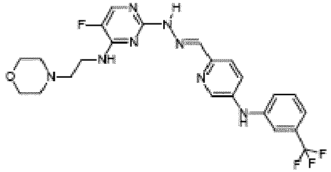

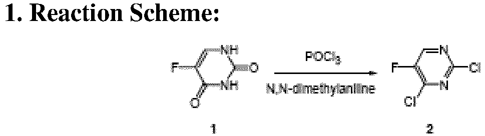

**Experimental Details:** A solution of 5-fluoro-1H-pyrimidine-2,4-dione (113 g, 0.5 mol) in N,N-dimethylaniline (70 mL) was treated with POCl₃ (500 mL), then was reflux for 2 h. After cooling to room temperature, the reaction mixture was poured onto ice-water. The resulting mixture was extracted with ethyl acetate (100 mL × 3). The combined organic layers were washed with saturated aqueous of NaHCO₃, then brine. The solvent was removed under reduced pressure to give compound **2.**

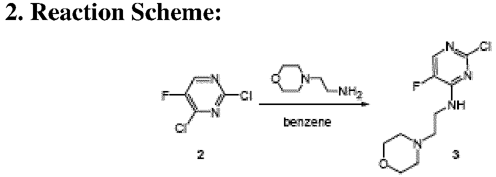

**Experimental Details:** To a solution of compound **2** (20.8 g, 0.194 mol) in ethanol (300 mL) was added morpholine (21.6g, 0.25 mol) drop wise at -10 °C over a period of 15 min. This mixture was stirred at room temperature for 0.5 h, heated then to 50 °C for 15 min. After cooling to room temperature and dilution with water, solid was precipitated. The solid was collected by filtrate and washed with water to give compound **3.**

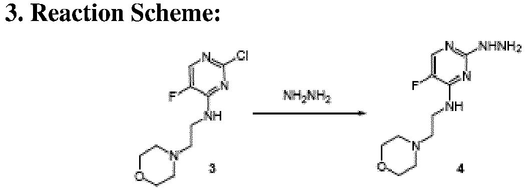

**Experimental Details:** A solution of **3** (4.6 g, 17.5 mmol) and hydrazine (8.75 g, 87.5 mmol) in ethanol (40 mL) was heated to reflux for 6 h. After cooling and precipitating, the precipitate was collected by filtrate and washed with ethanol to give compound **4.**

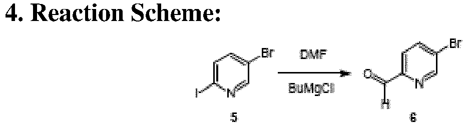

**Experimental Details:** A solution of compound **5** (14 g, 50 mmol) in anhydrous THF (100 mL) was treated with BuMgCl (37.5 mL, 60 mmol) at - 15 °C under N₂ atmosphere. After complete addition, this mixture was stirred at this temperature for 1 h. Anhydrous DMF (0.54 g, 75 mmol) was added to the reaction mixture at 0 oC over a period of 30 min, warmed then to room temperature for 1 h. The reaction mixture was quenched by adding 2 M HCl (80 mL). The result mixture was extracted with ethyl acetate (50 mL x 3). The combined organic layers were dried over Na₂SO₄. The solvent was concentrated to dryness. The residue was separated by column to give compound **6.**

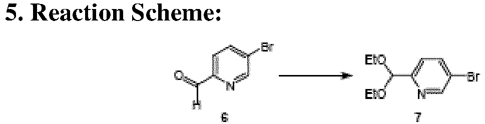

**Experimental Details:** A solution of compound **6** (4.5 g, 22.5 mmol) in triethyl orthoformate (15 mL) was heated in the presence of a trace of TsOH over night. The reaction mixture was diluted with ethyl acetate (100 mL) and washed with an aqueous of 5% Na₂CO₃. The organic layer was separated and dried over Na₂SO₄. The solvent was concentrated to give compound **7.**

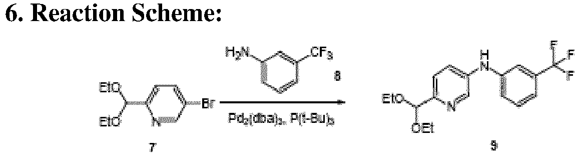

**Experimental Details:** To a stirred and degassed mixture of compound **7** (1.3 g, 5 mmol) and compound **8** (0.97 g, 6 mmol), and tBuONa (0.7 g, 7 mmol) and P(t-Bu)₃ (15 mg) in toluene (60 mL) was added Pd₂(dba)₃ (23 mgl) under N₂ atmosphere and stirred under reflux for 12 h. After filtrating off the solid, the filtrate was concentrated to dryness. The residue was purified by column to give product **9.**

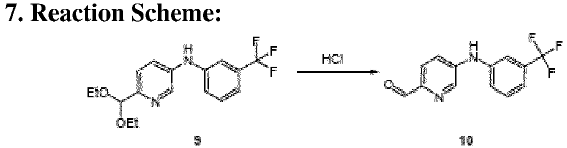

**Experimental Details:** A solution of compound **9** (200 mg, 0.58 mmol) in dichloromethane (10 mL) was treated with BBr₃ (146 mg, 0.6 mmol) at - 30 °C under N₂ atmosphere, then was stirred at room temperature for 4 h. The reaction was poured unto ice-water and then was brought by adding Na₂CO₃. The resulting mixture was extracted with dichloromethane (25 mL × 3), the combined organic layer was dried over Na₂SO₄. After filtrating off the Na₂SO₄, the filtrate was concentrated to give the crude product **10.**

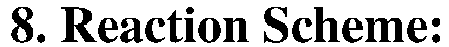

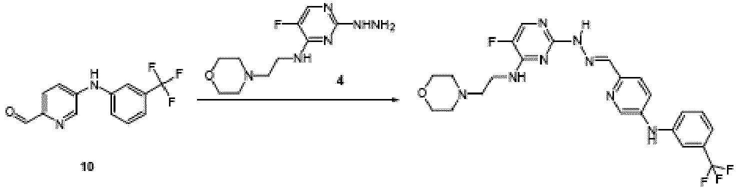

**Experimental Details:** A solution of compound **10** (48.7 mg, 0.2 mmol) and compound **4** (63 mg, 0.2 mmol) in anhydrous CH₂Cl₂ (300 mL) was stirred under reflux for 6 h. The solvent was removed under reduced pressure. The residue was separated by prep-TLC to give the desired compound.

### EXAMPLE 27.

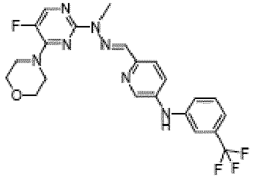

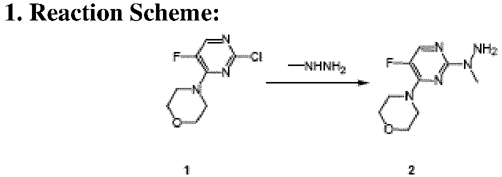

**Experimental Details:** A solution of **3** (2.4 g, 11 mmol) and methylhydrazine (2 g, 45 mmol) in ethanol (40 mL) was heated to reflux for 6 h. After cooling and precipitating, the precipitate was collected by filtrate and washed with ethanol to give compound **2.**

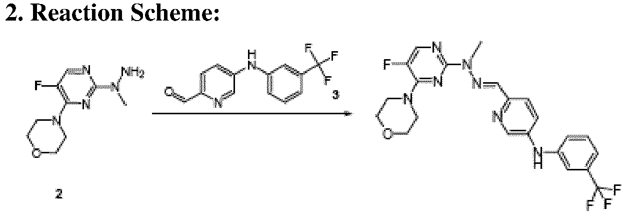

**Experimental Details:** A solution of compound **2** (28 mg, 0.1 mmol) and compound **3** (37 mg, 0.1 mmol) in anhydrous CH₂Cl₂ (300 mL) was stirred under reflux for 6 h. The solvent was removed under reduced pressure. The residue was separated by prep-TLC to give the desired compound.

### EXAMPLE 28.

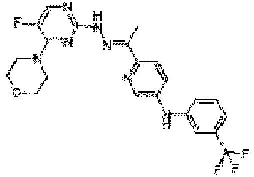

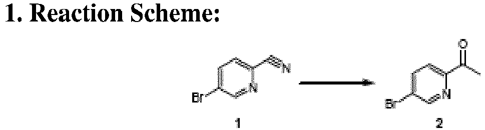

**Experimental Details:** A solution of **1** (2 g, 0.011 mol) in anhydrous THF (100 mL) was treated with MeMgCl (15 mL. 0.038 mol), at -20 °C and stirred for 2 h at this temperature. This reaction mixture was quenched by adding the saturated aqueous of NH₄Cl. The resulting mixture was extracted with ethyl acetate (100 mL × 3). The combined organic layers were washed with brine. The solvent was removed under reduced pressure to give compound **2.**

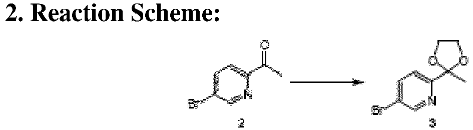

**Experimental Details:** A solution of compound **2** (2 g, 0.01 mol) and ethylene glycol (3 g, 0.048 mol) in anline (100 mL) was heated in the presence of a trace of TsOH for 3 h. The reaction mixture was diluted with ethyl acetate (100 mL) and washed with an aqueous of 5% Na₂CO₃. The organic layer was separated and dried over Na₂SO₄. The solvent was concentrated to give compound **3.**

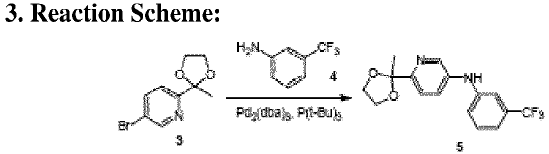

**Experimental Details:** To a stirred and degassed mixture of compound **3** (0.4 g, 1.6 mmol) and compound **4** (0.3 g, 1.9 mmol), and tBuONa (0.22 g, 2 mmol) and P(t-Bu)₃ (59 mg) in toluene (30 mL) was added Pd₂(dba)₃ (29 mgl) under N₂ atmosphere and stirred under reflux for 12 h. After filtrating off the solid, the filtrate was concentrated to dryness. The residue was purified by column to give product **5.**

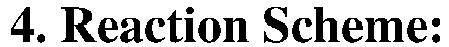

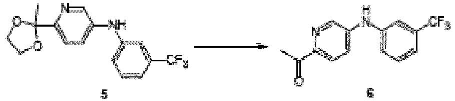

**Experimental Details:** A solution of compound **5** (100 mg, 0.3 mmol) in dichloromethane (10 mL) was treated with BBr₃ (146 mg, 0.6 mmol) at - 30 °C under N₂ atmosphere, then was stirred at room temperature for 4 h. The reaction was poured unto ice-water and then was brought by adding Na₂CO₃. The resulting mixture was extracted with dichloromethane (25 mL × 3), the combined organic layer was dried over Na₂SO₄. After filtrating off the Na₂SO₄, the filtrate was concentrated to give the crude product **6.**

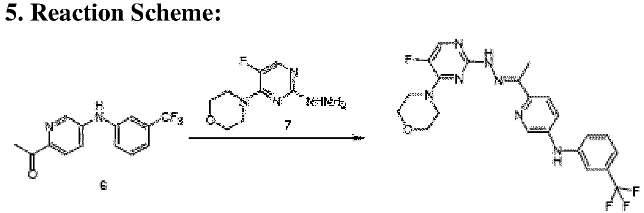

**Experimental Details:** A solution of compound **6** (64 mg, 0.2 mmol) and compound **7** (45 mg, 0.2 mmol) in anhydrous CH₂Cl₂ (300 mL) was stirred under reflux for 6 h. The solvent was removed under reduced pressure. The residue was separated by prep-TLC to give the desired compound.

### EXAMPLE 29.

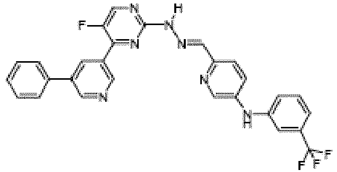

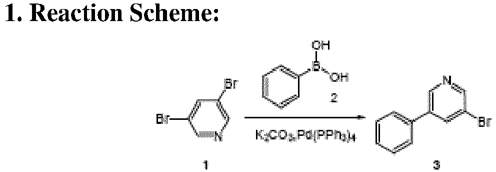

**Experimental Details:** A mixture of **1** (5.2 g, 22 mmol) and 2 (2.44 g, 20 mmol) in an aqueous of 2 M Na₂CO₃ (25 mL) and toluene (40 mL) was stirred with Pd(PPh₃)₄ (0.57 g, 0.05 mmol) under reflux over night. The reaction mixture was extracted with ethyl acetate (100 mL × 3). The combined organic layers were washed with brine. The solvent was removed under reduced pressure to dryness. The residue was purified by column to give **3.**

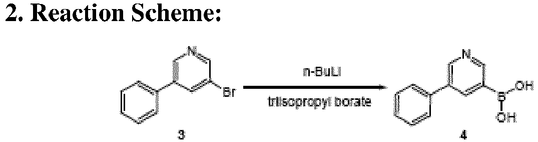

**Experimental Details:** A solution of compound **3** (0.78 g, 3.3 mmol) and triisopropyl borate (1 mL, 4 mmol) in anhydrous toluene ( 50 mL) was treated with n-BuLi ( 1.5 mL, 3.75 mmol) at -60 °C under N₂ atmosphere. After complete addition, theis mixture was stirred at -10 °C for 1 h. The reaction mixture was quenched by adding aqueous of 2 M HCl, and washed with toluene. The aqueous layer was brought pH = 8 by adding Na₂CO₃, extracted then with ethyl acetate (50 mL × 3). The combined organic layers were washed with brine. The solvent was removed under reduced pressure to dryness. The residue was separated by column to give **4.**

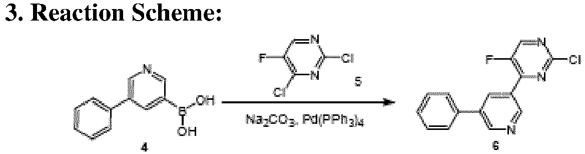

**Experimental Details:** To a stirred and degassed mixture of compound **4** (2.8 g, 14 mmol) and compound **5** (7 g, 42 mmol) in an aqueous of 2 M Na₂CO₃ (250 mL) and toluene (40 mL) was stirred with Pd(PPh₃)₄ (0.57 g, 0.05 mmol) under reflux over night. The reaction mixture was extracted with ethyl acetate (100 mL × 3). The combined organic layers were washed with brine. The solvent was removed under reduced pressure to dryness. The residue was separated by column to give **6.**

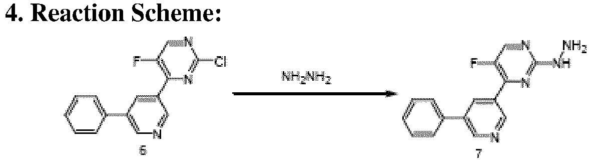

**Experimental Details:** A solution of **6** (0.53 g, 1.9 mmol) and hydrazine (0.52 g, 8.8 mmol) in ethanol (50 mL) was stirred under reflux for 6 h. After cooling and precipitating, the precipitate was collected by filtrate and washed with ethanol to give compound **7.**

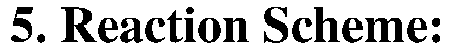

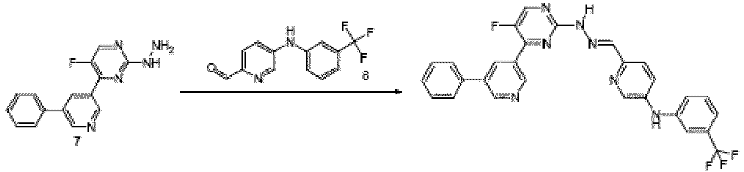

**Experimental Details:** A solution of compound **7** (53 mg, 0.13 mmol) and compound 8 (79 mg, 0.13 mmol) in anhydrous CH₂Cl₂ (300 mL) was stirred under reflux for 6 h. The solvent was removed under reduced pressure. The residue was purified by prep-TLC to give the desired compound.

### EXAMPLE 30.

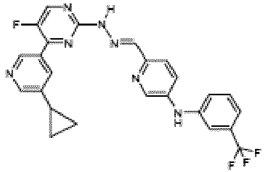

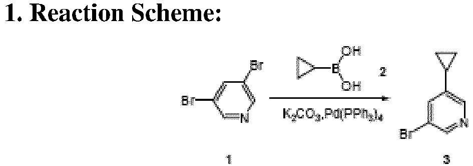

**Experimental Details:** A mixture of **1** (3.1 g, 13 mmol) and **2** (1 g, 12 mmpl) in an aqueous of 2 M Na₂CO₃ (15 mL) and toluene (30 mL) was stirred with Pd(PPh₃)₄ (0.4 g, 0.029 mmol) under reflux over night. The reaction mixture was extracted with ethyl acetate (100 mL × 3). The combined organic layers were washed with brine. The solvent was removed under reduced pressure to dryness. The residue was separated by column to give **3.**

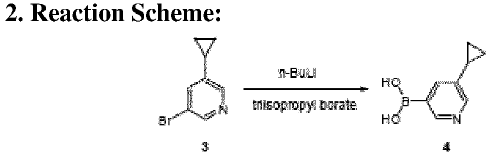

**Experimental Details:** A solution of compound **3** (2.0 g, 10 mmol) and triisopropyl borate ( 7 mL, 30 mmol) in anhydrous toluene ( 50 mL) was treated with n-BuLi ( 12 mL, 30 mmol) at -60 °C under N₂ atmosphere. After complete addition, this mixture was stirred at -10 °C for 1 h. The reaction mixture was quenched by adding an aqueous of 2 m HCl, and washed with toluene. The aqueous layer was brought pH = 8 by adding Na₂CO₃, extracted then with ethyl acetate (50 mL × 3). The combined organic layers were washed with brine. The solvent was removed under reduced pressure to dryness. The residue was separated by column to give **4.**

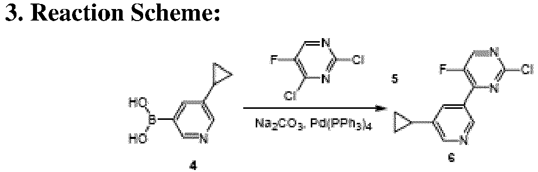

**Experimental Details:** To a stirred and degassed mixture of compound **4** (0.5 g, 3 mmol) and compound 5 (1.5 g, 9 mmol) in an aqueous of 2 M Na₂CO₃ (3.5 mL) and toluene (40 mL) was stirred with Pd(PPh₃)₄ (94 mg, 0.003 mmol) under reflux over night. The reaction mixture was extracted with ethyl acetate (100 mL × 3). The combined organic layers were washed with brine. The solvent was removed under reduced pressure to dryness. The residue was purified by column to give **6.**

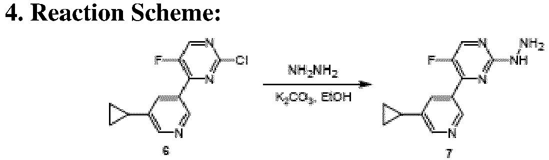

**Experimental Details:** A solution of **6** (0.24 g, 1 mmol) and hydrazine (0.3g, 4.7 mmol) in ethanol (50 mL) was stirred under reflux for 6 h. After cooling and precipitating, the precipitate was collected by filtrate and washed with ethanol to give compound **7.**

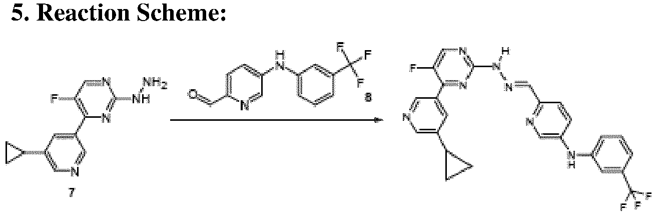

**Experimental Details:** A solution of compound **7** (70 mg, 0.29 mmol) and compound **8** (83 mg, 0.3 mmol) in anhydrous CH₂Cl₂ (300 mL) was stirred under reflux for 6 h. The solvent was removed under reduced pressure. The residue was purified by prep-TLC to give the desired compound.

### EXAMPLE 31.

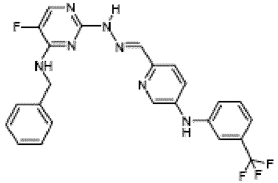

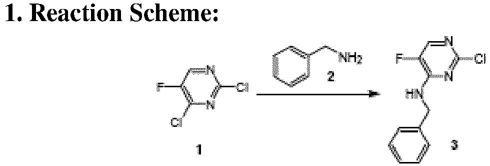

**Experimental Details:** To a solution of compound **2** (0.83 g, 5 mmol) in ethanol (100 mL) was added benzylamine (0.54 g, 5 mmol) drop wise. After stirring for 2 h, the reaction mixture was diluted with water. The resulting mixture was extracted wit ethyl acetate (50 mL × 3). The combined organic layers were dried over Na₂SO₄. The solvent was concentrated to give compound **3.**

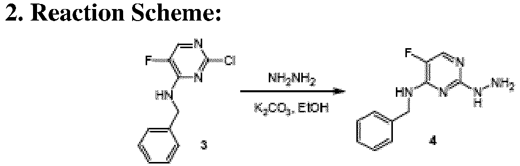

**Experimental Details:** A solution of **3** (1.18 g, 5 mmol) and hydrazine (5ml) in ethanol (40 mL) was heated to reflux for 6 h. After cooling and precipitating, the precipitate was collected by filtrate and washed with ethanol to give compound **4.**

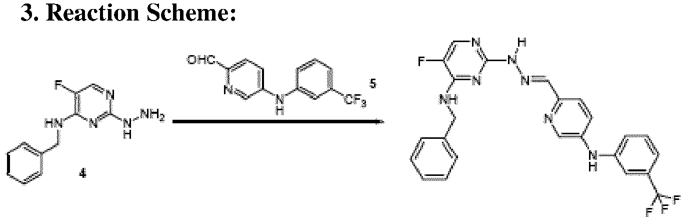

**Experimental Details:** A solution of compound **4** (48.7 mg, 2 mmol) and compound 5 (63 mg, 0.2 mmol) in anhydrous CH₂Cl₂ (300 mL) was stirred under reflux for 6 h. The solvent was removed under reduced pressure. The residue was separated by prep-TLC to give the desired compound.

### EXAMPLE 32.

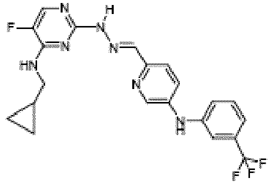

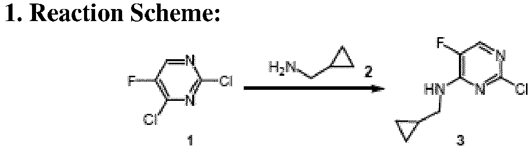

**Experimental Details:** To a solution of compound **2** (0.83 g, 5 mmol) in ethanol (100 mL) was added compound **2** (0.35 g, 5 mmol) dropwise. After stirring for 2 h, the reaction mixture was diluted with water. The resulting mixture was extracted wit ethyl acetate (50 mL × 3). The combined organic layers were dried over Na₂SO₄. The solvent was concentrated to give compound **3.**

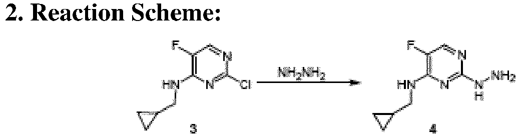

**Experimental Details:** A solution of **3** (1.0 g, 5 mmol) and hydrazine (5 mL) in ethanol (40 mL) was heated to reflux for 6 h. After cooling and precipitating, the precipitate was collected by filtrate and washed with ethanol to give compound **4.**

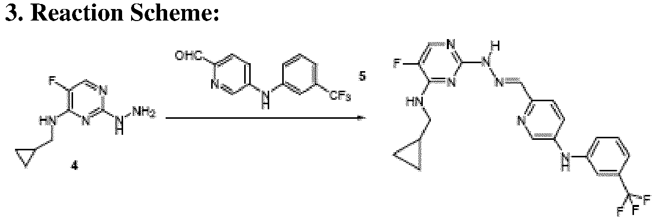

**Experimental Details:** A solution of compound **4** (480 mg, 2 mmol) and compound **5** (60 mg, 0.2 mmol) in anhydrous CH₂Cl₂ (300 mL) was stirred under reflux for 6 h. The solvent was removed under reduced pressure. The residue was separated by prep-TLC to give the desired compound.

### REFERENCES

Adcock, I.M., Lane, S.J. Mechanisms of Steroid Action and Resistance in Inflammation. Journal of Endocrinology, Volume 178 (September 2003) Pages 347-355
Allen, P.B., Wiedemann, L.M. An Activating Mutation in the ATP Binding Site of the ABL Kinase Domain. The Journal of Biological Chemistry, Volume 271 (August 9, 1996) Pages 19585-19591
Barthe, C., Cony-Makhoul, P., Melo, J.V., Reiffers, J., Mahon, F,X. Roots of Clinical Resistance to STI- 571 Cancer Therapy. Science, Volume 293 (September 21, 2001) Page 2163a
Branford, S., Rudzki, Z., Walsh, S., Grigg, A., Arthur, C., Taylor, K., Hermann, R., Lynch, K.P., Hughes, T.P. High Frequency of Point Mutations Clustered Within the Adenosine Triphosphate-Binding Region of BCR/ABL in Patients with Chronic Myeloid Leukemia or Ph-positive Acute Lymphoblastic Leukemia Who Develop Imatinib (STI571) Resistance. Blood, Volume 99 (May 1, 2002) Pages 3472-3475
Burbaum, J.J., Ohlmeyer, M.H., Reader, J.C., Henderson, I., Dillard, L.W., Li, G., Randle, T.L., Sigal, N.H., Chelsky, D., Baldwin, J.J. A paradigm for drug discovery employing encoded combinatorial libraries. Proceedings of the National Academy of Science USA, Volume 92 (June 20, 1995) Pages 6027-6031.
Carter, T.A., Wodicka, L.M., Shah, N.P., Velasco, A.M., Fabian, M.A., Treiber, D.K., Milanov, Z.V., Atteridge, C.E., Biggs, W.H. 3rd, Edeen, P.T., Floyd, M., Ford, J.M., Grotzfeld, R.M., Herrgard, S., Insko, D.E., Mehta, S.A., Patel, H.K., Pao, W., Sawyers, C.L., Varmus, H., Zarrinkar, P.P., Lockhart, D.J. Inhibition of drug-resistant mutants of ABL, KIT, and EGF receptor kinases. Proceedings of the National Academy of Science U S A, Volume 102 (August 2, 2005) Pages 11011-11016.
Corbin, A. S., Buchdunger, E., Pascal, F., Druker, B. J. Analysis of the Structural Basis of Specificity of Inhibition of the Abl Kinase by STI571. The Journal of Biological Chemistry, Volume 277 (August 30, 2002) Pages 32214-32219
Crossman, LC, O'Hare, T, Lange, T, Willis, SG, Stoffregen, EP, Corbin, AS, O'Brien, SG, Heinrich, MC, Druker, BJ, Middleton, PG, Deininger, MWN. A single nucleotide polymorphism in the coding region of ABL and its effects on sensitivity to imatinib. Leukemia, Volume 19 (September 8, 2005) Pages 1859-1862
Cunningham, B.C., De Vos, A.M., Mulkerrin, M.G., Ultsch, M, Wells, J.A. Selecting Ligand Agonists and Antagonists**.** U.S. Patent 5,506,107 (April 9,1996)
Cunningham, B.C., Wells, J.A., Clark, R. G., Olson, K., Fuh, G.G. Method for Inhibiting Growth Hormone Action**.** U.S. Patent 6,004,931 (December 21,1999)
Daley, G. Q., Van Etten, R. A., Baltimore, D. Induction of Chronic Myelogenous Leukemia in Mice by the P210 brc/abl Gene of the Philadelphia Chromosome. Science, Volume 247 (February 16, 1990) Pages 824-830
Druker, B. J., M.D., Talpaz, M., M.D., Resta, D.J., R.N., Peng, B., Ph.D., Buchdunger, E., Ph.D., Ford, J.M., M.D., Lydon, N. B., Ph.D., Kantarjian, H., M.D., Capdeville, R., M.D., Ohno-Jones, S., B.S., Sawyers, C. L., M.D. Efficacy and Safety of a Specific Inhibitor of the BCR-ABL Tyrosine Kinase in Chronic Myeloid Leukemia. The New England Journal of Medicine, Volume 344 (April 5, 2001) Pages 1031-1037
Druker, B. J., Tamura, S., Buchdunger, E., Ohno, S., Segal, G.M., Fanning, S., Zimmermann, J., Lydon, N.B. Effects of a Selective Inhibitor of the Abl Tyrosine Kinase on the Growth of Bcr-Abl Positive Cells. Nature Medicine, Volume 2 (May 1996) Pages 561-566
Druker, B.J., M.D., Sawyers, C.L., M.D., Kantarjian, H., M.D., Resta, D. J., R.N., Reese, S.F., M.D., Ford, J.M., M.D., Capdeville, R., M.D., Talpaz, M., M.D. Activity of a Specific Inhibitor of the BCR-ABL Tyrosine Kinase in the Blast Crisis of Chronic Myeloid Leukemia and Acute Lymphoblastic Leukemia with the Philadelphia Chromosome. The New England Journal of Medicine, Volume 344 (April 5, 2001) Pages 1038-1042
Faderl, S., M.D., Talpaz, M., M.D., Estrov, Z., M.D., O'Brien, S., M.D., Kurzrock, R., M.D., Kantarjian, H. M., M.D. The Biology of Chronic Myeloid Leukemia. The New England Journal of Medicine, Volume 341 (July 15,1999) Pages 164-172
Foreman, J. C. and Johansen, T. Textbook of Receptor Pharmacology. CRC Press, 2002; Boca Raton
Gambacorti-Passerini, C., Barni, R., Le Coutre, P., Zucchetti, M., Cabrita, G., Cleris, L., Rossi, F., Gianazza, E., Brueggen, J., Cozens, R., Pioltelli, P., Pogliani, E., Corneo, G., Formelli, F., D'Incalci, M. Role of α1 Acid Glycoprotein in the In Vivo Resistance of Human BCR-ABL+ . Leukemic Cells to the Abl Inhibitor STI571. Journal of the National Cancer Institute, Volume 92 (October 18, 2000) Pages 1641-1650.
Goodnow, R.A., Jr., Guba, W., Haap, W. Library design practices for success in lead generation with small molecule libraries. Combinatorial Chemistry and High Throughput Screening, Volume 6 (November 2003) Pages 649-660.
Gorre, M.E., Mohammed, M., Ellwood, K., Hsu, N., Paquette, R., Rao, P.N., Sawyers, C.L. Clinical Resistance to STI- 571 Cancer Therapy Caused by BCR- ABL Gene Mutation or Amplification. Science, Volume 293 (August 3, 2001) Pages 876-880.
Hanke, J.H., Gardner, J.P., Dow, R.L., Changelian, P.S., Brissette, W.H., Weringer, E.J., Pollok, B.A., Connelly, P.A. Discovery of a novel, potent, and Src family-selective tyrosine kinase inhibitor. Study of Lck- and FynT-dependent T cell activation. Journal of Biological Chemistry, Volume 271 (January 1996) Pages 695-701.
Hofmann, W. K., Jones, L.C., Lemp, N.A., DeVos, S., Gschaidmeier, H., Hoelzer, D., Ottmann, O. G., Koeffler, H. P. Ph+ Acute Lymphoblastic Leukemia Resistant to the Tyrosine Kinase Inhibitor STI571 has a Unique BCR-ABL Gene Mutation. Blood, Volume 99 (March 1, 2002) Pages 1860-1862.
Hou, Y.Y., Tan, Y.S., Sun, M.H., Wei, Y.K., Xu, J.F., Lu, S.H., A-Ke-Su, S.J., Zhou, Y.N., Gao, F., Zheng, A.H., Zhang, T.M., Hou, W.Z., Wang, J., Du, X., Zhu, X.Z. C-kit Gene Mutation in Human Gastrointestinal Stromal Tumors. World Journal of Gastroenterology, Volume 10 (May 1, 2004) Pages 1310-1314.
Housey GM. Method of Screening for Protein Inhibitors and Activators**.** U.S. Patent 4, 980, 281 (December 25, 1990)
Housey GM, Johnson MD, Hsiao WL, O'Brian CA, Murphy JP, Kirschmeier P, Weinstein IB. Overproduction of protein kinase C causes disordered growth control in rat fibroblasts. Cell, Volume 52 (February 12, 1988) Pages 343-54.
Kerkelä, R., Grazette, L., Yacobi, R., Iliescu, C., Patten, R., Beahm, C., Walters, B., Shevtsov, S., Pesant, S., Clubb, F.J., Rosenzweig, A., Salomon, R.N., Van Etten, R.A., Alroy, J., Durand, J.B., Force, T. Cardiotoxicity of the cancer therapeutic agent imatinib mesylate. Nature Medicine, Volume 12 (August 2006) Pages 908-916.
Knight, Z.A., Shokat, K.M. Features of Selective Kinase Inhibitors. Chemistry and Biology, Volume 12 (June 2005) Pages 621-637.
La Rosee, P., Corbin, A. S., Stoffregen, E. P., Deininger, M.W., Druker, B. J. Activity of the Bcr-Abl Kinase Inhibitor PD180970 Against Clinically Relevant Bcr-Abl Isoforms That Cause Resistance to Imatinib Mesylate (Gleevec, STI-571). Cancer Research, Volume 62 (December 15, 2002) Pages 7149-7153
Le Coutre, P., Tassi, E., Varella-Garcia, M., Barni, R., Mologni, L., Cabrita, G., Marchesi, E., Supino, R., Gambacorti-Passerini, C. Induction of Resistance to the Abelson Inhibitor STI571 in Human Leukemic Cells Through Gene Amplification. Blood, Volume 95 (March 1, 2000) Pages 1758-1766
Leonard, G.D., Fojo, T., Bates, S.E. The Role of ABC Transporters in Clinical Practice. The Oncologist, Volume 8 (2003) Pages 411-424
Loutfy, M.R., Walmsley, S.L. Salvage Antiretroviral Therapy in HIV Infection. Expert Opinion, Volume 3 (February 2002) Pages 81-90
Lynch, T.J., M.D., Bell, D.W., Ph.D., Sordella, R., Ph.D., Gurubhagavatula, S., M.D., Okimoto, R.A., B.S., Brannigan, B.W., B.A., Harris, P.L., M.S., Haserlat, S.M., B.A., Supko, J.G., Ph.D., Haluska, F.G., M.D., Ph.D., Louis, D.N., M.D., Christiani, D.C., M.D., Settleman, J., Ph.D., Haber, D.A., M.D., Ph.D. Activating Mutations in the Epidermal Growth Factor Receptor Underlying Responsiveness of Non-Small-Cell Lung Cancer to Gefitinib. The New England Journal of Medicine, Volume 350 (May 20, 2004) Pages 2129-2139
Mahon, F. X., Deininger, M. W.N., Schultheis, B., Chabrol, J., Reiffers, J., Goldman, J.M., Melo, J.V. Selection and Characterization of BCR-ABL Positive Cell Lines with Differential Sensitivity to the Tyrosine Kinase Inhibitor STI571: Diverse Mechanisms of Resistance. Blood, Volume 96 (August 1, 2000) Pages 1070-1079
Marx, J. Why a New Cancer Drug Works Well in Some Patients. Science, Volume 304 (April 30, 2004) Pages 658-659
Melo JV, Myint H, Galton DA, Goldman JM. P190BCR-ABL chronic myeloid leukaemia: the missing link with chronic myelomonocytic leukaemia? Leukemia, Volume 8 (January 1994) Pages 208-11
Noble, M.E.M., Endicott, J.A., Johnson, L.N. Protein Kinase Inhibitors: Insights into Drug Design from Structure. Science, Volume 303 (March 19, 2004) Pages 1800-1805
O'Hare T, Pollock R, Stoffregen EP, Keats JA, Abdullah OM, Moseson EM, Rivera VM, Tang H, Metcalf CA 3rd, Bohacek RS, Wang Y, Sundaramoorthi R, Shakespeare WC, Dalgarno D, Clackson T, Sawyer TK, Deininger MW, Druker BJ. Inhibition of wild-type and mutant Bcr-Abl by AP23464, a potent ATP-based oncogenic protein kinase inhibitor: implications for CML. Blood, Volume 104 (October 15, 2004) Pages 2532-2539
Paez, J.G., Jänne, P.A., Lee, J.C., Tracy, S., Greulich, H., Gabriel, S., Herman, P., Kaye, F.J., Lindeman, N., Boggon, T.J., Naoki, K., Sasaki, H., Fujii, Y., Eck, M.J., Sellers, W.R., Johnson, B.E., Meyerson, M. EGFR Mutations in Lung Cancer: Correlation with Clinical Response to Gefitinib Therapy. Sciencexpress (April 29, 2004) Pages 1-4
Ravandi F, Cortes J, Albitar M, Arlinghaus R, Qiang Guo J, Talpaz M, Kantarjian HM. Chronic myelogenous leukaemia with p185(BCR/ABL) expression: characteristics and clinical significance. British Journal of Haematology, Volume 107 (December 1999) Pages 581-586
Sambrook and Russell, Molecular Cloning: A Laboratory Manual. Cold Spring Harbor Laboratory Press, New York, 2001, Volumes 1-3
Sawyers,C.L. M.D. Chronic Myeloid Leukemia. The New England Journal of Medicine, Volume 340 (April 29,1999) Pages 1330-1340.
Sawyers, C.L., Gorre, M.E., Shah, N.P., Nicoll, J. Mutations in the BCR-ABL Tyrosine Kinase Associated with Resistance to STI-571**.** WO 02/102976 A2 Published December 27, 2002 (PCT/US02/18729 Filed June 14, 2002)
Schindler, T., Bornmann,W., Pellicena, P., Miller, W. T., Clarkson, B., Kuriyan, J. Structural Mechanism for STI-571 Inhibition of Abelson Tyrosine Kinase. Science, Volume 289 (September 15, 2000) Pages 1938-1942
Senechal, K., Halpern, J., Sawyers, C.L. The CRKL Adaptor Protein Transforms Fibroblasts and Functions in Transformation by the BCR-ABL Onocogene. The Journal of Biological Chemistry, Volume 271 (September 20, 1996) Pages 23255-23261
Senechal, K., Heaney, C., Druker, B., Sawyers, C.L. Structural Requirements for Function of the Crkl Adapter Protein in Fibroblasts and Hematopoietic Cells. Molecular and Cellular Biology, Volume 18 (September 1998) Pages 5082-5090.
Tipping AJ, Baluch S, Barnes DJ, Veach DR, Clarkson BM, Bornmann WG, Mahon FX, Goldman JM, Melo JV. Efficacy of dual-specific Bcr-Abl and Src-family kinase inhibitors in cells sensitive and resistant to imatinib mesylate. Leukemia, Volume 18 (August 2004) Pages 1352-1356
Von Bubnoff, N., Schneller, F., Peschel, C., Duyster, J. BCR-ABL Gene Mutations in Relation to Clinical Resistance of Philadelphia-Chromosome-Positive Leukemia to STI571: A Prospective Study. The Lancet, Volume 359 (February 9, 2002) Pages 487-491
Von Bubnoff, N., Veach DR, Van Der Kuip H, Aulitzky WE, Sanger J, Seipel P, Bornmann WG, Peschel C, Clarkson B, Duyster J. A cell-based screen for resistance of Bcr-Abl positive leukemia identifies the mutation pattern for PD166326, an alternative Abl kinase inhibitor. Blood, Volume 105 (February 15, 2005) Pages 1652-1659
Von Bubnoff, N, Veach, DR, Miller, WT, Wanqing, L, Sänger, J, Peschel, C, Bornmann, WG, Clarkson, B. Duyster, J. Inhibition of Wild-Type and Mutant Bcr-Abl by Pyrido-Pyrimidine Type Small Molecule Kinase Inhibitors. Cancer Research, Volume 63 (October 1, 2003) Pages 6395-6404
Wakai, T., Kanda, T., Hirota, S., Ohashi, A., Shirai, Y. Hatakeyama, K. Late Resistance to Imatinib Therapy in a Metastatic Gastrointestinal Stromal Tumour is Associated With a Second KIT Mutation. British Journal of Cancer, Volume 90 (June 1, 2004) Pages 2059-2061
Warmuth, M., Mathes, R., Hallek, M. Method for Selecting Enzyme Inhibitors**.** U.S. Patent Application 2003/0162222 A1 (August 28, 2003)
Weigel, U., Meyer, M., Sebald, W. Mutant Proteins of Human Interleukin 2: Renaturation Yield, Proliferative Activity and Receptor Binding. European Journal of Biochemistry, Volume 180 (March 15, 1989) Pages 295-300.
Weisberg E, Catley L, Kujawa J, Atadja P, Remiszewski S, Fuerst P, Cavazza C, Anderson K, Griffin JD. Histone deacetylase inhibitor NVP-LAQ824 has significant activity against myeloid leukemia cells in vitro and in vivo. Leukemia, Volume 18 (December 2004) Pages 1951-1963
Weisberg, E., Griffin, J. D. Mechanism of Resistance to the ABL Tyrosine Kinase Inhibitor STI 571 in BCR/ABL-Transformed Hematopoietic Cell Lines. Blood, Volume 95 (June 1, 2000) Pages 3498-3505
Weisberg E, Manley PW, Breitenstein W, Bruggen J, Cowan-Jacob SW, Ray A, Huntly B, Fabbro D, Fendrich G, Hall-Meyers E, Kung AL, Mestan J, Daley GQ, Callahan L, Catley L, Cavazza C, Azam M, Neuberg D, Wright RD, Gilliland DG, Griffin JD. Characterization of AMN107, a selective inhibitor of native and mutant Bcr-Abl. Cancer Cell, Volume 7 (February 2005) Pages 129-41
White, MF, Livingston, JM, Backer, Lauris, V, Dull, TJ, Ullrich A, Kahn, CR. Mutation of the Insulin Receptor at Tyrosine 960 Inhibits Signal Transmission but Does Not Affect Its Tyrosine Kinase Activity. Cell, Volume 54 (August 26, 1988) Pages 641-649
Wolff, NC, Veach, DR, Tong, WP, Bornmann, WG, Clarkson, B, Ilaria Jr, RL. P0166326, a novel tyrosine kinase inhibitor, has greater antileukemic activity than imatinib mesylate in a murine model of chronic myeloid leukemia. Blood, Volume 105 (May 10, 2005) Pages 3995-4003

## Claims

1. An *in vitro* cell-based screening method for identifying test compounds having improved cellular specificity as compared to a first compound that is an inhibitor or activator of a protein that is capable of eliciting a detectable phenotypic characteristic, which comprises:
a) providing a test cell which expresses the protein and is capable of eliciting a detectable phenotypic characteristic linked to the presence and functional activity of the protein in the cell and wherein said phenotypic characteristic is also capable of being modulated by an inhibitor or activator (phenoresponse);
b) providing a control cell which expresses the protein at a lower level or does not express the protein and is capable of eliciting the phenoresponse;
c) measuring the modulation of the phenoresponse of the test cell treated with the first compound,
d) measuring the modulation of the phenoresponse of the control cell treated with the first compound,
e) measuring the modulation of the phenoresponse identified and selected in steps a-d of the test cell treated with test compounds;
f) measuring the modulation of the phenoresponse identified and selected in steps a-d of the control cell treated with the test compounds;
g) determining the cellular specificity gap (CSG) of the test compounds by measuring the ability of the test compounds to modulate the selected phenoresponse in the test cell relative to the ability of said compounds to modulate the same phenoresponse in the control cell, wherein the CSG is quantified by dividing the IC₅₀ of the compounds on the phenoresponse of the control cell by the IC₅₀ of the compounds on the phenoresponse of the test cell; and
h) identifying a test compound as having improved cellular specificity if the CSG of the test compound is greater than the CSG of the first compound.

2. The method of claim 1, which comprises:
a) measuring the IC₅₀ of the first compound on the phenoresponse of a test cell;
b) measuring the IC₅₀ of the first compound on the phenoresponse of a control cell;
c) measuring the IC₅₀ of a test compound which shares the same scaffold as the first compound on the phenoresponse of the test cell; and
d) measuring the IC₅₀ of the test compound on the phenoresponse of the control cell.

3. The method of any one of claims 1 or 2, wherein the protein is P210^{Bcr-Abl-T315I} or p190^{Bcr-Abl} that contains the corresponding threonine to isoleucine mutation at the corresponding amino acid position.

4. The method of any one of claims 1-3, which comprises selecting the optimized compound of step g) and repeating steps c)-f).

5. The method of any one of claims 1-4 wherein the protein is an endogenous protein that harbors a mutation that renders said protein resistant to a drug (theramutein) that is known to inhibit or activate in a therapeutically effective manner its non-mutated counterpart.

6. The method of any one of claims 1-5, wherein the test compound has a higher IC₅₀ than the first compound.

7. The method of any one of claims 1-5, wherein the test compound has a lower IC₅₀ than the first compound.

8. The method of any one of claims 1-7, wherein the protein is selected from a protein kinase, tyrosine kinase, receptor tyrosine kinase, serine threonine protein kinase, dual specificity protein kinases, protease, matrix metalloproteinase, phosphatase, cell cycle control protein, docking protein such as an IRS family member, cell-surface receptor, G-protein, ion channel, DNA- and RNA-binding protein or polymerase.

9. The method of any one of claims 1-8, wherein the phenoresponse of the test cell comprises a growth characteristic of the test cell and the phenoresponse of the control cell comprises a growth characteristic of the control cell.

10. The method of any one of claims 1-9, wherein the phenoresponse of the test cell comprises proliferation of the test cell and the phenoresponse of the control cell comprises a proliferation of the control cell.

11. The method of any one of claims 1-8, wherein the phenoresponse of the test cell comprises a transformation state of the test cell and the phenoresponse of the control cell comprises a transformation state of the control cell.

12. The method of any one of claims 1-8, wherein the phenoresponse of the test cell comprises a differentiation state of the test cell and the phenoresponse of the control cell comprises a differentiation state of the control cell.

13. The method of any one of claims 1-8, wherein the phenoresponse of the test cell comprises a substrate phosphorylation state and the phenoresponse of the control cell comprises a substrate phosphorylation state of the control cell.

14. The method of any one of claims 1-8, wherein the phenoresponse of the test cell comprises a change
(a) of an ion flux across a membrane of the test cell and the phenoresponse of the control cell comprises a change of an ion flux across a membrane of the control cell;
(b) of pH within the test cell and the phenoresponse of the control cell comprises a change of pH within the control cell; and/or
(c) in concentration of an intracellular chemical species in the test cell and the phenoresponse of the control cell comprises a change in concentration of an intracellular chemical species in the control cell.

## Patentansprüche

1. Zellbasiertes in-vitro-Screening-Verfahren zur Identifizierung von Testverbindungen mit verbesserter zellulärer Spezifität im Vergleich zu einer ersten Verbindung, die ein Inhibitor oder Aktivator eines Proteins ist, das in der Lage ist, ein nachweisbares phänotypisches Merkmal hervorzurufen, welches umfasst:
a) Bereitstellen einer Testzelle, die das Protein exprimiert und in der Lage ist, ein nachweisbares phänotypisches Merkmal hervorzurufen, das mit dem Vorhandensein und der funktionellen Aktivität des Proteins in der Zelle verbunden ist, und wobei das phänotypische Merkmal auch durch einen Inhibitor oder Aktivator moduliert werden kann (Phenoresponse);
b) Bereitstellen einer Kontrollzelle, die das Protein in geringerem Maße oder gar nicht exprimiert und in der Lage ist, die Phenoresponse auszulösen;
c) Messen der Modulation der Phenoresponse der mit der ersten Verbindung behandelten Testzelle,
d) Messen der Modulation der Phenoresponse der mit der ersten Verbindung behandelten Kontrollzelle,
e) Messen der Modulation der in den Schritten a-d identifizierten und ausgewählten Phenoresponse der mit Testverbindungen behandelten Testzelle;
f) Messen der Modulation der in den Schritten a-d identifizierten und ausgewählten Phenoresponse der mit den Testverbindungen behandelten Kontrollzelle;
g) Bestimmen der zellulären Spezifitätslücke (CSG) der Testverbindungen durch Messen der Fähigkeit der Testverbindungen, die ausgewählte Phenoresponse in der Testzelle zu modulieren, relativ zu der Fähigkeit der Verbindungen, dieselbe Phenoresponse in der Kontrollzelle zu modulieren, wobei die CSG quantifiziert wird, indem die IC₅₀ der Verbindungen auf die Phenoresponse der Kontrollzelle durch die IC₅₀ der Verbindungen auf die Phenoresponse der Testzelle dividiert wird; und
h) Identifizieren einer Testverbindung als mit verbesserter zellulärer Spezifität, wenn die CSG der Testverbindung größer ist als die CSG der ersten Verbindung.

2. Verfahren nach Anspruch 1, das umfasst:
a) Messen der IC₅₀ der ersten Verbindung auf die Phenoresponse einer Testzelle;
b) Messen der IC₅₀ der ersten Verbindung auf die Phenoresponse einer Kontrollzelle;
c) Messen der IC₅₀ einer Testverbindung, die das gleiche Gerüst wie die erste Verbindung aufweist, auf die Phenoresponse der Testzelle; und
d) Messen der IC₅₀ der Testverbindung auf die Phenoresponse der Kontrollzelle.

3. Verfahren nach einem der Ansprüche 1 oder 2, wobei das Protein P210^{Bcr-Abl-T315I} oder p190^{Bcr-Abl} ist, das die entsprechende Threonin-zu-Isoleucin-Mutation an der entsprechenden Aminosäureposition enthält.

4. Verfahren nach einem der Ansprüche 1-3, das das Auswählen der verbesserten Verbindung aus Schritt g) und das Wiederholen der Schritte c)-f) umfasst.

5. Verfahren nach einem der Ansprüche 1-4, wobei das Protein ein endogenes Protein ist, das eine Mutation aufweist, die das Protein resistent gegen ein Medikament (Theramutein) macht, von dem bekannt ist, dass es sein nicht mutiertes Gegenstück in therapeutisch wirksamer Weise hemmt oder aktiviert.

6. Verfahren nach einem der Ansprüche 1-5, wobei die Testverbindung eine höhere IC₅₀ aufweist als die erste Verbindung.

7. Verfahren nach einem der Ansprüche 1-5, wobei die Testverbindung eine niedrigere IC₅₀ als die erste Verbindung aufweist.

8. Verfahren nach einem der Ansprüche 1-7, wobei das Protein ausgewählt ist aus einer Proteinkinase, Tyrosinkinase, Rezeptortyrosinkinase, Serin-Threonin-Proteinkinase, Doppelspezifitäts-Proteinkinasen, Protease, Matrix-Metalloproteinase, Phosphatase, Zellzyklus-Kontrollprotein, Andockprotein wie ein Mitglied der IRS-Familie, Zelloberflächenrezeptor, G-Protein, Ionenkanal, DNA- und RNA-bindendes Protein oder Polymerase.

9. Verfahren nach einem der Ansprüche 1-8, wobei die Phenoresponse der Testzelle ein Wachstumsmerkmal der Testzelle und die Phenoresponse der Kontrollzelle ein Wachstumsmerkmal der Kontrollzelle umfasst.

10. Verfahren nach einem der Ansprüche 1-9, wobei die Phenoresponse der Testzelle eine Proliferation der Testzelle und die Phenoresponse der Kontrollzelle eine Proliferation der Kontrollzelle umfasst.

11. Verfahren nach einem der Ansprüche 1-8, wobei die Phenoresponse der Testzelle einen Transformationszustand der Testzelle und die Phenoresponse der Kontrollzelle einen Transformationszustand der Kontrollzelle umfasst.

12. Verfahren nach einem der Ansprüche 1-8, wobei die Phenoresponse der Testzelle einen Differenzierungszustand der Testzelle und die Phenoresponse der Kontrollzelle einen Differenzierungszustand der Kontrollzelle umfasst.

13. Verfahren nach einem der Ansprüche 1-8, wobei die Phenoresponse der Testzelle einen Substratphosphorylierungszustand und die Phenoresponse der Kontrollzelle einen Substratphosphorylierungszustand der Kontrollzelle umfasst.

14. Verfahren nach einem der Ansprüche 1 bis 8, wobei die Phenoresponse der Testzelle eine Änderung
(a) eines Ionenflusses durch eine Membran der Testzelle und die Phenoresponse der Kontrollzelle eine Änderung eines Ionenflusses durch eine Membran der Kontrollzelle umfasst;
(b) des pH-Wertes in der Testzelle und die Phenoresponse der Kontrollzelle eine Änderung des pH-Wertes in der Kontrollzelle umfasst; und/oder
(c) in der Konzentration einer intrazellulären chemischen Spezies in der Testzelle und die Phenoresponse der Kontrollzelle eine Änderung der Konzentration einer intrazellulären chemischen Spezies in der Kontrollzelle umfasst.

## Revendications

1. Procédé de criblage *in vitro* à base de cellules pour identifier des composés de test présentant une spécificité cellulaire améliorée par comparaison à un premier composé qui est un inhibiteur ou un activateur d'une protéine qui est capable de susciter une caractéristique phénotypique détectable, qui comprend les étapes consistant à :
a) fournir une cellule de test qui exprime la protéine et est capable de susciter une caractéristique phénotypique détectable liée à la présence et à l'activité fonctionnelle de la protéine dans la cellule et dans laquelle ladite caractéristique phénotypique est également capable d'être modulée par un inhibiteur ou un activateur (phénoréponse) ;
b) fournir une cellule témoin qui exprime la protéine à un niveau inférieur ou n'exprime pas la protéine et est capable de susciter la phénoréponse ;
c) mesurer la modulation de la phénoréponse de la cellule de test traitée avec le premier composé,
d) la modulation de la phénoréponse de la cellule témoin traitée avec le premier composé,
e) mesurer la modulation de la phénoréponse identifiée et sélectionnée aux étapes a à d de la cellule de test traitée avec des composés de test ;
f) mesurer la modulation de la phénoréponse identifiée et sélectionnée aux étapes a à d de la cellule témoin traitée avec les composés de test ;
g) déterminer l'écart de spécificité cellulaire (CSG) des composés de test en mesurant la capacité des composés de test à moduler la phénoréponse sélectionnée dans la cellule de test par rapport à la capacité desdits composés à moduler la même phénoréponse dans la cellule témoin, dans lequel le CSG est quantifié en divisant la CI₅₀ des composés sur la phénoréponse de la cellule témoin par la CI₅₀ des composés sur la phénoréponse de la cellule de test ; et
h) identifier un composé de test comme ayant une spécificité cellulaire améliorée si le CSG du composé de test est supérieur au CSG du premier composé.

2. Procédé selon la revendication 1, comprenant en outre les étapes consistant à :
a) mesurer la CI₅₀ du premier composé sur la phénoréponse d'une cellule de test ;
b) mesurer la CI₅₀ du premier composé sur la phénoréponse d'une cellule témoin ;
c) mesurer la CI₅₀ d'un composé de test qui partage le même échafaudage que le premier composé sur la phénoréponse de la cellule de test ; et
d) mesurer la CI₅₀ du composé de test sur la phénoréponse de la cellule témoin.

3. Procédé selon l'une quelconque des revendications 1 ou 2, dans lequel la protéine est P210^{Bcr-Abl-T315I} ou p190^{Bcr-Abl} qui contient la mutation correspondante de thréonine en isoleucine à la position d'acide aminé correspondante.

4. Procédé selon l'une quelconque des revendications 1 à 3, qui comprend la sélection du composé optimisé de l'étape g) et la répétition des étapes c) à f).

5. Procédé selon l'une quelconque des revendications 1 à 4, dans lequel la protéine est une protéine endogène qui porte une mutation qui rend ladite protéine résistante à un médicament (thramutéine) qui est connu pour inhiber ou activer de manière thérapeutiquement efficace son homologue non muté.

6. Procédé selon l'une quelconque des revendications 1 à 5, dans lequel le composé de test présente une CI₅₀ plus élevée que le premier composé.

7. Procédé selon l'une quelconque des revendications 1 à 5, dans lequel le composé de test présente une CI₅₀ inférieure à celle du premier composé.

8. Procédé selon l'une quelconque des revendications 1 à 7, dans lequel la protéine est choisie parmi une protéine kinase, une tyrosine kinase, une tyrosine kinase réceptrice, une sérine thréonine protéine kinase, des protéine kinases à double spécificité, une protéase, une métalloprotéinase matricielle, une phosphatase, une protéine de contrôle du cycle cellulaire, une protéine d'amarrage telle qu'un membre de la famille IRS, un récepteur de surface cellulaire, une protéine G, un canal ionique, une protéine ou une polymérase de liaison à l'ADN et à l'ARN.

9. Procédé selon l'une quelconque des revendications 1 à 8, dans lequel la phénoréponse de la cellule de test comprend une caractéristique de croissance de la cellule de test et la phénoréponse de la cellule témoin comprend une caractéristique de croissance de la cellule témoin.

10. Procédé selon l'une quelconque des revendications 1 à 9, dans lequel la phénoréponse de la cellule de test comprend une prolifération de la cellule de test et la phénoréponse de la cellule témoin comprend une prolifération de la cellule témoin.

11. Procédé selon l'une quelconque des revendications 1 à 8, dans lequel la phénoréponse de la cellule de test comprend un état de transformation de la cellule de test et la phénoréponse de la cellule témoin comprend un état de transformation de la cellule témoin.

12. Procédé selon l'une quelconque des revendications 1 à 8, dans lequel la phénoréponse de la cellule de test comprend un état de différenciation de la cellule de test et la phénoréponse de la cellule témoin comprend un état de différenciation de la cellule témoin.

13. Procédé selon l'une quelconque des revendications 1 à 8, dans lequel la phénoréponse de la cellule de test comprend un état de phosphorylation de substrat et la phénoréponse de la cellule témoin comprend un état de phosphorylation de substrat de la cellule témoin.

14. Procédé selon l'une quelconque des revendications 1 à 8, dans lequel la phénoréponse de la cellule de test comprend un changement
a) d'un flux ionique à travers une membrane de la cellule de test et la phénoréponse de la cellule témoin comprend un changement d'un flux ionique à travers une membrane de la cellule témoin ;
b) du pH à l'intérieur de la cellule de test et la phénoréponse de la cellule témoin comprend un changement de pH à l'intérieur de la cellule témoin ; et/ou
c) dans la concentration d'une espèce chimique intracellulaire dans la cellule de test et la phénoréponse de la cellule témoin comprend un changement de concentration d'une espèce chimique intracellulaire dans la cellule témoin.
